(19) Europäisches Patentamt
European Patent Office
Office européen des brevets

(11) EP 2 637 503 B1

(12) **EUROPEAN PATENT SPECIFICATION**

(45) Date of publication and mention of the grant of the patent:
**14.09.2016 Bulletin 2016/37**

(21) Application number: **11840448.2**

(22) Date of filing: **08.11.2011**

(51) Int Cl.:
***C07H 9/06*** $^{(2006.01)}$ ***C07D 513/04*** $^{(2006.01)}$
***A61K 31/7056*** $^{(2006.01)}$

(86) International application number:
**PCT/US2011/059668**

(87) International publication number:
**WO 2012/064680 (18.05.2012 Gazette 2012/20)**

(54) **SELECTIVE GLYCOSIDASE INHIBITORS AND USES THEREOF**

SELEKTIVE GLYCOSIDASEHEMMER UND IHRE VERWENDUNG

INHIBITEURS SÉLECTIFS DE LA GLYCOSIDASE ET UTILISATIONS DE CEUX-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.10.2011 PCT/CN2011/080691**
**01.12.2010 US 418596 P**
**08.11.2010 PCT/CN2010/078528**

(43) Date of publication of application:
**18.09.2013 Bulletin 2013/38**

(73) Proprietors:
- **Alectos Therapeutics Inc.**
**Burnaby, British Columbia V5A 4B5 (CA)**
- **Merck Sharp & Dohme Corp.**
**Rahway, NJ 07065-0907 (US)**

(72) Inventors:
- **CHANG, Jiang**
**Beijing 100176 (CN)**
- **MCEACHERN, Ernest, J.**
**Burnaby**
**British Columbia V5A 4B5 (CA)**
- **MU, Changwei**
**Beijing 100176 (CN)**
- **SHI, Feng**
**Beijing 100176 (CN)**
- **VOCADLO, David, J.**
**Burnaby**
**British Columbia V5A 4B5 (CA)**
- **WANG, Yaode**
**Beijing 100176 (CN)**
- **WEI, Zhongyong**
**Beijing 100176 (CN)**
- **ZHOU, Yuanxi**
**Burnaby**
**British Columbia V5A 4B5 (CA)**
- **ZHU, Yongbao**
**Burnaby**
**British Columbia V5A 4B5 (CA)**

(74) Representative: **Buchan, Gavin MacNicol et al**
**Merck & Co., Inc.**
**European Patent Department**
**Hertford Road**
**Hoddesdon, Hertfordshire EN11 9BU (GB)**

(56) References cited:
**WO-A1-2013/028715 US-A1- 2010 016 386**

- **RAO ET AL.: 'Structural insights into the mechanism and inhibition of eukaryotic O-GlcNAc hydrolysis.' THE EMBO JOURNA 0261-4189 vol. 25, 2006, pages 1569 - 1578, XP055105437**
- **REMPEL ET AL.: 'Covalent inhibitors of glycosidases and their applications in biochemistry and biology.' GLYCOBIOLOGY vol. 18, no. 8, 2008, pages 570 - 586, XP055105451**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 637 503 B1

## Description

### FIELD OF THE INVENTION

This application relates to compounds which selectively inhibit glycosidases and uses thereof.

### BACKGROUND OF THE INVENTION

A wide range of cellular proteins, both nuclear and cytoplasmic, are post-translationally modified by the addition of the monosaccharide 2-acetamido-2-deoxy-ß-D-glucopyranoside (β-N-acetylglucosamine) which is attached via an O-glycosidic linkage.[1] This modification is generally referred to as O-linked N-acetylglucosamine or O-GlcNAc. The enzyme responsible for post-translationally linking β-N-acetylglucosamine (GlcNAc) to specific serine and threonine residues of numerous nucleocytoplasmic proteins is O-GlcNAc transferase (OGT).[2-5] A second enzyme, known as O-GlcNAcase[6,7] removes this post-translational modification to liberate proteins making the O-GlcNAc-modification a dynamic cycle occurring several times during the lifetime of a protein.[8]

O-GlcNAc-modified proteins regulate a wide range of vital cellular functions including, for example, transcription,[9-12] proteasomal degradation,[13] and cellular signaling.[14] O-GlcNAc is also found on many structural proteins.[15-17] For example, it has been found on a number of cytoskeletal proteins, including neurofilament protein,[18,19] synapsins,[6,20] synapsin-specific clathrin assembly protein AP-3,7 and ankyrinG.[14] O-GlcNAc modification has been found to be abundant in the brain.[21,22] It has also been found on proteins clearly implicated in the etiology of several diseases including Alzheimer's disease (AD) and cancer.

For example, it is well established that AD and a number of related tauopathies including Downs' syndrome, Pick's disease, Niemann-Pick Type C disease, and amyotrophic lateral sclerosis (ALS) are characterized, in part, by the development of neurofibrillary tangles (NFTs). These NFTs are aggregates of paired helical filaments (PHFs) and are composed of an abnormal form of the cytoskeletal protein "tau". Normally tau stabilizes a key cellular network of microtubules that is essential for distributing proteins and nutrients within neurons. In AD patients, however, tau becomes hyperphosphorylated, disrupting its normal functions, forming PHFs and ultimately aggregating to form NFTs. Six isoforms of tau are found in the human brain. In AD patients, all six isoforms of tau are found in NFTs, and all are markedly hyperphosphorylated.[23,24] Tau in healthy brain tissue bears only 2 or 3 phosphate groups, whereas those found in the brains of AD patients bear, on average, 8 phosphate groups.[25,26] A clear parallel between NFT levels in the brains of AD patients and the severity of dementia strongly supports a key role for tau dysfunction in AD.27,28 The precise causes of this hyperphosphorylation of tau remain elusive. Accordingly, considerable effort has been dedicated toward: a) elucidating the molecular physiological basis of tau hyperphosphorylation;[29] and b) identifying strategies that could limit tau hyperphosphorylation in the hope that these might halt, or even reverse, the progression of Alzheimer's disease[30-33] Thus far, several lines of evidence suggest that up-regulation of a number of kinases may be involved in hyperphosphorylation of tau,[21,34,35] although very recently, an alternative basis for this hyperphosphorylation has been advanced.[21]

In particular, it has recently emerged that phosphate levels of tau are regulated by the levels of O-GlcNAc on tau. The presence of O-GlcNAc on tau has stimulated studies that correlate O-GlcNAc levels with tau phosphorylation levels. The recent interest in this field stems from the observation that O-GlcNAc modification has been found to occur on many proteins at amino acid residues that are also known to be phosphorylated.[36-38] Consistent with this observation, it has been found that increases in phosphorylation levels result in decreased O-GlcNAc levels and conversely, increased O-GlcNAc levels correlate with decreased phosphorylation levels.[39] This reciprocal relationship between O-GlcNAc and phosphorylation has been termed the "Yin-Yang hypothesis"[40] and has gained strong biochemical support by the recent discovery that the enzyme OGT[4] forms a functional complex with phosphatases that act to remove phosphate groups from proteins.[41] Like phosphorylation, O-GlcNAc is a dynamic modification that can be removed and reinstalled several times during the lifespan of a protein. Suggestively, the gene encoding O-GlcNAcase has been mapped to a chromosomal locus that is linked to AD.[7,42] Hyperphosphorylated tau in human AD brains has markedly lower levels of O-GlcNAc than are found in healthy human brains.[21] Very recently, it has been shown that O-GlcNAc levels of soluble tau protein from human brains affected with AD are markedly lower than those from healthy brain.[21] Furthermore, PHF from diseased brain was suggested to lack completely any O-GlcNAc modification whatsoever.[21] The molecular basis of this hypoglycosylation of tau is not known, although it may stem from increased activity of kinases and/or dysfunction of one of the enzymes involved in processing O-GlcNAc. Supporting this latter view, in both PC-12 neuronal cells and in brain tissue sections from mice, a nonselective N-acetylglucosamindase inhibitor was used to increase tau O-GlcNAc levels, whereupon it was observed that phosphorylation levels decreased.[21] The implication of these collective results is that by maintaining healthy O-GlcNAc levels in AD patients, such as by inhibiting the action of O-GlcNAcase, one should be able to block hyperphosphorylation of tau and all of the associated effects of tau hyperphosphorylation, including the formation of NFTs and downstream effects. However, because the proper functioning of the β-hexosaminidases is critical, any potential therapeutic intervention for the treatment of AD that blocks the action of O-GlcNAcase would have

to avoid the concomitant inhibition of both hexosaminidases A and B.

Neurons do not store glucose and therefore the brain relies on glucose supplied by blood to maintain its essential metabolic functions. Notably, it has been shown that within brain, glucose uptake and metabolism decreases with aging.[43] Within the brains of AD patients marked decreases in glucose utilization occur and are thought to be a potential cause of neurodegeneration.[44] The basis for this decreased glucose supply in AD brain[45-47] is thought to stem from any of decreased glucose transport,[48,49] impaired insulin signaling,[50,51] and decreased blood flow.[52]

In light of this impaired glucose metabolism, it is worth noting that of all glucose entering into cells, 2-5% is shunted into the hexosamine biosynthetic pathway, thereby regulating cellular concentrations of the end product of this pathway, uridine diphosphate-N-acetylglucosamine (UDP-GlcNAc).[53] UDP-GlcNAc is a substrate of the nucleocytoplasmic enzyme O-GlcNAc transferase (OGT),[2-5] which acts to post-translationally add GlcNAc to specific serine and threonine residues of numerous nucleocytoplasmic proteins. OGT recognizes many of its substrates[54,55] and binding partners[41,56] through its tetratricopeptide repeat (TPR) domains.[57,58] As described above, O-GlcNAcase[6,7] removes this post-translational modification to liberate proteins making the O-GlcNAc-modification a dynamic cycle occurring several times during the lifetime of a protein.[8] O-GlcNAc has been found in several proteins on known phosphorylation sites,[10,37,38,59] including tau and neurofilaments.[60] Additionally, OGT shows unusual kinetic behaviour making it exquisitely sensitive to intracellular UDP-GlcNAc substrate concentrations and therefore glucose supply.[41]

Consistent with the known properties of the hexosamine biosynthetic pathway, the enzymatic properties of OGT, and the reciprocal relationship between O-GlcNAc and phosphorylation, it has been shown that decreased glucose availability in brain leads to tau hyperphosphorylation.[44] Therefore the gradual impairment of glucose transport and metabolism, whatever its causes, leads to decreased O-GlcNAc and hyperphosphorylation of tau (and other proteins). Accordingly, the inhibition of O-GlcNAcase should compensate for the age related impairment of glucose metabolism within the brains of health individuals as well as patients suffering from AD or related neurodegenerative diseases.

These results suggest that a malfunction in the mechanisms regulating tau O-GlcNAc levels may be vitally important in the formation of NFTs and associated neurodegeneration. Good support for blocking tau hyperphosphorylation as a therapeutically useful intervention[61] comes from recent studies showing that when transgenic mice harbouring human tau are treated with kinase inhibitors, they do not develop typical motor defects[33] and, in another case,[32] show decreased levels of insoluble tau. These studies provide a clear link between lowering tau phosphorylation levels and alleviating AD-like behavioural symptoms in a murine model of this disease. Indeed, pharmacological modulation of tau hyperphosphorylation is widely recognized as a valid therapeutic strategy for treating AD and other neurodegenerative disorders.[62]

Recent studies[63] support the therapeutic potential of small-molecule O-GlcNAcase inhibitors to limit tau hyperphosphorylation for treatment of AD and related tauopathies. Specifically, the O-GlcNAcase inhibitor thiamet-G has been implicated in the reduction of tau phosphorylation in cultured PC-12 cells at pathologically relevant sites.[63] Moreover, oral administration of thiamet-G to healthy Sprague-Dawley rats has been implicated in reduced phosphorylation of tau at Thr231, Ser396 and Ser422 in both rat cortex and hippocampus.[63].

There is also a large body of evidence indicating that increased levels of O-GlcNAc protein modification provides protection against pathogenic effects of stress in cardiac tissue, including stress caused by ischemia, hemorrhage, hypervolemic shock, and calcium paradox. For example, activation of the hexosamine biosynthetic pathway (HBP) by administration of glucosamine has been demonstrated to exert a protective effect in animals models of ischemia/reperfusion,[64-70] trauma hemorrhage,[71-73] hypervolemic shock,[74] and calcium paradox.[64,75] Moreover, strong evidence indicates that these cardioprotective effects are mediated by elevated levels of protein O-GlcNAc modification.[64,65,67,70,72,75-78] There is also evidence that the O-GlcNAc modification plays a role in a variety of neurodegenerative diseases, including Parkinson's disease and Huntington's disease.[79]

Humans have three genes encoding enzymes that cleave terminal β-N-acetyl-glucosamine residues from glycoconjugates. The first of these encodes the enzyme O-glycoprotein 2-acetamido-2-deoxy-β-D-glucopyranosidase (O-GlcNAcase). O-GlcNAcase is a member of family 84 of glycoside hydrolases that includes enzymes from organisms as diverse as prokaryotic pathogens to humans (for the family classification of glycoside hydrolases see Coutinho, P.M. & Henrissat, B. (1999) Carbohydrate-Active Enzymes server at URL: http://afmb.cnrs-mrs.fr/CAZY/.[27,28] O-GlcNAcase acts to hydrolyse O-GlcNAc off of serine and threonine residues of post-translationally modified proteins.[1,6,7,80,81] Consistent with the presence of O-GlcNAc on many intracellular proteins, the enzyme O-GlcNAcase appears to have a role in the etiology of several diseases including type II diabetes,[14,82] AD,[16,21,83] and cancer.[22,84] Although O-GlcNAcase was likely isolated earlier on,[18,19] about 20 years elapsed before its biochemical role in acting to cleave O-GlcNAc from serine and threonine residues of proteins was understood.[6] More recently O-GlcNAcase has been cloned,[7] partially characterized,[20] and suggested to have additional activity as a histone acetyltransferase.[20] However, little was known about the catalytic mechanism of this enzyme.

The other two genes, HEXA and HEXB, encode enzymes catalyzing the hydrolytic cleavage of terminal β-N-acetylglucosamine residues from glycoconjugates. The gene products of HEXA and HEXB predominantly yield two dimeric isozymes, hexosaminidase A and hexosaminidase B, respectively. Hexosaminidase A (αß), a heterodimeric

isozyme, is composed of an α- and a ß-subunit. Hexosaminidase B (ßß), a homodimeric isozyme, is composed of two ß-subunits. The two subunits, α- and ß-, bear a high level of sequence identity. Both of these enzymes are classified as members of family 20 of glycoside hydrolases and are normally localized within lysosomes. The proper functioning of these lysosomal β-hexosaminidases is critical for human development, a fact that is underscored by the tragic genetic illnesses, Tay-Sach's and Sandhoff diseases which stem from a dysfunction in, respectively, hexosaminidase A and hexosaminidase B.[85] These enzymatic deficiencies cause an accumulation of glycolipids and glycoconjugates in the lysosomes resulting in neurological impairment and deformation. The deleterious effects of accumulation of gangliosides at the organismal level are still being uncoveed.[86]

As a result of the biological importance of these β-*N*-acetyl-glucosaminidases, small molecule inhibitors of glycosidases[87-90] have received a great deal of attention,[91] both as tools for elucidating the role of these enzymes in biological processes and in developing potential therapeutic applications. The control of glycosidase function using small molecules offers several advantages over genetic knockout studies including the ability to rapidly vary doses or to entirely withdraw treatment.

However, a major challenge in developing inhibitors for blocking the function of mammalian glycosidases, including O-GlcNAcase, is the large number of functionally related enzymes present in tissues of higher eukaryotes. Accordingly, the use of non-selective inhibitors in studying the cellular and organismal physiological role of one particular enzyme is complicated because complex phenotypes arise from the concomitant inhibition of such functionally related enzymes. In the case of ß-N-acetylglucosaminidases, existing compounds that act to block O-GlcNAcase function are non-specific and act potently to inhibit the lysosomal β-hexosaminidases.

A few of the better characterized inhibitors of β-*N*-acetyl-glucosaminidases which have been used in studies of O-GlcNAc post-translational modification within both cells and tissues are streptozotocin (STZ), 2'-methyl-α-D-glucopyrano-[2,1-*d*]-Δ2'-thiazoline (NAG-thiazoline) and *O*-(2-acetamido-2-deoxy-$_D$-glucopyranosylidene)amino *N*-phenylcarbamate (PUGNAc).[14,92-95]

STZ has long been used as a diabetogenic compound because it has a particularly detrimental effect on ß-islet cells.[96] STZ exerts its cytotoxic effects through both the alkylation of cellular DNA[96,97] as well as the generation of radical species including nitric oxide.[98] The resulting DNA strand breakage promotes the activation of poly(ADP-ribose) polymerase (PARP)99 with the net effect of depleting cellular NAD+ levels and, ultimately, leading to cell death.[100,101] Other investigators have proposed instead that STZ toxicity is a consequence of the irreversible inhibition of O-GlcNAcase, which is highly expressed within ß-islet cells.[92,102] This hypothesis has, however, been brought into question by two independent research groups.[103,104] Because cellular O-GlcNAc levels on proteins increase in response to many forms of cellular stress[105] it seems possible that STZ results in increased O-GlcNAc-modification levels on proteins by inducing cellular stress rather than through any specific and direct action on O-GlcNAcase. Indeed, Hanover and coworkers have shown that STZ functions as a poor and somewhat selective inhibitor of O-GlcNAcase[106] and although it has been proposed by others that STZ acts to irreversibly inhibit O-GlcNAcase,[107] there has been no clear demonstration of this mode of action. Recently, it has been shown that STZ does not irreversibly inhibit O-GlcNAcase.[108]

NAG-thiazoline has been found to be a potent inhibitor of family 20 hexosaminidases,[90,109] and more recently, the family 84 O-GlcNAcases.[108] Despite its potency, a downside to using NAG-thiazoline in a complex biological context is that it lacks selectivity and therefore perturbs multiple cellular processes.

PUGNAc is another compound that suffers from the same problem of lack of selectivity, yet has enjoyed use as an inhibitor of both human O-GlcNAcase[6,110] and the family 20 human β-hexosaminidases.[111] This molecule, developed by Vasella and coworkers, was found to be a potent competitive inhibitor of the β-*N*-acetyl-glucosaminidases from *Canavalia ensiformis*, *Mucor rouxii*, and the β-hexosaminidase from bovine kidney.88 It has been demonstrated that administration of PUGNAc in a rat model of trauma hemorrhage decreases circulating levels of the pro-inflammatory cytokines TNF-α and IL-6.112 It has also been shown that administration of PUGNAc in a cell-based model of lymphocyte activation decreases production of the cytokine IL-2.[113] Recent studies have indicated that PUGNAc can be used in an animal model to reduce myocardial infarct size after left coronary artery occlusions.[114] Of particular significance is the fact that elevation of O-GlcNAc levels by administration of PUGNAc, an inhibitor of O-GlcNAcase, in a rat model of trauma hemorrhage improves cardiac function.[112,115] In addition, elevation of O-GlcNAc levels by treatment with PUGNAc in a cellular model of ischemia/reperfusion injury using neonatal rat ventricular myocytes improved cell viability and reduced necrosis and apoptosis compared to untreated cells.[116]

More recently, it has been suggested that the selective O-GlcNAcase inhibitor NButGT exhibits protective activity in cell-based models of ischemia/reperfusion and cellular stresses, including oxidative stress.[117] This study suggests the use of O-GlcNAcase inhibitors to elevate protein O-GlcNAc levels and thereby prevent the pathogenic effects of stress in cardiac tissue.

International patent applications PCT/CA2006/000300, filed 1 March 2006, published under No. WO 2006/092049 on 8 September 2006; PCT/CA2007/001554, filed 31 August 2007, published under No. WO 2008/025170 on 6 March 2008; PCT/CA2009/001087, filed 31 July 2009, published under No. WO 2010/012106 on 4 Februrary 2010; PCT/CA2009/001088, filed 31 July 2009, published under WO 2010/012107 on 4 Februrary 2010; and

PCT/CA2009/001302, filed 16 September 2009, published under WO 2010/037207 on 8 April 2010, describe selective inhibitors of O-GlcNAcase. WO 2013/028715 (which is prior art under Art. 54(3) EPC for some subject matter disclosed herein) describes a group of pyrano[3,2-d]thiazoles which are glycosidase inhibitors.

## SUMMARY OF THE INVENTION

The invention is directed to compounds for selectively inhibiting glycosidases, uses of the compounds and pharmaceutical compositions including the compounds. Methods of treating diseases and disorders related to deficiency or overexpression of O-GlcNAcase, and/or accumulation or deficiency of O-GlcNAc are also disclosed.

## DETAILED DESCRIPTION

The invention encompasses compounds of Formula (I) or a pharmaceutically acceptable salt thereof:

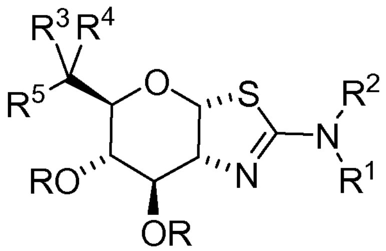

(I)

wherein each variable is as defined in claim 1 hereinafter.

The invention also encompasses a genus of compounds of Formula (Ia) or a pharmaceutically acceptable salt thereof:

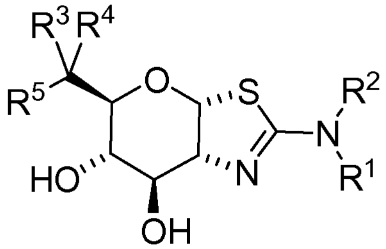

(Ia)

wherein the variables are as defined in claim 1 hereinafter

Within the genus, the invention encompasses a first sub-genus of compounds of Formula (Ia) wherein: $R^1$ and $R^2$ are independently $C_{1-4}$alkyl; $R^3$ is $C_{1-6}$alkyl; $R^4$ is selected from the group consisting of: H and $C_{1-6}$alkyl; and $R^5$ is OH. Within the first subgenus, the invention further encompasses compounds of Formula (Ia) wherein: $R^1$ and $R^2$ are independently methyl or ethyl; $R^3$ is methyl or ethyl; and $R^4$ is selected from the group consisting of: H, methyl and ethyl.

Also within the genus, the invention encompasses a sub-genus of compounds of Formula (Ia) wherein $R^3$ is $CF_3$ and $R^5$ is OH.

The invention also encompasses the compounds that follow or pharmaceutically acceptable salts thereof.

An embodiment of the invention encompasses compounds of Formula (I) wherein: each R is H, $R^5$ is OH, $R^3$ is $C_{1-6}$alkyl, optionally mono-substituted with fluoro or hydroxy, and $R^4$ is H.

An embodiment of the invention encompasses compounds of Formula (I) wherein: each R is H, $R^5$ is H, $R^3$ is $C_{1-6}$alkyl, optionally mono-substituted with fluoro or hydroxy, and $R^4$ is H or $C_{1-6}$alkyl.

An embodiment of the invention encompasses compounds of Formula (I) wherein $R^1$ is $C_{1-6}$alkyl, optionally mono-substituted with hydroxy, and $R^2$ is H.

An embodiment of the invention encompasses compounds of Formula (I) wherein $R^1$ is $C_{2-6}$alkenyl and $R^2$ is H.

An embodiment of the invention encompasses compounds of Formula (I) wherein each R is H and $R^5$ is F.

The invention also encompasses a pharmaceutical composition comprising the compound of Formula (I) or (Ia) in combination with a pharmaceutically acceptable carrier.

Also disclosed is a method of selectively inhibiting O-GlcNAcase in a subject in need thereof, the method comprising administering to the subject an effective amount of a compound of Formula (I) or (Ia), or a pharmaceutically acceptable salt thereof.

Also disclosed is a method of elevating the level of O-GlcNAc in a subject in need thereof, the method comprising administering to the subject an effective amount of a compound of Formula (I) or (Ia), or a pharmaceutically acceptable salt thereof.

Also disclosed is a method of treating a condition that is modulated by O-GlcNAcase, in a subject in need thereof, the method comprising administering to the subject an effective amount of a compound of Formula (I) or (Ia), or a pharmaceutically acceptable salt thereof. For example, the condition is selected from one or more of the group consisting of an inflammatory disease, an allergy, asthma, allergic rhinitis, hypersensitivity lung diseases, hypersensitivity pneumonitis, eosinophilic pneumonias, delayed-type hypersensitivity, atherosclerosis, interstitial lung disease (ILD), idiopathic pulmonary fibrosis, ILD associated with rheumatoid arthritis, systemic lupus erythematosus, ankylosing spondylitis, systemic sclerosis, Sjogren's syndrome, polymyositis or dermatomyositis, systemic anaphylaxis or hypersensitivity response, drug allergy, insect sting allergy, autoimmune disease, rheumatoid arthritis, psoriatic arthritis, multiple sclerosis, Guillain-Barré syndrome, systemic lupus erythematosus, myastenia gravis, glomerulonephritis, autoimmune thyroiditis, graft rejection, allograft rejection, graft-versus-host disease, inflammatory bowel disease, Crohn's disease, ulcerative colitis, spondyloarthropathy, scleroderma, psoriasis, T-cell mediated psoriasis, inflammatory dermatosis, dermatitis, eczema, atopic dermatitis, allergic contact dermatitis, urticaria, vasculitis, necrotizing, cutaneous, and hypersensitivity vasculitis, eosinphilic myotis, eosiniphilic fasciitis, solid organ transplant rejection, heart transplant rejection, lung transplant rejection, liver transplant rejection, kidney transplant rejection, pancreas transplant rejection, kidney allograft, lung allograft, epilepsy, pain, fibromyalgia, stroke, neuroprotection.

Also disclosed is a method of treating a condition selected from the group consisting of a neurodegenerative disease, a tauopathy, cancer and stress, in a subject in need thereof, the method comprising administering to the subject an effective amount of a compound of Formula (I) or (Ia), or a pharmaceutically acceptable salt thereof. For example, the condition is selected from one or more of the group consisting of Alzheimer's disease, Amyotrophic lateral sclerosis (ALS), Amyotrophic lateral sclerosis with cognitive impairment (ALSci), Argyrophilic grain dementia, Bluit disease, Corticobasal degeneration (CBD), Dementia pugilistica, Diffuse neurofibrillary tangles with calcification, Down's syndrome, Familial British dementia, Familial Danish dementia, Frontotemporal dementia with parkinsonism linked to chromosome 17 (FTDP-17), Gerstmann-Straussler-Scheinker disease, Guadeloupean parkinsonism, Hallevorden-Spatz disease (neurodegeneration with brain iron accumulation type 1), Multiple system atrophy, Myotonic dystrophy, Niemann-Pick disease (type C), Pallido-ponto-nigral degeneration, Parkinsonism-dementia complex of Guam, Pick's disease (PiD), Post-encephalitic parkinsonism (PEP), Prion diseases (including Creutzfeldt-Jakob Disease (CJD), Variant Creutzfeldt-Jakob Disease (vCJD), Fatal Familial Insomnia, and Kuru), Progressive supercortical gliosis, Progressive supranuclear palsy (PSP), Richardson's syndrome, Subacute sclerosing panencephalitis, Tangle-only dementia, Huntington's disease, Parkinson's disease, Schizophrenia, Mild Cognitive Impairment (MCI), Neuropathy (including peripheral neuropathy, autonomic neuropathy, neuritis, and diabetic neuropathy), or Glaucoma. Also disclosed is this method wherein the stress is a cardiac disorder. In another aspect, the cardiac disorder is selected from one or more of the group consisting of ischemia; hemorrhage; hypovolemic shock; myocardial infarction; an interventional cardiology procedure; cardiac bypass surgery; fibrinolytic therapy; angioplasty; and stent placement.

The compounds of the invention are capable of inhibiting an O-glycoprotein 2-acetamido-2-deoxy-β-D-glucopyranosidase (O-GlcNAcase). In some embodiments, the O-GlcNAcase is a mammalian O-GlcNAcase, such as a rat, mouse or human O-GlcNAcase. In some embodiments, the β-hexosaminidase is a mammalian β-hexosaminidase, such as a rat, mouse or human β-hexosaminidase.

Compounds of the invention selectively inhibit the activity of a mammalian O-GlcNAcase over a mammalian β-hexosaminidase. A compound that "selectively" inhibits an O-GlcNAcase is a compound that inhibits the activity or biological function of an O-GlcNAcase, but does not substantially inhibit the activity or biological function of a β-hexosaminidase. For example, in some embodiments, a selective inhibitor of an O-GlcNAcase selectively inhibits the cleavage of 2-acetamido-2-deoxy-β-D-glucopyranoside (O-GlcNAc) from polypeptides. In some embodiments, a selective inhibitor of an O-GlcNAcase selectively binds to an O-GlcNAcase. In some embodiments, a selective inhibitor of an O-GlcNAcase inhibits hyperphosphorylation of a tau protein and/or inhibits formations of NFTs. By "inhibits," "inhibition" or "inhibiting" means a decrease by any value between 10% and 90%, or of any integer value between 30% and 60%, or over 100%, or a decrease by 1-fold, 2-fold, 5-fold, 10-fold or more. It is to be understood that the inhibiting does not require full inhibition. In some embodiments, a selective inhibitor of an O-GlcNAcase elevates or enhances O-GlcNAc levels e.g., O-GlcNAc-modified polypeptide or protein levels, in cells, tissues, or organs (e.g., in brain, muscle, or heart (cardiac) tissue) and in animals. By "elevating" or "enhancing" is meant an increase by any value between 10% and 90%, or of any integer value between 30% and 60%, or over 100%, or an increase by 1-fold, 2-fold, 5-fold, 10-fold, 15-fold, 25-fold, 50-fold, 100-fold or more. In some embodiments, a selective inhibitor of an O-GlcNAcase exhibits a selectivity ratio, as described herein, in the range 10 to 100000, or in the range 100 to 100000, or in the range 1000 to 100000, or at least 10, 20, 50, 100, 200, 500, 1000, 1500, 2000, 2500, 3000, 3500, 4000, 4500, 5000, 6000, 7000, 10,000, 25,000, 50,000, 75,000, or any value within or about the described range.

The compounds of the present invention elevate O-GlcNAc levels on O-GlcNAc-modified polypeptides or proteins *in vivo* specifically via interaction with an O-GlcNAcase enzyme, and are effective in treating conditions which require or respond to inhibition of O-GlcNAcase activity.

In some embodiments, the compounds of the present invention are useful as agents that produce a decrease in tau phosphorylation and NFT formation. In some embodiments, the compounds are therefore useful to treat Alzheimer's disease and related tauopathies. In some embodiments, the compounds are thus capable of treating Alzheimer's disease and related tauopathies by lowering tau phosphorylation and reducing NFT formation as a result of increasing tau O-GlcNAc levels. In some embodiments, the compounds produce an increase in levels of O-GlcNAc modification on O-GlcNAc-modified polypeptides or proteins, and are therefore useful for treatment of disorders responsive to such increases in O-GlcNAc modification; these disorders include without limitation neurodegenerative, inflammatory, cardiovascular, and immunoregulatory diseases. In some embodiments, the compounds are also useful as a result of other biological activites related to their ability to inhibit the activity of glycosidase enzymes. In alternative embodiments, the compounds of the invention are valuable tools in studying the physiological role of O-GlcNAc at the cellular and organismal level.

Also disclosed are methods of enhancing or elevating levels of protein O-GlcNAc modification in animal subjects, such as, veterinary and human subjects. Also disclosed are methods of selectively inhibiting an O-GlcNAcase enzyme in animal subjects, such as, veterinary and human subjects. Also disclosed are methods of inhibiting phosphorylation of tau polypeptides, or inhibiting formation of NFTs, in animal subjects, such as, veterinary and human subjects.

The invention also encompasses the compounds of the invention for use in treating one or more of the diseases or conditions described herein. The invention also encompasses the use of the compounds of the invention for the manufacture of a medicament for treating one or more diseases or conditions described herein.

As will be appreciated by a person skilled in the art, Formula (I) above may also be represented alternatively as follows:

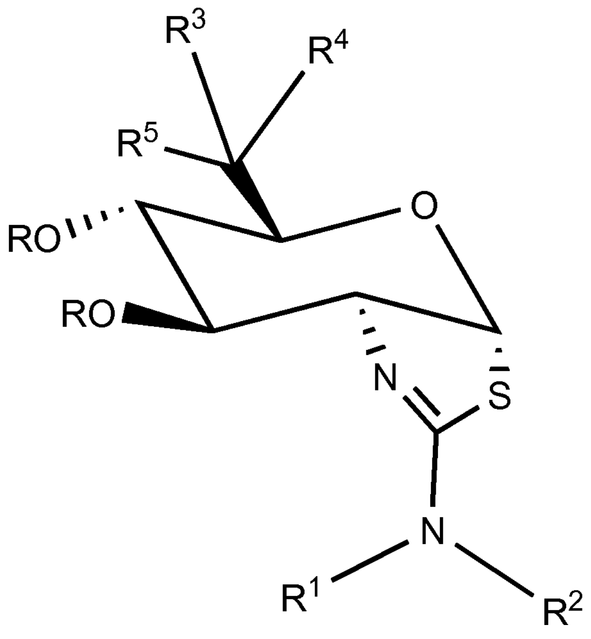

As used herein the singular forms "a", "and", and "the" include plural referents unless the context clearly dictates otherwise. For example, "a compound" refers to one or more of such compounds, while "the enzyme" includes a particular enzyme as well as other family members and equivalents thereof as known to those skilled in the art.

In some embodiments, all of the compounds of the invention contain at least one chiral center. In some embodiments, the formulations, preparation, and compositions including compounds according to the invention include mixtures of stereoisomers, individual stereoisomers, and enantiomeric mixtures, and mixtures of multiple stereoisomers. In general, the compound may be supplied in any desired degree of chiral purity.

"Alkyl" refers to a straight or branched hydrocarbon chain group consisting solely of carbon and hydrogen atoms, containing no unsaturation and including, for example, from one to ten carbon atoms, such as 1, 2, 3, 4, 5, 6, 7,

8, 9, or 10 carbon atoms, and which is attached to the rest of the molecule by a single bond. Unless stated otherwise specifically in the specification, the alkyl group may be optionally substituted by one or more substituents as described herein. Unless stated otherwise specifically herein, it is understood that the substitution can occur on any carbon of the alkyl group.

"Alkenyl" refers to a straight or branched hydrocarbon chain group consisting solely of carbon and hydrogen atoms, containing at least one double bond and including, for example, from two to ten carbon atoms, such as 2, 3, 4, 5, 6, 7, 8, 9, or 10 carbon atoms, and which is attached to the rest of the molecule by a single bond or a double bond. Unless stated otherwise specifically in the specification, the alkenyl group may be optionally substituted by one or more substituents as described herein. Unless stated otherwise specifically herein, it is understood that the substitution can occur on any carbon of the alkenyl group.

"Alkynyl" refers to a straight or branched hydrocarbon chain group consisting solely of carbon and hydrogen atoms, containing at least one triple bond and including, for example, from two to ten carbon atoms. Unless stated otherwise specifically in the specification, the alkenyl group may be optionally substituted by one or more substituents as described herein.

"Aryl" means mono- or bicyclic aromatic rings containing only carbon atoms, including for example, 6-14 members. Examples of aryl include phenyl, naphthyl, indanyl, indenyl, tetrahydronaphthyl, 2,3-dihydrobenzofuranyl, dihydrobenzopyranyl, 1,4-benzodioxanyl, and the like. Unless stated otherwise specifically herein, the term "aryl" is meant to include aryl groups optionally substituted by one or more substituents as described herein.

"Heteroaryl" refers to a single or fused aromatic ring group containing one or more heteroatoms in the ring, for example N, O, S, including for example, 5-14 members, such as 5, 6, 7, 8, 9, 10, 11, 12, 13 or 14 members. Examples of heteroaryl groups include furan, thiophene, pyrrole, oxazole, thiazole, imidazole, pyrazole, isoxazole, isothiazole, 1,2,3-oxadiazole, 1,2,3-triazole, 1,2,4-triazole, 1,3,4-thiadiazole, tetrazole, pyridine, pyridazine, pyrimidine, pyrazine, 1,3,5-triazine, imidazole, benzimidazole, benzoxazole, benzothiazole, indolizine, indole, isoindole, benzofuran, benzothiophene, 1H-indazole, purine, 4H-quinolizine, quinoline, isoquinoline, cinnoline, phthalazine, quinazoline, quinoxaline, 1,8-naphthyridine, pteridine. Unless stated otherwise specifically herein, the term "heteroaryl" is meant to include heteroaryl groups optionally substituted by one or more substituents as described herein.

"Cycloalkyl" refers to a stable monovalent monocyclic, bicyclic or tricyclic hydrocarbon group consisting solely of carbon and hydrogen atoms, having for example from 3 to 15 carbon atoms, and which is saturated and attached to the rest of the molecule by a single bond. Unless otherwise stated specifically herein, the term "cycloalkyl" is meant to include cycloalkyl groups which are optionally substituted as described herein.

The term "3 to 7-membered carbocyclic or heterocyclic ring" means a monocylic carbon ring of 3 to 7 atoms or a monocyclic ring of 3 to 7 atoms containing one or more heteroatoms selected from O, N and S.

"Optional" or "optionally" means that the subsequently described event of circumstances may or may not occur, and that the description includes instances where said event or circumstance occurs and instances in which it does not. For example, "optionally substituted alkyl" means that the alkyl group may or may not be substituted and that the description includes both substituted alkyl groups and alkyl groups having no substitution. Examples of optionally substituted alkyl groups include, without limitation, methyl, ethyl, propyl, etc. and including cycloalkyls such as cyclopropyl, cyclobutyl, cyclopentyl, cyclohexyl, cycloheptyl, etc.; examples of optionally substituted alkenyl groups include allyl, crotyl, 2-pentenyl, 3-hexenyl, 2-cyclopentenyl, 2-cyclohexenyl, 2-cyclopentenylmethyl, 2-cyclohexenylmethyl, etc. In some embodiments, optionally substituted alkyl and alkenyl groups include $C_{1-6}$ alkyls or alkenyls.

"Halo" refers to bromo, chloro, fluoro, iodo, etc. In some embodiments, suitable halogens include fluorine or chlorine.

Examples of optionally substituted carbonyl groups, or sulfonyl groups include optionally substituted forms of such groups formed from various hydrocarbyls such as alkyl, alkenyl and 5- to 6-membered monocyclic aromatic group (e.g., phenyl, pyridyl, etc.), as described herein.

Therapeutic Indications

There are disclosed methods of treating conditions that are modulated, directly or indirectly, by an O-GlcNAcase enzyme or by O-GlcNAc-modified protein levels, for example, a condition that is benefited by inhibition of an O-GlcNAcase enzyme or by an elevation of O-GlcNAc-modified protein levels. Such conditions include, without limitation, glaucoma, schizophrenia, tauopathies, such as Alzheimer's disease, neurodegenerative diseases, cardiovascular diseases, diseases associated with inflammation, diseases associated with immunosuppression and cancers. The compounds of the invention are also useful in the treatment of diseases or disorders related to deficiency or over-expression of O-GlcNAcase or accumulation or depletion of O-GlcNAc, or any disease or disorder responsive to glycosidase inhibition therapy. Such diseases and disorders include, but are not limited to, glaucoma, schizophrenia, neurodegenerative disorders, such as Alzheimer's disease (AD), or cancer. Such diseases and disorders may also include diseases or disorders related to the accumulation or deficiency in the enzyme OGT. Also included is a method of protecting or treating target cells

expressing proteins that are modified by O-GlcNAc residues, the dysregulation of which modification results in disease or pathology. The term "treating" as used herein includes treatment, prevention, and amelioration.

Also disclosed are methods of enhancing or elevating levels of protein O-GlcNAc modification in animal subjects, such as, veterinary and human subjects. This elevation of O-GlcNAc levels can be useful for the prevention or treatment of Alzheimer's disease; prevention or treatment of other neurodegenerative diseases (e.g. Parkinson's disease, Huntington's disease); providing neuroprotective effects; preventing damage to cardiac tissue; and treating diseases associated with inflammation or immunosuppression.

In general, the methods are effected by administering a compound according to the invention to a subject in need thereof, or by contacting a cell or a sample with a compound according to the invention, for example, a pharmaceutical composition comprising a therapeutically effective amount of the compound according to Formula (I) or (Ia). More particularly, they are useful in the treatment of a disorder in which the regulation of O-GlcNAc protein modification is implicated, or any condition as described herein. Disease states of interest include Alzheimer's disease (AD) and related neurodegenerative tauopathies, in which abnormal hyperphosphorylation of the microtubule-associated protein tau is involved in disease pathogenesis. In some embodiments, the compounds may be used to block hyperphosphorylation of tau by maintaining elevated levels of O-GlcNAc on tau, thereby providing therapeutic benefit.

The effectiveness of the compounds in treating pathology associated with the accumulation of toxic tau species (for example, Alzheimer's disease and other tauopathies) may be confirmed by testing the ability of the compounds to block the formation of toxic tau species in established cellular[118-120] and/or transgenic animal models of disease.[32,33] Tauopathies that may be treated with the compounds of the invention include: Alzheimer's disease, Amyotrophic lateral sclerosis (ALS), Amyotrophic lateral sclerosis with cognitive impairment (ALSci), Argyrophilic grain dementia, Bluit disease, Corticobasal degeneration (CBD), Dementia pugilistica, Diffuse neurofibrillary tangles with calcification, Down's syndrome, Familial British dementia, Familial Danish dementia, Frontotemporal dementia with parkinsonism linked to chromosome 17 (FTDP-17), Gerstmann-Straussler-Scheinker disease, Guadeloupean parkinsonism, Hallevorden-Spatz disease (neurodegeneration with brain iron accumulation type 1), Multiple system atrophy, Myotonic dystrophy, Niemann-Pick disease (type C), Pallido-ponto-nigral degeneration, Parkinsonism-dementia complex of Guam, Pick's disease (PiD), Post-encephalitic parkinsonism (PEP), Prion diseases (including Creutzfeldt-Jakob Disease (CJD), Variant Creutzfeldt-Jakob Disease (vCJD), Fatal Familial Insomnia, and Kuru), Progressive supercortical gliosis, Progressive supranuclear palsy (PSP), Richardson's syndrome, Subacute sclerosing panencephalitis, Tangle-only dementia, and Glaucoma.

The compounds of this invention are also useful in the treatment of conditions associate with tissue damage or stress, stimulating cells, or promoting differentiation of cells. Accordingly, in some embodiments, the compounds of this invention may be used to provide therapeutic benefit in a variety of conditions or medical procedures involving stress in cardiac tissue, including but not limited to: ischemia; hemorrhage; hypovolemic shock; myocardial infarction; an interventional cardiology procedure; cardiac bypass surgery; fibrinolytic therapy; angioplasty; and stent placement. The effectiveness of the compounds in treating pathology associated with cellular stress (including ischemia, hemorrhage, hypovolemic shock, myocardial infarction, and other cardiovascular disorders) may be confirmed by testing the ability of the compounds to prevent cellular damage in established cellular stress assays,[105,116,117] and to prevent tissue damage and promote functional recovery in animal models of ischemia-reperfusion,[70,114] and trauma-hemorrhage.[72,112,115]

Compounds that selectively inhibit O-GlcNAcase activity may be used for the treatment of diseases that are associated with inflammation, including but not limited to, inflammatory or allergic diseases such as asthma, allergic rhinitis, hypersensitivity lung diseases, hypersensitivity pneumonitis, eosinophilic pneumonias, delayed-type hypersensitivity, atherosclerosis, interstitial lung disease (ILD) (e.g., idiopathic pulmonary fibrosis, or ILD associated with rheumatoid arthritis, systemic lupus erythematosus, ankylosing spondylitis, systemic sclerosis, Sjogren's syndrome, polymyositis or dermatomyositis); systemic anaphylaxis or hypersensitivity responses, drug allergies, insect sting allergies; autoimmune diseases, such as rheumatoid arthritis, psoriatic arthritis, multiple sclerosis, Guillain-Barré syndrome, systemic lupus erythematosus, myastenia gravis, glomerulonephritis, autoimmune thyroiditis, graft rejection, including allograft rejection or graft-versus-host disease; inflammatory bowel diseases, such as Crohn's disease and ulcerative colitis; spondyloarthropathies; scleroderma; psoriasis (including T-cell mediated psoriasis) and inflammatory dermatoses such as dermatitis, eczema, atopic dermatitis, allergic contact dermatitis, urticaria; vasculitis (e.g., necrotizing, cutaneous, and hypersensitivity vasculitis); eosinphilic myotis, eosiniphilic fasciitis; and cancers.

In addition, compounds that affects levels of protein O-GlcNAc modification may be used for the treatment of diseases associated with immunosuppression, such as in individuals undergoing chemotherapy, radiation therapy, enhanced wound healing and burn treatment, therapy for autoimmune disease or other drug therapy (e.g., corticosteroid therapy) or combination of conventional drugs used in the treatment of autoimmune diseases and graft/transplantation rejection, which causes immunosuppression; or immunosuppression due to congenital deficiency in receptor function or other causes.

The compounds of the invention may be useful for treatment of neurodegenerative diseases, including Parkinson's disease and Huntington's disease. Other conditions that may be treated are those triggered, affected, or in any other way correlated with levels of O-GlcNAc post-translational protein modification. It is expected that the compounds of this invention may be useful for the treatment of such conditions and in particular, but not limited to, the following for which a association with O-GlcNAc levels on proteins has been established: graft rejection, in particular but not limited to solid organ transplants, such as heart, lung, liver, kidney, and pancreas transplants (e.g. kidney and lung allografts); cancer, in particular but not limited to cancer of the breast, lung, prostate, pancreas, colon, rectum, bladder, kidney, ovary; as well as non-Hodgkin's lymphoma and melanoma; epilepsy, pain, fibromyalgia, or stroke, e.g., for neuroprotection following a stroke.

## Pharmaceutical & Veterinary Compositions, Dosages, And Administration

Pharmaceutical compositions including compounds according to the invention, or for use according to the invention, are contemplated as being within the scope of the invention. In some embodiments, pharmaceutical compositions including an effective amount of a compound of Formula (I) or (Ia) are provided.

The compounds of Formula (I) or (Ia) and their pharmaceutically acceptable salts, stereoisomers, solvates, and derivatives are useful because they have pharmacological activity in animals, including humans. In some embodiments, the compounds according to the invention are stable in plasma, when administered to a subject.

In some embodiments, compounds according to the invention, or for use according to the invention, may be provided in combination with any other active agents or pharmaceutical compositions where such combined therapy is useful to modulate O-GlcNAcase activity, for example, to treat neurodegenerative, inflammatory, cardiovascular, or immunoregulatory diseases, or any condition described herein. In some embodiments, compounds according to the invention, or for use according to the invention, may be provided in combination with one or more agents useful in the prevention or treatment of Alzheimer's disease. Examples of such agents include, without limitation,

- acetylcholine esterase inhibitors (AChEIs) such as Aricept® (Donepezil), Exelon® (Rivastigmine), Razadyne® (Razadyne ER®, Reminyl®, Nivalin®, Galantamine), Cognex® (Tacrine), Dimebon, Huperzine A, Phenserine, Debio-9902 SR (ZT-1 SR), Zanapezil (TAK0147), ganstigmine, NP7557, etc.;
- NMDA receptor antagonists such as Namenda® (Axura®, Akatinol®, Ebixa®, Memantine), Dimebon, SGS-742, Neramexane, Debio-9902 SR (ZT-1 SR), etc.; gamma-secretase inhibitors and/or modulators such as Flurizan™ (Tarenflurbil, MPC-7869, R-flurbiprofen), LY450139, MK 0752, E2101, BMS-289948, BMS-299897, BMS-433796, LY-411575, GSI-136, etc.;
- beta-secretase inhibitors such as ATG-Z1, CTS-21166, etc.;
- alpha-secretase activators, such as NGX267, etc;
- amyloid-β aggregation and/or fibrillization inhibitors such as Alzhemed™ (3APS, Tramiprosate, 3-amino-1-propanesulfonic acid), AL-108, AL-208, AZD-103, PBT2, Cereact, ONO-2506PO, PPI-558, etc.;
- tau aggregation inhibitors such as methylene blue, etc.;
  microtubule stabilizers such as AL-108, AL-208, paclitaxel, etc.;
- RAGE inhibitors, such as TTP488, etc.;
- 5-HT1a receptor antagonists, such as Xaliproden, Lecozotan, etc.;
- 5-HT4 receptor antagonists, such as PRX-03410, etc.;
- kinase inhibitors such as SRN-003-556, amfurindamide, LiCl, AZD1080, NP031112, SAR-502250, etc.
- humanized monoclonal anti-Aβ antibodies such as Bapineuzumab (AAB-001), LY2062430, RN1219, ACU-5A5, etc.;
- amyloid vaccines such as AN-1792, ACC-001
- neuroprotective agents such as Cerebrolysin, AL-108, AL-208, Huperzine A, etc.;
- L-type calcium channel antagonists such as MEM-1003, etc.;
- nicotinic receptor antagonists, such as AZD3480, GTS-21, etc.;
- nicotinic receptor agonists, such as MEM 3454, Nefiracetam, etc.;
- peroxisome proliferator-activated receptor (PPAR) gamma agonists such as Avandia® (Rosglitazone), etc.;
- phosphodiesterase IV (PDE4) inhibitors, such as MK-0952, etc.;
- hormone replacement therapy such as estrogen (Premarin), etc.;
- monoamine oxidase (MAO) inhibitors such as NS2330, Rasagiline (Azilect®), TVP-1012, etc.;
- AMPA receptor modulators such as Ampalex (CX 516), etc.;
  nerve growth factors or NGF potentiators, such as CERE-110 (AAV-NGF), T-588, T-817MA, etc.;
- agents that prevent the release of luteinizing hormone (LH) by the pituitary gland, such as leuoprolide (VP-4896), etc.;
- GABA receptor modulators such as AC-3933, NGD 97-1, CP-457920, etc.; benzodiazepine receptor inverse agonists such as SB-737552 (S-8510), AC-3933, etc.;
- noradrenaline-releasing agents such as T-588, T-817MA, etc.

It is to be understood that combination of compounds according to the invention, or for use according to the invention, with Alzheimer's agents is not limited to the examples described herein, but includes combination with any agent useful for the treatment of Alzheimer's disease. Combination of compounds according to the invention, or for use according to the invention, and other Alzheimer's agents may be administered separately or in conjunction. The administration of one agent may be prior to, concurrent to, or subsequent to the administration of other agent(s).

Compounds according to the invention, or for use according to the invention, can be provided alone or in combination with other compounds in the presence of a liposome, an adjuvant, or any pharmaceutically acceptable carrier, diluent or excipient, in a form suitable for administration to a subject such as a mammal, for example, humans, cattle, sheep, etc. If desired, treatment with a compound according to the invention may be combined with more traditional and existing therapies for the therapeutic indications described herein. Compounds according to the invention may be provided chronically or intermittently. "Chronic" administration refers to administration of the compound(s) in a continuous mode as opposed to an acute mode, so as to maintain the initial therapeutic effect (activity) for an extended period of time. "Intermittent" administration is treatment that is not consecutively done without interruption, but rather is cyclic in nature. The terms "administration," "administrable," or "administering" as used herein should be understood to mean providing a compound of the invention to the subject in need of treatment.

"Pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier that has been approved, for example, by the United States Food and Drug Administration or other governmental agency as being acceptable for use in humans or domestic animals.

The compounds of the present invention may be administered in the form of pharmaceutically acceptable salts. In such cases, pharmaceutical compositions in accordance with this invention may comprise a salt of such a compound, preferably a physiologically acceptable salt, which are known in the art. In some embodiments, the term "pharmaceutically acceptable salt" as used herein means an active ingredient comprising compounds of Formula (I) or (Ia) used in the form of a salt thereof, particularly where the salt form confers on the active ingredient improved pharmacokinetic properties as compared to the free form of the active ingredient or other previously disclosed salt form.

A "pharmaceutically acceptable salt" includes both acid and base addition salts. A "pharmaceutically acceptable acid addition salt" refers to those salts which retain the biological effectiveness and properties of the free bases, which are not biologically or otherwise undesirable, and which are formed with inorganic acids such as hydrochloric acid, hydrobromic acid, sulfuric acid, nitric acid, phosphoric acid and the like, and organic acids such as acetic acid, trifluoroacetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like.

A "pharmaceutically acceptable base addition salt" refers to those salts which retain the biological effectiveness and properties of the free acids, which are not biologically or otherwise undesirable. These salts are prepared from addition of an inorganic base or an organic base to the free acid. Salts derived from inorganic bases include, but are not limited to, the sodium, potassium, lithium, ammonium, calcium , magnesium, iron, zinc, copper, manganese, aluminium salts and the like. Preferred inorganic salts are the ammonium, sodium, potassium, calcium, and magnesium salts. Salts derived from organic bases include, but are not limited to, salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, dicyclohexylamine, lysine, arginine, histidine, caffeine, procaine, hydrabamine, choline, betaine, ethylenediamine, glucosamine,methylglucamine, theobromine, purines, piperazine, piperidine, N-ethylpiperidine, polyamine resins and the like.

Thus, the term "pharmaceutically acceptable salt" encompasses all acceptable salts including but not limited to acetate, lactobionate, benzenesulfonate, laurate, benzoate, malate, bicarbonate, maleate, bisulfate, mandelate, bitartarate, mesylate, borate, methylbromide, bromide, methylnitrite, calcium edetate, methylsulfate, camsylate, mucate, carbonate, napsylate, chloride, nitrate, clavulanate, N-methylglucamine, citrate, ammonium salt, dihydrochloride, oleate, edetate, oxalate, edisylate, pamoate (embonate), estolate, palmitate, esylate, pantothenate, fumarate, phosphate/diphosphate, gluceptate, polygalacturonate, gluconate, salicylate, glutame, stearate, glycollylarsanilate, sulfate, hexylresorcinate, subacetate, hydradamine, succinate, hydrobromide, tannate, hydrochloride, tartrate, hydroxynaphthoate, teoclate, iodide, tosylate, isothionate, triethiodide, lactate, panoate, valerate, and the like.

Pharmaceutically acceptable salts of the compounds of the present invention can be used as a dosage for modifying solubility or hydrolysis characteristics, or can be used in sustained release or prodrug formulations. Also, pharmaceutically acceptable salts of the compounds of this invention may include those formed from cations such as sodium, potassium, aluminum, calcium, lithium, magnesium, zinc, and from bases such as ammonia, ethylenediamine, N-methyl-glutamine, lysine, arginine, ornithine, choline, N,N'-dibenzylethylene-diamine, chloroprocaine, diethanolamine, procaine, N-benzylphenethyl-amine, diethylamine, piperazine, tris(hydroxymethyl)aminomethane, and tetramethylammonium hydroxide.

Pharmaceutical formulations will typically include one or more carriers acceptable for the mode of administration of the preparation, be it by injection, inhalation, topical administration, lavage, or other modes suitable for the selected treatment. Suitable carriers are those known in the art for use in such modes of administration.

Suitable pharmaceutical compositions may be formulated by means known in the art and their mode of administration and dose determined by the skilled practitioner. For parenteral administration, a compound may be dissolved in sterile water or saline or a pharmaceutically acceptable vehicle used for administration of non-water soluble compounds such as those used for vitamin K. For enteral administration, the compound may be administered in a tablet, capsule or dissolved in liquid form. The table or capsule may be enteric coated, or in a formulation for sustained release. Many suitable formulations are known, including, polymeric or protein microparticles encapsulating a compound to be released, ointments, gels, hydrogels, or solutions which can be used topically or locally to administer a compound. A sustained release patch or implant may be employed to provide release over a prolonged period of time. Many techniques known to skilled practitioners are described in Remington: the Science & Practice of Pharmacy by Alfonso Gennaro, 20th ed., Williams & Wilkins, (2000). Formulations for parenteral administration may, for example, contain excipients, polyalkylene glycols such as polyethylene glycol, oils of vegetable origin, or hydrogenated naphthalenes. Biocompatible, biodegradable lactide polymer, lactide/glycolide copolymer, or polyoxyethylene-polyoxypropylene copolymers may be used to control the release of the compounds. Other potentially useful parenteral delivery systems for modulatory compounds include ethylene-vinyl acetate copolymer particles, osmotic pumps, implantable infusion systems, and liposomes. Formulations for inhalation may contain excipients, for example, lactose, or may be aqueous solutions containing, for example, polyoxyethylene-9-lauryl ether, glycocholate and deoxycholate, or may be oily solutions for administration in the form of nasal drops, or as a gel.

The compounds or pharmaceutical compositions according to the present invention may be administered by oral or non-oral, e.g., intramuscular, intraperitoneal, intravenous, intracisternal injection or infusion, subcutaneous injection, transdermal or transmucosal routes. In some embodiments, compounds or pharmaceutical compositions in accordance with this invention or for use in this invention may be administered by means of a medical device or appliance such as an implant, graft, prosthesis, stent, etc. Implants may be devised which are intended to contain and release such compounds or compositions. An example would be an implant made of a polymeric material adapted to release the compound over a period of time. The compounds may be administered alone or as a mixture with a pharmaceutically acceptable carrier *e.g*., as solid formulations such as tablets, capsules, granules, powders, etc.; liquid formulations such as syrups, injections, etc.; injections, drops, suppositories, pessaryies. In some embodiments, compounds or pharmaceutical compositions in accordance with this invention or for use in this invention may be administered by inhalation spray, nasal, vaginal, rectal, sublingual, or topical routes and may be formulated, alone or together, in suitable dosage unit formulations containing conventional non-toxic pharmaceutically acceptable carriers, adjuvants and vehicles appropriate for each route of administration.

The compounds of the invention may be used to treat animals, including mice, rats, horses, cattle, sheep, dogs, cats, and monkeys. However, compounds of the invention can also be used in other organisms, such as avian species (*e.g.*, chickens). The compounds of the invention may also be effective for use in humans. The term "subject" or alternatively referred to herein as "patient" is intended to be referred to an animal, preferably a mammal, most preferably a human, who has been the object of treatment, observation or experiment. However, the compounds, methods and pharmaceutical compositions of the present invention may be used in the treatment of animals. Accordingly, as used herein, a "subject" may be a human, non-human primate, rat, mouse, cow, horse, pig, sheep, goat, dog, cat, etc. The subject may be suspected of having or at risk for having a condition requiring modulation of O-GlcNAcase activity.

An "effective amount" of a compound according to the invention includes a therapeutically effective amount or a prophylactically effective amount. A "therapeutically effective amount" refers to an amount effective, at dosages and for periods of time necessary, to achieve the desired therapeutic result, such as inhibition of an O-GlcNAcase, elevation of O-GlcNAc levels, inhibition of tau phosphorylation, or any condition described herein. A therapeutically effective amount of a compound may vary according to factors such as the disease state, age, sex, and weight of the individual, and the ability of the compound to elicit a desired response in the individual. Dosage regimens may be adjusted to provide the optimum therapeutic response. A therapeutically effective amount is also one in which any toxic or detrimental effects of the compound are outweighed by the therapeutically beneficial effects. A "prophylactically effective amount" refers to an amount effective, at dosages and for periods of time necessary, to achieve the desired prophylactic result, such as inhibition of an O-GlcNAcase, elevation of O-GlcNAc levels, inhibition of tau phosphorylation, or any condition described herein. Typically, a prophylactic dose is used in subjects prior to or at an earlier stage of disease, so that a prophylactically effective amount may be less than a therapeutically effective amount. A suitable range for therapeutically or prophylactically effective amounts of a compound may be any integer from 0.1 nM-0.1M, 0.1 nM-0.05M, 0.05 nM-15$\mu$M or 0.01 nM-10$\mu$M.

In alternative embodiments, in the treatment or prevention of conditions which require modulation of O-GlcNAcase activity, an appropriate dosage level will generally be about 0.01 to 500 mg per kg subject body weight per day,

and can be administered in singe or multiple doses. In some embodiments, the dosage level will be about 0.1 to about 250 mg/kg per day. It will be understood that the specific dose level and frequency of dosage for any particular patient may be varied and will depend upon a variety of factors including the activity of the specific compound used, the metabolic stability and length of action of that compound, the age, body weight, general health, sex, diet, mode and time of administration, rate of excretion, drug combination, the severity of the particular condition, and the patient undergoing therapy.

It is to be noted that dosage values may vary with the severity of the condition to be alleviated. For any particular subject, specific dosage regimens may be adjusted over time according to the individual need and the professional judgement of the person administering or supervising the administration of the compositions. Dosage ranges set forth herein are exemplary only and do not limit the dosage ranges that may be selected by medical practitioners. The amount of active compound(s) in the composition may vary according to factors such as the disease state, age, sex, and weight of the subject. Dosage regimens may be adjusted to provide the optimum therapeutic response. For example, a single bolus may be administered, several divided doses may be administered over time or the dose may be proportionally reduced or increased as indicated by the exigencies of the therapeutic situation. It may be advantageous to formulate parenteral compositions in dosage unit form for ease of administration and uniformity of dosage. In general, compounds of the invention should be used without causing substantial toxicity, and as described herein, the compounds exhibit a suitable safety profile for therapeutic use. Toxicity of the compounds of the invention can be determined using standard techniques, for example, by testing in cell cultures or experimental animals and determining the therapeutic index, i.e., the ratio between the LD50 (the dose lethal to 50% of the population) and the LD100 (the dose lethal to 100% of the population). In some circumstances however, such as in severe disease conditions, it may be necessary to administer substantial excesses of the compositions

In the compounds of generic Formula (I) or (Ia), the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the compounds of generic Formula (I) or (Ia). For example, different isotopic forms of hydrogen (H) include protium ($^1H$), deuterium ($^2H$) and tritium ($^3H$). Protium is the predominant hydrogen isotope found in nature. Enriching for deuterium may afford certain therapeutic advantages, such as increasing *in vivo* half-life or reducing dosage requirements, or may provide a compound useful as a standard for characterization of biological samples. Isotopically-enriched compounds within generic Formula (I) or (Ia) can be prepared without undue experimentation by conventional techniques well known to those skilled in the art or by processes analogous to those described in the Schemes and Examples herein using appropriate isotopically-enriched reagents and/or intermediates.

Other Uses and Assays

A compound of Formula (I) or (Ia) may be used in screening assays for compounds which modulate the activity of glycosidase enzymes, preferably the O-GlcNAcase enzyme. The ability of a test compound to inhibit O-GlcNAcase-dependent cleavage of O-GlcNAc from a model substrate may be measured using any assays, as described herein or known to one of ordinary skill in the art. For example, a fluoresence or UV-based assay known in the art may be used. A "test compound" is any naturally-occurring or artificially-derived chemical compound. Test compounds may include, without limitation, peptides, polypeptides, synthesised organic molecules, naturally occurring organic molecules, and nucleic acid molecules. A test compound can "compete" with a known compound such as a compound of Formula (I) or (Ia) by, for example, interfering with inhibition of O-GlcNAcase-dependent cleavage of O-GlcNAc or by interfering with any biological response induced by a compound of Formula (I) or (Ia).

Generally, a test compound can exhibit any value between 10% and 200%, or over 500%, modulation when compared to a compound of Formula (I) or (Ia) or other reference compound. For example, a test compound may exhibit at least any positive or negative integer from 10% to 200% modulation, or at least any positive or negative integer from 30% to 150% modulation, or at least any positive or negative integer from 60% to 100% modulation, or any positive or negative integer over 100% modulation. A compound that is a negative modulator will in general decrease modulation relative to a known compound, while a compound that is a positive modulator will in general increase modulation relative to a known compound.

In general, test compounds are identified from large libraries of both natural products or synthetic (or semi-synthetic) extracts or chemical libraries according to methods known in the art. Those skilled in the field of drug discovery and development will understand that the precise source of test extracts or compounds is not critical to the method(s) of the invention. Accordingly, virtually any number of chemical extracts or compounds can be screened using the exemplary methods described herein. Examples of such extracts or compounds include, but are not limited to, plant-, fungal-, prokaryotic- or animal-based extracts, fermentation broths, and synthetic compounds, as well as modification of existing compounds. Numerous methods are also available for generating random or directed synthesis (e.g., semi-synthesis or total synthesis) of any number of chemical compounds, including, but not limited to, saccharide-, lipid-,

peptide-, and nucleic acid-based compounds. Synthetic compound libraries are commercially available. Alternatively, libraries of natural compounds in the form of bacterial, fungal, plant, and animal extracts are commercially available from a number of sources, including Biotics (Sussex, UK), Xenova (Slough, UK), Harbor Branch Oceanographic Institute (Ft. Pierce, FL, USA), and PharmaMar, MA, USA. In addition, natural and synthetically produced libraries are produced, if desired, according to methods known in the art, e.g., by standard extraction and fractionation methods. Furthermore, if desired, any library or compound is readily modified using standard chemical, physical, or biochemical methods.

When a crude extract is found to modulate inhibition of O-GlcNAcase-dependent cleavage of O-GlcNAc, or any biological response induced by a compound of Formula (I) or (Ia), further fractionation of the positive lead extract is necessary to isolate chemical constituents responsible for the observed effect. Thus, the goal of the extraction, fractionation, and purification process is the careful characterization and identification of a chemical entity within the crude extract having O-GlcNAcase- inhibitory activities. The same assays described herein for the detection of activities in mixtures of compounds can be used to purify the active component and to test derivatives thereof. Methods of fractionation and purification of such heterogeneous extracts are known in the art. If desired, compounds shown to be useful agents for treatment are chemically modified according to methods known in the art. Compounds identified as being of therapeutic, prophylactic, diagnostic, or other value may be subsequently analyzed using a suitable animal model, as described herein on known in the art.

In some embodiments, the compounds are useful in the development of animal models for studying diseases or disorders related to deficiencies in O-GlcNAcase, over-expression of O-GlcNAcase, accumulation of O-GlcNAc, depletion of O-GlcNAc, and for studying treatment of diseases and disorders related to deficiency or over-expression of O-GlcNAcase, or accumulation or depletion of O-GlcNAc. Such diseases and disorders include neurodegenerative diseases, including Alzheimer's disease, and cancer.

Various alternative embodiments and examples of the invention are described herein. These embodiments and examples are illustrative and should not be construed as limiting the scope of the invention.

## Examples

The following examples are intended to illustrate embodiments of the invention and are not intended to be construed in a limiting manner.

## Abbreviations

| | |
|---|---|
| AIBN | = 2,2'-Azobisisobutyronitrile |
| DAST | = (Diethylamino)sulfur trifluoride |
| DCM | = dichloromethane |
| DIBAL-H | = Diisobutylaluminum hydride |
| DMAP | = 4-dimethylaminopyridine |
| DMF | = *N,N*-dimethylformamide |
| DMP | = Dess-Martin periodinane |
| DMSO | = dimethyl sulfoxide |
| EDC | = 1-(3-Dimethylaminopropyl)-3-ethlycarbodiimide hydrochloride |
| NBS | = N-bromosuccinimide |
| PMBBr | = para-methoxy benzyl bromide |
| TBAB | = tetra-n-butylammonium bromide |
| TBAF | = tetra-*n*-butylammonium fluoride |
| TEA | = triethylamine |
| TEAF | = tetraethylammonium fluoride |
| TEMPO | = 2,2,6,6-tetramethyl-piperidin-1-oxy free radical |
| TFA | = 2,2,2-trifluoroacetic acid |
| THF | = tetrahydrofuran |

**Synthesis of intermediate** (**5**)

**1,3,4,6-tetra-O-acetyl-2-deoxy-2-isothiocyanate-β-D-glueopyranose (5)**

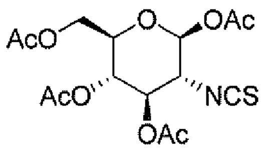

**Scheme I**

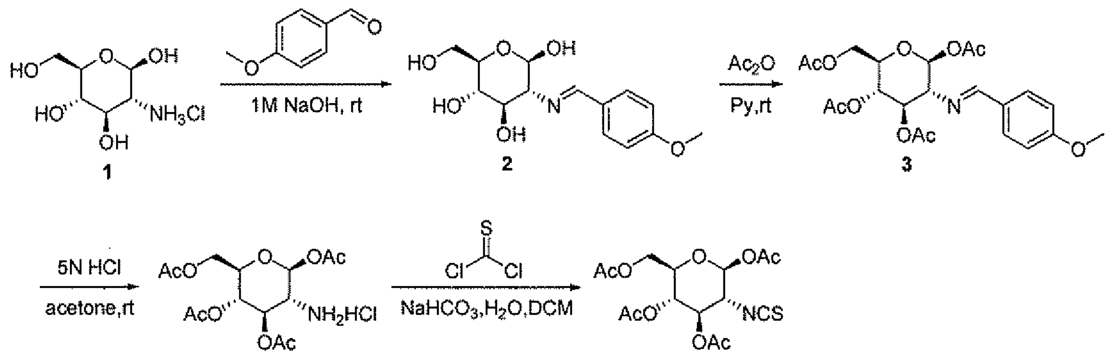


1 2 3 4 5

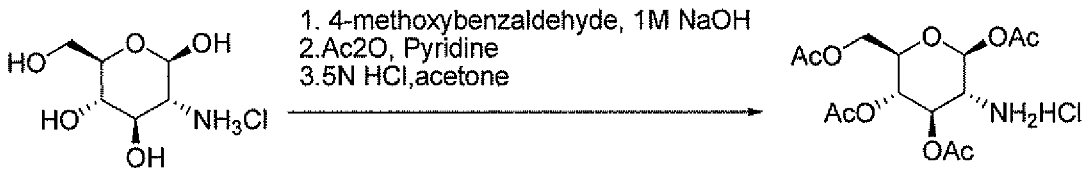


1 4

**(2S,3R,4R,5S,6R)-6-(acetoxymethyl)-3-amino-tetrahydro-2H-pyran-2,4,5-triyl triacetate hydrochloride (4)** was prepared from compound **1** according to publication: D. J. Silva etc. J. Org. Chem., 1999, 64(16), 5926-5929.

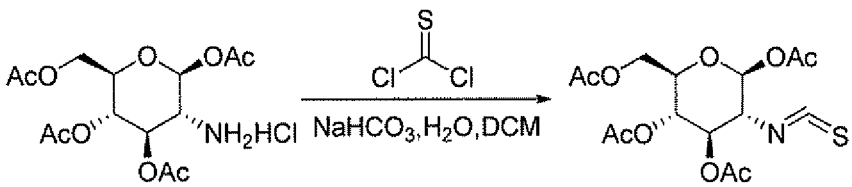


5

4

**(25,3R,4R,5S,6R)-6-(acetoxymethyl)-3-isothiocyanato-tetrahydro-2H-pyran-2,4,5-triyl triacetate** ( **5)** was prepared from compound **4** according to publication: M.V. Gonzalez etc. Carbohydrate Research, 1986, 154, 49-62.

**Example 1**

**(3aR,5R,6S,7R,7aR)-2-(Dimethylamino)-5-((S)-1-hydroxyethyl)-5,6,7,7a-tetrahydro-3aH-pyrano[3,2-d]thiazole-6,7-diol**

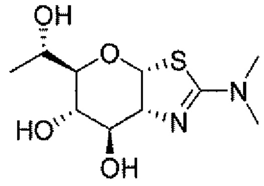

**Scheme II**

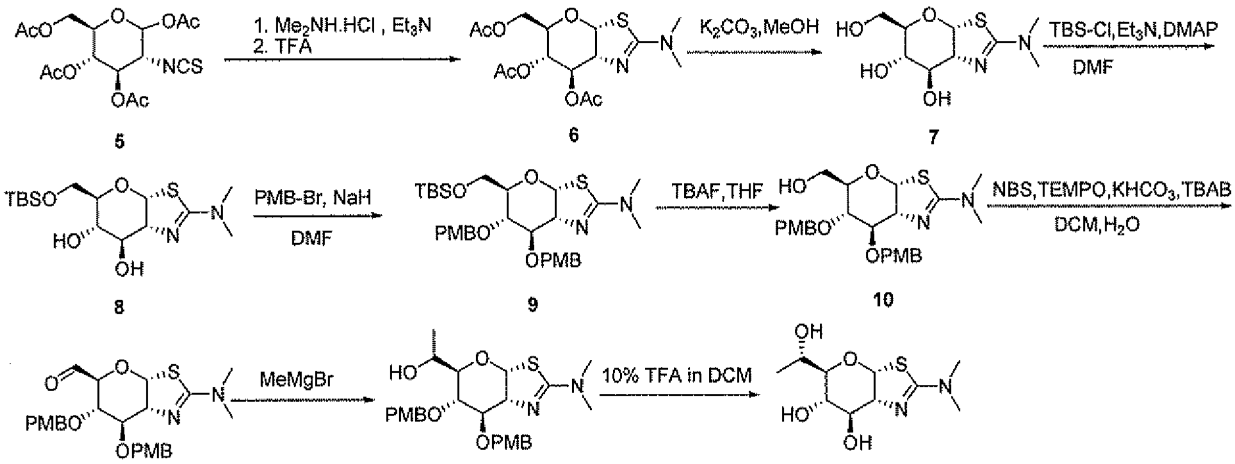


5 6 7 8 9 10 11 12 13

**Step 1**

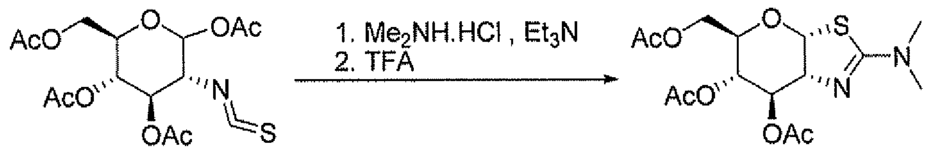


5 6

**(3aR,5R,6S,7R,7aR)-5-(Acetoxymethyl)-2-(dimethylamino)-5,6,7,7a-tetrahydro-3aH-pyrano[3,2-d]thiazole-6,7-diyl diacetate (6):** To a solution of (3R,4R,5S,6R)-6-(acetoxymethyl)-3-isothiocyanato-tetrahydro-2H-pyran-2,4,5-triyl triacetate (2 g, 5.14 mmol) in dichloromethane (20 mL) was added dimethylamine hydrochloride (460 mg, 5.64 mmol) and triethylamine (675 mg, 6.68 mmol) at 5~10 °C. After stirred for 3 h, the reaction mixture was treated with TFA (1.6 g, 14 mmol) overnight at room temperature. The reaction mixture was washed with saturated sodium bicarbonate (50 mL), dried over anhydrous magnesium sulfate, and concentrated under vacuum to provide a residue, which was purified by silica gel column, eluted with 1 % MeOH in dichloromethane to give compound **6** as yellow oil (1.65 g, 85 %). (ES, *m/z*): $[M+H]^+$ 374.9; $^1H$ NMR (300 MHz, $CDCl_3$) $\delta$ 6.24 - 6.26 (d, *J* = 6.6 Hz, 1H), 5.31 - 5.43 (m, 1H), 4.94 - 4.99 (m, 1H), 4.34 - 4.38 (t, *J* = 10.8 Hz, 1H), 4.16 - 4.22 (m, 2H), 4.38 - 4.39 (m, 1H), 3.02 (s, 6H), 2.06 - 2.12 (m ,9H).

**Step 2**

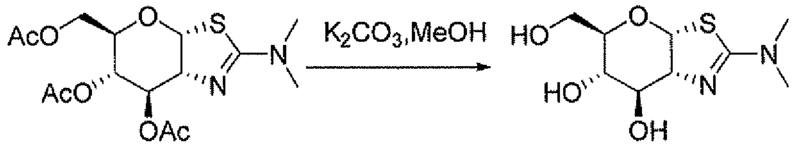


6 7

**(3aR,5R,6S,7R,7aR)-2-(Dimethylamino)-5-(hydroxymethyl)-5,6,7,7a-tetrahydro-3aH-pyrano[3,2-d]thiazole-6,7-diol (7):** To a solution of (3aR,5R,6S,7R,7aR)-5-(acetoxymethyl)-2-(dimethylamino)-5,6,7,7a-tetrahydro-3aH-pyrano[3,2-d]thiazole-6,7-diyl diacetate (1.65 g, 4.41 mmol) in methanol (20 mL) was added potassium carbonate (25 mg, 0.18 mmol). The resulting mixture was stirred overnight at room temperature to yield a solid. This was collected by filtration, washed with cold methanol and dried. The product **7** was obtained as a light yellow solid (1.05 g, 94 %). (ES, *m/z*): $[M+H]^+$ 248.9; $^1H$ NMR (300 MHz, $CDCl_3$) $\delta$ 6.33 - 6.35 (d, *J* = 6.3 Hz, 1H), 4.29 - 4.33 (t, *J* = 6.0 Hz 1H), 4.16 (s, 1H), 3.76 - 3.89 (m, 2H), 3.70 (s, 2H), 3.03 (s, 6H).

**Step 3**

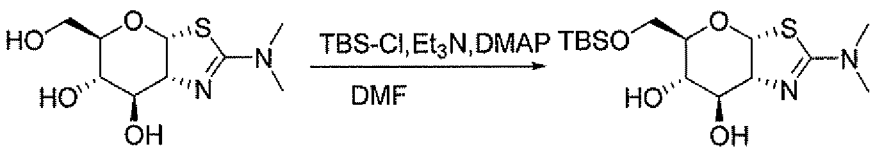


7 8

**(3aR,5R,6S,7R,7aR)-5-((tert-Butyldimethylsilyloxy)methyl)-2-(dimethylamino)-5,6,7,7a-tetrahydro-3aH-pyrano[3,2-d]thiazole-6,7-diol (8):** To a solution of (3aR,5R,6S,7R,7aR)-2-(dimethylamino)-5-(hydroxymethyl)-5,6,7,7a-tetrahydro-3aH-pyrano[3,2-d]thiazole-6,7-diol (1 g, 4.03 mmol), DMAP (49.2 mg, 0.40 mmol) and triethylamine (611 mg, 6.05 mmol) in DMF (50mL) was added tert-butylchlorodimethylsilane (665 mg, 4.43 mmol). After stirred overnight at 50 °C, the resulting mixture was concentrated under vacuum to provide a residue, which was purified by silica gel column, eluted with 2 -5% MeOH in dichloromethane to give compound 8 as a yellow solid (1.0 g, 65 %). (ES, *m/z*): $[M+H]^+$ 263.0; $^1H$ NMR (300 MHz, $CDCl_3$) $\delta$ 6.33 - 6.35 (d, *J* = 6.3 Hz, 1H), 4.35 - 4.39 (t, *J* = 5.7 Hz, 1H), 4.18 - 4.21 (t, *J* = 4.5 Hz, 1H), 3.81 - 3.84 (m, 3H), 3.62 - 3.67 (m, 1H), 3.05 (s, 6H), 0.93 (s, 9H), 0.11 (s, 6H).

**Step 4**

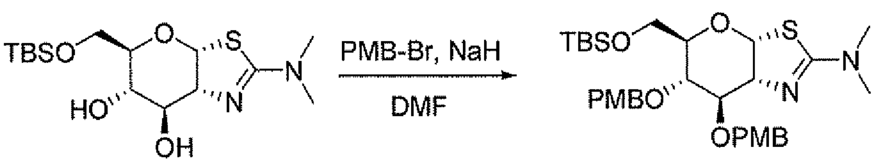


8 9

**(3aR,5R,6S,7R,7aR)-5-((tert-Butyldimethylsilyloxy)methyl)-6,7-bis(4-methoxybenzyloxy)-N,N-dimethyl-5,6,7,7a-tetrahydro-3aH-pyrano[3,2-d]thiazol-2-amine (9):** To a solution of (3aR,5R,6S,7R,7aR)-5-((tert-butyldimethylsilyloxy)methyl)-2-(dimethylamino)-5,6,7,7a-tetrahydro-3aH-pyrano[3,2-d]thiazole-6,7-diol (1 g, 2.76 mmol) in DMF (20 mL) was added sodium hydride (568 mg, 16.6 mmol, 70 %) at 15 °C, and followed by addition of 1-(bromomethyl)-4-methoxybenzene (2.22 g, 11.0 mmol). The resulting solution was stirred for 3 h at room temperature, quenched by addition of cold water (50 mL), and extracted with dichloromethane (3 x 50 mL). The combined organic layers were dried over anhydrous magnesium sulfate and concentrated to give a residue, which was purified by silica gel column, eluted with 10 -25 % ethyl acetate in petroleum ether to give compound **9** as a yellow oil (1.2 g, 64 %). (ES, *m/z*): $[M+H]^+$ 603.1; $^1H$ NMR (300 MHz, $CDCl_3$) $\delta$ 7.22 - 7.35 (m, 4H), 6.84 - 6.92 (m. 4H), 6.27 - 6.29 (d, *J* = 6.6 Hz, 1H), 4.60 - 4.76 (m, 4H),

4.36 - 4.43 (m, 2H), 4.10 - 4.17 (m, 2H), 3.81 (s, 6H), 3.72 (m, 1H), 3.61 (m, 1H), 2.99 (s, 6H), 0.83 (s, 9H), 0.07 (s, 6H).

**Step 5**

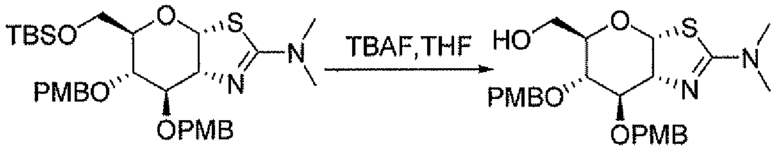


9 10

**((3aR,5R,6S,7R,7aR)-2-(Dimethylamino)-6,7-bis(4-methoxybenzyloxy)-5,6,7,7a-tetrahydro-3aH-pyrano[3,2-d]thiazol-5-yl)methanol (10):** (3aR,5R,6S,7R,7aR)-6,7-bis(4-methoxybenzyloxy)-5-((tert-butyldimethylsilyloxy)methyl)-N,N-dimethyl-5,6,7,7a-tetrahydro-3aH-pyrano[3,2-d]thiazol-2-amine (9.5 g, 15.8 mmol) in THF (100 mL) was treated with TBAF (8.27 g, 31. 6 mmol) overnight at room temperature. The resulting solution was diluted with brine (200 mL), extracted with ethyl acetate (2 x200 mL), and dried over anhydrous magnesium sulfate. After removal of solvents, the residue was purified by silica gel column, eluted with 1 -2.5 % MeOH in dichloromethane to give compound **10** as a yellow oil (7.0 g, 86 %). (ES, *m/z):* $[M+H]^+$ 489.0; $^1H$ NMR (300 MHz, $CDCl_3$) δ 7.32 - 7.62 (m, 2H), 7.22 - 7.28 (m, 2H), 6.85 - 6.91 (m, 4H), 6.26 - 6.28 (d, *J* = 6.6 Hz, 1 H), 4.52 - 4.73 (m, 4H), 4.31 - 4.34 (d, *J* = 11.4 Hz, 1H), 4.23 (s, 1H), 3.81 (s, 6H), 3.53 - 3.76 (m, 4H), 3.01 (m, 6H), 1.78 - 1.82 (m, 1H).

**Step 6**

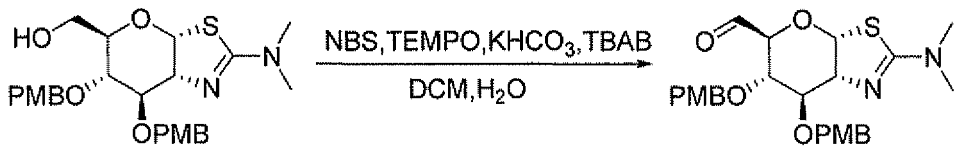


10 11

**(3aR,5S,6S,7R,7aR)-2-(Dimethylamino)-6,7-bis(4-methoxybenzyloxy)-5,6,7,7a-tetrahydro-3aH-pyrano[3,2-d]thiazole-5-carbaldehyde (11):** To a mixture of ((3aR,5R,6S,7R,7aR)-6,7-bis(4-methoxybenzyloxy)-2-(dimethylamino)-5,6,7,7a-tetrahydro-3aH-pyrano[3,2-d]thiazol-5-yl)methanol (500 mg, 1.0 mmol), TBAB (16.5 mg, 0.05 mmol), $KHCO_3$ (461 mg, 4.6 mmol) and TEMPO (8 mg, 0.05 mmol) in dichloromethane (25 mL) and $H_2O$ (5 mL) was added NBS (201 mg, 1.13 mmol) at 15 °C. After stirred for 30 mm, the reaction mixture was quenched by saturated $Na_2SO_3$ (5 mL). The organic layer was dried over anhydrous magnesium sulfate and condensed to provide a residue, which was purified by silica gel column, eluted with 20 -30 % ethyl acetate in dichloromethane to give compound **11** as a yellow syrup (320 mg, 75 % pure). (ES, *m/z*): $[M+H]^+$ 487.0. $^1H$ NMR (300 MHz, $CDCl_3$) δ 9.61 (s, 1H), 7.22 - 7.34 (m, 4H), 6.83 - 6.92 (m, 4H), 6.11 - 6.13 (d, *J* = 6.0 Hz, 1H), 4.17 - 4.67 (m, 8H), 3.83 (s, 6H), 3.00 - 3.04 (s, 6H).

**Step 7**

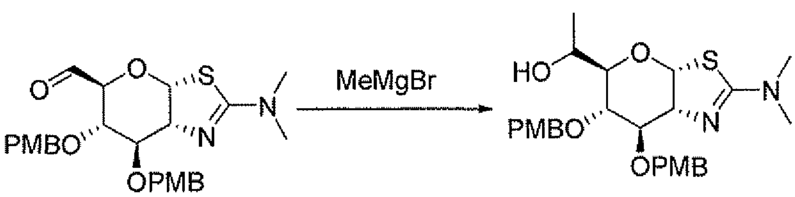


11 12

**1-((3aR,5R,6S,7R,7aR)-6,7-Bis(4-methoxybenzyloxy)-2-(dimethylamino)-5,6,7,7a-tetrahydro-3aH-pyrano[3,2-d]thiazol-5-yl)ethanol (12):** To a solution of (3aR,5S,6S,7R,7aR)-6,7-bis(4-methoxybenzyloxy)-2-(dimethylamino)-5,6,7,7a-tetrahydro-3aH-pyrano[3,2-d]thiazole-5-carbaldehyde (260 mg, 0.53 mmol) in THF (10 mL) was added methylmagnesium bromide (0.3 mL, 3M in THF). After stirred for 2 h at room temperature, the reaction mixture was quenched with sat.$NH_4Cl$ (aq, 20 mL), extracted with ethyl acetate (3 x 20 mL). The combined organic layers were dried over anhydrous magnesium sulfate, concentrated under vacuum to give a residue, which was purified by silica gel column, eluted with 1 -2 % MeOH in dichloromethane to give compound **12** as a yellow syrup (250 mg, 74 %, two diastereomers, faster moving one: slower moving one = 1 : 5). (ES, *m/z*): $[M+H]^+$ 503.0; $^1H$ NMR (300 MHz, $CDCl_3$) $\delta$ 7.26 - 7.35 (m, 2H), 7.22 - 7.28 (m, 2H), 6.85 - 6.91 (m, 4H), 6.29 - 6.31 (d, *J*= 6.9 Hz, 1H), 4.52 - 4.73 (m, 4H), 4.31 - 4.34 (d, *J* = 11.4 Hz, 1H), 4.23 (s, 1H), 3.81 (s, 6H), 3.53 - 3.76 (m, 4H), 3.01 (m, 6H), 1.19 - 1.21 (d , *J* = 6.6 Hz, 3H).

**Step 8**

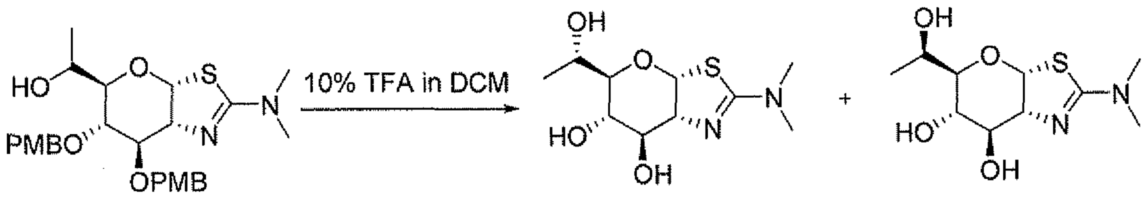


**12** **13** **13a**

Slower eluting isomer Faster eluting isomer

**(3aR,5R,6S,7R,7aR)-2-(Dimethylamino)-5-((R)-1-hydroxyethyl)-5,6,7,7a-tetrahydro-3aH-pyrano[3,2-d]thiazole-6,7-diol (13a,) and (3aR,5R,6S,7R,7aR)-2-(dimethylamino)-5-((S)-1-hydroxyethyl)-5,6,7,7a-tetrahydro-3aH-pyrano[3,2-d]thiazole-6,7-diol (13, Example 1):** A solution of 1-((3aR,5R,6S,7R,7aR)-6,7-bis(4-methoxybenzyloxy)-2-(dimethylamino)-5,6,7,7a-tetrahydro-3aH-pyrano[3,2-d]thiazol-5-yl)ethanol (315 mg, 0.63 mmol, a 1:5 mixture of two diastereomers) in dichloromethane (20 mL) was treated with TFA (2 mL) for 1 h at room temperature. The reaction mixture was concentrated under vacuum to give a residue, which was purified by Prep-HPLC under the following conditions [(3#-Agilent 1200 prep HPLC): Column, SunFire Prep C18,19*50mm 5um; mobile phase, WATER with 0.03 % $NH_4OH$ and $CH_3CN$(10 % $CH_3CN$ up to 45 % in 10 min); Detector, UV 220nm] to afford
the faster eluting isomer as a white solid (**13a**, 6.9 mg, 4.2 %): (ES, *m/z*): $[M+H]^+$ 262.9; $^1H$ NMR ( 300 MHz, $CDCl_3$) $\delta$ 6.21 - 6.23 (d, *J*= 6.6 Hz, 1H), 4.12 - 4.16 (m, 1H), 3.92 - 3.99 (m, 2H), 3.48 - 3.59 (m, 2H), 2.9 (s, 6H), 1.07 - 1.10 (d, *J* = 6.6 Hz, 3H);
and the slower eluting isomer as white solid (**13**, **Example 1**, 52.8 mg, **32** %): (ES, *m/z*): $[M+H]^+$ 262.9; $^1H$ NMR (slower eluting isomer, 300 MHz, $CDCl_3$) $\delta$ 6.18 - 6.21 (d, *J* = 6.6 Hz, 1H), 4.13 - 4.16 (m, 1H), 3.95 -3.98 (m, 1H), 3.84 - 3.88 (m, 1H), 3.58 - 3.63 (m, 1H), 3.21 - 3.26 (m, 1H), 2.89 (s, 6H), 1.11 - 1.13 (d, *J* = 6.6 Hz, 3H).

**Example 2**

**(3aR, 5R, 6S,7R,7aR)-5-((S)-1-hydroxyethyl)-2-(propylamino)-5,6,7,7a-tetrahydro-3aH-pyrano[3,2-d]thiazole-6,7-diol**

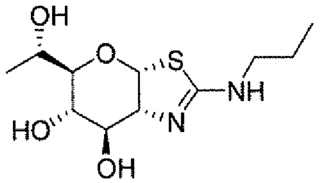

Scheme III

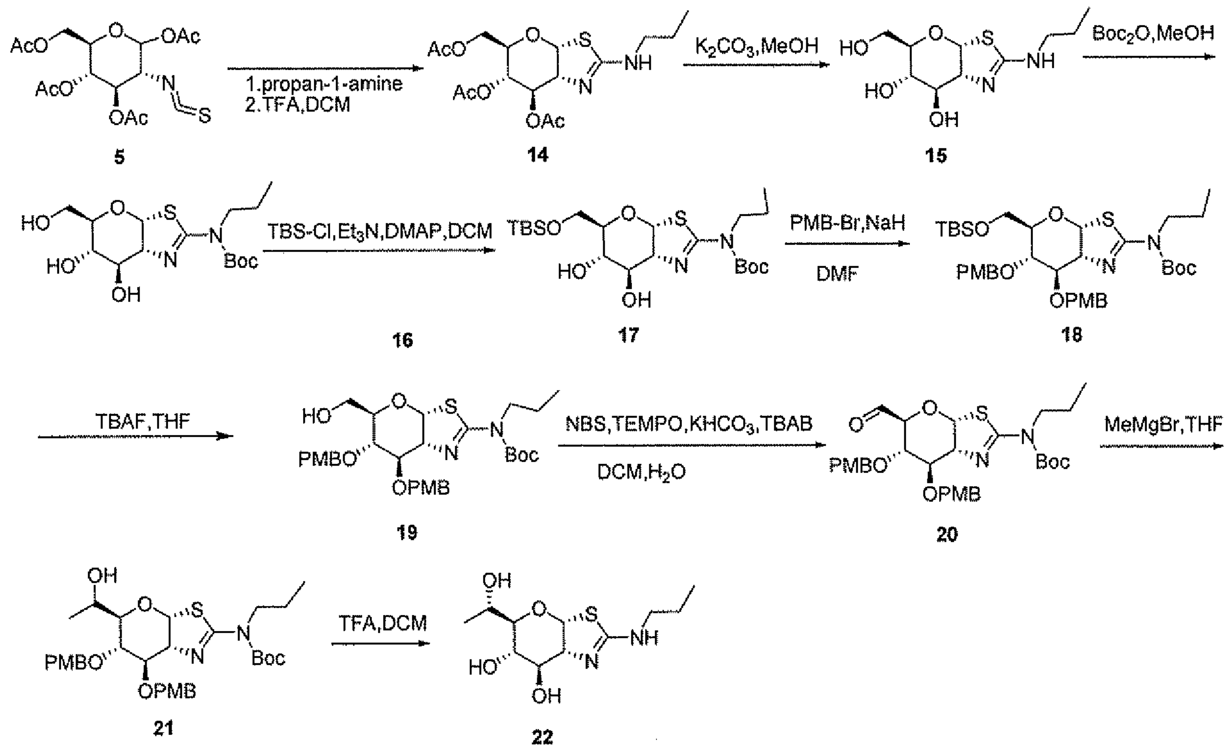


5 14 15 16 17 18 19 20 21 22

## Step 1

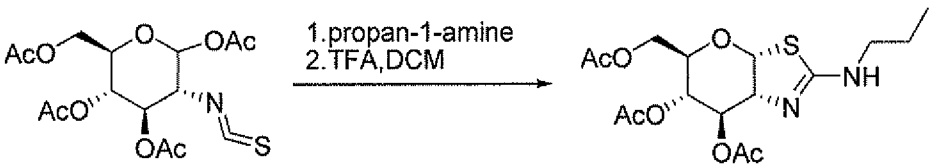


5 14

**(3aR,5R,6S,7R,7aR)-5-(Acetoxymethyl)-2-(propylamino)-5,6,7,7a-tetrahydro-3aH-pyrano[3,2-d]thiazole-6,7-diyl diacetate (14):** To a solution of (3R,4R,5S,6R)-6-(acetoxymethyl)-3-isothiocyanato-tetrahydro-2H-pyran-2,4,5-triyl triacetate (59 g, 151 mmol) in dichloromethane (500 mL) was added propan-1-amine (9.4 g, 159 mmol) dropwise with stirring at 0 °C. After stirred for 1 h, the reaction solution was treated with trifluoroacetic acid (130 g, 1.34 mol) overnight at room temperature. The reaction mixture was washed with saturated sodium bicarbonate (300 mL), dried over anhydrous magnesium sulfate, and concentrated under vacuum to provide a residue, which was purified by silica gel column, eluted with 1 % MeOH in dichloromethane to give compound **14** as a yellow oil (50 g, 84 %). (ES, *m/z*): $[M+H]^+$ 388.9; $^1H$ NMR (300 MHz, $CDCl_3$) $\delta$ 6.14 - 6.16 (d, *J*= 6.6 Hz, 1H), 5.33 - 5.35 (m, 1H), 5.05 - 5.19 (m, 1H), 4.85 -4.89 (m, 1H), 4.27 - 4.30 (m, 1H), 4.08 (s, 2H), 3.75 - 3.81 (m, 1H), 3.06 - 3.28 (m, 2H), 1.97 - 2.04 (m ,9H), 1.49 - 1.58 (m, 2H), 0.82 - 0.91 (m, 3H).

## Step 2

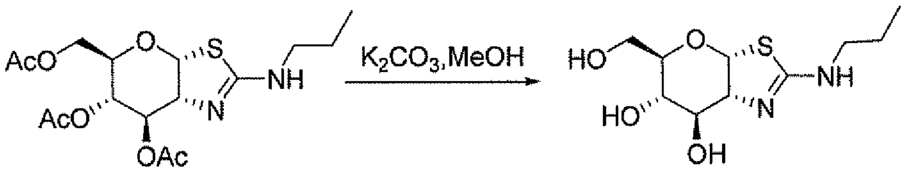


14 15

**(3aR,5R,6S,7R,7aR)-5-(Hydroxyrnethyl)-2-(prapylamino)-5,6,7,7a-tetrahydro-3aH-pyrano[3,2-d]thiazole-6,7-diol (15):** To a solution of (3aR,5R,6S,7R,7aR)-5-(acetoxymethyl)-2-(propylamino)-5,6,7,7a-tetrahydro-3aH-pyrano[3,2-d]thiazole-6,7-diyl diacetate (100 g, 257 mmol) in methanol (800 mL) was added potassium carbonate (18 g, 130 mmol). The resulting solution was stirred overnight at 40 °C and then cooled down to 0 °C to yield a solid This was collected by filtration, washed with cold methanol and dried. The product **15** was obtained as a white solid (50 g, 74 %). (ES, *m*/*z*): $[M+H]^+$ 262.9; $^1H$ NMR (300 MHz, D2O) δ 6.17 - 6.19 (d, *J* = 6.3 Hz, 1H), 4.07 - 4.10 (m, 1H), 3.93 - 3.96 (m, 1H), 3.71 - 3.76 (m, 1H), 3.45 - 3.69 (m, 3H), 3.04 - 3.13 (m, 2H), 1.39 - 1.51 (m, 2H), 0.82 - 0.91 (m, 3H).

**Step 3**

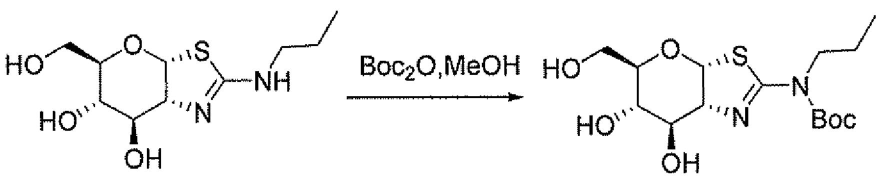


15 16

**tert-Butyl (3aR,5R,6S,7R,7aR)-6,7-dihydroxy-5-(hydroxymethyl)-5,6,7,7a-tetrahydro-3aH-pyrano[3,2-d]thiazol-2-yl(propyl)carbamate (16):** To a solution of (3aR,5R,6S,7R,7aR)-5-(hydroxymethyl)-2-(propylamino)-5,6,7,7a-tetrahydro-3aH-pyrano[3,2-d]thiazole-6,7-diol (67.6 g, 258 mmol) in methanol (800 mL) was added $(Boc)_2O$ (83 g, 384 mmol). After stirred for 3 h at room temperature, the resulting mixture was concentrated under vacuum to provide a residue, which was purified by silica gel column, eluted with 1 % MeOH in dichloromethane to give compound **16** as yellow oil (53 g, 46 %). (ES, *m*/*z*): $[M+H]^+$ 363.0. $^1H$ NMR (300 MHz, $CDCl_3$) δ 6.11 - 6.14 (d, *J* = 6.6 Hz, 1H), 4.16 - 4.26 (m, 2H), 3.75 - 3.96 (m, 6H), 3.58 - 3.63 (m, 1H), 1.50 - 1.57 (m, 12H), 0.89 - 0.94 (m, 3H).

**Step 4**

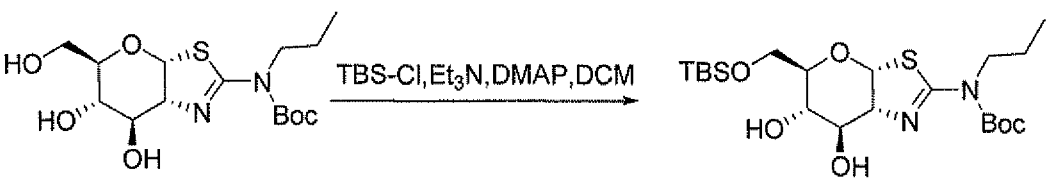


16 17

**tert-Butyl (3aR,5R,6S,7R,7aR)-5-((tert-butyldimethylsilyloxy)methyl)-6,7-dihydroxy-5,6,7,7a-tetrahydro-3aH-pyrano[3,2-d]thiazol-2-yl(propyl)carbamate (17):** To a stirred solution of tert-butyl (3aR,5R,6S,7R,7aR)-6,7-dihydroxy-5-(hydroxymethyl)-5,6,7,7a-tetrahydro-3aH-pyrano[3,2-d]thiazol-2-yl(propyl)carbamate (45 g, 124 mmol), DMAP (760 mg, 6 mmol) and triethylamine (25.1 g, 248 mmol) in dichloromethane (300 mL) was added tert-butylchlorodimethylsilane (22.4 g, 149 mmol). After stirred for 6 h at room temperature, the mixture was condensed to provide a residue, which was purified by silica gel column, eluted with 10 -30 % ethyl acetate in petroleum ether to give compound 17 as a yellow oil (43 g. 65 %). (ES, *m*/*z*): $[M+H]^+$ 477.1; $^1H$ NMR (300 MHz, $CDCl_3$) δ 6.12 - 6.14 (d, *J* = 6.3 Hz, 1H), 4.40 - 4.45 (m, 1 H), 3.84 - 3.96 (m, 4H), 3.64 - 3.71 (m, 1 H), 3.13 - 3.30 (m, 3H), 1.67 - 1.74 (m, 2H), 1.53 (s, 9H), 0.93 - 0.98

(m, 3H), 0.92 (s ,9H), 0.12 (s, 6H).

**Step 5**

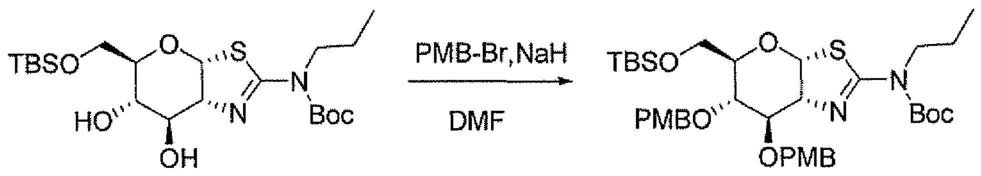


**17** **18**

**tert-Butyl (3aR,5R,6S,7R,7aR)-5-((tert-butyldimethylsilyloxy)methyl)-6,7-bis(4-methoxybenzyloxy)-5,6,7,7a-tetrahydro-3aH-pyrano[3,2-d]thiazol-2-yl(propyl)carbamate (18):** To a solution of tert-butyl (3aR,5R,6S,7R,7aR)-5-((tert-butyldimethylsilyloxy)methyl)-6,7-dihydroxy-5,6,7,7a-tetrahydro-3aH-pyrano[3,2-d]thiazol-2-yl(propyl)carbamate (20 g, 42 mmol) in DMF (300 mL) was added sodium hydride (10 g, 417 mmol) at 15 °C in portions, followed by addition of 1-(bromomethyl)-4-methoxybenzene (33.8 g, 168 mmol). The resulting solution was stirred for 1.5 h at room temperature, quenched by addition of cold water (100 mL), and extracted with dichloromethane (5x100 mL). The combined organic layers were dried over anhydrous magnesium sulfate and concentrated under vacuum to provide a residue, which was purified by silica gel column, eluted with 10 -25 % ethyl acetate in petroleum ether to give compound **18** as a yellow oil (20 g, 64.8%). (ES, *m/z*): $[M+H]^+$ 717.1; $^1$H NMR (300 MHz, $CDCl_3$) δ 7.32 - 7.35 (d, *J*= 8.7 Hz, 2H), 7.19 - 7.22 (d, *J*= 8.7 Hz, 2H), 6.83 - 6.92 (m. 4H), 6.05 - 6.07 (d, *J* = 6.9 Hz, 1H), 4.57 - 4.74 (m, 3H), 4.30 - 4.39 (m, 2H), 4.14 - 4.17 (t, *J* = 4.2 Hz ,1H), 3.68 - 3.84 (m, 11 H), 3.41 - 3.45 (m, 1H), 1.67 - 1.74 (m , 2H), 1.53 (s, 9H), 0.89 (s, 9H), 0.84 (m, 3H), 0.08 (s, 6H).

**Step 6**

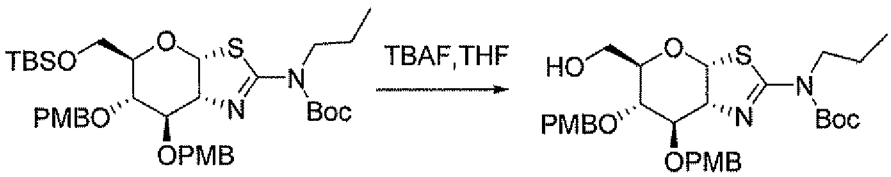


**18** **19**

**tert-Butyl (3aR,5R,6S,7R,7aR)-5-(hydroxymethyl)-6,7-bis(4-methoxybenzyloxy)-5,6,7,7a-tetrahydro-3aH-pyrano[3,2-d]thiazol-2-yl(propyl)carbamate (19):** To a solution of tert-butyl (5R,6S,7R)-6,7-bis(4-methoxybenzyloxy)-5-((tert-butyldimethylsilyloxy)methyl)-5,6,7,7a-tetrahydro-3aH-pyrano[3,2-d]thiazol-2-yl(propyl)carbamate (19 g, 26.50 mmol) in tetrahydrofuran (100 mL) was treated with TBAF (13.8 g, 53 mmol) overnight at room temperature. The resulting solution was diluted with brine (200 mL), extracted with ethyl acetate (2 x200 mL), and dried over anhydrous magnesium sulfate. After removal of solvents, the residue was purified by silica gel column, eluted with 10-30 % ethyl acetate in petroleum ether to give compound **19** as a yellow oil (14.5 g, 91 %). (ES, *m/z*): $[M+H]^+$ 603.0; $^1$H NMR (300 MHz, $CDCl_3$) δ 7.32 - 7.35 (d, *J* = 8.7 Hz, 2H), 7.17 - 7.20 (d, *J* = 8.7 Hz, 2H), 6.83 - 6.92 (m. 4H), 6.04 - 6.06 (d, *J* = 6.9 Hz, 1H), 4.64 - 4.66 (m, 2H), 4.40 - 4.48 (m, 1H), 4.17 - 4.38 (m, 3H), 3.72 - 3.83 (m, 9H), 3.45 - 3.60 (m, 2H), 3.43 -. 3.45 (m, 1H), 1.58 - 1.63 (m, 2H), 1.53 (s, 9H), 0.78 - 0.83 (t, *J*= 7.2 Hz ,3H).

**Step 7**

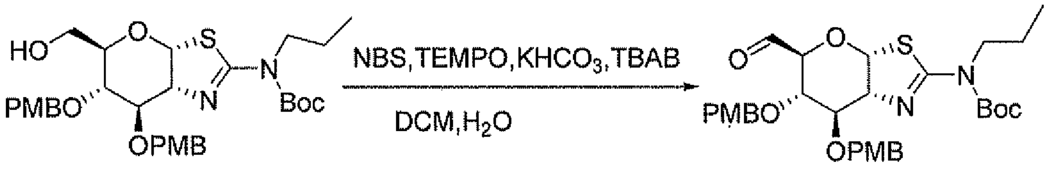


19 20

**tert-Butyl (3aR,5S,6S,7R,7aR)-5-formyl-6,7-bis(4-methoxybenzyloxy)-5,6,7,7a-tetrahydro-3aH-pyrano[3,2-d]thiazol-2-yl(propyl)carbamate (20) :** To a mixture of tert-butyl (5R,6S,7R)-6,7-bis(4-methoxybenzyloxy)-5-(hydroxymethyl)-5,6,7,7a-tetrahydro-3aH-pyrano[3,2-d]thiazol-2-yl(propyl) carbamate (2 g, 3.32 mmol), $KHCO_3$ (1.49bg, 15 mmol), TBAB (53 mg, 0.16 mmol) and TEMPO (26 mg, 0.17 mmol) in dichloromethane (60 mL) and $H_2O$ (12 mL) was added NBS (592 mg, 3.33 mmol) at 15 °C. After stirred for 30 min, the reaction mixture was quenched by saturated $Na_2SO_3$ (10 mL). The organic layer was dried over anhydrous magnesium sulfate and condensed to provide a residue, which was purified by silica gel column, eluted with 20 -30 % AcOEt in dichloromethane to give compound **20** as a yellow syrup (1.2 g, purity 60 %). (ES, *m/z*): [M+H] + 601.1. $^1H$ NMR (300 MHz, $CDCl_3$) δ 9.61 (s, 1H), 7.23 - 7.28 (m, 4H), 6.82 - 6.86 (m, 4H), 6.19 - 6.21 (d, *J* = 6.9 Hz, 1H), 4.60 - 4.67 (m, 2H), 4.41 - 4.55 (m, 3H), 4.02 - 4.03 (m, 1H), 3.88 - 3.93 (m, 1H), 3.68 - 3.81 (m, 9H), 1.46 - 1.53 (m, 11H), 0.83 - 0.86 (t, *J* = 7.5 Hz, 3H).

**Step 8**

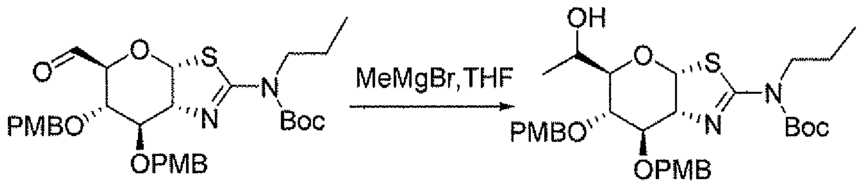


20 21

**tert-Butyl (3aR,5R,6S,7R,7aR)-1-hydroxyethyl)-6,7-bis(4-methoxybenzyloxy)-5,6,7,7a tetrahyd ro-3aH-pyrano[3,2-d]thiazol-2-yl(propyl)carbamate (21):** To a solution of tert-butyl (3aR,5S,6S,7R,7aR)-6,7-bis(4-methoxybenzyloxy)-5-formyl-5,6,7,7a-tetrahydro-3aH-pyrano[3,2-d]thiazol-2-yl(propyl)carbamate (600 mg, 1.00 mmol, 60 % purity) in tetrahydrofuran (20 mL) was added methylmagnesium bromide (1 mL, 3M in THF, 3 mmol). After stirred for 10 min at r.t, the reaction was quenched with sat.$NH_4Cl$ (aq, 20 mL), extracted with dichloromethane (4x10 mL). The combined organic layers were dried over anhydrous magnesium sulfate, and concentrated under vacuum to provide a residue, which was purified by silica gel column, eluted with 30 % AcOEt in dichloromethane to give compound **21** as a yellow syrup (300 mg, 81 %, two diastereomers, faster moving one: slower moving one = 1 : 3). (ES, *m/z*): $[M+H]^+$ 617.0; $^1H$ NMR (300 MHz, $CDCl_3$) δ 7.16 - 7.72 (m, 5H), 6.83 - 6.93 (m, 4H), 6.05 - 6.08 (m, 1H), 4.67 (s, 2H), 4.30 - 4.44 (m, 2H), 4.27 - 4.35 (m, 2H), 3.67 - 3.95 (m, 10H), 3.20 - 3.31 (m, 1H), 1.71 - 1.76 (m, 1H), 1.53-1.55 (m, 14H), 0.78 - 0.83 (m, 3H).

**Step 9**

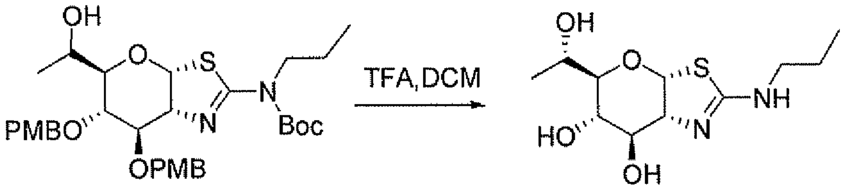


21 22

**(3aR, 5R, 6S,7R,7aR)-5-((S)-1-Hydroxyethyl)-2-(propylamino)-5,6,7,7a-tetrahydro-3aH-pyrano[3,2-d]thiazole-6,7-diol (22):** A solution of tert-butyl (3aR,5R,6S,7R,7aR)-6,7-bis(4-methoxybenzyloxy)-1-hydroxyethyl)-5,6,7,7a-tetrahydro-3aH-pyrano[3,2-d]thiazol-2-yl(propyl)carbamate (300 mg, 0.49 mmol, a mixture of two diastereomers, faster moving one: slower moving one = 1 : 3) in 20 mL dichloromethane was treated with 2 mL TFA. After stirred overnight at room temperature, the resulting mixture was concentrated under vacuum to give a crude product, which was purified by Prep-HPLC with the following conditions [(Agilent 1200 detecl), Column, 19*150mm; mobile phase, WATER WITH 0.03% $NH_4OH$ and $CH_3CN$ (10% $CH_3CN$ up to 45 % in 10 min); Detector, 254nm 220nm] to afford compound **22 (Example 2)** as white solid (TFA salt, 143.1 mg, 75 %). (ES, *m/z*): $[M+H]^+$ 277.0; $^1H$ NMR (300MHz, $D_2O$): δ: 6.49 - 6.45 (m, 1H), 4.14 - 4.69 (m, 1H), 3.86 - 3.99 (m, 2H), 3.59 - 3.66 (m, 1H), 3.32 - 3.36 (m, 1H), 3.24 - 3.30 (m, 2H), 1.47 - 1.59 (m, 3H), 1.10 - 1.13 (m, 3H).

**Example 3**

**(3aR,5S,6S,7R,7aR)-5-(2-Hydroxypropan-2-yl)-2-(methylamino)-5,6,7,7a-tetrahydro-3aH-pyrano[3,2-d]thiazole-6,7-diol**

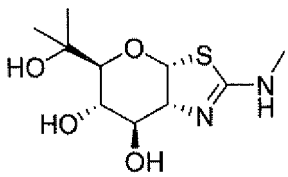

**Scheme IV**

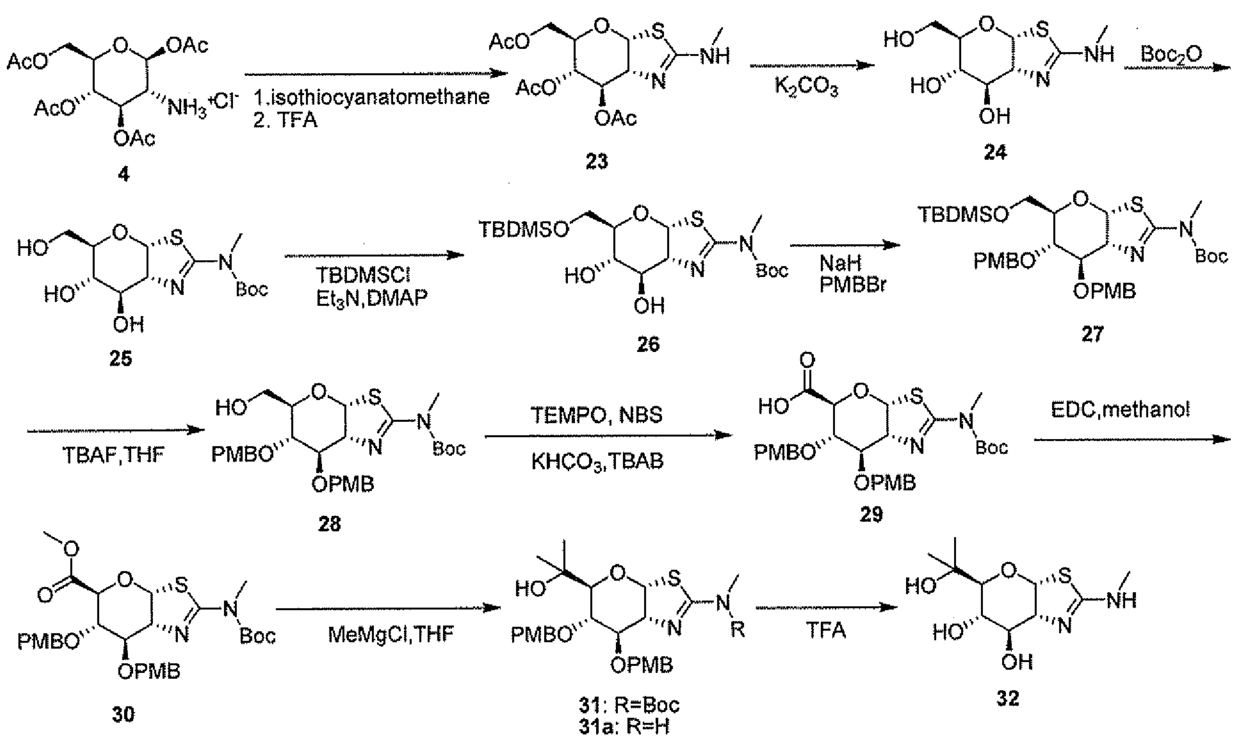

4
23
24
25
26
27
28
29
30
31: R=Boc
31a: R=H
32

**Step 1**

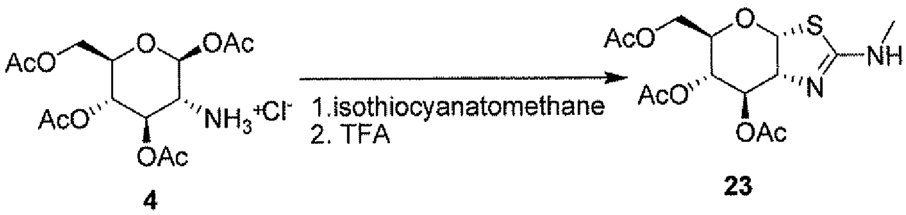


4

23

**(3aR,5R,6S,7R,7aR)-5-(Acetoxymethyl)-2-(methylamino)-5,6,7,7a-tetrahydro-3aH-pyrano[3,2-d]thiazole-6,7-diyl diacetate (23):** To a solution of (3R,4R,5S,6R)-2,4,5-triacetoxy-6-(acetoxymethyl)-tetrahydro-2H-pyran-3-aminium chloride (100 g, 261 mmol) in $CH_3CN$ (1 L) was added methyl isothiocyanate (21 g, 287 mmol), triethylamine (29 g, 287 mmol). After stirred for 12 h at 60 °C, the resulting solution was treated with TFA (110 g, 0.96 mol) at room temperature overnight, and then washed with saturated sodium bicarbonate (1 L). The organic layer was dried over magnesium sulfate and concentrated under vacuum to provide a residue, which was purified by silica gel column, eluted with 1 % MeOH in dichloromethane to give compound **23** as a yellow oil (150 g, 87 %). (ES, *m/z*): $[M+H]^+$ 360.9; $^1$H NMR (300MHz, $CDCl_3$) δ 6.31 - 6.33 (d, *J*= 6.6 Hz, 1H), 5.41 - 5.48 (t, *J* = 8.4 Hz, 1H), 4.97 - 5.00 (m, 1H), 4.31 - 4.37 (t, *J* = 5.7 Hz, 1H), 4.21 (m, 2H), 3.97 (m, 1H), 2.99 (s, 3H), 2.00 - 2.20 (m, 9H).

**Step 2**

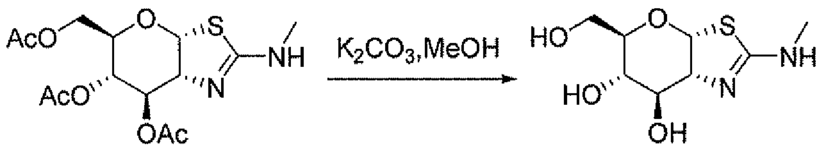


23

24

**(3aR,5R,6S,7R,7aR)-5-(Hydroxymethyl)-2-(methylamino)-5,6,7,7a-tetrahydro-3aH-pyrano[3,2-d]thiazole-6,7-diol(24):** A solution of (3aR,5R,6S,7R,7aR)-5-(acetoxymethyl)-2-(methylamino)-5,6,7,7a-tetrahydro-3aH-pyrano[3,2-d]thiazole-6,7-diyl diacetate (150 g, 417 mmol) in methanol (1 L) was treated with potassium carbonate (11.5 g, 83 mmol). The resulting mixture was stirred overnight at room temperature to yield a solid. This was collected by filtration, washed with cold methanol and dried. The product **24** was obtained as a yellow solid (85 g, 87 %). (ES, *m/z*): $[M+H]^+$ 235.1; $^1$H NMR (300MHz, $D_2O$) δ 6.14 - 6.16 (d, *J* = 6.3 Hz, 1H), 4.03 - 4.07 (m, 1H), 3.89 - 3.92 (m, 1H), 3.65 - 3.70 (m, 1H), 3.48 - 3.56 (m, 2H), 3.41 - 3.45 (m, 1H), 2.69 (s, 3H).

**Step 3**

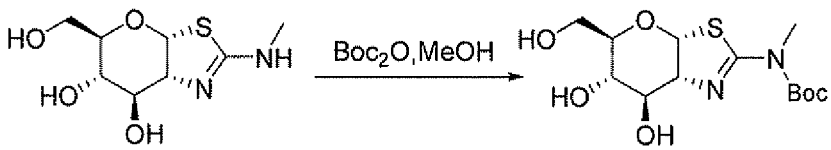


24

25

**tert-Butyl (3aR,5R,6S,7R,7aR)-6,7-dihydroxy-5-(hydroxymethyl)-5,6,7,7a-tetrahydro-3aH-pyrano[3,2-d]thiazol-2-yl(methyl)carbamate (25):** A solution of (3aR,5R,6S,7R,7aR)-5-(hydroxymethyl)-2-(methylamino)-5,6,7,7a-tetrahydro-3aH-pyrano[3,2-d]thiazole-6,7-diol (85 g, 363 mmol) in methanol (600 mL) was treated with $Boc_2O$ (117.7 g, 540 mmol) and triethylamine (73.3 g, 726 mmol). The resulting solution was stirred overnight at 45 °C, and then concentrated under vacuum to give a residue, which was purified by silica gel column, eluted with 2.5 % methanol in dichloromethane to give compound **25** as a yellow solid (90 g, 74 %). (ES, *m/z*): $[M+H]^+$ 334.8; $^1$H NMR (300MHz, $CDCl_3$) δ 6.14 - 6.17 (d, *J* = 6.9 Hz, 1H), 4.19 - 4.23 (t, *J* = 6.3 Hz, 1H), 4.10 - 4.14 (t, *J* = 5.4 Hz, 1H), 3.79 - 3.84 (m, 3H), 3.60 - 3.64 (m, 2H), 3.14 (s, 3H), 1.55 (s, 9H).

Step 4

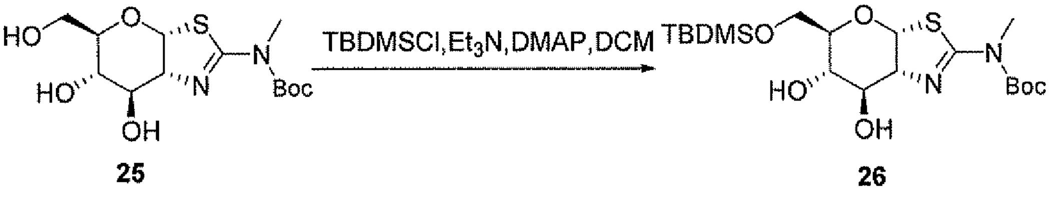


**tert-Butyl (3aR,5R,6S,7R,7aR)-5-((tert-butyldimethylsilyloxy)methyl)-6,7-dihydroxy-5,6,7,7a-tetrahydro-3aH-pyrano[3,2-d]thiazol-2-yl(methyl)carbamate (26):** To a mixture of tert-butyl (3aR,5R,6S,7R,7aR)-6,7-dihydroxy-5-(hydroxymethyl)-5,6,7,7a-tetrahydro-3aH-pyrano[3,2-d]thiazol-2-yl(methyl)carbamate (30 g, 90 mmol), DMAP (550 mg, 4.5 mmol) and triethylamine (18.2 g, 180 mmol) in dichloromethane (200 mL) was added tert-butylchlorodimethylsilane (16.3 g, 108 mmol) at 0 °C. The resulting solution was stirred for 4 h at room temperature, and then concentrated under vacuum to provide a residue, which was purified by silica gel column, eluted with 1 % methanol in dichloromethane to give compound **26** as a white solid (20 g, 50 %). (ES, *m/z*): $[M+H]^+$ 449.0; $^1H$ NMR (300MHz, $CDCl_3$) $\delta$ 6.10 - 6.12 (d, $J$ = 6.9 Hz, 1H), 4.15 - 4.24 (m, 2H), 3.83 - 3.97 (m, 5H), 3.60 (m, 1H), 2.58 - 2.66 (m, 2H), 1.55 (s, 9H), 1.18 (m, 3H), 0.91 (s, 9H), 0.09 (s, 6H).

Step 5

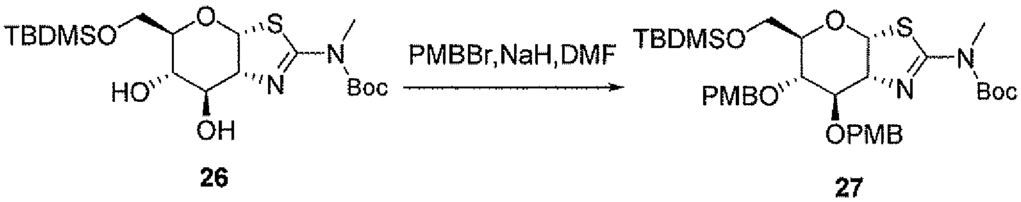


**tert-Butyl (3aR,5R,6S,7R,7aR)-5-((tert-butyldimethylsilyloxy)methyl)-6,7-bis(4-methoxybenzyloxy)-5,6,7,7a-tetrahydro-3aH-pyrano[3,2-d]thiazol-2-yl(methyl)carbamate (27):** To a solution of (3aR,5R,6S,7R,7aR)-5-((tert-butyldimethylsilyloxy)methyl)-6,7-dihydroxy-5,6,7,7a-tetrahydro-3aH-pyrano[3,2-d]thiazol-2-yl(methyl)carbamate (20 g, 45 mmol) in DMF (150 mL) was added sodium hydride (10.7 g, 446 mmol) in portion at 0 °C, and followed by addition of 1-(bromomethyl)-4-methoxybenzene (36 g, 179 mmol). The resulting solution was stirred for 2 h at room temperature, quenched with cold water (200 mL), and extracted with ethyl acetate (3 x 500 mL). The combined organic layers were washed with brine (5 x 200 mL), dried over anhydrous magnesium sulfate, and concentrated under vacuum to afford a residue, which was purified by silica gel column, eluted with 10 % ethyl acetate in petroleum ether to give compound **27** as a yellow liquid (21 g, 68 %). (ES, *m/z*): $[M+H]^+$ 689.1. $^1H$ NMR (300MHz, $CDCl_3$) $\delta$ 7.31 - 7.37 (m, 2H), 7.22 - 7.28 (m, 2H), 6.10 - 6.12 (d, $J$ = 6.9 Hz, 1H), 4.60 - 4.74 (m, 4H), 4.20 - 4.47 (m, 4H), 3.82 (s, 6H), 3.69 (s, 3H), 3.33 - 3.37 (m, 2H), 1.56 (s, 9H), 0.89 (s, 9H), 0.05 (s , 6H).

Step 6

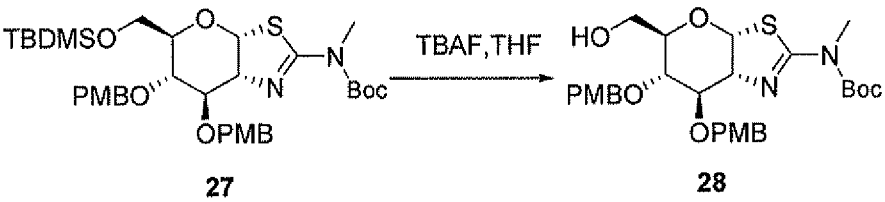

**tert-Butyl (3aR,5R,6S,7R,7aR)-5-(hydroxymethyl)-6,7-bis(4-methoxybenzyloxy)-5,6,7,7a-tetrahydro-3aH-pyrano[3,2-d]thiazol-2-yl(methyl)carbamate (28):** A solution of (3aR,5R,6S,7R,7aR)-5-((tert-butyldimethylsilyloxy)methyl)-6,7-bis(4-methoxybenzyloxy)-5,6,7,7a-tetrahydro-3aH-pyrano[3,2-d]thiazol-2-yl(methyl)carbamate (1.54 g, 2.24 mmol) in THF (30 mL) was treated with TBAF (1.17 g, 4.5 mmol) overnight at room temperature. The resulting solution was diluted with brine (20 mL), extracted with ethyl acetate (2 x 50 mL), and dried over anhydrous magnesium sulfate. After removal of solvents, the residue was purified by silica gel column, eluted with 20 % ethyl acetate in petroleum ether to give compound **28** as a yellow solid (0.8 g, 62 %). (ES, *m/z*): $[M+H]^+$ 575.2, $^1H$ NMR (300MHz, $CDCl_3$) δ 7.33 - 7.36 (d, *J* = 7.8 Hz, 2H), 7.20 - 7.22 (d, *J* = 8.1 Hz, 2H), 6.08 - 6.10 (d, *J* = 6.3 Hz, 1H), 4.27 - 4.71 (m, 6H), 3.82 (s, 6H), 3.48 - 3.70 (m, 4H), 3.33 (s, 3H), 1.76 - 1.80 (t, *J*= 7.2 Hz, 1H), 1.54 (s, 9H).

**Step 7**

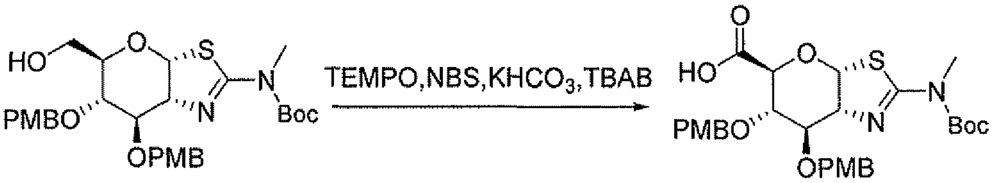


28 29

**(3aR,5S,6S,7R,7aR)-2-(tert-Butoxycarbonyl(methyl)amino)-6,7-bis(4-methoxybenzyloxy)-5,6,7,7a-tetrahydro-3aH-pyrano[3,2-d]thiazole-5-carboxylic acid (29):** A mixture of tert-butyl (3aR,5R,6S,7R,7aR)-5-(hydroxymethyl)-6,7-bis(4-methoxybenzyloxy)-5,6,7,7a-tetrahydro-3aH-pyrano[3,2-d]thiazol-2-yl(methyl)carbamate (1 g, 1.74 mmol), $KHCO_3$ (780 mg, 7.8 mmol), TBAB (28 mg, 0.09 mmol) and TEMPO (14 mg, 0.09 mmol) in dichloromethane(30 mL) and $H_2O$ (6 mL) was treated with NBS (620 mg, 3.5 mmol) overnight at room temperature. The reaction mixture was adjusted to acidic (pH at 3) with hydrochloric acid, and then extracted with dichloromethane(3 x 20 mL). The combined organic layers were dried over anhydrous sodium sulfate and concentrated under vacuum to provide a residue, which was purified by a silica gel column, eluted with 20~50 % ethyl acetate in petroleum ether to give compound **29** as a white solid (600 mg, 58 %). (ES, *m/z*): $[M+H]^+$ 589.0, $^1H$ NMR (300MHz, $CDCl_3$) δ 7.25 - 7.32 (m, 2H), 6.85 - 6.90 (m, 2H), 6.08 - 6.10 (d, *J* = 6.3 Hz, 1H), 4.50 - 4.62 (m, 5H), 4.22 - 4.29 (m, 2H), 3.97 (m, 1H), 3.81(s, 6H), 3.34 (s, 3H), 1.54 (s, 9H).

**Step 8**

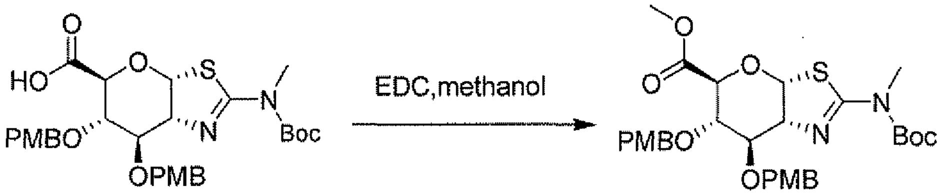


29 30

**(3aR,5S,6S,7R,7aR)-Methyl 6,7-bis(4-methoxybenzyloxy)-2-(tert-butoxycarbonyl)-5,6,7,7a-tetrahydro-3aH-pyrano[3,2-d]thiazole-5-carboxylate (30):** A solution of (3aR,5S,6S,7R,7aR)-2-(tert-butoxycarbonyl(methyl)amino)-6,7-bis(4-methoxybenzyloxy)-5,6,7,7a-tetrahydro-3aH-pyrano[3,2-d]thiazole-5-carboxylic acid (600 mg, 1.02 mmol) in methanol (30 mL) was treated with EDC (392 mg, 2.04 mmol) for 2 h at room temperature. After removal of solvents, the residue was purified by a silica gel column, eluted with 20 % ethyl acetate in petroleum ether to give compound **30** (510 mg, 83 %). (ES, *m/z*): $[M+H]^+$ 603.0, $^1H$ NMR (300MHz, $CDCl_3$) δ 7.22 - 7.29 (m, 4H), 6.83 - 6.91 (m, 4H), 6.07 - 6.09 (d, *J* = 5.7 Hz, 1 H), 4.48 - 4.61 (m, 4H), 4.30 - 4.31 (m, 1H), 4.22 - 4.29 (m, 2H), 4.17-4.19 (t, *J* = 4.8 Hz,1H), 3.82 (s, 6H), 3.67 (s, 3H), 3.30 (s, 3H), 1.54 (s, 9H).

**Step 9**

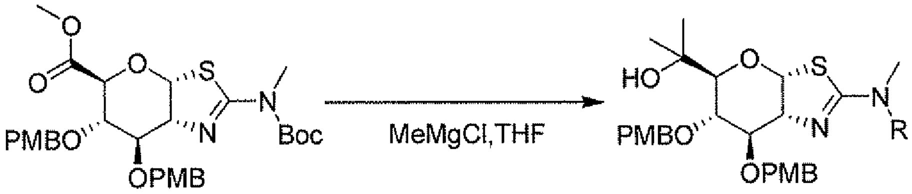


30

31: R=Boc
31a: R=H

**tert-Butyl (3aR,5S,6S,7R,7aR)-6,7-bis(4-methoxybenzyloxy)-5-(2-hydroxypropan-2-yl)-5,6,7,7a-tetrahydro-3aH-pyrano[3,2-d]thiazol-2-yl(methyl)carbamate (31) and 2-((3aR,5S,6S,7R,7aR)-6,7-bis(4-methoxybenzyloxy)-2-(methylamino)-5,6,7,7a-tetrahydro-3aH-pyrano[3,2-d]thiazol-5-yl)propan-2-ol (31a):** Compound **30** (510 mg) in THF (20 mL) was treated with methylmagnesium chloride (1 mL, 3M in THF) for 2 h at room temperature. The reaction was quenched with brine (20 mL), and extracted with dichloromethane (3 x 20 mL). The combined organic layers were dried over anhydrous magnesium sulfate and concentrated under vacuum to provide a residue, which was purified by a silica gel column, eluted with 20 % ethyl acetate in petroleum ether to give compound **31** as a yellow oil (308 mg, 57 %). (ES, *m/z*): $[M+H]^+$ 603.3; $^1$H NMR (300MHz, $CDCl_3$) $\delta$ 7.34 - 7.37 (d, *J* = 8.4 Hz, 2H), 7.19 - 7.22 (d, *J* = 8.4 Hz, 2H), 6.90 - 6.93 (dd, *J* = 2.1 Hz, 1.8 Hz, 2H), 6.83 - 6.86 (dd, *J* = 2.1 Hz, 1.8 Hz, 2H), 6.15 - 6.18 (d, *J* = 7.2 Hz, 1H), 4.68 (s, 2H), 4.35 - 4.48 (m,3H),4.23 - 4.26 (d, *J* = 4.5 Hz,1H), 3.86 (s, 6H), 3.26 (s, 3H), 3.21 (d, *J* = 8.1 Hz, 1H), 1.54 (s, 9H); and then eluted with 1% MeOH in dichloromethane to give **31a** as a white solid (152 mg, 31 %). (ES, *m/z*): $[M+H]^+$ 503.3; $^1$H NMR (300MHz, $CDCl_3$) $\delta$ 7.28 - 7.34 (m, 4H), 6.85 - 6.92 (m, 4H), 6.34 - 6.37 (d, *J* = 7.2 Hz, 1H), 4.49 - 4.68 (m, 5H), 4.25 - 4.29 (m, 2H), 3.76 - 3.86 (m, 8H), 3.34 - 3.37 (m, 1H), 3.90 (s, 3H).

**Step 10**

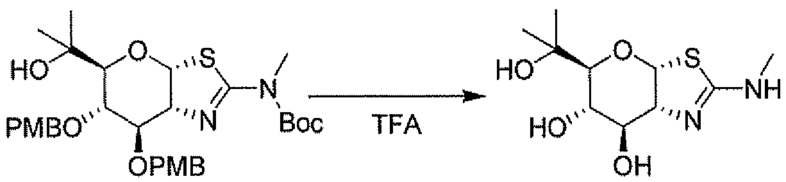


31

32

**(3aR,5S,6S,7R,7aR)-5-(2-Hydroxypropan-2-yl)-2-(methylamino)-5,6,7,7a-tetrahydro-3aH-pyrano[3,2-d]thiazole-6,7-diol (32):** A solution of tert-butyl (3aR,5S,6S,7R,7aR)-5-(2-hydroxypropan-2-yl)-6,7-bis(4-methoxybenzyloxy)-5,6,7,7a-tetrahydro-3aH-pyrano[3,2-d]thiazol-2-yl(methyl)carbamate (200 mg, 0.33 mmol) in DCM (20 mL) was treated with trifluoroacetic acid (2 mL) overnight at room temperature. The reaction mixture was condensed to give a residue, which was purified by Prep-HPLC with the following conditions [(3#-Agilent 1200 detect prep HPLC): Column, C18, 19*150mm, 5um; mobile phase, water with 0.03% ammonia and $CH_3CN$ (10 % $CH_3CN$ up to 45 % in 10 min); Detector, UV220nm] to give compound **32 (Example 3)** as a white solid (67.9 mg, 78 %). (ES, *m/z*): $[M+H]^+$ 263.1; $^1$H NMR (300 MHz, $D_2O$) $\delta$ 6.20 - 6.22 (d, *J* = 6.3 Hz, 1H), 4.31 (m, 1H), 4.28 - 4.30 (m, 1H), 3.68 - 3.71 (m, 1H), 3.14 - 3.17 (d, 2H), 2.71 (s, 3H), 1.10 (s, 3H), 1.07 (s, 3H).

**Example 4**

**(3aR,5S,6S,7R,7aR)-2-(dimethylamino)-5-(2-hydroxypropan-2-yl)-5,6,7,7a-tetrahydro-3aH-pyrano[3,2-d]thiazole-6,7-diol**

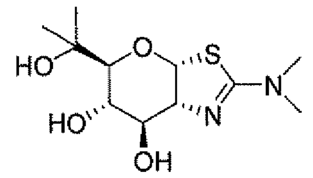

**Scheme V**

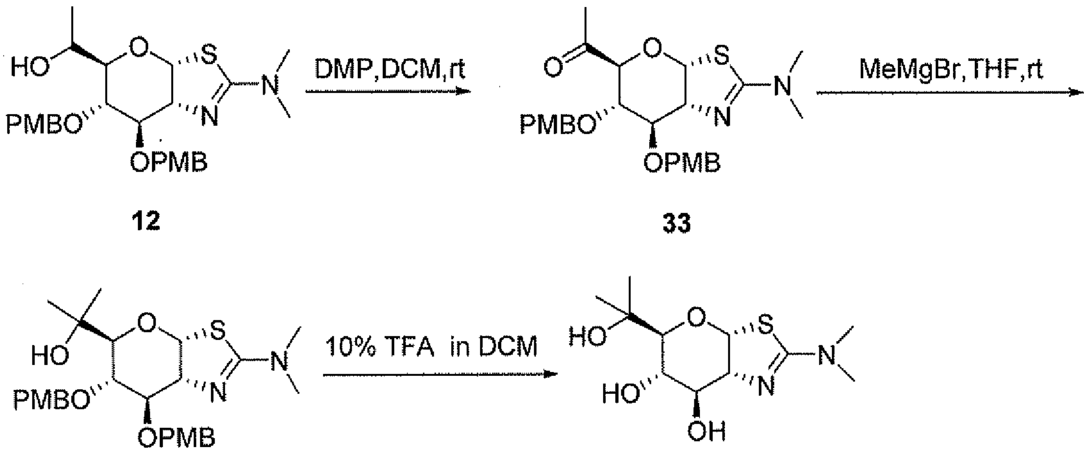


**12** **33**

**34** **35**

**Step 1**

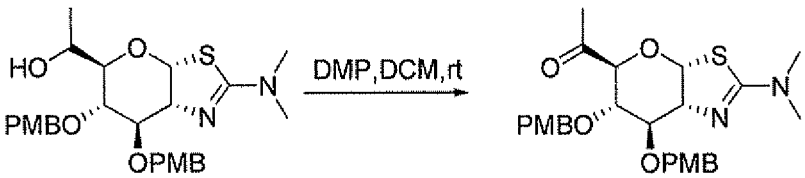


**12** **33**

**1-((3aR,5S,6S,7R,7aR)-2-(dimethylamino)-6,7-bis(4-methoxybenzyloxy)-5,6,7,7a-tetrahydro-3aH-pyrano[3,2-d]thiazol-5-yl)ethanone (33):** A solution of 1-((3aR,5R,6S,7R,7aR)-6,7-bis(4-methoxybenzyloxy)-2-(dimethylamino)-5,6,7,7a-tetrahydro-3aH-pyrano[3,2-d]thiazol-5-yl)ethanol (700 mg, 1.39 mmol, a mixture of diastereomers: faster moving one: slower moving one = 1 : 5) in dichloromethane (25 mL) was treated with DMP (1.18 g, 2.78 mmol) for 2 h at room temperature. The reaction mixture was quenched by saturated $Na_2S_2O_4$ (25 mL) and saturated $NaHCO_3$ (25 mL), extracted with dichloromethane(2 x 50 mL). The combined organic layers were concentrated under vacuum to provide a residue, which was purified by a silica gel column with 10 % AcOEt in dichloromethane to give compound **33** as a yellow oil (550 mg, 71 %). (ES, *m/z*): $[M+H]^+$ 500.9; $^1H$ NMR (300 MHz, $CDCl_3$) $\delta$ 7.24 - 7.30 (m, 4H), 6.83 - 6.90 (m, 4H), 6.27 - 6.29 (d, *J* = 6.3 Hz, 1H), 3.80 - 4.60 (m, 14H), 3.02 (s, 6H), 2.16 (s, 3H).

**Step 2**

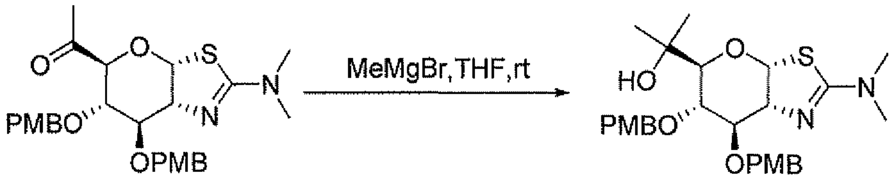


**33** **34**

**2-((3aR,5S,6S,7R,7aR)-2-(Dimethylamino)-6,7-bis(4-methoxybenzyloxy)-5,6,7,7a-tetrahydro-3aH-pyrano[3,2-d]thiazol-5-yl)propan-2-ol (34):** A solution of 1-((3a,R,5S,6S,7R,7aR)-6,7-bis(4-methoxybenzyloxy)-2-(dimethylamino)-5,6,7,7a-tetrahydro-3aH-pyrano[3,2-d]thiazol-5-yl)ethanone (500 mg, 1.00 mmol) in THF (20 mL) was treated with methylmagnesium bromide (1 mL, 3M in THF) for 1 h at room temperature, then quenched by brine (50 mL), and extracted with ethyl acetate (3 x 50 mL). The combined organic layers were dried over anhydrous magnesium sulfate and concentrated under vacuum to provide a residue, which was purified by a silica gel column with 1 % methanol in dichloromethane to give compound **34** as a yellow oil (480 mg, 84 %). (ES, *m/z*): $[M+H]^+$ 516.9; $^1H$ NMR (300MHz, $CDCl_3$) δ 7.33 - 7.36 (d, *J*= 8.7 Hz, 2H), 6.83 - 6.94 (m, 4H), 6.32 - 6.34 (d, *J*= 6.9 Hz, 1H), 4.47 - 4.71 (m, 5H), 4.28 - 4.36 (m, 2H), 3.82 (s, 6H), 3.36 - 3.49 (m, 1H), 3.29 (s, 6H), 1.18 (s, 6H).

**Step 3**

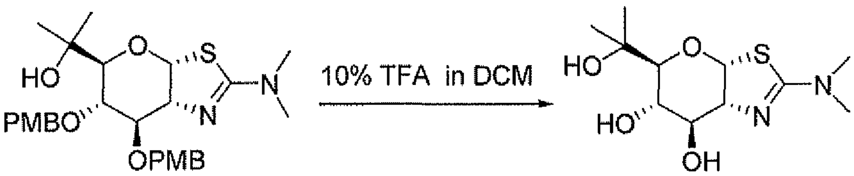


**34** **35**

**(3aR,5S,6S,7R,7aR)-2-(Dimethylamino)-5-(2-hydroxypropan-2-yl)-5,6,7,7a-tetrahydro-3aH-pyrano[3,2-d]thiazole-6,7-diol (35 as its TFA salt):** A solution of 2-((3aR,5S,6S,7R,7aR)-6,7-bis(4-methoxybenzyloxy)-2-(dimethylamino)-5,6,7,7a-tetrahydro-3aH-pyrano[3,2-d]thiazol-5-yl)propan-2-ol (480 mg, 0.93 mmol) in dichloromethane (20 mL) was treated with TFA (2 mL) for 1 h at room temperature. The reaction mixture was condensed to give a residue, which was purified by Prep-HPLC with the following conditions [(3#-Agilent 1200 prep HPLC): Column, SunFire Prep C18,19*50mm 5um; mobile phase, WATER with 0.03 % $NH_4OH$ and $CH_3CN$ (10 % $CH_3CN$ up to 45 % in 10 min; Detector, UV 220nm)] to give compound **35 (Example 4)** as a white solid (TFA salt, 238 mg, 92 %). (ES, *m/z*): $[M+H]^+$ 276.9, $^1H$ NMR (300MHz, $CDCl_3$) δ 6.46 - 6.48 (d, *J* = 7.5 Hz, 1H), 4.46 - 4.49 (m, 1H), 4.08 - 4.10 (m, 1H), 3.81 - 3.87 (m, 1H), 3.28 - 3.30 (d, *J* = 8.4 Hz, 1H), 3.18 (s, 3H), 3.12 (s, 3H), 1.07 - 1.20 (m, 6H).

**Example 5**

**(3aR,5R,6S,7R,7aR)-2-(Dimethylamino)-5-(2-hydroxyethyl)-5,6,7,7a-tetrahydro-3aH-pyrano[3,2-d]thiazole-6,7-diol**

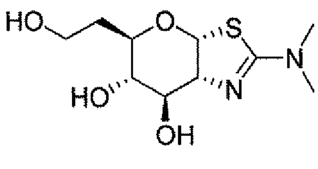

**Scheme VI**

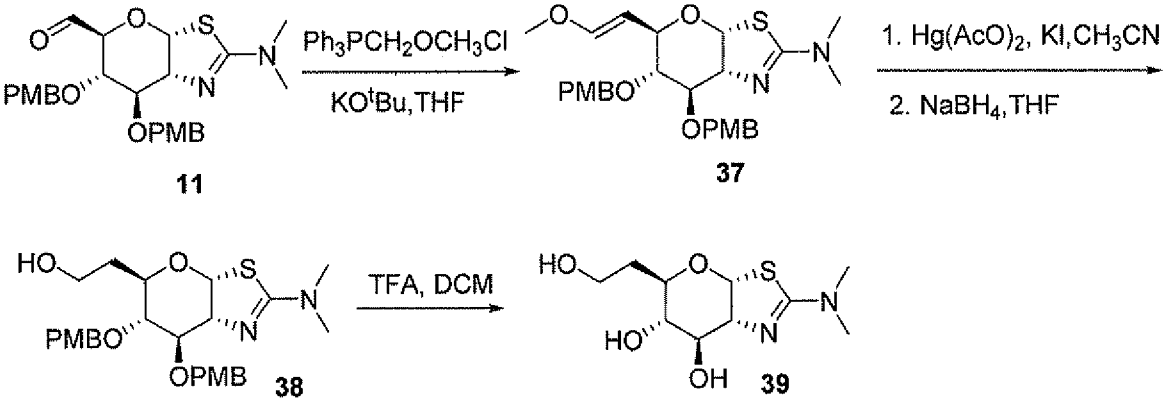


11 37 38 39

Step **1**

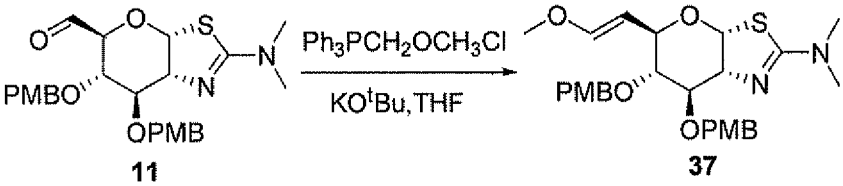


11 37

**(3aR,5R,6R,7R,7aR,E)-6,7-Bis(4-methoxybenzyloxy)-5-(2-methoxyvinyl)-N,N-dimethyl-5,6,7,7a-tetrahydro-3aH-pyrano[3,2-d]thiazol-2-amine (37) :** A solution of $Ph_3PCH_2OCH_3Br$ (2.0 g, 5.90 mmol) in THF (30 mL) was treated with t-BuOK (645 mg, 5.76 mmol) at -10 °C for 30 min, and followed by addition of (3aR, 5S, 6S, 7R, 7aR)-2-(dimethylamino)-6, 7-bis(4-methoxybenzyloxy)-5, 6, 7, 7a-tetrahydro-3aH-pyrano [3, 2-d] thiazole-5-carbaldehyde (700 mg, 1.37 mmol). After stirred for 3 h at 0°C, the resulting solution was quenched with ice-water (20 mL), and extracted with ethyl acetate (2 x 20 mL). The combined organic layers were dried over anhydrous magnesium sulfate and concentrated to give a residue, which was purified by a silica gel column, eluted with 10 - 50 % ethyl acetate in petroleum ether to give the product **37** as a yellow oil (100 mg, 14 %). (ES, *m*/*z*)$[M+H]^+$ 515.0; $^1H$ NMR (300 MHz, $CDCl_3$) δ 7.15 - 7.35 (m, 4H), 6.84-6.98 (m, 4H), 6.45 (d, *J* = 7.8 Hz, 1H ), 6.28 (d, *J* = 6.3 Hz, 1H), 5.21 (m, 1H), 4.45 - 4.89 (m, 4H), 4.18 (t, *J* = 5.7 Hz, 1H), 3.94 (t, *J* = 4.8 Hz, 1H), 3.87 - 3.91 (m, 1 H), 3.78 (s, 6H), 3.56 - 3.65 (m, 1H), 3.52 (s, 3H), 2.92 (s, 6H).

**Step 2**

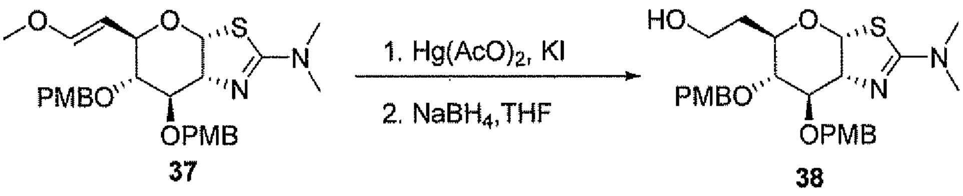


37 38

**2-((3aR,5R,6R,7R,7aR)-2-(Dimethylamino)-6,7-bis(4-methoxybenzyloxy)-5,6,7,7a-tetrahydro-3aH-pyrano[3,2-d]thiazol-5-yl)ethanol (38):** To a cold solution of 37 (100 mg, 0.18 mmol) in acetonitrile (20 mL) at -5 °C was added a solution of $Hg(OAc)_2$ (68 mg, 0.21 mmol) in water (10 mL) dropwise. After stirred at 0 °C for 4 h, the reaction mixture was treated with potassium iodide (40 mg, 0.24 mmol) in water (1.6 mL) for 10 min. The reaction solution was quenched with water, extracted with dichloromethane (40 mL), and dried over $MgSO_4$. Removal of solvents gave crude 2-((3aR,5R,6R,7R,7aR)-6,7-bis(4-methoxybenzyloxy)-2-(dimethylamino)-5,6,7,7a-tetrahydro-3aH-pyrano[3,2-d]thiazol-5-yl)acetaldehyde. To a solution of the crude product in dry THF (20 mL) was added $NaBH_4$ (16 mg, 0.42 mmol) at 0 °C in portions. After stirred at 0 °C overnight, the reaction solution was quenched with water (25 mL) and extracted with ethyl acetate (2 x 20 mL). The combined organic phases were washed with saturated aqueous $NH_4Cl$, dried over $MgSO_4$, and concentrated to give a residue, which was purified by silica gel chromatography, eluted with 20 - 50 % ethyl acetate in petroleum ether to give the product **38** as a light yellow oil (50 mg, 48 %).(ES, *m/z*)[M+H]$^+$ 503.0; $^1$H NMR (300 MHz, $CDCl_3$) δ 7.15 - 7.35 (m, 4H), 6.84 - 6.98 (m, 4H), 6.28 (d, *J* = 6.3 Hz, 1H), 4.45 - 4.89 (m, 4H), 4.18 (t, *J*= 5.7 Hz, 1H), 3.94 (t, *J* = 4.8 Hz, 1H), 3.87 - 3.91 (m, 1H), 3.78 (s, 6H), 3.56 - 3.65 (m, 3H), 2.92 (s, 6H), 1.89 - 1.98 (m, 1H), 1.62 - 1.73 (m, 1H).

**Step 3**

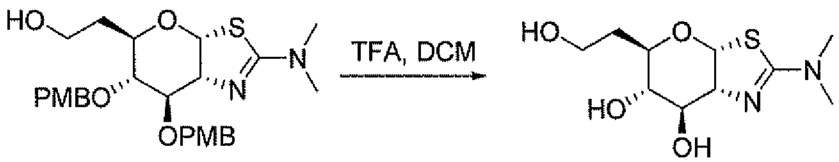


**38** **39**

**(3aR,5R,6S,7R,7aR)-2-(Dimethylamino)-5-(2-hydroxyethyl)-5,6,7,7a-tetrahydro-3aH-pyrano[3,2-d]thiazole-6,7-diol (39):** A solution of 2-((3aR,5R,6R,7R,7aR)-2-(dimethylamino)-6,7-bis(4-methoxybenzyloxy)-5,6,7,7a-tetrahydro-3aH-pyrano[3, 2-d]thiazol-5-yl)ethanol (100 mg, 0.18 mmol) in dichloromethane (20 mL) was treated with TFA overnight at room temperature. Removal of solvents provided a residue, which was purified by Prep-HPLC with the following conditions: (Agilent 1200 prep HPLC: Column, Sun fire prep. C18; mobile phase, water with 0.03 % trifluoroacetic acid and $CH_3CN$ (10 % up to 20 % in time 10); Detector, 220 nm.) to give the product **39 (Example 5)** as a white solid (5.0 mg, 11 %). (ES, *m/z*)[M+H]$^+$ 263.0; $^1$H NMR (300 MHz, $D_2O$) δ 6.29 (d, *J* = 6.3 Hz, 1H), 4.18 (t, *J* = 5.7 Hz, 1H), 3.98 (t, *J* = 4.8 Hz, 1H), 3.56 - 3.65 (m, 3H), 3.39 - 3.43 (m, 1H), 2.92 (s, 6H), 1.89 - 1.98 (m, 1H), 1.62 - 1.72 (m, 1H).

**Example 6 & 7**

**(3aR,5R,6S,7R,7aR)-2-(Dimethylamino)-5-ethyl-5,6,7,7a-tetrahydro-3aH-pyrano[3,2-d]thiazole-6,7-diol and (3aR,5R,6S,7R,7aR)-2-(dimethylamino)-5-vinyl-5,6,7,7a-tetrahydro-3aH-pyrano[3,2-d]thiazole-6,7-diol**

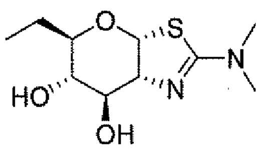

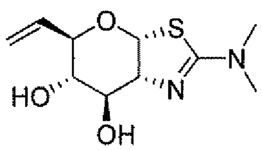

## Scheme VII

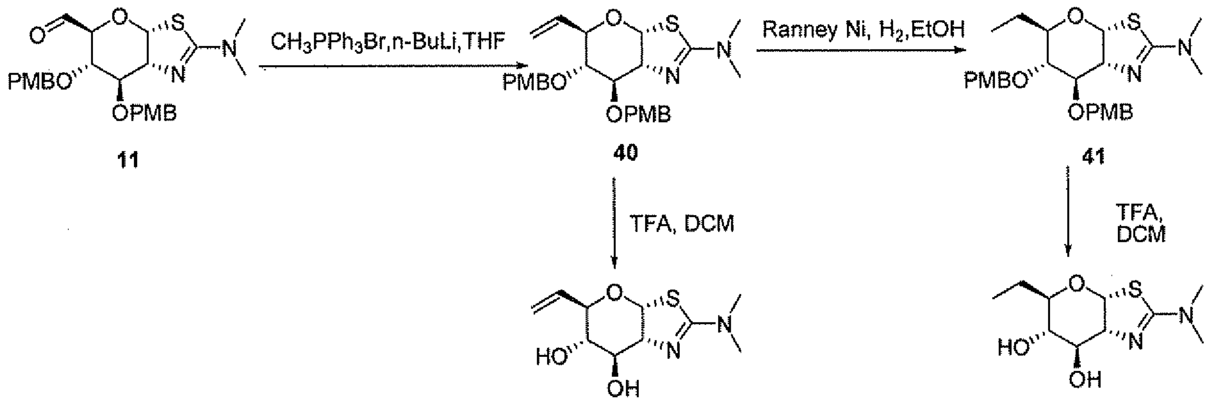


11 40 41

43 42

### Step 1

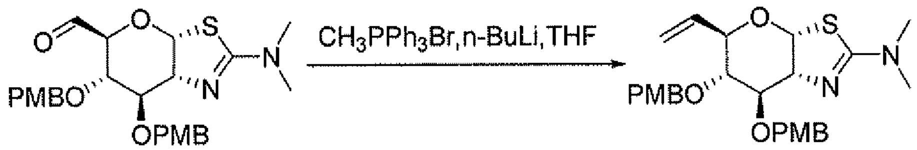


11 40

**(3aR,5R,6R,7R,7aR)-6,7-Bis(4-methoxybenzyloxy)-N,N-dimethyl-5-vinyl-5,6,7,7a-tetrahydro-3aH-pyrano[3,2-d]thiazol-2-amine (40):** To a suspension of methyltriphenylphosphonium bromide (5.9 g, 16.5 mmol) in THF (70 mL) was treated with n-BuLi (5.9 ml, 2.5 M in THF) for 30 min at 0 °C, and followed by addition of (3aR,5S,6S,7R,7aR)-2-(dimethylamino)-6,7-bis(4-methoxybenzyloxy)-5,6,7,7a-tetrahydro-3aH-pyrano[3,2-d]thiazole-5-carbaldehyde (1.6 g, 3.29 mmol) in THF (10 mL). After stirred overnight at room temperature, the reaction mixture was quenched with water (50 mL), extracted with dichloromethane (3 x 50 mL). The organic phases was combined and dried over $MgSO_4$, concentrated under vacuum to give a residue, which was purified by a silica gel column, eluted with 30 % ethyl acetate in petroleum ether to give compound **40** as a yellow oil (520 mg, purity 57 % by LC-MS). This material was used in the next step without further purification. (ES, *m/z*): $[M+H]^+$ 485.0.

### Step 2

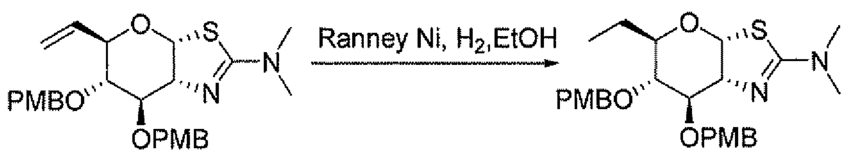


40 41

**(3aR,5R,6R,7R,7aR)-5-Ethyl-6,7-bis(4-methoxybenzyloxy)-N,N-dimethyl-5,6,7,7a-tetrahydro-3aH-pyrano[3,2-d]thiazol-2-amine (41):** A solution of above crude compound **2** (300 mg, purity 57 % by LC - MS) was dissolved into ethanol (20 mL) and treated with Ranney Ni (200 mg) under $H_2$ atmosphere overnight at room temperature. After filtration, the organic solution was concentrated to give crude compound **41** (260 mg, purity 54 % by LC-MS), which was used in the next step without further purification. (ES, *m/z*): $[M+H]^+$ 487.0.

**Step 3**

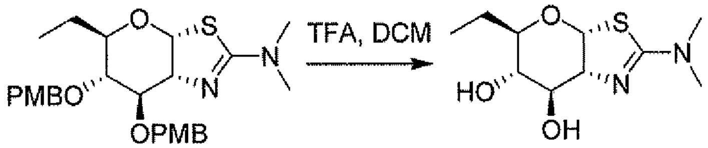


41 42

**(3aR,5R,6S,7R,7aR)-2-(Dimethylamino)-5-ethyl-5,6,7,7a-tetrahydro-3aH-pyrano[3,2-d]thiazole-6,7-diol (42) :** A solution of above crude product **41** (260 mg, purity 54 % by LC - MS) in dichloromethane (10 mL) was treated with TFA (1 mL) for 4 hours at room temperature. The solution was adjusted to pH at 8 with $NH_4OH$, and then condensed to give a crude product, which was purified by Prep-HPLC with the following conditions (Agilent 1200 prep HPLC: Column, SunFire Prep C18,19*50mm 5um; mobile phase, WATER with 0.03 %$NH_4OH$ and $CH_3CN$(10 %$CH_3CN$ up to 45 % in 10 min; Detector, UV 220nm) to provide compound **42 (Example 6**) as a white solid (38 mg). (ES, *m*/*z*): $[M+H]^+$ 247.0. $^1H$ NMR (300 MHz, $D_2O$) δ 6.18 - 6.21 (d, *J* = 6.6 Hz, 1H), 4.09 - 4.13 (t, *J* = 6.0 Hz, 1H), 3.91 - 3.94 (t, *J* = 4.8 Hz, 1H), 3.42 - 3.44 (m, 2H), 2.92 (s, 6H), 1.69 - 1.77 (m, 1H), 1.40-1.50 (m, 1 H), 0.84 - 0.89 (t, *J* = 7.5 Hz, 3H).

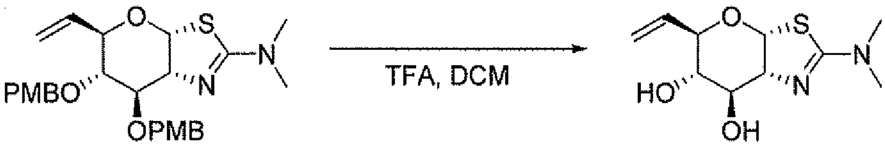


40 43

**(3aR,5R,6S,7R,7aR)-2-(Dimethylamino)-5-vinyl-5,6,7,7a-tetrahydro-3aH-pyrano[3,2-d]thiazole-6,7-diol (43)** : A solution of above crude product **40** (220 mg, purity 57 % by LC-MS) in dichloromethane (10 mL) was treated with TFA (1 mL) for 4 hours at room temperature. The solution was adjusted to pH at 8 with $NH_4OH$, and then condensed to give a crude product, which was purified by Prep-HPLC with the following conditions (Agilent 1200 prep HPLC: Column, SunFire Prep C18,19*50mm 5um; mobile phase, WATER with 0.03 % $NH_4OH$ and $CH_3CN$(10 % $CH_3CN$ up to 45 % in 10 min; Detector, UV 220nm) to provide compound **43 (Example 7)** as a white solid (35.7 mg). (ES, *m*/*z*): $[M+H]^+$ 245.0. $^1H$ NMR (300 MHz, $D_2O$) δ 6.20 - 6.22 (d, *J* = 6.3 Hz, 1H), 5.79 - 5.87 (m, 1H), 5.30 - 5.38 (m, 2H), 3.98 - 4.08 (m, 2H), 3.87 - 3.91 (t, *J*= 6.0 Hz, 1H), 3.46 - 3.52 (m, 1H), 2.93 (s, 6H).

**Example 8**

**(3aR,5R,6S,7R,7aR)-5-Ethyl-2-(methylamino)-5,6,7,7a-tetrahydro-3aH-pyrano [3,2-d]thiazole-6,7-diol**

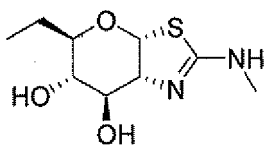

**Scheme VIII**

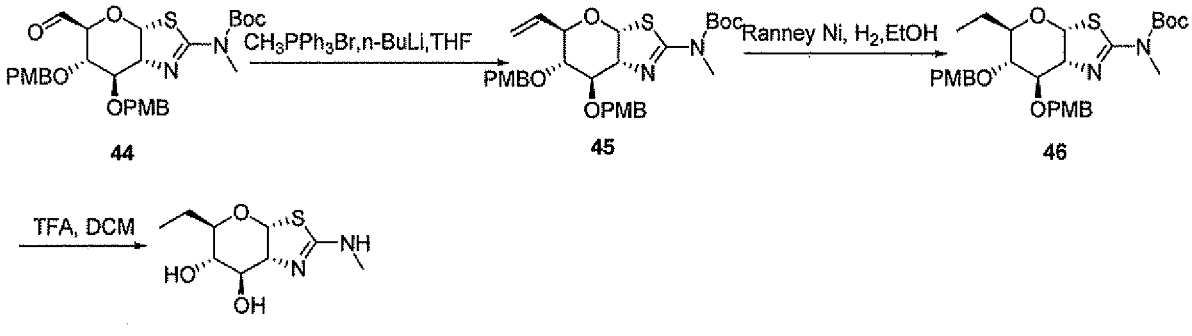


44 45 46 47

**Step 1**

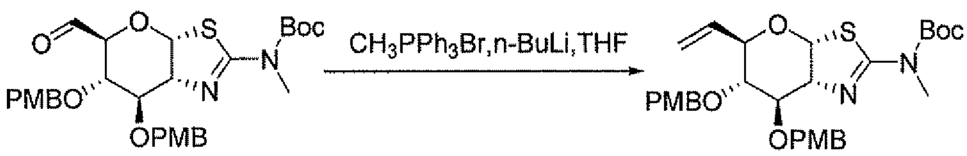


44 45

**tert-Butyl (3aR,5R,6R,7R,7aR)-6,7-bis(4-methoxybenzyloxy)-5-vinyl-5,6,7,7a-tetrahydro-3aH-pyrano[3,2-d]thiazol-2-yl(methyl)carbamate (45)** : A suspension of methyltriphenylphosphonium bromide (1.85 g, 5.18 mmol) in tetrahydrofuran (35 mL) was treated with n-BuLi (1.9 ml, 2.5M) for 30 min at 0 °C, and followed by addition of tert-butyl (3aR,5S,6S,7R,7aR)-5-formyl-6,7-bis(4-methoxybenzyloxy)-5,6,7,7a-tetrahydro-3aH-pyrano[3,2-d]thiazol-2-yl(methyl)carbamate (**44**) prepared from compound **28** by a procedure identical to that desribed for the conversion of **10** to **11**) (0.6 g, 1.05 mmol) in THF (5 mL). The resulting solution was stirred overnight at room temperature, quenched by water (25 mL), and extracted with dichloromethane (3 x 25 ml). The combined organic layers were dried over magnesium sulfate and concentrated under vacuum to give a residue, which was purified by a silica gel column, eluted with 20 % ethyl acetate in petroleum ether to give crude compound **45** as a yellow oil (220 mg, purity 50 % by LC-MS). (ES, *m/z*): $[M+H]^+$ 571.0.

**Step 2**

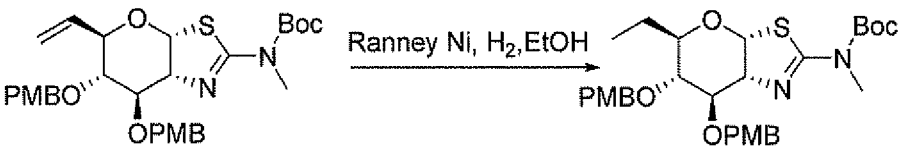


45 46

**tert-Butyl (3aR,5R,6R,7R,7aR)-5-ethyl-6,7-bis(4-methoxybenzyloxy)-5,6,7,7a-tetrahydro-3aH-pyrano[3,2-d]thiazol-2-yl(methyl)carbamate (46) :** A solution of above crude compound **45** (220 mg, purity 50 %) was dissolved into ethanol (15 mL) and treated with Ranney Ni (100 mg) under $H_2$ atmosphere overnight at room temperature. After filtration, the organic solution was concentrated to give crude compound **46** (200 mg, purity 48 % by LC-MS), which was used in the next step without further purification. (ES, *m/z*): $[M+H]^+$ 573.0.

**Step 3**

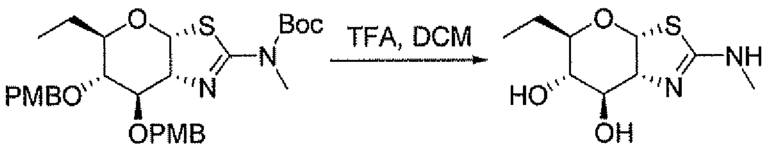


**46** **47**

**(3aR,5R,6S,7R,7aR)-5-Ethyl-2-(methylamino)-5,6,7,7a-tetrahydro-3aH-pyrano[3,2-d]thiazole-6,7-diol (47) :** A solution of above crude product **46** (200 mg, purity 48 % by LC-MS) in dichloromethane (10 mL) was treated with TFA (1 mL) for 4 hours at room temperature. The solution was adjusted to pH at 8 with $NH_4OH$, and then condensed to give a crude product, which was purified by Prep-HPLC with the following conditions (Agilent 1200 prep HPLC: Column, SunFire Prep C18,19*50mm 5um; mobile phase, WATER with 0.03 % $NH_4OH$ and $CH_3CN$(10 % $CH_3CN$ up to 45 % in 10 min; Detector, UV 220nm) to provide the title compound **47 (Example 8)** as a white solid (7.6 mg). (ES, *m*/*z*): $[M+H]^+$ 233.0. $^1H$ NMR (300 MHz, $D_2O$) δ 6.18 - 6.20 (d, *J* = 6.3 Hz, 1H), 4.09 - 4.13 (t, *J* = 6.0 Hz, 1H), 3.92 - 3.95 (t, *J* = 4.8 Hz, 1H), 3.36 - 3.45 (m, 2H), 2.75 (s, 3H), 1.65 - 1.75 (m, 1H), 1.38 - 1.48 (m, 1H), 0.81 - 0.86 (t, *J* = 7.5 Hz, 3H).

**Example 9**

**(3aR,5S,6S,7R,7aR)-2-(Dimethylamino)-5-((S)-1-fluoroethyl)-5,6,7,7a-tetrahydro-3aH-pyrano [3,2-d]thiazole-6,7-diol**

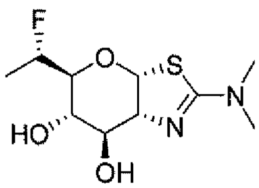

## Scheme IX

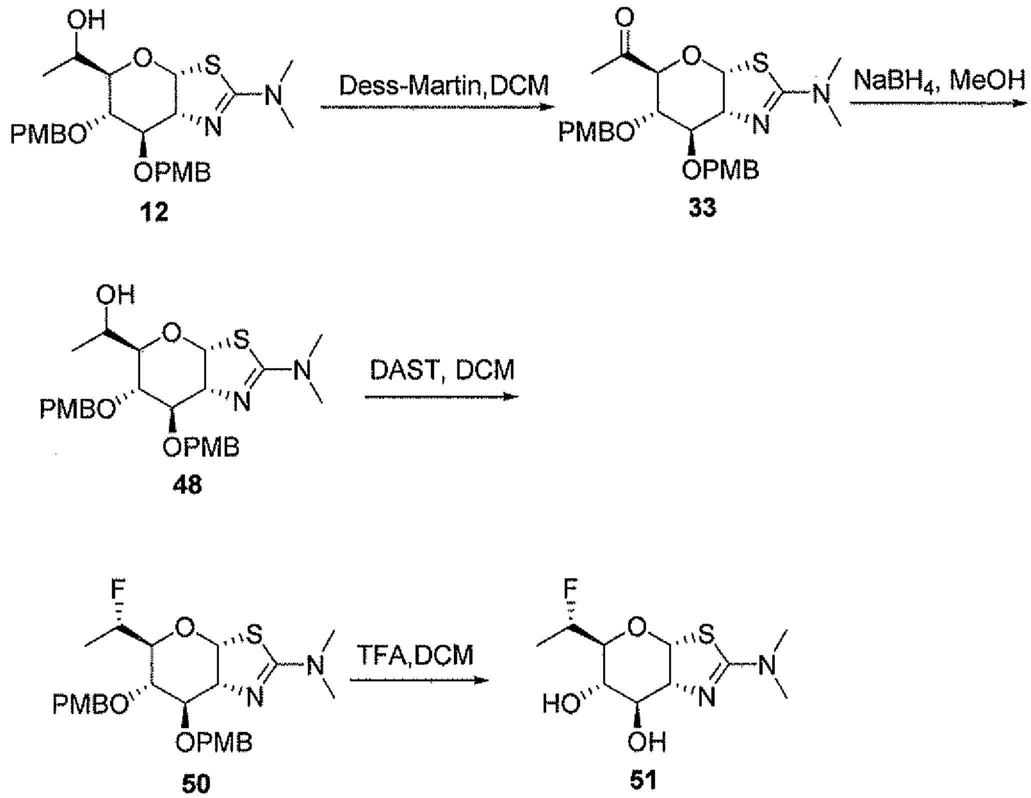


12 33 48 50 51

### Step 1

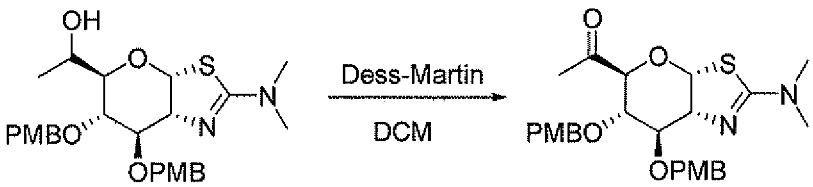


12 33

**1-((3aR,5S,6S,7R,7aR)-2-(Dimethylamino)-6,7-bis(4-methoxybenzyloxy)-5,6,7,7a-tetrahydro-3aH-pyrano[3,2-d]thiazol-5-yl)ethanone (33) :** A solution of 1-((3aR,5R,6S,7R,7aR)-2-(dimethylamino)-6,7-bis(4-methoxybenzyloxy)-5,6,7,7a-tetrahydro-3aH-pyrano[3,2-d]thiazol-5-yl) ethanol (450 mg, 0.90 mmol, a mixture of diastereomers, faster eluting isomer: slower eluting isomer = 1 : 5 by HPLC and $^{1}$HNMR) in dichloromethane (20 mL) was treated with Dess-Martin reagent (760 mg, 1.80 mmol) for 2 hours at room temperature. The reaction mixture was quenched by sat. aqueous $Na_2S_2O_3$ solution (20 mL) and sat. aqueous $NaHCO_3$ solution (20 mL), and extracted with dichloromethane (2 x 20 mL). The combined, organic layers were dried over anhydrous sodium sulfate and concentrated under reduced pressure to provide a residue, which was purified by silica gel column, eluted with 2 - 3 % methanol in dichloromethane to give compound **33** as a yellow syrup (400 mg, 88 %). (ES, *m/z*): $[M+H]^+$ 501.0; $^{1}$H NMR (300 MHz, $CDCl_3$) δ 7.25 - 7.30 (m, 4H), 6.83 - 6.90 (m, 4H), 6.29 - 6.31 (d, *J*= 6.0 Hz, 1H), 4.26 - 4.64 (m, 6H), 3.81 - 3.98 (m, 8H), 2.96 - 3.02 (m, 6H), 2.17 (s, 3H).

### Step 2

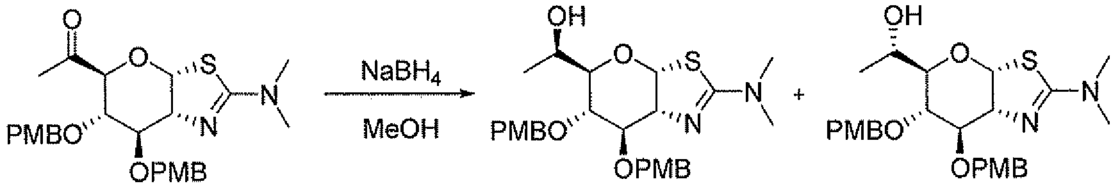


33 48 48a

**(R)-1-((3aR,5R,6S,7R,7aR)-2-(Dimethylamino)-6,7-bis(4-methoxybenxyloxy)-5,6,7,7a-tetrahydro-3aH-pyrano[3,2-d]thiazol-5-yl)ethanol (48) and (S)-1-((3aR,5R,6S,7R,7aR)-2-(dimethylamino)-6,7-bis(4-methoxybenzyloxy)-5,6,7,7a-tetrahydro-3aH-pyrano[3,2-d]thiazol-5-yl)ethanol (48a):** A solution of 1-((3aR,5S,6S,7R,7aR)-2-(dimethylamino)-6,7-bis(4-methoxybenzyloxy)-5,6,7,7a-tetrahydro-3aH-pyrano[3,2-d]thiazol-5-yl)ethanone (400 mg, 0.80 mmol) in methanol (10 mL) was treated with $NaBH_4$ (150 mg, 3.95 mmol) for 1 hour at room temperature. The reaction was quenched by water (10 mL), and extracted with ethyl acetate (3 x 20 mL). The combined organic layers were dried over anhydrous sodium sulfate and concentrated under reduced pressure to provide a residue, which was purified by Chiral- HPLC, eluted with 20 % ethanol in hexane to give compound **48** as a light yellow oil (slower eluting isomer by Chiral-HPLC, 270 mg, 67 %). (ES, *m/z*): $[M+H]^+$ 503.0; $^1H$ NMR (300 MHz, $CDCl_3$) $\delta$ 7.22 - 7.36 (m, 4H), 6.84 - 6.92 (m, 4H), 6.34 (broad, 1H), 4.53 - 4.71 (m, 4H), 4.25 - 4.35 (m, 2H), 3.76 - 3.94 (m, 8H), 3.45 - 3.49 (dd, *J* = 4.2 Hz, 4.5 Hz, 1H), 3.01 (s, 6H), 1.19 - 1.21 (d, *J* = 6.0 Hz, 1H), 1.11 - 1.13 (d, *J* = 6.0 Hz, 3H); and compound **48a** as a light yellow oil (faster eluting isomer by Chiral-HPLC, 113 mg, 28 %).(ES, *m/z*): $[M+H]^+$ 503.0; $^1H$ NMR (300 MHz, $CDCl_3$) $\delta$ 7.32 - 7.35 (d, *J* = 8.4 Hz, 2H), 7.24 - 7.27 (d, *J* = 8.7 Hz, 2H), 6.83 - 6.91 (m, 4H), 6.31 - 6.33 (d, *J* = 6.6 Hz, 1H), 4.60 - 4.71 (m, 2H), 4.53 - 4.57 (m, 2H), 4.25 - 4.35 (m, 2H), 3.85 - 3.87 (m, 7H), 3.73 - 3.75 (d, *J* = 8.7 Hz, 1H), 3.32 - 3.36 (dd, *J* = 3.6 Hz, 3.3 Hz, 1H), 3.01 (s, 6H), 1.92 - 1.94 (d, *J* = 7.5 Hz, 1H), 1.19 - 1.21 (d, *J* = 6.3 Hz, 3H).

**Step 3**

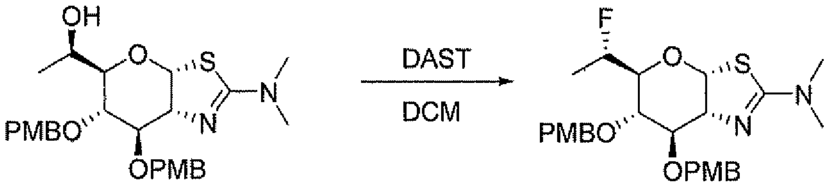


48 50

**(3aR,5S,6S,7R,7aR)-5-((S)-1-Fluoroethyl)-6,7-bis(4-methoxybenzyloxy)-N,N-dimethyl-5,6,7,7a-tetrahydro-3aH-pyrano[3,2-d]thiazol-2-amine (50) :** A solution of (R)-1-((3aR,5R,6S,7R,7aR)-2-(dimethylamino)-6,7-bis(4-methoxybenzyloxy)-5,6,7,7a-tetrahydro-3aH-pyrano[3,2-d]thiazol-5-yl)ethanol (280 mg, 0.56 mmol) in dichloromethane (10 mL) was treated with DAST (900 mg, 5.59 mmol) at -78 °C for 10 min and at 0 °C for 1 hr. The reaction was quenched by sat. aqueous $Na_2CO_3$ solution (30 mL), and extracted with dichloromethane (2 x 20 mL). The combined organic layers were dried over anhydrous sodium sulfate and concentrated under reduced pressure to provide a residue, which was purified by silica gel column, eluted with 1 - 2 % methanol in dichloromethane to give compound **50** as a yellow syrup (240 mg, 85 %). (ES, *m/z*): $[M+H]^+$ 505.0; $^1H$ NMR (300 MHz, $CDCl_3$) $\delta$ 7.22 - 7.43 (m, 4H), 6.83 - 6.94 (m, 4H), 6.38 - 6.50 (m, 1H), 4.58 - 4.78 (m, 6H), 3.75 - 3.90 (m, 8H), 3.46 - 3.50 (m, 1H), 3.05 - 3.18 (m, 6H), 1.27 - 1.36 (m, 3H).

**Step 4**

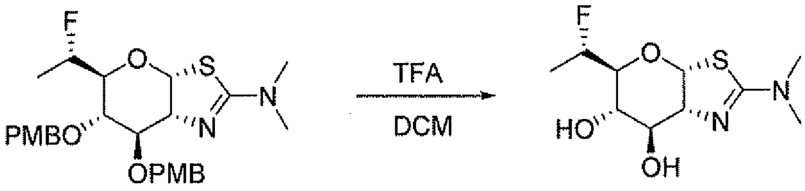


50 51

**(3aR,5S,6S,7R,7aR)-2-(Dimethylamino)-5-((S)-1-fluoroethyl)-5,6,7,7a-tetrahydro-3aH-pyrano[3,2-d]thiazole-6,7-diol (51) :** A solution of (3aR,5S,6S,7R,7aR)-5-((S)-1-fluoroethyl)-6,7-bis(4-methoxybenzyloxy)-N,N-dimethyl-5,6,7,7a-tetrahydro-3aH-pyrano[3,2-d]thiazol-2-amine (240 mg, 0.48 mmol) in dichloromethane (20 mL) was treated with TFA (2 mL) for 1 h at room temperature. The reaction mixture was concentrated under reduced pressure to give a residue, which was purified by Prep-HPLC under the following conditions [(Agilent 1200 prep HPLC): Column, SunFire Prep C18, 19 * 50 mm 5 um; mobile phase, water with 0.03 % $NH_4OH$ and $CH_3CN$ (10 % $CH_3CN$ up to 45 % in 10 min); Detector, UV 220nm] to afford compound **51 (Example 9)** as a white solid (30 mg, 24 %). (ES, *m/z*): $[M+H]^+$ 265.0; $^1H$ NMR (300 MHz, $D_2O$) $\delta$ 6.23 - 6.25 (d, *J*= 6.0 Hz, 1H), 4.93 - 4.99 (m, 0.5H), 4.77 - 4.84 (m, 0.5H), 4.17 (t, *J* = 6.0 Hz, 1H), 4.00 (t, *J* = 6.0 Hz, 1H), 3.71 - 3.74 (m, 1H), 3.36 - 3.49 (m, 1H), 2.94 (s, 1H), 1.25 - 1.35 (dd, *J* = 6.6 Hz, 6.3 Hz, 3H).

**Example 10 and 11**

**(3aR,5S,6S,7R,7aR)-2-(dimethylamino)-5-((S)-2,2,2-trifluoro-1-hydroxyethyl)-5,6,7,7a-tetrahydro-3aH-pyrano[3,2-d]thiazole-6,7-diol and (3aR,5S,6S,7R,7aR)-2-(dimethylamino)-5-((R)-2,2,2-trifluoro-1-hydroxyethyl)-5,6,7,7a-tetrahydro-3aH-pyrano[3,2-d]thiazole-6,7-diol**

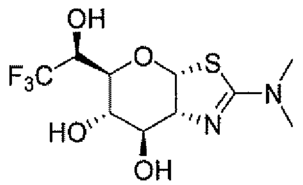

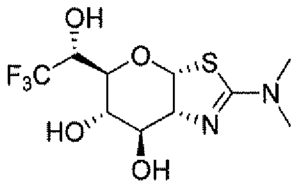

## Scheme X

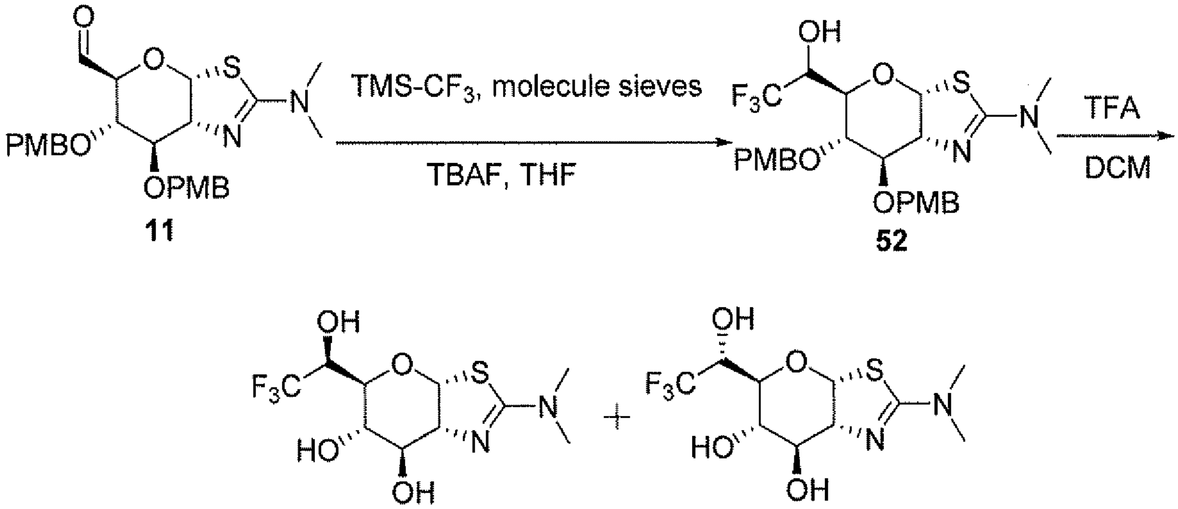


11 52

53 54

**Step** 1

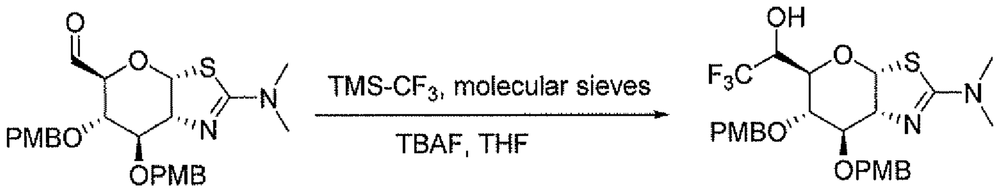


11 52

**1-((3aR,5R,6S,7R,7aR)-2-(Dimethylamino)-6,7-bis(4-methoxybenzyloxy)-5,6,7,7a-tetrahydro-3aH-pyrano[3,2-d]thiazol-5-yl)-2,2,2-trifluoroethanol (52, mixture of two diastereomers):** To a stirred mixture of TBAF (107 mg, 0.41 mmol) and 4Å molecule sieves in THF (20 mL) was added a solution of (3aR,5S,6S,7R,7aR)-2-(dimethylamino)-6,7-bis(4-methoxybenzyloxy)-5,6,7,7a-tetrahydro-3aH-pyrano[3,2-d]thiazole-5-carbaldehyde **(1)** (400 mg, 0.82 mmol) and TMS-$CF_3$ (230 mg, 1.64 mmol) in THF (5 mL) at 0 °C. After stirring for 4 hours at 0 °C, the reaction was quenched by brine (30 mL), and extracted with ethyl acetate (3 x 20 mL). The combined organic layers were dried over anhydrous sodium sulfate and concentrated under reduced pressure to provide a residue, which was purified by silica gel column, eluted with 2 - 3 % methanol in dichloromethane to give compound **52** as a yellow syrup (300 mg, 65 %, a mixture of diastereomers, faster eluting isomer : slower moving isomer = 1: 2 by Chiral-HPLC). (ES, *m/z*): $[M+H]^+$ 557.0; $^1H$ NMR (300 MHz, $CDCl_3$) δ 7.20 - 7.35 (m, 4H), 6.85 - 6.92 (m, 4H), 6.25 - 6.27 (d, *J* = 6.6 Hz, 1H), 4.56 - 4.69 (m, 5H), 4.30 - 4.36 (m, 2H), 3.82 - 3.83 (m, 8H), 2.99 - 3.00 (m, 6H).

**Step 2**

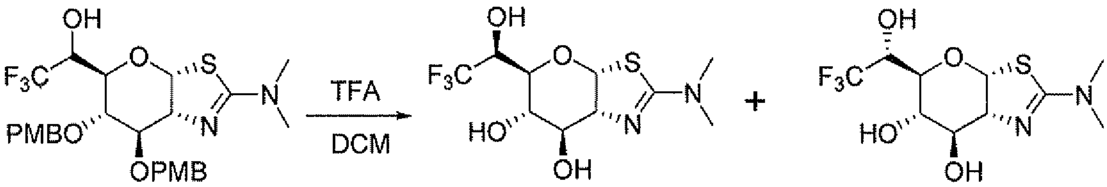


52 53 54

**(3aR,5S,6S,7R,7aR)-2-(dimethylamino)-5-((S)-2,2,2-trifluoro-1-hydroxyethyl)-5,6,7,7a-tetrahydro-3aH-pyrano[3,2-d]thiazole-6,7-diol (53) and (3aR,5S,6S,7R,7aR)-2-(dimethylamino)-5-((R)-2,2,2-trifluoro-1-hydroxyethyl)-5,6,7,7a-tetrahydro-3aH-pyrano[3,2-d]thiazole-6,7-diol (54) :** A solution of 1-((3aR,5R,6S,7R,7aR)-2-(dimethylamino)-6,7-bis(4-methoxybenzyloxy)-5,6,7,7a-tetrahydro-3aH-pyrano[3,2-d]thiazol-5-yl)-2,2,2-trifluoroethanol **(52, a mixture of two diastereomers from previous step)** (400 mg, 0.72 mmol) in dichloromethane (20 mL) was treated with TFA (2 mL) for 1 h at room temperature. The reaction mixture was concentrated under reduced pressure to give a residue, which was purified by Prep-HPLC under the following conditions [(Agilent 1200 prep HPLC): Column, SunFire Prep C18, 19 * 50 mm 5 um; mobile phase, water with 0.03 % $NH_4OH$ and $CH_3CN$ (10 % $CH_3CN$ up to 45 % in 10 min); Detector, UV 220nm] to afford compound **53 (Example 10)** as a white solid (faster eluting isomer, 62 mg, 27 %): (ES, *m/z*): $[M+H]^+$ 316.9; $^1H$ NMR (300 MHz, $D_2O$) δ 6.19 - 6.21 (d, *J* = 6.6 Hz, 1H), 4.22 - 4.27 (m, 1H), 4.04 (t, *J* = 6.6 Hz, 1H), 3.86 - 3.90 (m, 1H), 3.71 - 3.76 (m, 1H), 2.93 (s, 6H); Compound **54 (Example 11)** as a white solid (slower eluting isomer, 55 mg, 24 %).(ES, *m/z*): $[M+H]^+$ 316.9; $^1H$ NMR (300 MHz, $D_2O$) δ 6.25 - 6.27 (d, *J* = 6.3 Hz, 1H), 4.26 - 4.34 (m, 1H), 4.10 (t, *J* = 6.0 Hz, 1 H), 3.94 (t, *J* = 5.4 Hz, 1H), 3.72 - 3.82 (m, 1H), 2.92 (s, 6H).

**Example 12**

**(3aR,5S,6S,7R,7aR)-5-(1,1-Difluoroethyl)-2-(dimethylamino)-5,6,7,7a-tetrahydro-3aH-pyrano[3,2-d]thiazole-6,7-diol**

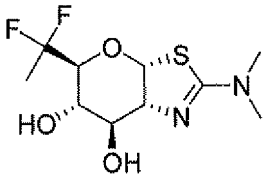

**SchemeXI**

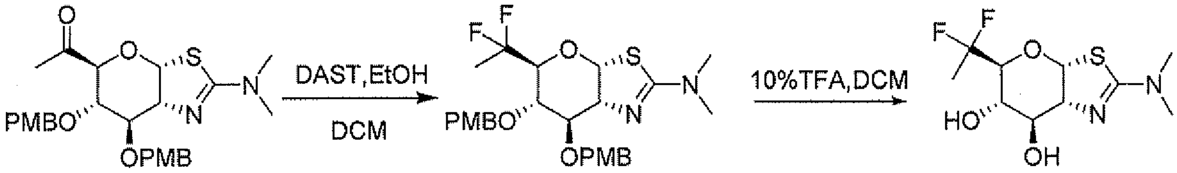


33 55 56

**Step 1**

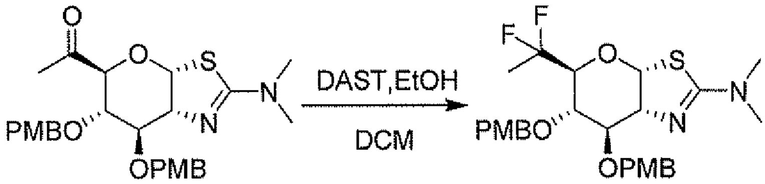


33 55

**(S)-1-((3aR,5R,6S,7R,7aR)-2-(Dimethylamino)-6,7-bis(4-methoxybenzyloxy)-5,6,7,7a-tetrahydro-3aH-pyrano[3,2-d]thiazol-5-yl)ethanol (55) :** A solution of 1-((3aR,5S,6S,7R,7aR)-2-(dimethylamino)-6,7-bis(4-methoxybenzyloxy)-5,6,7,7a-tetrahydro-3aH-pyrano[3,2-d]thiazol-5-yl)ethanone (280 mg, 0.56 mmol) in dichloromethane (10 mL) was treated with (diethylamino)sulfur trifluoride (451 g, 2.80 mol) and ethanol (5.2 mg, 0.11 mmol) overnight at room temperature. The reaction was then quenched by addition of sat. aqueous sodium carbonate solution (50 mL), and extracted with dichloromethane (3 x 20 mL). The combined organic layers were dried over anhydrous magnesium sulfate and condensed under vacuum to give a crude product **55** (140 mg), which was used in the next step without further purification. (ES, *m/z*) $[M+H]^+$ 523.0; $^1H$ NMR (300 MHz, $CD_3Cl$) δ $^1H$ NMR (300 MHz, $CDCl_3$) δ 7.24 - 7.30 (m, 4H), 6.84 - 6.95 (m, 4H), 6.30 - 6.32 (m, 1H), 4.57 - 4.62 (m, 4H), 4.27 - 4.35 (m, 2H), 3.81 - 3.91 (m, 8H), 2.99 - 3.01 (m, 6H), 1.56 - 1.62 (m, 3H).

**Step 2**

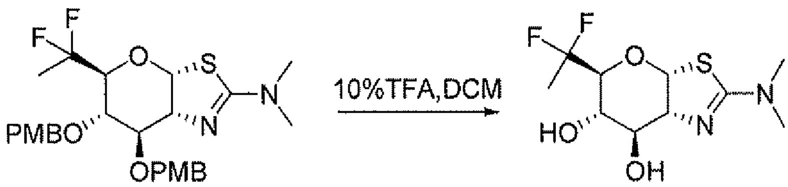


55 56

**1-((3aR,5S,6S,7R,7aR)-2-(Dimethylamino)-6,7-bis(4-methoxybenzyloxy)-5,6,7,7a-tetrahydro-3aH-pyrano[3,2-d]thiazol-5-yl)ethanone (56)** : A solution of crude product **2** (140 mg, 0.27 mmol, 1.00 equiv) in dichloromethane (10 mL) was treated with TFA (1 mL) for 2 hrs at room temperature, and then condensed o give a residue, which was purified by Prep-HPLC with the following conditions [(Prep-HPLC): Column, 19* 150mm; mobile phase, Water with 0.03% ammonia and CH3CN (10 % CH3CN up to 35 % in 10 min); Detector, 220nm.] to give the title compound **56 (Example 12)**

as a white solid (76.3 mg, 40 %). (ES, *m/z*) $[M+H]^+$ 282.9; $^1H$ NMR (300 MHz, $D_2O$) δ 6.23 (d, *J* = 6.6 Hz, 1H), 4.35 (t, *J* = 5.1 Hz, 1H), 4.14 (t, *J* = 2.7 Hz, 1H), 3.91 - 3.95 (m, 1H), 3.57 - 3.67 (m, 1H), 2.94 (s, 6H), 1.59 (t, *J* = 19.8 Hz, 3H).

The following examples were synthesized according to procedures analogous to the schemes and examples outlined above.

Table 1

| Example | structure | Name | MH+ |
|---|---|---|---|
| 13 | 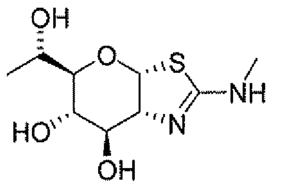 | (3aR,5R,6S,7R,7aR)-5-((S)-1-hydroxyethyl)-2-(methylamino)-5,6,7,7a-tetrahydro-3aH-pyrano[3,2-d]thiazole-6,7-diol(Slower eluting isomer) | 249.1 |
| 14 | 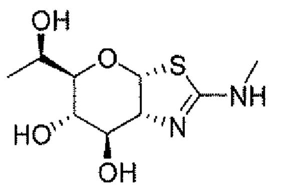 | (3aR,5R,6S,7R,7aR)-5-((R)-1-hydroxyethyl)-2-(methylamino)-5,6,7,7a-tetrahydro-3aH-pyrano[3,2-d]thiazole-6,7-diol(Faster eluting isomer) | 249.0 |
| 15 | 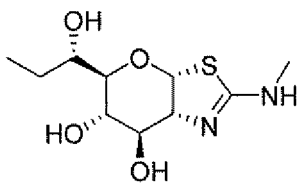 | (3aR,5R,6S,7R,7aR)-5-((S)-1-hydroxypropyl)-2-(methylamino)-5,6,7,7a-tetrahydro-3aH-pyrano[3,2-d]thiazole-6,7-diol(Slower eluting isomer) | 262.1 |
| 16 | 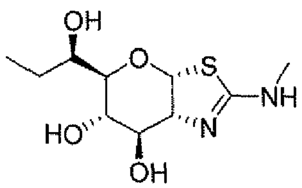 | (3aR,5R,6S,7R,7aR)-5-((R)-1-hydroxypropyl)-2-(methylamino)-5,6,7,7a-tetrahydro-3aH-pyrano[3,2-d]thiazole-6,7-diol(Faster eluting isomer) | 262.1 |
| 17 | 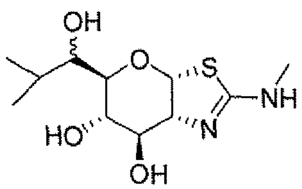 | (3aR,5R,6S,7R,7aR)-5-(1-hydroxy-2-methylpropyl)-2-(methylamino)-5,6,7,7a-tetrahydro-3aH-pyrano[3,2-d]thiazole-6,7-diol (faster moving isomer: slower moving isomer = 30:1) | 277.1 |
| 18 | 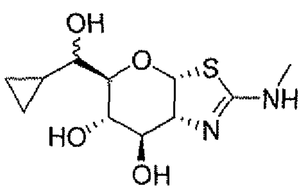 | (3aR,5R,6S,7R,7aR)-5-(cyclopropyl(hydroxy)met hyl)-2-(methylamino)-5,6,7,7a-tetrahydro-3aH-pyrano[3,2-d]thiazole-6,7-diol (faster moving isomer: slower moving isomer = 1:49) | 275.1 |
| 19 | 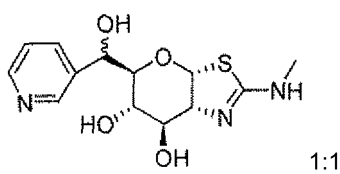 1:1) | (3aR,5R,6S,7R,7aR)-5-(hydroxy(pyridin-3-yl)methyl)-2-(methylamino)-5,6,7,7a-tetrahydro-3aH-pyrano[3,2-d]thiazole-6,7-diol (faster moving isomer: slower moving isomer = 1:1) | 312.1 |

(continued)

| Example | structure | Name | MH+ |
|---|---|---|---|
| 20 | 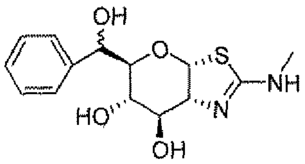 | (3aR,5R,6S,7R,7aR)-5-(hydroxy(phenyl)methyl)-2-(methylamino)-5,6,7,7a-tetrahydro-3aH-pyrano[3,2-d]thiazole-6,7-diol (faster moving isomer: slower moving isomer = 1:18) | 311.1 |
| 21 | 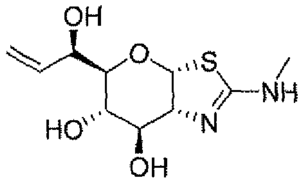 | (3aR,5R,6S,7R,7aR)-5-((R)-1-hydroxyallyl)-2-(methylamino)-5,6,7,7a-tetrahydro-3aH-pyrano[3,2-d]thiazole-6,7-diol (Faster eluting isomer) | 261.1 |
| 22 | 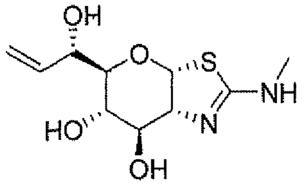 | (3aR,5R,6S,7R,7aR)-5-((S)-1-hydroxyallyl)-2-(methylamino)-5,6,7,7a-tetrahydro-3aH-pyrano[3,2-d]thiazole-6,7-diol (Slower eluting isomer) | 261.1 |
| 23 | 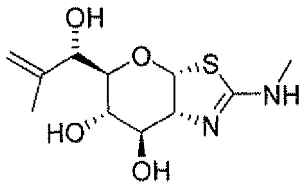 | (3aR,5R,6S,7R,7aR)-5-((S)-1-hydroxy-2-methylallyl)-2-(methylamino)-5,6,7,7a-tetrahydro-3aH-pyrano[3,2-d]thiazole-6,7-diol (Slower eluting isomer) | 275.1 |
| 24 | 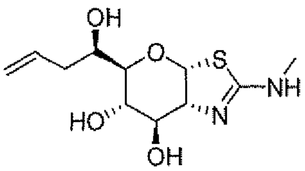 | (3aR,5R,6S,7R,7aR)-5-((R)-1-hydroxybut-3-enyl)-2-(methylamino)-5,6,7,7a-tetrahydro-3aH-pyrano[3,2-d]thiazole-6,7-diol (Faster eluting isomer) | 275.0 |
| 25 | 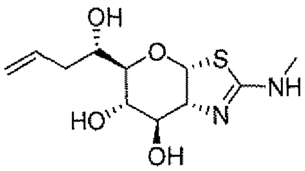 | (3aR,5R,6S,7R,7aR)-5-((S)-1-hydroxybut-3-enyl)-2-(methylamino)-5,6,7,7a-tetrahydro-3aH-pyrano[3,2-d]thiazole-6,7-diol(Slower eluting isomer) | 275.0 |
| 26 | 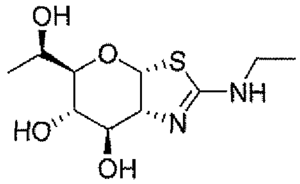 | (3aR,5R,6S,7R,7aR)-2-(ethylamino)-5-((R)-1-hydroxyethyl)-5,6,7,7a-tetrahydro-3aH-pyrano[3,2-d]thiazole-6,7-diol(Faster eluting isomer) | 263.1 |
| 27 | 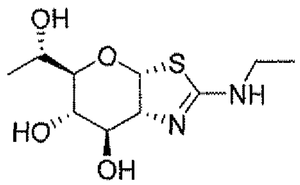 | (3aR,5R,6S,7R,7aR)-2-(ethylamino)-5-((S)-1-hydroxyethyl)-5,6,7,7a-tetrahydro-3aH-pyrano[3,2-d]thiazole-6,7-diol(Slower eluting isomer) | 263.1 |

(continued)

| Example | structure | Name | MH+ |
|---|---|---|---|
| 28 | 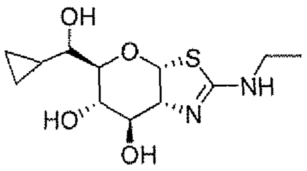 | (3aR,5R,6S,7R,7aR)-5-(cyclopropyl(hydroxy)met hyl)-2-(ethylamino)-5,6,7,7a-tetrahydro-3aH-pyrano[3,2-d]thiazole-6,7-diol | 289.1 |
| 29 | 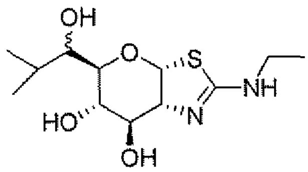 | (3aR,5R,6S,7R,7aR)-2-(ethylamino)-5-(1-hydroxy-2-methylpropyl)-5,6,7,7a-tetrahydro-3aH-pyrano[3,2-d]thiazole-6,7-diol (faster moving isomer: slower moving isomer = 31:1) | 291.2 |
| 30 | 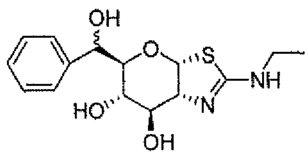 | (3aR,5R,6S,7R,7aR)-2-(ethylamino)-5-(hydroxy(phenyl)methyl)-5,6,7,7a-tetrahydro-3aH-pyrano[3,2-d]thiazole-6,7-diol (faster moving isomer: slower moving isomer = 1:20) | 325.2 |
| 31 | 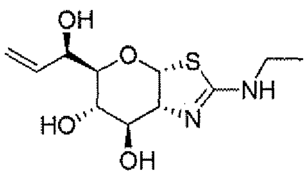 | (3aR,5R,6S,7R,7aR)-2-(ethylamino)-5-((R)-1-hydroxyallyl)-5,6,7,7a-tetrahydro-3aH-pyrano[3,2-d]thiazole-6,7-diol (Faster eluting isomer) | 275.2 |
| 32 | 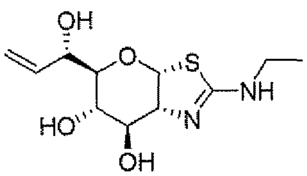 | (3aR,5R,6S,7R,7aR)-2-(ethylamino)-5-((S)-1-hydroxyallyl)-5,6,7,7a-tetrahydro-3aH-pyrano[3,2-d]thiazole-6,7-diol (Slower eluting isomer) | 275.1 |
| 33 | 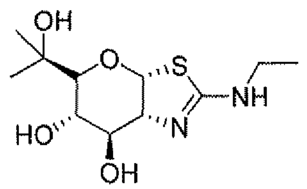 | (3aR,5R,6S,7R,7aR)-2-(ethylamino)-5-(2-hydroxypropan-2-yl)-5,6,7,7a-tetrahydro-3aH-pyrano[3,2-d]thiazole-6,7-diol | 277.1 |
| 34 | 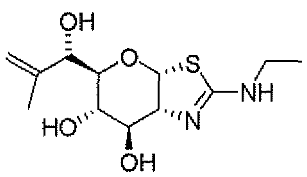 | (3aR,5R,6S,7R,7aR)-2-(ethylamino)-5-(S)(-1-hydroxy-2-methylallyl)-5,6,7,7a-tetrahydro-3aH-pyrano[3,2-d]thiazole-6,7-diol(Slower eluting isomer) | 289.2 |
| 35 | 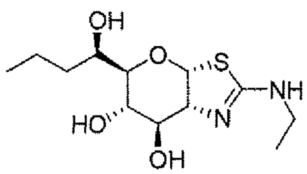 | (3aR,5R,6S,7R,7aR)-2-(ethylamino)-5-((R)-1-hydroxybutyl)-5,6,7,7a-tetrahydro-3aH-pyrano[3,2-d]thiazole-6,7-diol (Faster eluting isomer) | 291.0 |

(continued)

| Example | structure | Name | MH+ |
|---|---|---|---|
| 36 | 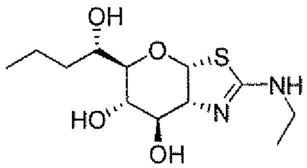 | (3aR,5R,6S,7R,7aR)-2-(ethylamino)-5-((S)-1-hydroxybutyl)-5,6,7,7a-tetrahydro-3aH-pyrano[3,2-d]thiazole-5,7-diol (Slower eluting isomer) | 291.0 |
| 37 | 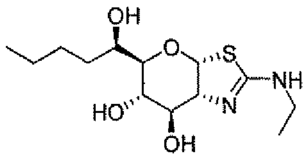 | (3aR,5R,6S,7R,7aR)-2-(ethylamino)-5-((R)-1-hydroxypentyl)-5,6,7,7a-tetrahydro-3aH-pyrano[3,2-d]thiazole-6,7-diol (Faster eluting isomer) | 305.0 |
| 38 | 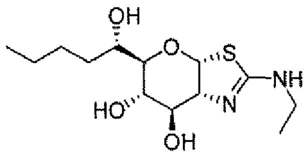 | (3aR,5R,6S,7R,7aR)-2-(ethylamino)-5-((S)-1-hydroxypentyl)-5,6,7,7a-tetrahydro-3aH-pyrano[3,2-d]thiazole-6,7-diol (Slower eluting isomer) | 305.0 |
| 39 | 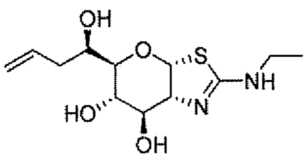 | (3aR,5R,6S,7R,7aR)-2-(ethylamino)-5-((R)-1-hydroxybut-3-enyl)-5,6,7,7a-tetrahydro-3aH-pyrano[3,2-d]thiazole-6,7-diol(Faster eluting isomer) | 289.0 |
| 40 | 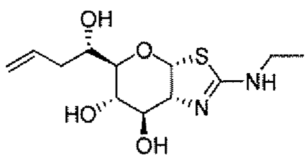 | (3aR,5R,6S,7R,7aR)-2-(ethylamino)-5-((S)-1-hydroxybut-3-enyl)-5,6,7,7a-tetrahydro-3aH-pyrano[3,2-d]thiazole-6,7-diol(Slower eluting isomer) | 289.0 |
| 41 | 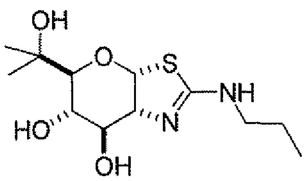 | (3aR,5S,6S,7R,7aR)-5-(2-hydroxypropan-2-yl)-2-(propylamino)-5,6,7,7a-tetrahydro-3aH-pyrano[3,2-d]thiazole-6,7-diol | 291.0 |
| 42 | 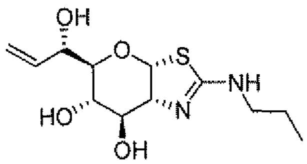 | (3aR,5R,6S,7R,7aR)-5-((S)-1-hydroxyallyl)-2-(propylamino)-5,6,7,7a-tetrahydro-3aH-pyrano[3,2-d]thiazole-5,7-diol (Slower eluting isomer) | 289.0 |
| 43 | 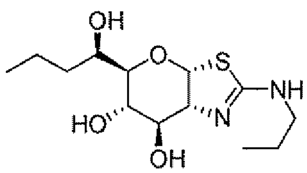 | (3aR,5R,6S,7R,7aR)-5-((R)-1-hydroxybutyl)-2-(propylamino)-5,6,7,7a-tetrahydro-3aH-pyrano[3,2-d]thiazole-6,7-diol (Faster eluting isomer) | 305.0 |
| 44 | 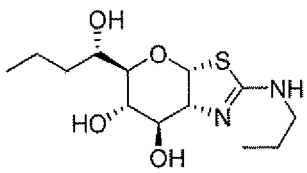 | (3aR,5R,6S,7R,7aR)-5-((S)-1-hydroxybutyl)-2-(propylamino)-5,6,7,7a-tetrahydro-3aH-pyrano[3,2-d]thiazole-6,7-diol (Slower eluting isomer) | 305.0 |

(continued)

| Example | structure | Name | MH+ |
|---|---|---|---|
| 45 | 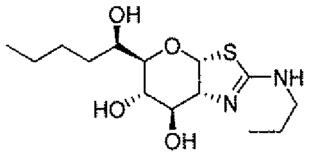 | (3aR,5R,6S,7R,7aR)-5-((R)-1-hydroxypentyl)-2-(propylamino)-5,6,7,7a-tetrahydro-3aH-pyrano[3,2-d]thiazole-6,7-diol (Faster eluting isomer) | 319.0 |
| 46 | 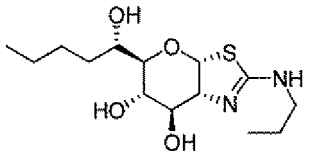 | (3aR,5R,6S,7R,7aR)-5-((S)-1-hydroxypentyl)-2-(propylamino)-5,6,7,7a-tetrahydro-3aH-pyrano[3,2-d]thiazole-6,7-diol (Slower eluting isomer) | 319.0 |
| 47 | 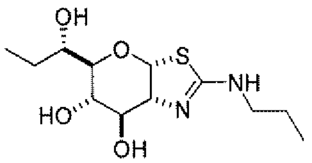 | (3aR,5R,6S,7R,7aR)-5-(1-hydroxypropyl)-2-(propylamino)-5,6,7,7a-tetrahydro-3aH-pyrano[3,2-d]thiazole-6,7-diol (Slower eluting isomer) | 291.0 |
| 48 | 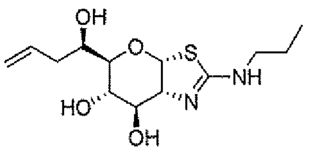 | (3aR,5R,6S,7R,7aR)-5-((R)-1-hydroxybut-3-enyl)-2-(propylamino)-5,6,7,7a-tetrahydro-3aH-pyrano[3,2-d]thiazole-6,7-diol(Faster eluting isomer) | 303.0 |
| 49 | 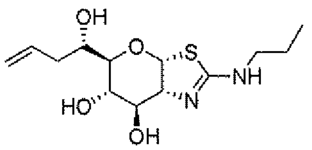 | (3aR,5R,6S,7R,7aR)-5-((S)-1-hydroxybut-3-enyl)-2-(propylamino)-5,6,7,7a-tetrahydro-3aH-pyrano[3,2-d]thiazole-6,7-diol(Slower eluting isomer) | 303.0 |
| 50 | 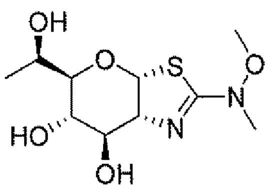 | (3aR,5R,6S,7R,7aR)-5-((R)-1-hydroxyethyl)-2-(methoxy(methyl)amino)-5,6,7,7a-tetrahydro-3aH-pyrano[3,2-d]thiazole-6,7-diol(Faster eluting isomer) | 279.0 |
| 51 | 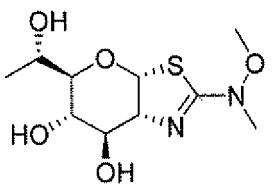 | (3aR,5R,6S,7R,7aR)-5-((S)-1-hydroxyethyl)-2-(methoxy(methyl)amino)-5,6,7,7a-tetrahydro-3aH-pyrano[3,2-d]thiazole-6,7-diol(Slower eluting isomer) | 279.0 |
| 52 | 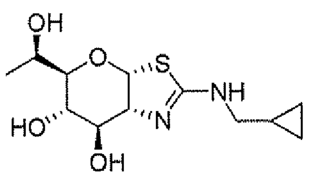 | (3aR,5R,6S,7R,7aR)-2-(cyclopropylmethylamino)-5-((R)-1-hydroxyethyl)-5,6,7,7a-tetrahydro-3aH-pyrano[3,2-d]thiazole-6,7-diol(Faster eluting isomer) | 289.0 |
| 53 | 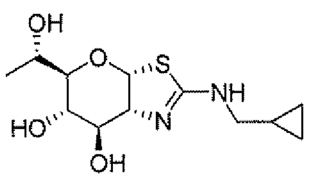 | (3aR,5R,6S,7R,7aR)-2-(cyclopropylmethylamino)-5-((S)-1-hydroxyethyl)-5,6,7,7a-tetrahydro-3aH-pyrano[3,2-d]thiazole-6,7-diol(Slower eluting isomer) | 289.0 |

(continued)

| Example | structure | Name | MH+ |
|---|---|---|---|
| 54 | 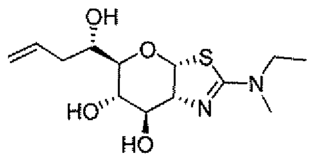 | (3aR,5R,6S,7R,7aR)-2-(ethyl(methyl)amino)-5-((S)-1-hydroxybut-3-enyl)-5,6,7,7a-tetrahydro-3aH-pyrano[3,2-d]thiazole-6,7-diol (Slower eluting isomer) | 303.0 |
| 55 | 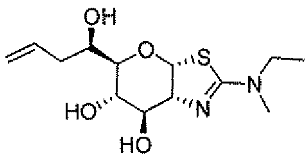 | (3aR,5R,6S,7R,7aR)-2-(ethyl(methyl)amino)-5-((R)-1-hydroxybut-3-enyl)-5,6,7,7a-tetrahydro-3aH-pyrano[3,2-d]thiazole-6,7-diol (Faster eluting isomer) | 303.0 |
| 56 | 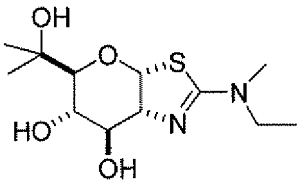 | (3aR,5S,6S,7R,7aR)-2-(ethyl(methyl)amino)-5-(2-hydroxypropan-2-yl)-5,6,7,7a-tetrahydro-3aH-pyrano[3,2-d]thiazole-6,7-diol | 290.9 |
| 57 | 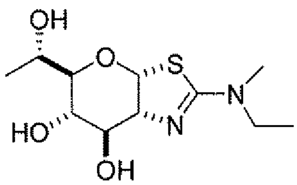 | (3aR,5R,6S,7R,7aR)-2-(ethyl(methyl)amino)-5-(1-hydroxyethyl)-5,6,7,7a-tetrahydro-3aH-pyrano[3,2-d]thiazole-6,7-diol (Slower eluting isomer) | 276.9 |
| 58 | 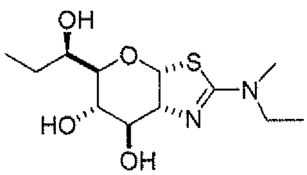 | (3aR,5R,6S,7R,7aR)-2-(ethyl(methyl)amino)-5-((R)-1-hydroxypropyl)-5,6,7,7a-tetrahydro-3aH-pyrano[3,2-d]thiazole-6,7-diol (Faster eluting isomer) | 291.0 |
| 59 | 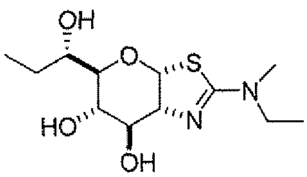 | (3aR,5R,6S,7R,7aR)-2-(ethyl(methyl)amino)-5-(1-hydroxypropyl)-5,6,7,7a-tetrahydro-3aH-pyrano[3,2-d]thiazole-6,7-diol (Slower eluting isomer) | 290.9 |
| 60 | 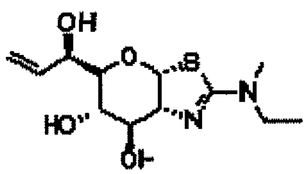 | (3aR,5S,6S,7R,7aR)-2-(ethyl(methyl)amino)-5-((R)-1-hydroxyallyl)-5,6,7,7a-tetrahydro-3aH-pyrano[3,2-d]thiazole-6,7-diol (Faster eluting isomer) | 289.0 |
| 61 | 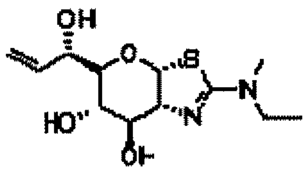 | (3aR,5S,6S,7R,7aR)-2-(ethyl(methyl)amino)-5-(1-hydroxyallyl)-5,6,7,7a-tetrahydro-3aH-pyrano[3,2-d]thiazole-6,7-diol (Slower eluting isomer) | 289.0 |

(continued)

| Example | structure | Name | MH+ |
|---|---|---|---|
| 62 | 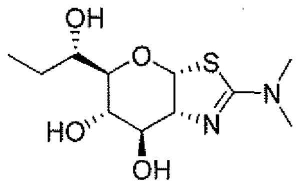 | (3aR,5R,6S,7R,7aR)-2-(dimethylamino)-5-((S)-1-hydroxypropyl)-5,6,7,7a-tetrahydro-3aH-pyrano[3,2-d]thiazole-6,7-diol (Slower eluting isomer) | 276.9 |
| 63 | 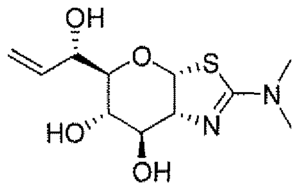 | (3aR,5R,6S,7R,7aR)-2-(dimethylamino)-5-((S)-1-hydroxyallyl)-5,6,7,7a-tetrahydro-3aH-pyrano[3,2-d]thiazole-6,7-diol (Slower eluting isomer) | 274.9 |
| 64 | 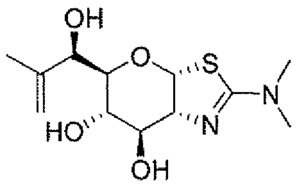 | (3aR,5R,6S,7R,7aR)-2-(dimethylamino)-5-((R)-1-hydroxy-2-methylallyl)-5,6,7,7a-tetrahydro-3aH-pyrano[3,2-d]thiazole-6,7-diol(Faster eluting isomer) | 289.0 |
| 65 | 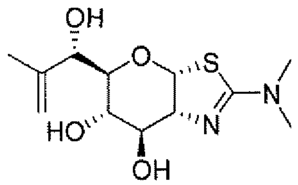 | (3aR,5R,6S,7R,7aR)-2-(dimethylamino)-5-((S)-1-hydroxy-2-methylallyl)-5,6,7,7a-tetrahydro-3aH-pyrano[3,2-d]thiazole-6,7-diol(Slower eluting isomer) | 289.0 |
| 66 | 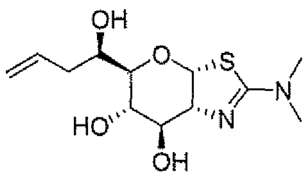 | (3aR,5R,6S,7R,7aR)-2-(dimethylamino)-5-((R)-1-hydroxybut-3-enyl)-5,6,7,7a-tetrahydro-3aH-pyrano[3,2-d]thiazole-6,7-diol(Faster eluting isomer) | 289.0 |
| 67 | 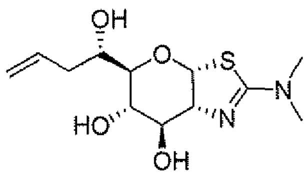 | (3aR,5R,6S,7R,7aR)-2-(dimethylamino)-5-((S)-1-hydroxybut-3-enyl)-5,6,7,7a-tetrahydro-3aH-pyrano[3,2-d]thiazole-6,7-diol(Slower eluting isomer) | 289.0 |
| 68 | 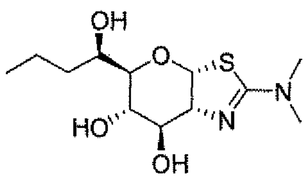 | (3aR,5R,6S,7R,7aR)-2-(dimethylamino)-5-((R)-1 -hydroxybutyl)-5,6,7,7a-tetrahydro-3aH-pyrano[3,2-d]thiazole-6,7-diol (Faster eluting isomer) | 291.0 |
| 69 | 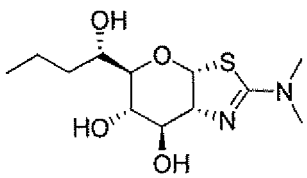 | (3aR,5R,6S,7R,7aR)-2-(dimethylamino)-5-((S)-1-hydroxybutyl)-5,6,7,7a-tetrahydro-3aH-pyrano[3,2-d]thiazole-6,7-diol (Slower eluting isomer) | 291.0 |

(continued)

| Example | structure | Name | MH+ |
|---|---|---|---|
| 70 | 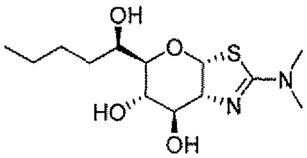 | (3aR,5R,6S,7R,7aR)-2-(dimethylamino)-5-((R)-1-hydroxypentyl)-5,6,7,7a-tetrahydro-3aH-pyrano[3,2-d]thiazole-6,7-diol (Faster eluting isomer) | 305.0 |
| 71 | 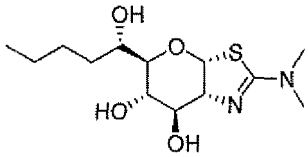 | (3aR,5R,6S,7R,7aR)-2-(dimethylamino)-5-((S)-1-hydroxypentyl)-5,6,7,7a-tetrahydro-3aH-pyrano[3,2-d]thiazole-6,7-diol (Slower eluting isomer) | 305.0 |
| 72 | 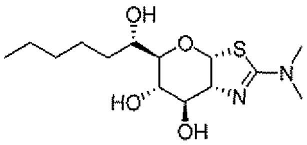 | (3aR,5R,6S,7R,7aR)-2-(dimethylamino)-5-((S)-1-hydroxyhexyl)-5,6,7,7a-tetrahydro-3aH-pyrano[3,2-d]thiazole-6,7-diol(Slower eluting isomer) | 319.0 |
| 73 | 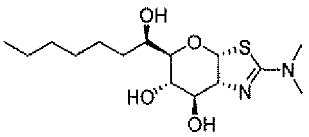 | (3aR,5R,6S,7R,7aR)-2-(dimethylamino)-5-((R)-1-hydroxyheptyl)-5,6,7,7a-tetrahydro-3aH-pyrano[3,2-d]thiazole-6,7-diol(Faster eluting isomer) | 333.0 |
| 74 | 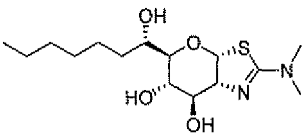 | (3aR,5R,6S,7R,7aR)-2-(dimethylamino)-5-((S)-1-hydroxyheptyl)-5,6,7,7a-tetrahydro-3aH-pyrano[3,2-d]thiazole-6,7-diol(Slower eluting isomer) | 333.0 |
| 75 | 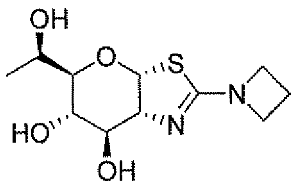 | (3aR,5R,6S,7R,7aR)-2-(azetidin-1-yl)-5-((R)-1-hydroxyethyl)-5,6,7,7a-tetrahydro-3aH-pyrano[3,2-d]thiazole-6,7-diol (Faster eluting isomer) | 274.9 |
| 76 | 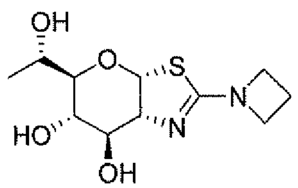 | (3aR,5R,6S,7R,7aR)-2-(azetidin-1-yl)-5-((S)-1-hydroxyethyl)-5,6,7,7a-tetrahydro-3aH-pyrano[3,2-d]thiazole-6,7-diol (Slower eluting isomer) | 274.9 |
| 77 | 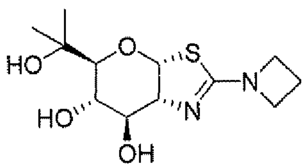 | (3aR,5S,6S,7R,7aR)-2-(azetidin-1-yl)-5-(2-hydroxypropan-2-yl)-5,6,7,7a-tetrahydro-3aH-pyrano[3,2-d]thiazole-6,7-diol | 288.9 |
| 78 | 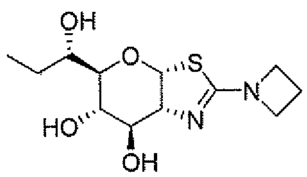 | (3aR,5R,6S,7R,7aR)-2-(azetidin-1-yl)-5-((S)-1-hydroxypropyl)-5,6,7,7a-tetrahydro-3aH-pyrano[3,2-d]thiazole-6,7-diol (Slower eluting isomer) | 288.9 |

(continued)

| Example | structure | Name | MH+ |
|---|---|---|---|
| 79 | 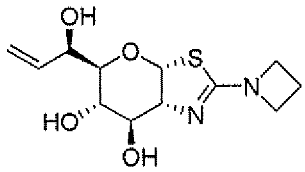 | (3aR,5R,6S,7R,7aR)-2-(azetidin-1-yl)-5-((R)-1-hydroxyallyl)-5,6,7,7a-tetrahydro-3aH-pyrano[3,2-d]thiazole-6,7-diol(Faster eluting isomer) | 286.9 |
| 80 | 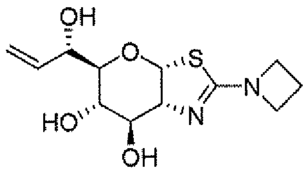 | (3aR,5R,6S,7R,7aR)-2-(azetidin-1-yl)-5-((S)-1-hydroxyallyl)-5,6,7,7a-tetrahydro-3aH-pyrano[3,2-d]thiazole-6,7-diol(Slower eluting isomer) | 286.9 |
| 81 | 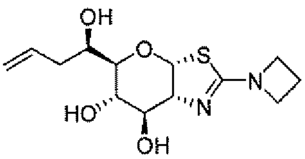 | (3aR,5R,6S,7R,7aR)-2-(azetidin-1-yl)-5-((R)-1-hydroxybut-3-enyl)-5,6,7,7a-tetrahydro-3aH-pyrano[3,2-d]thiazole-6,7-diol(Faster eluting isomer) | 301.0 |
| 82 | 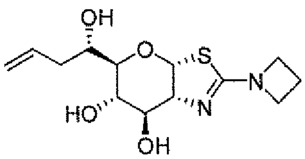 | (3aR,5R,6S,7R,7aR)-2-(azetidin-1-yl)-5-((S)-1-hydroxybut-3-enyl)-5,6,7,7a-tetrahydro-3aH-pyrano[3,2-d]thiazole-6,7-diol(Slower eluting isomer) | 301.0 |
| 83 | 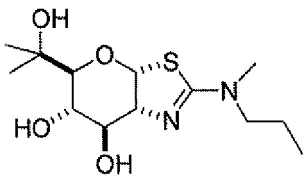 | (3aR,5S,6S,7R,7aR)-5-(2-hydroxypropan-2-yl)-2-(methyl(propyl)amino)-5,6,7,7a-tetrahydro-3aH-pyrano[3,2-d]thiazole-6,7-diol | 305.0 |
| 84 | 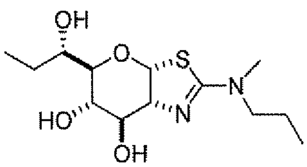 | (3aR,5R,6S,7R,7aR)-5-(1-hydroxypropyl)-2-(methyl(propyl)amino) -5,6,7,7a-tetrahydro-3aH-pyrano[3,2-d]thiazole-6,7-diol (Slower eluting isomer) | 305.0 |
| 85 | 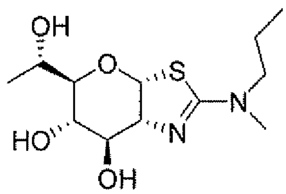 | (3aR,5R,6S,7R,7aR)-5-((S)-1-hydroxyethyl)-2-(methyl(propyl)amino)-5,6,7,7a-tetrahydro-3aH-pyrano[3,2-d]thiazole-6,7-diol(Slower eluting isomer) | 291.0 |
| 86 | 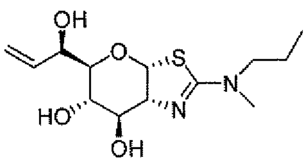 | (3aR,5R,6S,7R,7aR)-5-((R)-1-hydroxyallyl)-2-(methyl(propyl)amino)-5,6,7,7a-tetrahydro-3aH-pyrano[3,2-d]thiazole-6,7-diol(Faster eluting isomer) | 303.0 |

(continued)

| Example | structure | Name | MH+ |
|---|---|---|---|
| 87 | 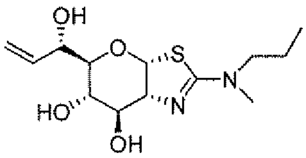 | (3aR,5R,6S,7R,7aR)-5-((S)-1-hydroxyallyl)-2-(methyl(propyl)amino)-5,6,7,7a-tetrahydro-3aH-pyrano[3,2-d]thiazole-6,7-diol(Slower eluting isomer) | 303.0 |
| 88 | 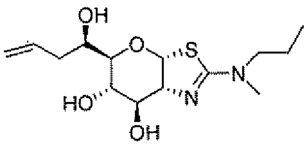 | (3aR,5R,6S,7R,7aR)-5-((R)-1-hydroxybut-3-enyl)-2-(methyl(propyl)amino)-5,6,7,7a-tetrahydro-3aH-pyrano[3,2-d]thiazole-6,7-diol(Faster eluting isomer) | 317.0 |
| 89 | 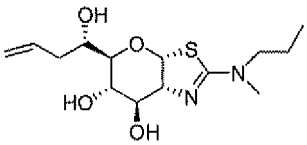 | (3aR,5R,6S,7R,7aR)-5-((S)-1-hydroxybut-3-enyl)-2-(methyl(propyl)amino)-5,6,7,7a-tetrahydro-3aH-pyrano[3,2-d]thiazole-6,7-diol(Slower eluting isomer) | 317.0 |
| 90 | 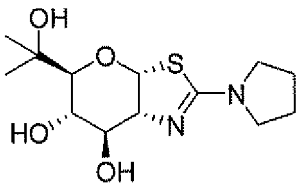 | (3aR,5S,6S,7R,7aR)-5-(2-hydroxypropan-2-yl)-2-(pyrrolidin-1-yl)-5,6,7,7a-tetrahydro-3aH-pyrano[3,2-d]thiazole-6,7-diol | 302.9 |
| 91 | 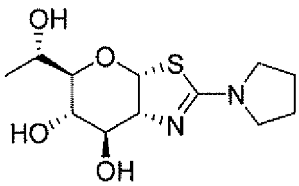 | (3aR,5R,6S,7R,7aR)-5-((S)-1-hydroxyethyl)-2-(pyrrolidin-1-yl)-5,6,7,7a-tetrahydro-3aH-pyrano [3,2-d]thiazole-6,7-diol (Slower eluting isomer) | 289.0 |
| 92 | 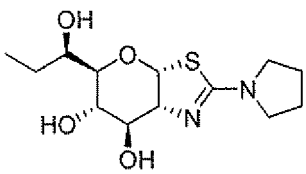 | (3aR,5R,6S,7R,7aR)-5-((R)-1-hydroxypropyl)-2-(pyrrolidin-1-yl)-5,6,7,7a-tetrahydro-3aH-pyrano[3,2-d]thiazole-6,7-diol (Faster eluting isomer) | 302.9 |
| 93 | 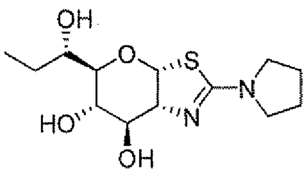 | (3aR,5R,6S,7R,7aR)-5-((S)-1-hydroxypropyl)-2-(pyrrolidin-1-yl)-5,6,7,7a-tetrahydro-3aH-pyrano[3,2-d]thiazole-6,7-diol (Slower eluting isomer) | 302.9 |
| 94 | 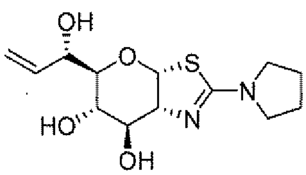 | (3aR,5R,6S,7R,7aR)-5-((S)-1-hydroxyallyl)-2-(pyrrolidin-1-yl)-5,6,7,7a-tetrahydro-3aH-pyrano[3,2-d]thiazole-6,7-diol(Slower eluting isomer) | 301.0 |

(continued)

| Example | structure | Name | MH+ |
|---|---|---|---|
| 95 | 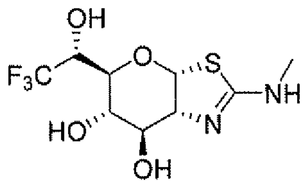 | (3aR,5S,6S,7R,7aR)-2-(methylamino)-5-((R)-2,2,2-trifluoro-1-hydroxyethyl)-5,6,7,7a-tetrahydro-3aH-pyrano[3,2-d]thiazole-6,7-diol | 303.0 |
| 97 | 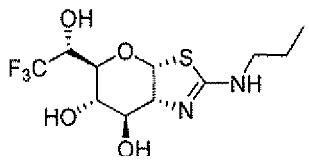 | (3aR,5S,6S,7R,7aR)-2-(propylamino)-5-((R)-2,2,2-trifluoro-1-hydroxyethyl)-5,6,7,7a-tetrahydro-3aH-pyrano[3,2-d]thiazole-6,7-diol | 331.0 |
| 98 | 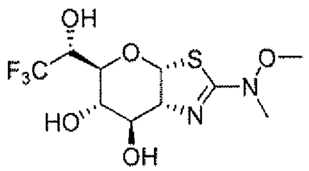 | (3aR,5S,6S,7R,7aR)-2-(methoxy(methyl)amino)-5-((R)-2,2,2-trifluoro-1-hydroxyethyl)-5,6,7,7a-tetrahydro-3aH-pyrano[3,2-d]thiazole-6,7-diol | 332.9 |
| 99 | 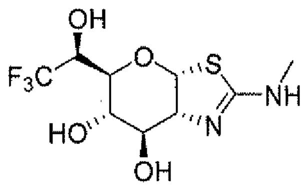 | (3aR,5S,6S,7R,7aR)-2-(methylamino)-5-((S)-2,2,2-trifluoro-1-hydroxyethyl)-5,6,7,7a-tetrahydro-3aH-pyrano[3,2-d]thiazole-6,7-diol | 303.0 |
| | | | |
| 101 | 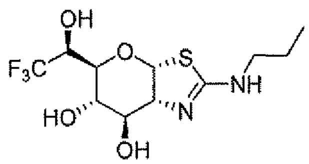 | (3aR,5S,6S,7R,7aR)-2-(propylamino)-5-((S)-2,2,2-trifluoro-1-hydroxyethyl)-5,6,7,7a-tetrahydro-3aH-pyrano[3,2-d]thiazole-6,7-diol | 331.0 |
| 102 | 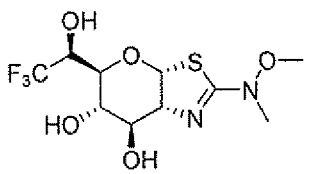 | (3aR,5S,6S,7R,7aR)-2-(methoxy(methyl)amino)-5-((S)-2,2,2-trifluoro-1-hydroxyethyl)-5,6,7,7a-tetrahydro-3aH-pyrano[3,2-d]thiazole-6,7-diol | 332.9 |
| 103 | 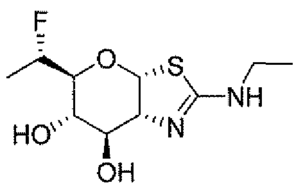 | (3aR,5S,6S,7R,7aR)-2-(ethylamino)-5-((S)-1-fluoroethyl)-5,6,7,7a-tetrahydro-3aH-pyrano[3,2-d]thiazole-6,7-diol | 265.7 |
| 104 | 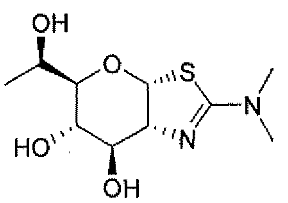 | (3aR,5R,6S,7R,7aR)-2-(dimethylamino)-5-((R)-1-hydroxyethyl)-5,6,7,7a-tetrahydro-3aH-pyrano[3,2-d]thiazole-6,7-diol | 262.9 |

(continued)

| Example | structure | Name | MH+ |
|---|---|---|---|
| 105 | 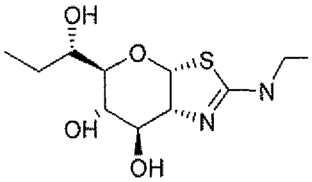 | (3aR,5R,6S,7R,7aR)-2-(ethylamino)-5-((S)-1-hydroxypropyl)-5,6,7,7a-tetrahydro-3aH-pyrano[3,2-d]thiazole-6,7-diol | 277.2 |
| 106 | 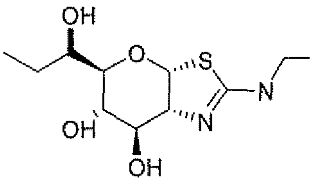 | (3aR,5R,6S,7R,7aR)-2-(ethylamino)-S-((R)-1-hydroxypropyl)-5,6,7,7a-tetrahydro-3aH-pyrano[3,2-d]thiazole-6,7-diol | 277.2 |
| 107 | 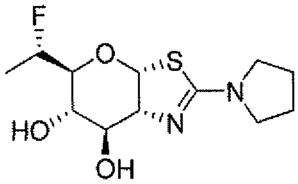 | (3aR,5S,6S,7R,7aR)-5-((S)-1-fluoroethyl)-2-(pyrrolidin-1-yl)-5,6,7,7a-tetrahydro-3aH-pyrano[3,2-d]thiazole-6,7-diol(Slower eluting isomer) | 291.0 |
| 108 | 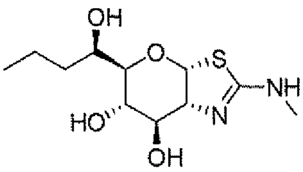 | (3aR,5R,6S,7R,7aR)-5-((R)-1-hydroxybutyl)-2-(methylamino)-5,6,7,7a-tetrahydro-3aH-pyrano[3,2-d]thiazole-6,7-diol(Faster eluting isomer) | 305.0 |
| 109 | 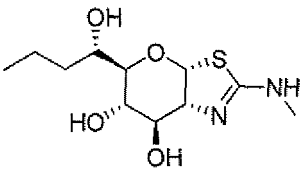 | (3aR,5R,6S,7R,7aR)-5-((S)-1-hydroxybutyl)-2-(methylamino)-5,6,7,7a-tetrahydro-3aH-pyrano[3,2-d]thiazole-6,7-diol(Slower eluting isomer) | 305.0 |
| 110 | 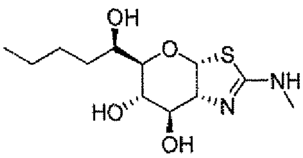 | (3aR,5R,6S,7R,7aR)-5-(R)-1-hydroxypentyl)-2-(methylamino)-5,6,7,7a-tetrahydro-3aH-pyrano[3,2-d]thiazole-6,7-diol(Faster eluting isomer) | 291.0 |
| 111 | 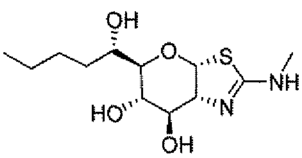 | (3aR,5R,6S,7R,7aR)-5-((S)-1-hydroxypentyl)-2-(methylamino)-5,6,7,7a-tetrahydro-3aH-pyrano[3,2-d]thiazole-6,7-diol(Slower eluting isomer) | 291.0 |
| 112 | 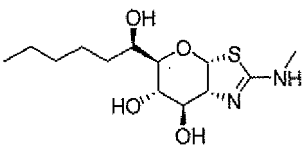 | (3aR,5R,6S,7R,7aR)-5-((R)-1-hydroxyhexyl)-2-(methylamino)-5,6,7,7a-tetrahydropyrano[3,2-d]thiazole-6,7-diol(Faster eluting isomer) | 305.0 |

(continued)

| Example | structure | Name | MH+ |
|---|---|---|---|
| 113 | 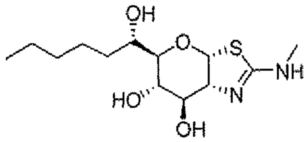 | (3aR,5R,6S,7R,7aR)-5-((S)-1-hydroxyhexyl)-2-(methylamino)-5,6,7,7a-tetrahydro-3aH-pyrano[3,2-d]thiazole-6,7-diol(Slower eluting isomer) | 305.0 |
| 114 | 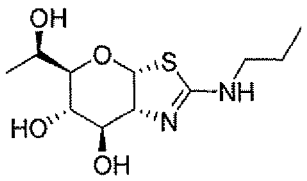 | (3aR,5R,6S,7R,7aR)-5-((R)-1-hydroxyethyl)-2-(propylamino)-5,6,7,7a-tetrahydro-3aH-pyrano[3,2-d]thiazole-6,7-diol(Faster eluting isomer) | 277.0 |
| 115 | 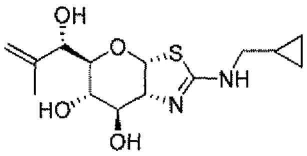 | (3aR,5R,6S,7R,7aR)-2-(cyclopropylmethylamino)-5-((S)-1-hydroxy-2-methylallyl)-5,6,7,7a-tetrahydro-3aH-pyrano[3,2-d]thiazole-6,7-diol(Slower eluting isomer) | 315.0 |
| 116 | 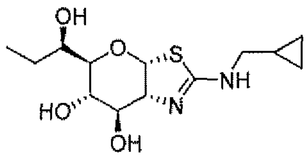 | (3aR,5R,6S,7R,7aR)-2-(cyclopropylmethylamino)-5-((R)-1-hydroxypropyl)-5,6,7,7a-tetrahydro-3aH-pyrano[3,2-d]thiazole-6,7-diol(Faster eluting isomer) | 303.0 |
| 117 | 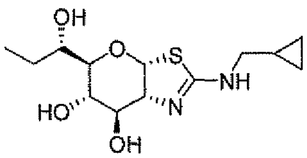 | (3aR,5R,6S,7R,7aR)-2-(cyclopropylmethylamino)-5-((S)-1-hydroxypropyl)-5,6,7,7a-tetrahydro-3aH-pyrano[3,2-d]thiazole-6,7-diol(Slower eluting isomer) | 303.0 |
| 118 | 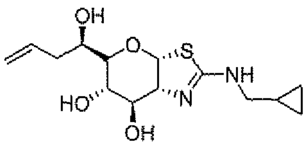 | (3aR,5S,6S,7R,7aR)-2-(cyclopropylmethylamino)-5-((R)-1-hydroxybut-3-enyl)-5,6,7,7a-tetrahydro-3aH-pyrano[3,2-d]thiazole-6,7-diol(Faster eluting isomer) | 315.0 |
| 119 | 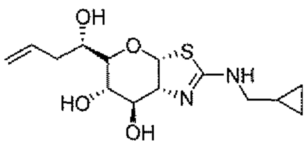 | (3aR,5S,6S,7R,7aR)-2-(cyclopropylmethylamino)-5-((S)1-hydroxybut-3-enyl)-5,6,7,7a-tetrahydro-3aH-pyrano[3,2-d]thiazole-6,7-diol(Slower eluting isomer) | 315.0 |
| 120 | 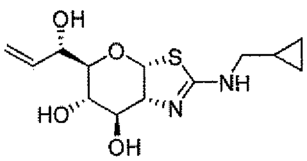 | (3aR,5R,6S,7R,7aR)-2-(cyclopropylmethylamino)-5-((S)-1-hydroxyallyl)-5,6,7,7a-tetrahydro-3aH-pyrano[3,2-d]thiazole-6,7-diol(Slower eluting isomer) | 301.0 |
| 121 | 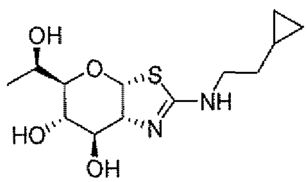 | (3aR,5R,6S,7R,7aR)-2-(2-cyclopropylethylamino)-5-((R)-1-hydroxyethyl)-5,6,7,7a-tetrahydro-3aH-pyrano[3,2-d]thiazole-6,7-diol(Faster eluting isomer) | 303.0 |

(continued)

| Example | structure | Name | MH+ |
|---|---|---|---|
| 122 | 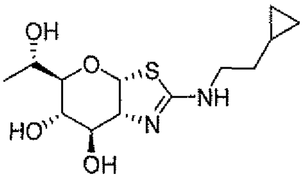 | (3aR,5R,6S,7R,7aR)-2-(2-cyclopropylethylamino)-5-((S)-1-hydroxyethyl)-5,6,7,7a-tetrahydro-3aH-pyrano[3,2-d]thiazole-6,7-diol(Slower eluting isomer) | 303.0 |
| 123 | 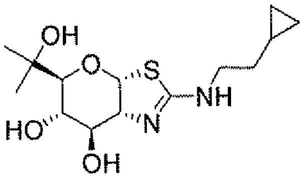 | (3aR,5S,6S,7R,7aR)-2-(2-cyclopropylethylamino)-5-(2-hydroxypropan-2-yl)-5,6,7,7a-tetrahydro-3aH-pyrano[3,2-d]thiazole-6,7-diol | 317.0 |
| 124 | 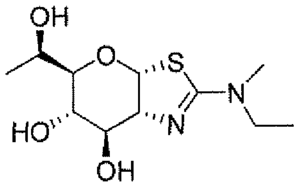 | (3aR,5R,6S,7R,7aR)-2-(ethyl(methyl)amino)-5-((R)-1-hydroxyethyl)-5,6,7,7a-tetrahydro-3aH-pyrano[3,2-d]thiazole-6,7-diol(Faster eluting isomer) | 276.9 |
| 125 | 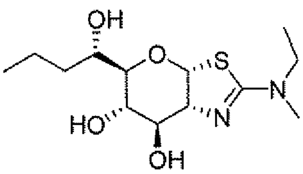 | (3aR,5R,6S,7R,7aR)-2-(ethyl(methyl)amino)-5-((S)-1-hydroxybutyl)-5,6,7,7a-tetrahydro-3aH-pyrano[3,2-d]thiazole-6,7-diol(Slower eluting isomer) | 305.0 |
| 126 | 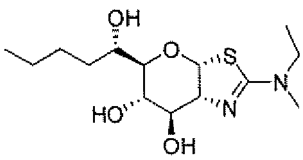 | (3aR,SR,6S,7R,7aR)-2-(ethyl(methyl)amino)-5-5,6,7,7a-tetrahydro-3aH-pyrano[3,2-d]thiazole-6,7-diol(Slower eluting isomer) | 319.0 |
| 127 | 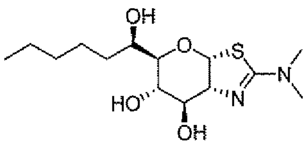 | (3aR,5R,6S,7R,7aR)-2-(dimethylamino)-5-((R)-1-hydroxyhexyl)-5,6,7,7a-tetrahydro-3aH-pyrano[3,2-d]thiazole-6,7-diol(Faster eluting isomer) | 319.0 |
| 128 | 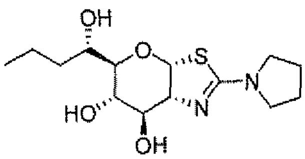 | (3aR,5R,6S,7R,7aR)-5-(S)-1-hydroxybutyl)-2-(pyrrolidin-1-yl)-5,6,7,7a-tetrahydro-3aH-pyrano[3,2-d]thiazole-6,7-diol(Slower eluting isomer) | 317.0 |
| 129 | 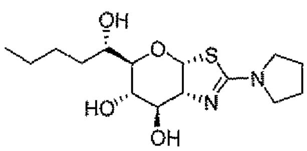 | (3aR,5R,6S,7R,7aR)-5-((S)-1-hydroxypentyl)-2-(pyrrolidin-1-yl)-5,6,7,7a-tetrahydro-3aH-pyrano[3,2-d]thiazole-6,7-diol(Slower eluting isomer) | 331.0 |

(continued)

| Example | structure | Name | MH+ |
|---|---|---|---|
| 130 | 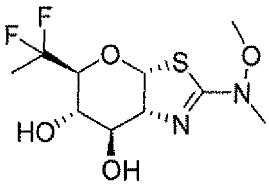 | (3aR,5S,6S,7R,7aR)-5-(1,1-difluoroethyl)-2-(methoxy(methyl)amino)-5,6,7,7a-tetrahydro-3aH-pyrano[3,2-d]thiazole-6,7-diol | 299.0 |
| 131 | 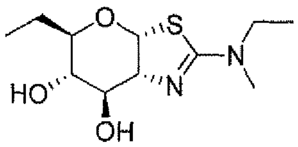 | (3aR,5R,6S,7R,7aR)-5-ethyl-2-5,6,7,7a-tetrahydro-3aH-pyrano[3,2-d]thiazole-6,7-diol | 261.0 |
| 132 | 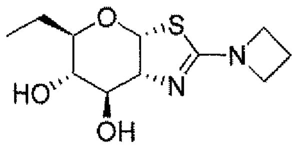 | (3aR,5R,6S,7R,7aR)-2-(azetidin-1-yl)-5-ethy-5,6,7,7a-tetrahydro-3aH-pyrano[3,2-d]thiazole-6,7-diol | 259.0 |
| 133 | 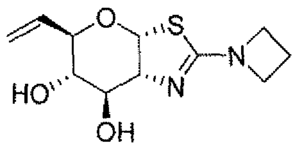 | (3aR,5R,6S,7R,7aR)-2-(azetidin-1-yl)-5-vinyl-5,6,7,7a-tetrahydro-3aH-pyrano[3,2-d]thiazole-6,7-diol | 257.0 |
| 134 | 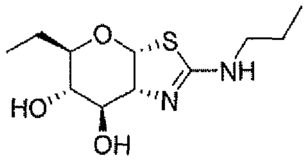 | (3aR,5R,6S,7R,7aR)-5-ethyl-2-(propylamino)-5,6,7,7a-tetrahydro-3aH-pyrano[3,2-d]thiazole-6,7-diol | 261.0 |
| 135 | 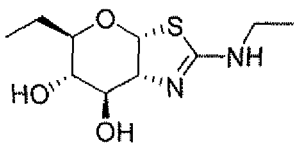 | (3aR,5R,6S,7R,7aR)-5-ethyl-2-(ethylamino)-5,6,7,7a-tetrahydro-3aH-pyrano[3,2-d]thiazole-6,7-diol | 247.0 |
| 136 | 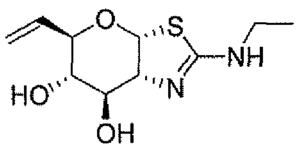 | (3aR,5R,6S,7R,7aR)-2-(ethylamino)-5 -vinyl-5,6,7,7a-tetrahydro-3aH-pyrano[3,2-d]thiazole-6,7-diol | 245.0 |

**Examples 137 and 138**

**(3aR,5S,6S,7R,7aR)-5-(1-hydroxycyclopentyl)-2-(methylamino)-5,6,7,7a-tetrahydro-3aH-pyrano[3,2-d]thiazole-6,7-diol and (3aR,5S,6S,7R,7aR)-5-(1-hydroxycyclopent-3-enyl)-2-(methylamino)-5,6,7,7a-tetrahydro-3aH-pyrano[3,2-d]thiazole-6,7-diol**

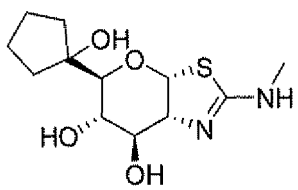

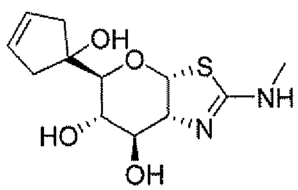

**Scheme XII**

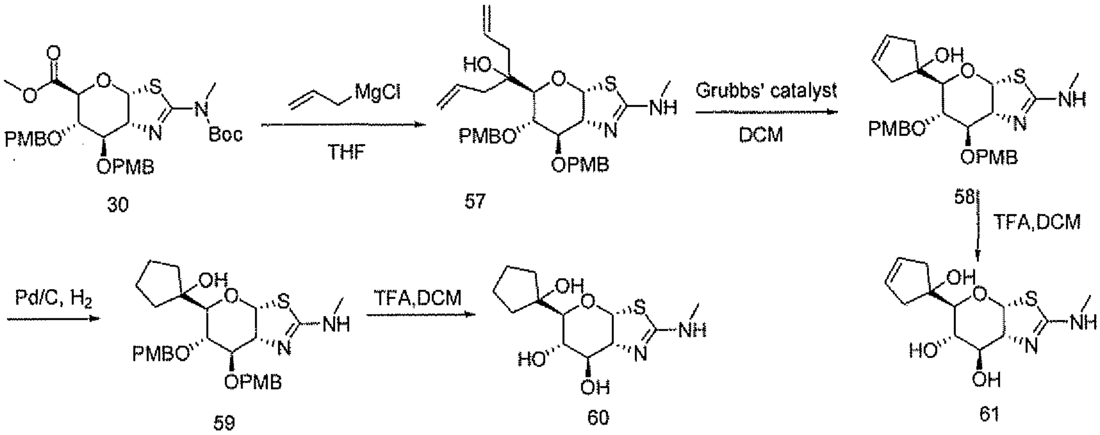


30 57 58 59 60 61

**Step 1**

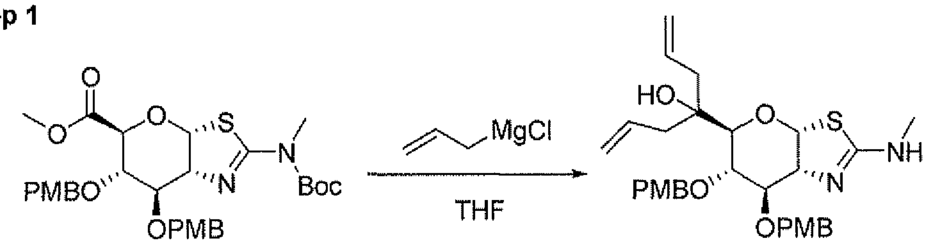


30 57

**4-((3aR,5S,6S,7R,7aR)-6,7-bis(4-methoxybenzyloxy)-2-(methylamino)-5,6,7,7a-tetrahydro-3aH-pyrano[3,2-d]thiazol-5-yl)hepta-1,6-dien-4-ol.** A solution of (3aR,5S,6S,7R,7aR)-methyl2-(tert-butoxycarbonyl(methyl)amino)-6,7-bis(4-metho xybenzyloxy)-5,6,7,7a-tetrahydro-3aH-pyrano[3,2-d]thiazole-5-carboxylate (1.0 g, 1.66 mmol) in THF (15 mL) was treated with allylmagnesium chloride (8.0 mL, 0.5 M in THF) for 1 hour at room temperature. The reaction was quenched by aqueous $NH_4Cl$ (20 mL), and extracted with ethyl acetate (2 x 30 mL). The combined organic layers were dried over anhydrous sodium sulfate and concentrated under reduced pressure to give a residue, which was purified by a silica gel column with 1 % methanol in dichloromethane to afford the title compound as a yellow oil (0.75 g, 81 %). (ES, *m/z*) $[M+H]^+$ 555.0; $^1H$ NMR (300 MHz, $CDCl_3$) $\delta$ 7.23 - 7.35 (m, 4H), 6.84 - 6.93 (m, 4H), 6.34 - 6.37 (d, *J*= 6.9 Hz, 1H), 5.86 - 5.92 (m, 2H), 5.06 - 5.11 (m, 4H), 4.65-4.66 (m, 2H), 4.50 - 4.54 (m, 2H), 4.26 - 4.33 (m, 2H), 3.96 - 3.99 (d, *J* = 8.4 Hz, 1H), 3.81 - 3.83 (m, 6H), 3.51 - 3.54 (d, *J* = 8.4 Hz, 1H), 2.92 (s, 3H), 2.22 - 2.31 (m, 4H).

**Step 2**

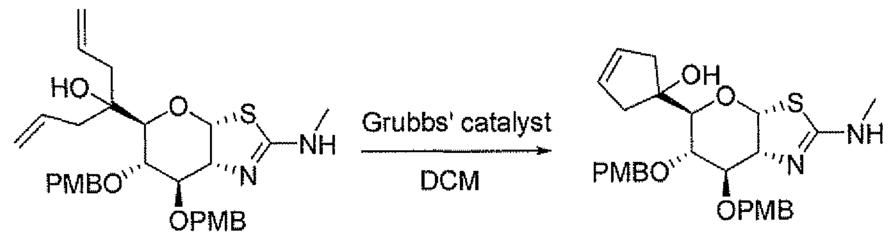


57 58

**1-((3aR,5S,6S,7R,7aR)-6,7-bis(4-methoxybenzyloxy)-2-(methylamino)-5,6,7,7a-tetrahydro-3aH-pyrano[3,2-d]thiazol-5-yl)cyclopent-3-enol.** To a solution of compound **57** (0.60 g, 1.08 mmol) in dichloromethane (10 mL) was added Grubbs' catalyst (0.19 g, 0.22 mmol) under nitrogen atmosphere. After stirring for 12 hours at room temperature, the reaction mixture was concentrated under reduced pressure to afford a residue, which was purified by a silica gel column with 30 % - 40 % ethyl acetate in petroleum ether to afford compound **58** as a grey oil (0.35 g, 61 %). (ES, *m/z*) $[M+H]^+$ 527.0; $^1H$ NMR (300 MHz, $CDCl_3$) $\delta$ 7.24 - 7.35 (m, 4H), 6.85 - 6.92 (m, 4H), 6.34 - 6.36 (d, *J* = 6.9 Hz, 1H), 5.66 - 5.67 (m, 2H), 4.49 - 4.65 (m, 4H), 4.28 - 4.31 (m, 2H), 3.90 - 3.93 (d, *J* = 8.4 Hz, 1H), 3.81 - 3.82 (m, 6H), 3.55 - 3.57 (d, *J* = 8.4 Hz, 1H), 2.92 (s, 3H), 2.58 - 2.78 (m, 2H), 2.23 - 2.34 (m, 2H).

Step 3

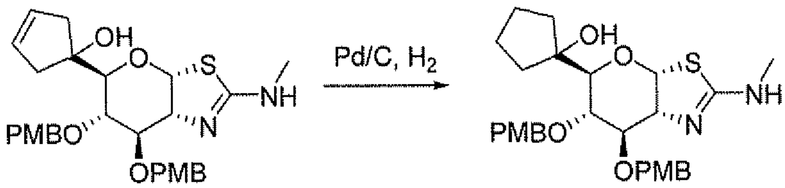


58 59

**1-((3aR,5S,6S,7R,7aR)-6,7-bis(4-methoxybenzyloxy)-2-(methylamino)-5,6,7,7a-tetrahydro-3aH-pyrano[3,2-d]thiazol-5-yl)cyclopentanol.** A mixture of compound 58 (0.35 g, 0.67 mmol) and Pd/C (0.10 g) in methanol (20 mL) was stirred under hydrogen atmosphere for 20 hours at room temperature. The reaction mixture was filtered and the filtrate was concentrated under vacuum to afford a residue, which was purified by a silica gel column with 30 % - 40 % ethyl acetate in petroleum ether to afford crude compound 59 as a yellow oil (300 mg), which was employed in the next step without further purification. $[M+H]^+$ 529.0.

**Step 4**

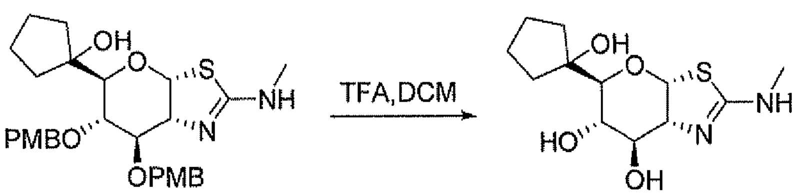


59 60

**(3aR,5S,6S,7R,7aR)-5-(1-hydroxycyclopentyl)-2-(methylamino)-5,6,7,7a-tetrahydro-3aH-pyrano[3,2-d]thiazole-6,7-diol :** A solution of the above compound **59** (100 mg) in dichloromethane (5 mL) was treated with TFA (0.5 mL) for 4 hours at room temperature. The reaction solution was concentrated under reduced pressure. The residue was purified by Prep-HPLC with the following conditions [(Agilent 1200 prep HPLC): Column, SunFire Prep C18,19 * 50 mm 5 um; mobile phase, $H_2O$ with 0.03 % $NH_4OH$ and $CH_3CN$ (10 % $CH_3CN$ up to 45 % in 10 min); Detector, UV 220 nm] to afford compound **60 (Example 137)** as a white solid (5.0 mg). (ES, *m/z*): $[M+H]^+$ 289.0; $^1H$ NMR (300 MHz, $D_2O$) $\delta$ 6.22 - 6.24 (d, *J* = 6.9 Hz, 1H), 4.29 - 4.32 (m, 1H), 4.12 - 4.14 (m, 1H), 3.80 - 3.83 (m, 1H), 3.30 - 3.33 (d, *J* = 8.4 Hz, 1H), 2.74 (s, 3H), 1.51 -1.66 (m, 8H).

**Step 5**

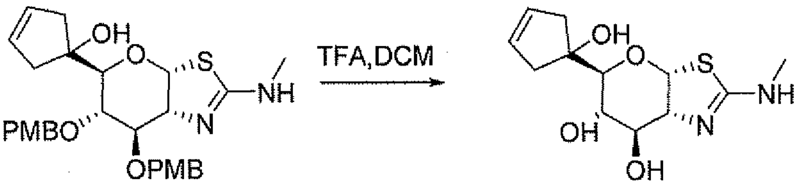


58 61

**(3aR,5S,6S,7R,7aR)-5-(1-hydroxycyclopent-3-enyl)-2-(methylamino)-5,6,7,7a-tetrahydro-3aH-pyrano[3,2-d]thiazole-6,7-diol** : A solution of compound **58** (100 mg, 0.19 mmol)) in dichloromethane (5 mL) was treated with TFA (0.5 mL) for 4 hours at room temperature. The reaction solution was concentrated under reduced pressure. The residue was purified by Prep-HPLC with the following conditions [(Agilent 1200 prep HPLC): Column, SunFire Prep C18,19 * 50 mm 5 um; mobile phase, $H_2O$ with 0.03 % $NH_4OH$ and $CH_3CN$ (10 % $CH_3CN$ up to 45 % in 10 min); Detector, UV 220 nm] to afford compound **61 (Example 138)** as a white solid (15.0 mg, 27 %). (ES, *m/z*): $[M+H]^+$ 287.0; $^1H$ NMR (300 MHz,

$D_2O$) δ 6.25 6.28 (d, *J* = 6.9 Hz, 1H), 5.62 - 5.64 (m, 2H), 4.31 - 4.37 (m, 1H), 4.13 - 4.1 (m, 1H), 3.76 - 3.81 (m, 1 H), 3.44 - 3.47 (d, *J* = 8.4 Hz, 1H), 2.76 (s, 3H), 2.58 - 2.64 (m, 2H), 2.16 - 2.31 (m, 2H).

**Examples 139 and 140**

**(3aR,5S,6S,7R,7aR)-5-(2-fluoropropan-2-yl)-2-(methylamino)-5,6,7,7a-tetrahydro-3aH-pyrano[3,2-d]thiazole-6,7-diol and (3aR,5R,6S,7R,7aR) -2- (methylamino)-5-(prop-1-en-2-yl)-5,6,7,7a-tetrahydro-3aH-pyrano[3,2-d]thiazole-6,7-diol.**

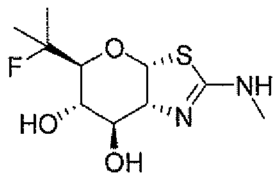

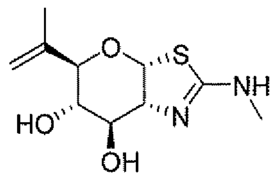

## Scheme XIII

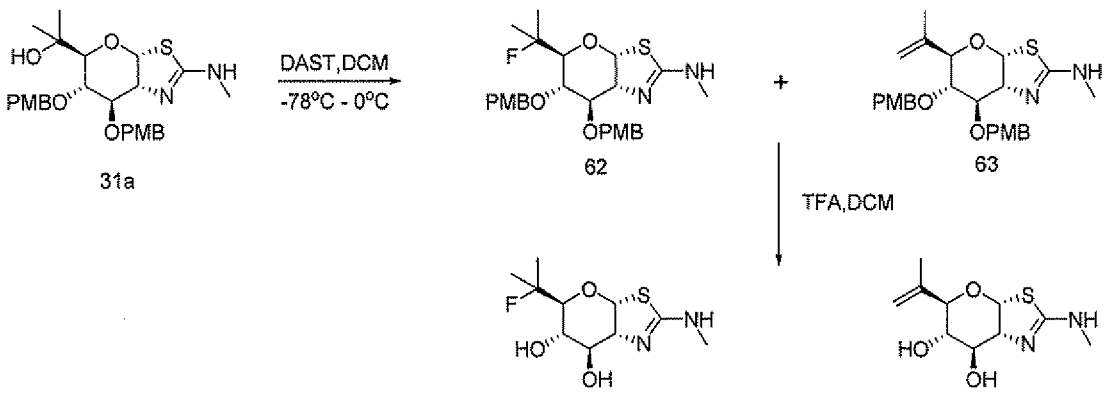


31a

62

63

64

65

**Step 1**

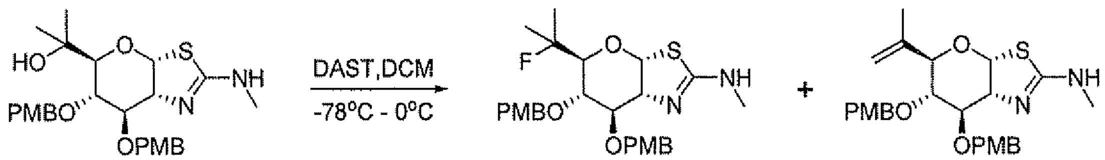


31a

62

63

**(3aR,5S,6S,7R,7aR)-5-(2-fluoropropan-2-yl)-6,7-bis(4-methoxybenzyloxy)-N-methyl-5,6,7,7a-tetrahydro-3aH-pyrano[3,2-d]thiazol-2-amine and (3aR,5R,6R,7R,7aR)-6,7-bis(4-methoxybenzyloxy)-N-methyl-5-(prop-1-en-2-yl)-5,6,7,7a-tetrahydro-3aH-pyrano[3,2-d]thiaxol-2-amine.** To a solution of 2-((3aR,5S,5S,7R,7aR)-6,7-bis(4-methoxybenzyloxy)-2-(methylamino)-5,6,7,7a-tetrahydro-3aH-pyrano[3,2-d]thiazol-5-yl)propan-2-ol (450.0 mg, 0.90 mmol) in dichloromethane (20 mL) was added DAST (720.0 mg, 4.47 mmol) at -78°C. After stirred for 1 hour at 0 °C, the reaction was quenched by aqueous potassium carbonate, extracted with dichloromethane (2 x 50 mL), dried over anhydrous sodium sulfate, and concentrated under reduced pressure to afford a mixture of **62** and **63** as a yellow oil (400.0 mg), which was used for next step without further purification: (ES, *m*/*z*) $[M+H]^+$ 505.0 and (ES, *m*/*z*) $[M+H]^+$ 485.0.

**Step 2**

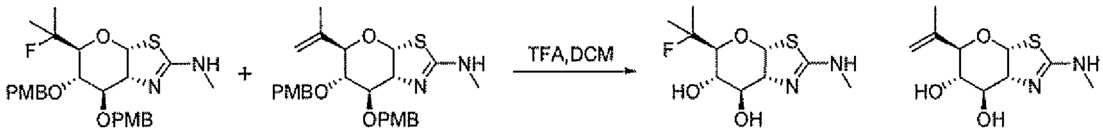


62 63 64 65

**(3aR,5S,6S,7R,7aR)-5-(2-fluoropropan-2-yl)-2-(methylamino)-5,6,7,7a-tetrahydro-3aH-pyrano[3,2-d]thiazole-6,7-diol and (3aR,5R,6S,7R,7aR) -2- (methylamino)-5-(prop-1-en-2-yl)-5,6,7,7a-tetrahydro-3aH-pyrano[3,2-d]thiazole-6,7-diol.** A solution of the above crude products (400.0 mg) in dichloromethane (20 mL) was treated with trifluoroacetic acid (2 mL) for 2 hours at room temperature. Removal of volatiles gave a residue, which was purified by Prep-HPLC with the following conditions [(Agilent 1200 prep HPLC): Column, SunFire Prep C18,19 * 50 mm 5 um; mobile phase, $H_2O$ with 0.03 % TFA and $CH_3CN$ (10 % $CH_3CN$ up to 45 % in 10 min); Detector, UV 220 nm] to afford compound **64 (Example 139)** as its TFA salt as a white solid (50.0 mg). (ES, *m/z*): $[M+H]^+$ 265.0; $^1H$ NMR (300 MHz, $CD_3OD$) $\delta$ 6.56 - 6.58 (d, *J* = 7.2 Hz, 1H), 4.48 - 4.49 (m, 1H), 4.12 - 4.14 (m, 1H), 3.92 - 3.94 (m, 1H), 3.44 - 3.51 (m, 1H), 3.07 (s, 3H), 1.45 - 1.47 (d, *J* = 6.3 Hz, 3H), 1.37 - 1.39 (d, *J* = 6.6 Hz, 3H); and compound **65 (Example 140)** as its TFA salt as a white solid (17.8 mg). (ES, *m/z*): $[M+H]^+$ 245.0; $^1H$ NMR (300 MHz, $D_2O$) $\delta$ 6.57 - 6.61 (m, 1H), 5.10 - 5.11 (m, 2H), 4.09 - 4.19 (m, 2H), 3.84 - 3.94 (m, 1H), 3.58 - 3.66 (m, 1H), 2.97 (s, 3H), 1.71 (s, 3H).

**Example 141**

**(3aR,5R,6S,7R,7aR)-2-(2-Fluoroethylamino)-5-((S)-1-hydroxyethyl)-5,6,7,7a-tetrahydro-3aH-pyrano[3,2-d]thiazole-6,7-diol**

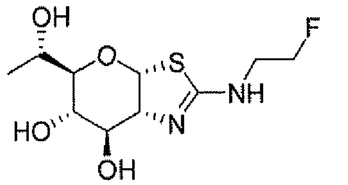

Scheme XIV

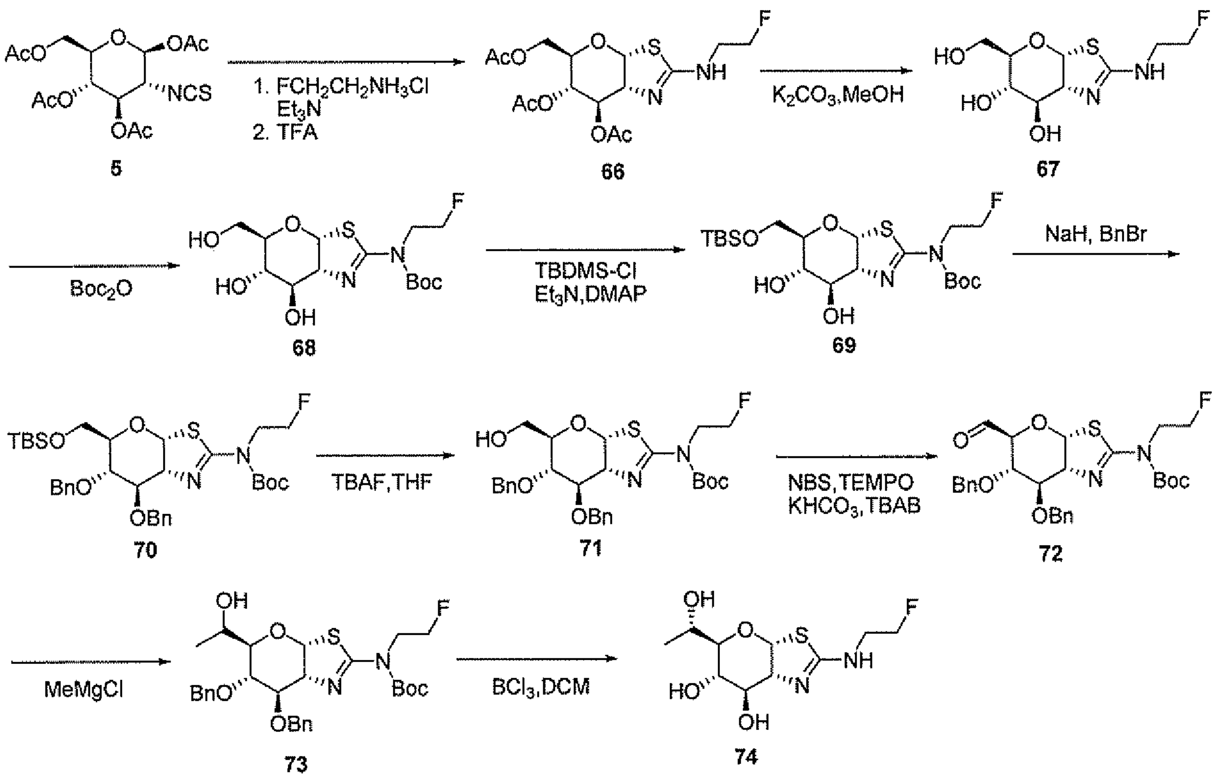


5 66 67 68 69 70 71 72 73 74

**Step 1**

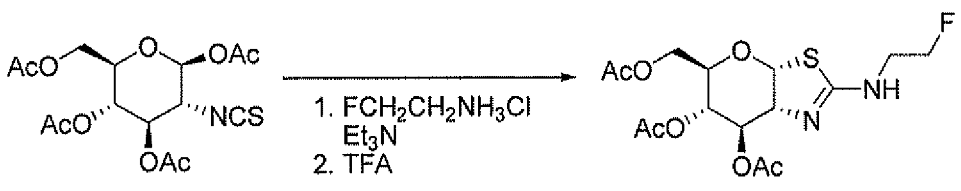


5 66

**(3aR,5R,6S,7R,7aR)-5-(Acetoxymethyl)-2-(2-fluoroethylamino)-5,6,7,7a-tetrahydro-3aH-pyrano[3,2-d]thiazole-6,7-diyl diacetate (66):** A solution of (2S,3R,4R,5S,6R)-6-(acetoxymethyl)-3-isothiocyanato-tetrahydro-2H-pyran-2,4,5-triyl triacetate (30 g, 77 mmol) in dichloromethane (100 mL) was added 2-fluoroethylamine hydrochloride (8.4 g, 84 mmol) and triethylamine (11.7 mL, 115 mmol). The resulting solution was stirred for 1 hour at room temperature, and then condensed to give a light yellow foam, which was dissolved into dichloromethane (100 mL) and treated with 2,2,2-trifluoroacetic acid (75.6 g, 663 mmol) overnight at room temperature. The reaction mixture was quenched by saturated aqueous sodium carbonate (500 mL), washed with sodium chloride (3 x 50 mL), dried over anhydrous magnesium sulfate, and concentrated under vacuum to give a residue, which was purified by a silica gel column, eluting with 50 % ethyl acetate in petroleum ether to provide compound **66** as a light yellow oil (24 g, 70 %). (ES, *m/z*) $[M+H]^+$ 393.0; $^1H$ NMR (300 MHz, $CDCl_3$) $\delta$ 6.27 - 6.29 (d, *J* = 6.60 Hz, 1H), 5.42 - 5.44 (t, *J* = 2.7 Hz, 1H), 4.95 - 4.99 (dd, *J* = 1.8 Hz, 9.5 Hz, 1H), 4.70 - 4.73 (m, 1 H), 4.54 - 4.65 (m, 1H), 4.37 - 4.39 (t, *J* = 0.9 Hz, 1H), 4.16 - 4.17 (m, 2H), 3.81 - 3.87 (m, 1H), 3.56 - 3.71 (m, 2H), 2.09 (s, 3H), 2.11 (s, 3H), 2.14 (s, 3H).

Step 2

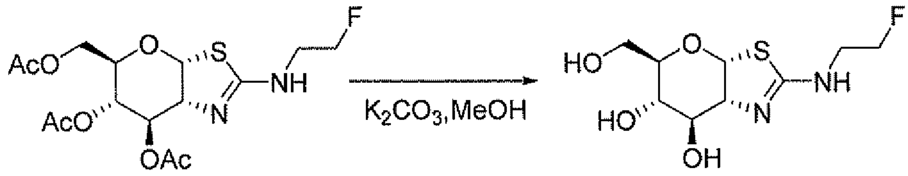


66 67

**(3aR,5R,6S,7R,7aR)-2-(2-Fluoroethylamino)-5-(hydroxymethyl)-5,6,7,7a-tetrahydro-3aH-pyrano[3,2-d]thiaznle-6,7-diol** (**67**): A solution of (3aR,5R,6S,7R,7aR)-5-(acetoxymethyl)-2-(2-fluoroethylamino)-5,6,7,7a-tetrahydro-3aH-pyrano[3,2-d]thiazole-6,7-diyl diacetate (29 g, 74 mmol) in methanol (200 mL) was treated with potassium carbonate (1 g, 7 mmol) overnight at room temperature. The solids were collected by filtration and washed with dichloromethane (3 x 50 mL) to afford compound **67** as a white solid (18 g, 92 %). (ES, *m/z*) $[M+H]^+$ 266.9; $^1H$ NMR (300 MHz, $D_2O$) δ 6.21 - 6.23 (d, *J* = 6.3 Hz, 1H), 4.55 - 4.59 (m, 1H), 4.40 - 4.44 (m, 1H), 4.10 - 4.14 (t, *J* = 5.7 Hz, 1H), 3.95 - 3.98 (t, *J*= 5.4 Hz, 1H), 3.70 - 3.75 (m, 1H), 3.40 - 3.62 (m, 5H).

Step 3

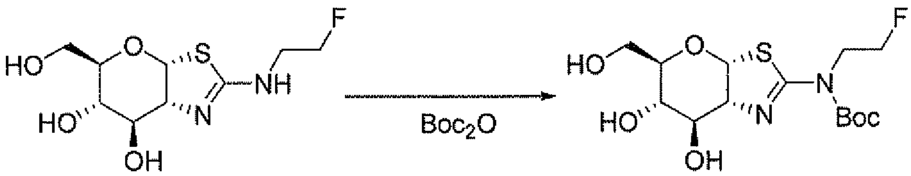


67 68

**tert-Butyl (3aR,5R,6S,7R,7aR)-6,7-dihydroxy-5-(hydroxymethyl)-5,6,7,7a-tetrahydro-3aH-pyrano[3,2-d]thiazol-2-yl(2-fluoroethyl)carbamateb** (**68**)**:** To a solution of (3aR,5R,6S,7R,7aR)-2-(2-fluoroethylamino)-5-(hydroxymethyl)-5,6,7,7a-tetrahydro-3aH-pyrano[3,2-d]thiazole-6,7-diol (13.7 g, 51 mmol) in methanol (100 mL) was added di-tert-butyl dicarbonate (22 g, 102 mmol) and triethylamine (8.6 mL). After stirred overnight at room temperature, the resulting mixture was concentrated under vacuum to give a residue, which was purified by a silica gel column, eluting with 20 % methanol in dichloromethane to give compound **68** as a white syrup (16 g, 85 %). (ES, *m/z*) $[M+H]^+$ 367.0; $^1H$ NMR (300 MHz, $CDCl_3$) δ 6.18 - 6.20 (d, *J* = 6.3 Hz, 1H), 4.70 - 4.76 (m, 1H), 4.55 - 4.60 (m, 1H), 4.43 - 4.48 (m, 1H), 4.27 - 4.30 (m, 1H), 3.86 - 3.98 (m, 3H), 3.71 - 3.79 (m, 2H), 3.49 - 3.62 (m, 2H), 1.57 (s, 9H).

Step 4

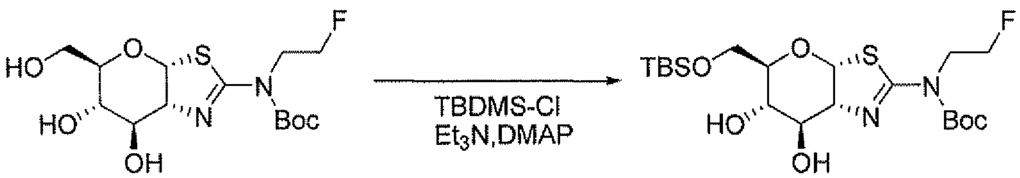


68 69

**tert-Butyl (3aR,5R,6S,7R,7aR)-5-((tert-butyldimethylsilyloxy)methyl)-6,7-dihydroxy-5,6,7,7a-tetrahydro-3aH-pyrano[3,2-d]thiazol-2-yl(2-fiuoroethyl)carbamate (69):**

To a solution of tert-butyl (3aR,5R,6S,7R,7aR)-6,7-dihydroxy-5-(hydroxymethyl)-5,6,7,7a-tetrahydro-3aH-pyrano[3,2-d]thiazol-2-yl(2-fluoroethyl)carbamate (16 g, 43.7 mmol) in dichloromethane (100 mL) was added tert-butylchlorodimethylsilane (7.6 g, 50.7 mmol), 4-dimethylaminopyridine (530 mg, 4.34 mmol) and triethylamine (6.6 g, 65 mmol). After stirred for 6 h at room temperature, the resulting mixture was concentrated under vacuum to give a residue, which was purified by a silica gel column, eluting with 20 % petroleum in ethyl acetate to give compound **69** as pale yellow oil (10 g, 48 %). (ES, *m/z)* $[M+H]^+$ 481.0; $^1H$ NMR (300 MHz, $CDCl_3$) δ 6.17 - 6.19 (d, *J* = 6.3 Hz, 1H), 4.42 - 4.74 (m, 3H), 3.52 - 4.26 (m, 7H), 1.56 (s, 9H), 0.92 (s, 9H), 0.10 (s, 6H).

**Step 5**

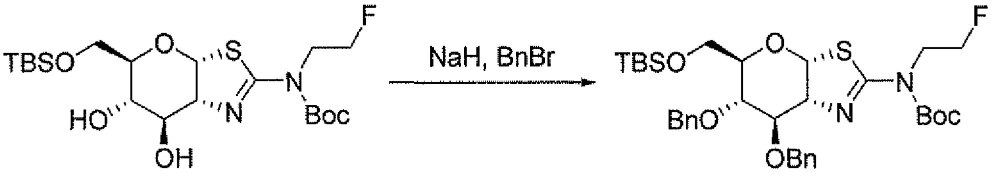


69 70

**tert-Butyl (3aR,5R,6S,7R,7aR)-6,7-bis(benzyloxy)-5-((tert-butyldimethylsilyloxy)methyl)-5,6,7,7a-tetrahydro-3aH-pyrano[3,2-d]thiazol-2-yl(2-fluoroethyl)carbamate (70):** A solution of tert-butyl (3aR,5R,6S,7R,7aR)-5-((tert-butyldimethylsilyloxy)methyl)-6,7-dihydroxy-5,6,7,7a-tetrahydro-3aH-pyrano[3,2-d]thiazol-2-yl(2-fluoroethyl)carbamate (2.0 g, 4.2 mmol) in N,N-dimethylformamide (30 mL) was treated with sodium hydride (510 mg, 21 mmol) for 10 min at 10 °C in a nitrogen atmosphere, and followed by addition of 1-(bromomethyl)benzene (2.2 g, 13 mmol). After stirred for 15 min, the reaction was quenched with saturated aqueous $NH_4Cl$ and extracted with dichloromethane (3 x 30 mL). The organic layers were dried over anhydrous magnesium sulfate and concentrated under vacuum to give a residue, which was purified by a silica gel column, eluting with 1.5 - 3 % ethyl acetate in petroleum ether to provide compound 70 as a yellow oil (0.7 g, 25 %). (ES, *m*/*z*) $[M+H]^+$ 660.8; $^1H$ NMR (300 MHz, $CDCl_3$) δ 7.28 - 7.43 (m, 10H), 6.11 - 6.13 (d, *J* = 6.3 Hz, 1H), 4.60 - 4.74 (m, 4H), 4.30 - 4.41 (m, 6H), 3.73 - 3.75 (m, 3H), 3.37 - 3.44 (m, 1H), 1.54 (s, 9H), 0.90 (s, 9H), 0.06 (s, 6H).

**Step 6**

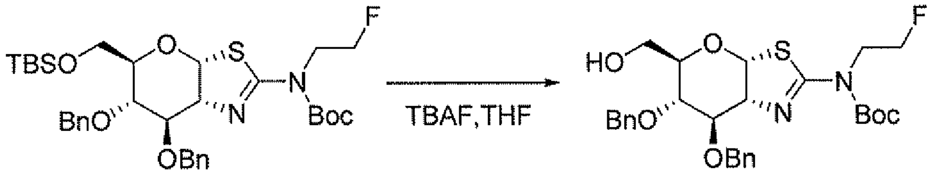


70 71

**tert-Butyl (3aR,5R,6S,7R,7aR)-6,7-bis(benzyloxy)-5-(hydroxymethyl)-5,6,7,7a-tetrahydro-3aH-pyrano[3,2-d]thiazol-2-yl(2-fluoroethyl)carbamate (71):** A solution of tert-butyl (3aR,5R,6S,7R,7aR)-6,7-bis(benzyloxy)-5-((tert-butyldimethylsilyloxy)methyl)-5,6,7,7a-tetrahydro-3aH-pyrano[3,2-d]thiazol-2-yl(2-fluoroethyl)carbamate (1.36 g, 2.06 mmol) in THF (30 mL) was treated with tetrabutylammonium fluoride (1.1 g, 4.2 mmol) for 2 hrs at room temperature. The reaction mixture was quenched with $H_2O$ (10 mL), extracted with ethyl acetate (3 x 50 mL), dried over anhydrous magnesium sulfate, and concentrated under vacuum to give a residue, which was purified by a silica gel column, eluting with 10 % - 20 % ethyl acetate in petroleum ether to provide compound **71** as a pale yellow oil (600 mg, 53 %). (ES, *m*/*z*) $[M+H]^+$ 546.8; $^1H$ NMR (300 MHz, $CDCl_3$) δ 7.24 - 7.43 (m, 10H), 6.09 - 6.11 (d, *J* = 4.2 Hz, 1H), 4.60 - 4.66 (m, 2H), 4.50 - 4.54 (m, 1H), 4.46 - 4.47 (m, 1H), 4.40 - 4.45 (m, 2H), 4.27 - 4.39 (m, 2H), 4.12 - 4.22 (m, 2H), 3.72 - 3.79 (m, 1H), 3.67 - 3.71 (m, 1H), 3.55 - 3.63 (m, 1H), 3.40 - 3.45 (m, 1H), 1.54 (s, 9H).

**Step 7**

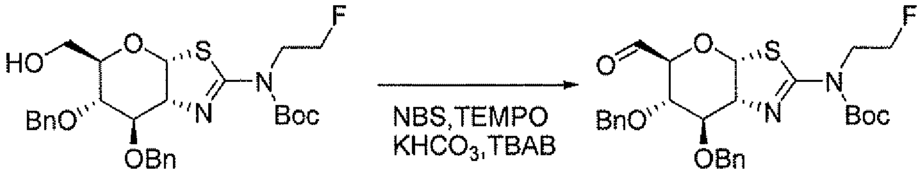


71 72

**tert-Butyl (3aR,5S,6S,7R,7aR)-6,7-bis(benzyloxy)-5-formyl-5,6,7,7a-tetrahydro-3aH-pyrano[3,2-d]thiazol-2-yl(2-fluoroethyl)carbamate (72):** A mixture of tert-butyl (3aR,5R,6S,7R,7aR)-6,7-bis(benzyloxy)-5-(hydroxymethyl)-5,6,7,7a-tetrahydro-3aH-pyrano[3,2-d]thiazol-2-yl(2-fluoroethyl)carbamate (600 mg, 1.10 mmol), $KHCO_3$ (495 mg, 4.95 mmol), TBAB (18 mg, 0.06 mmol) and TEMPO (9 mg, 0.06 mmol) in dichloromethane (17 mL) and water (2mL) was treated with NBS (215 mg, 1.21 mmol) for 15 min at 10 - 15 °C. The reaction mixture was quenched by saturated aqueous $Na_2SO_3$ (5 mL), extracted with dichloromethane (3 x 15 mL), dried over magnesium sulfate, and concentrated under vacuum to give a residue, which was purified by a silica gel column, eluting with 20 % ethyl acetate in petroleum ether to provide compound 72 as a yellow syrup (0.4 g, 67 %). (ES, *m/z*) $[M+H]^+$ 545.0; $^1H$ NMR (300 MHz, $CDCl_3$) δ 9.64 (s, 1H), 7.23 - 7.39 (m, 10H), 6.09 - 6.11 (d, *J* = 6.3 Hz, 1H), 4.55 - 4.75 (m, 5H), 4.34 - 4.45 (m, 2H), 4.13 - 4.28 (m, 3H), 3.90 - 4.12 (m, 2H), 1.54 (s, 9H).

**Step 8**

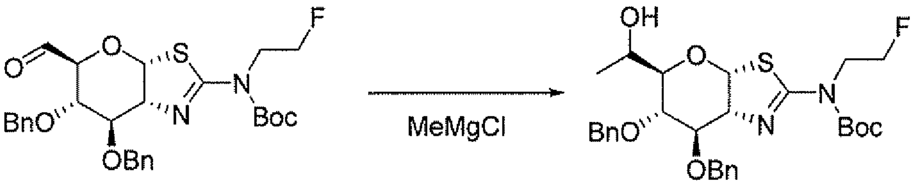


72 73

**tert-Butyl (3aR,5R,6S,7R,7aR)-6,7-bis(benzyloxy)-5-(1-hydroxyethyl)-5,6,7,7a-tetrahydro-3aH-pyrano[3,2-d]thiazol-2-yl(2-fluoroethyl)carbamate (73):** To a solution of tert-butyl (3aR,5S,6S,7R,7aR)-6,7-bis(benzyloxy)-5-formyl-5,6,7,7a-tetrahydro-3aH-pyrano[3,2-d]thiazol-2-yl(2-fluoroethyl)carbamate (400 mg, 0.73 mmol) in THF (20 mL) was added methylmagnesium chloride (1 mL, 3M in THF). After stirred for 3 hours at 0-5 °C, the resulting solution was quenched with saturated aqueous $NH_4Cl$ (10 mL) and extracted with ethyl acetate (3 x 30 mL). The organic layers were dried over anhydrous magnesium sulfate and concentrated under vacuum to give a residue, which was purified by a silica gel column, eluting with 20 % ethyl acetate in petroleum ether to give compound **73** as a yellow oil (0.37 g, 90 %, diasteromers' ratio is 1:4). (ES, *m/z*) $[M+H]^+$ 561.0; $^1H$ NMR (300 MHz, $CDCl_3$) δ 7.25 - 7.44 (m, 10H), 6.13 - 6.15 (d, *J* = 6.9 Hz, 1H), 4.73 - 4.78 (m, 2H), 4.54 - 4.68 (m, 2H), 4.28 - 4.49 (m, 4H), 4.07 - 4.25 (m, 2H), 3.75 - 3.97 (m, 2H), 3.12 - 3.17 (m, 1H), 1.54(s, 9H), 1.22 - 1.24 (d, *J* = 6.3 Hz, 3H).

**Step 9**

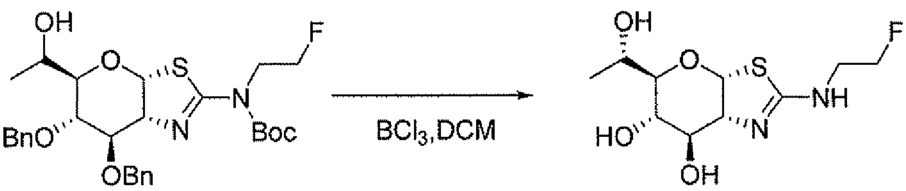


73 74

**(3aR,5R,6S,7R,7aR)-2-(2-Fluoroethylamino)-5-((S)-1-hydroxyethyl)-5,6,7,7a-tetrahydro-3aH-pyrano[3,2-d]thia-**

**zole-6,7-diol 2,2,2-trifluoroacetate (74):** A solution of tert-butyl (3aR,5R,6S,7R,7aR)-6,7-bis(benzyloxy)-5-(1-hydroxyethyl)-5,6,7,7a-tetrahydro-3aH-pyrano[3,2-d]thiazol-2-yl(2-fluoroethyl)carbamate (250 mg, 0.45 mmol) in dichloromethane (7 mL) was treated with $BCl_3$ (7 mL, 1M in dichloromethane) at -20 - -10 °C for 2 hrs. The reaction was quenched with methanol and concentrated under vacuum to give a crude product (0.25 g), which was purified by Prep-HPLC (Agilent 1200 prep HPLC; Column, X-Bridge 19*150mm 5um; mobile phase, water with 0.05 % trifluoroacetic acid and $CH_3CN$ (10 % up to 20 % in time 10); Detector, 220 nm.) to provide compound **74 (Example 141)** as a white solid (55.7 mg, 45 %). (ES, *m*/*z*) $[M+H]^+$ 281.0; $^1H$ NMR (300 MHz, $D_2O$) δ 6.53 - 6.55 (d, *J* = 6.9 Hz, 1H), 4.61 - 4.78 (m, 1H), 4.47 - 4.52 (m, 1H), 4.22 - 4.30 (m, 1H), 3.93 - 4.04 (m, 2H), 3.58 - 3.73 (m, 3H), 3.38 - 3.42 (m, 1H), 1.15 - 1.17 (d, *J* = 6.6 Hz, 3H).

**Example 142**

**(3aR,5R,6S,7R,7aR)-2-(3-Fluoropropylamino)-5-((S)-1-hydroxyethyl)-5,6,7,7a-tetrahydro-3aH-pyrano[3,2-d]thiazole-6,7-diol**

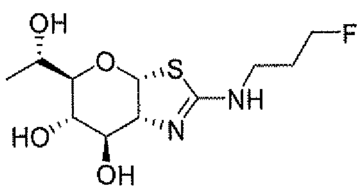

The title compound was synthesized following procedures ananalgous to Example 141. (ES, m/z) $[M+H]^+$ 295.0; 1H NMR (300 MHz, $D_2O$) δ 6.51 - 6.57 (m, 1H), 4.60 - 4.67 (m, 1H), 4.41 - 4.45 (m, 1H), 4.22 - 4.30 (m, 1H), 3.96 - 4.00 (m, 2H), 3.58 - 3.65 (m, 4H), 1.89 - 2.07 (m, 2H), 1.15 - 1.19 (m, 3H).

**Example 143**

**(3aR,5R,6S,7R,7aR)-5-(3-Hydroxypropyl)-2-(methylamino)-5,6,7,7a-tetrahydro-3aH-pyrano[3,2-d]thiazole-6,7-diol**

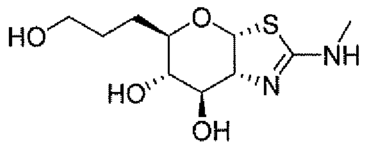

Scheme XV

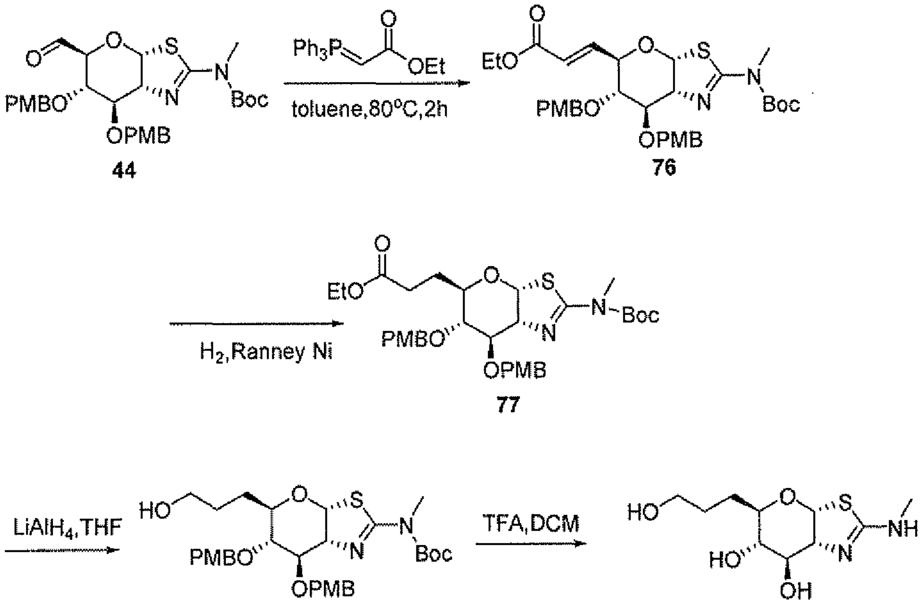


44 76 77 78 79

**Step 1**

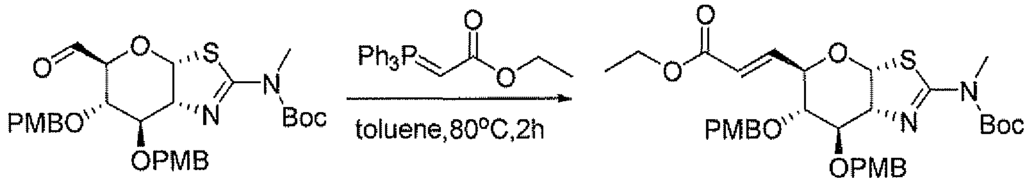


44 76

(E)-Ethyl **3-((3aR,5R,6R,7R,7aR)-2-(tert-butoxycarbonyl(methyl)amino)-6,7-bis(4-methoxybenzyloxy)-5,6,7,7a-tetrahydro-3aH-pyrano[3,2-d]thiazol-5-yl)acrylate (76):A** solution of 44 (420 mg, 0.51 mmol) in toluene (20 mL) was treated with (carbethoxymethylene)triphenylphosphorane (280 mg, 0.81 mmol) overnight at 80 °C. The reaction mixture was concentrated under vacuum to give a residue, which was purified by a silica gel column, eluting with 10 % ethyl acetate in petroleum ether to provide compound 76 as a light yellow solid (231 mg, 70 %). (ES, *m/z*) $[M+H]^+$ 643.3; $^1H$ NMR (300 MHz, $CDCl_3$) δ 7.31 - 7.34 (d, *J* = 8.4 Hz, 2H), 7.20 - 7.22 (d, *J* = 8.7 Hz, 2H), 6.85 - 6.96 (m, 5H), 6.02 - 6.08 (m, 2H), 4.63 - 4.71 (m, 2H), 4.53 - 4.59 (m, 1H), 4.35 - 4.41 (m, 1H), 4.16 - 4.35 (m, 4H), 3.98 -3.99 (m, 1H), 3.82 (s, 6H), 3.48 - 3.52 (m, 1H), 3.20 (s, 3H), 1.54 (s, 9H), 1.26 1.31 (t, *J* = 6.9 Hz, 3H).

**Step 2**

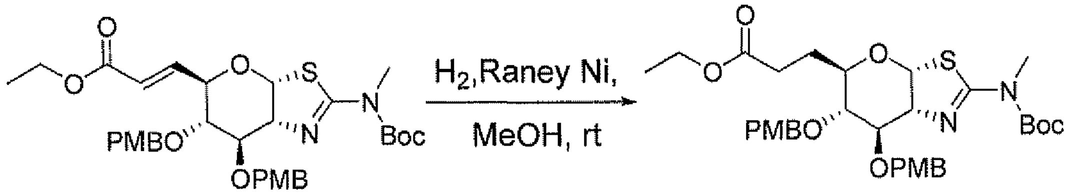


76 77

**Ethyl 3-((3aR,5R,6R,7R,7aR)-2-(tert-butoxycarbonyl(methyl)amino)-6,7-bis(4-methoxybenzyloxy)-5,6,7,7a-tetrahydro-3aH-pyrano[3,2-d]thiazol-5-yl)propanoate** (77): A mixture of compound **76** (100 mg, 0.16 mmol) and Raney Ni (20 mg) in methanol (10 mL) was stirred under hydrogen atmosphere overnight at room temperature. After removal of solids, the solution was concentrated under vacuum to give **77** as a yellow oil (80 mg, 76 %). (ES, *m/z*) $[M+H]^+$ 645.3; $^1H$ NMR (300 MHz, $CDCl_3$) δ 7.34 - 7.35 (d, *J* = 6.6 Hz, 2H), 7.19 - 7.20 (d, *J* = 6.6 Hz, 2H), 6.84 - 6.93 (m, 4H), 6.02 - 6.04 (d, *J* = 6.9 Hz, 1H), 4.60 - 4.71 (m, 2H), 4.49 - 4.52 (d, *J* = 8.1 Hz, 1H), 4.37 - 4.40 (m, 1H), 4.27 - 4.30 (d, *J* = 8.1 Hz, 1H), 4.22 - 4.24 (m, 1H), 4.07 - 4.14 (m, 2H), 3.82 (s, 6H), 3.80 - 3.81 (m, 1H), 3.40 - 3.43 (m, 1H), 3.10 (s, 3H), 2.25 - 2.45 (m, 2H), 2.01 - 2.09 (m, 1H), 1.66 - 1.74 (m, 1H), 1.54 (s, 9H), 1.22 - 1.29 (t, *J* = 6.9 Hz, 3H).

**Step 3**

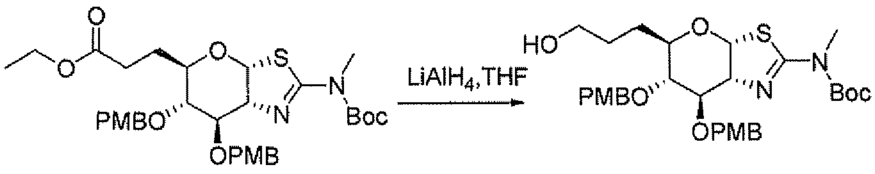


**77** **78**

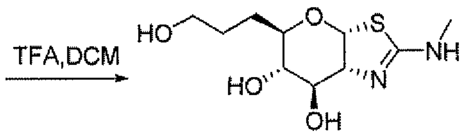


**79**

**(3aR,5R,6S,7R,7aR)-5-(3-Hydroxypropyl)-2-(methylamino)-5,6,7,7a-tetrahydro-3aH-pyrano[3,2-d]thiazole-6,7-diol (79):** A solution of compound 77 (150 mg, 0.23 mmol) in THF (10 mL) was treated with $LiAlH_4$ (26.6 mg, 0.70 mmol) for 3 h at room temperature. The reaction mixture was then quenched by addition of saturated aqueous $NH_4Cl$ (20 mL), extracted with dichloromethane (3 x 30 mL), and dried over anhydrous magnesium sulfate. The filtrate was concentrated under vacuum to give crude compound **78** as a yellow oil (90 mg), which was dissolved into dichloromethane (10 mL) and treated with TFA (1 mL) overnight at room temperature. Removal of volatiles gave a residue, which was purified by Prep-HPLC with the following conditions: [(Agilent 1200 prep HPLC: Column, Sun fire prep. C18; mobile phase, water with 0.03 % trifluoroacetic acid and $CH_3CN$ (10 % up to 20 % in time 10); Detector, 220 nm.)] to give the title compound **79 (Example 143)** as a white solid (10.8 mg, 23 %). (ES, *m/z*) $[M+H]^+$ 263.1; $^1H$ NMR (300 MHz, $D_2O$) δ 6.18 - 6.20 (d, *J* = 6.3 Hz, 1H), 4.08 - 4.12 (m, 1H), 3.89 - 3.93 (m, 1H), 3.36 - 3.51 (m, 4H), 2.74 - 2.76 (m, 3H), 1.68 - 1.76 (m, 1H), 1.33 - 1.62 (m, 3H).

**Example 144**

**(3aR,5R,6S,7R,7aR)-2-(Ethylamino)-5-(2-hydroxyethyl)-5,6,7,7a-tetrahydro-3aH-pyrano[3,2-d]thiazole-6,7-diol**

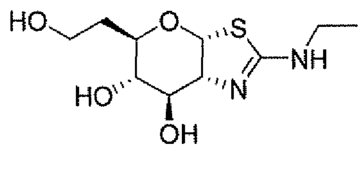

## Scheme XVI

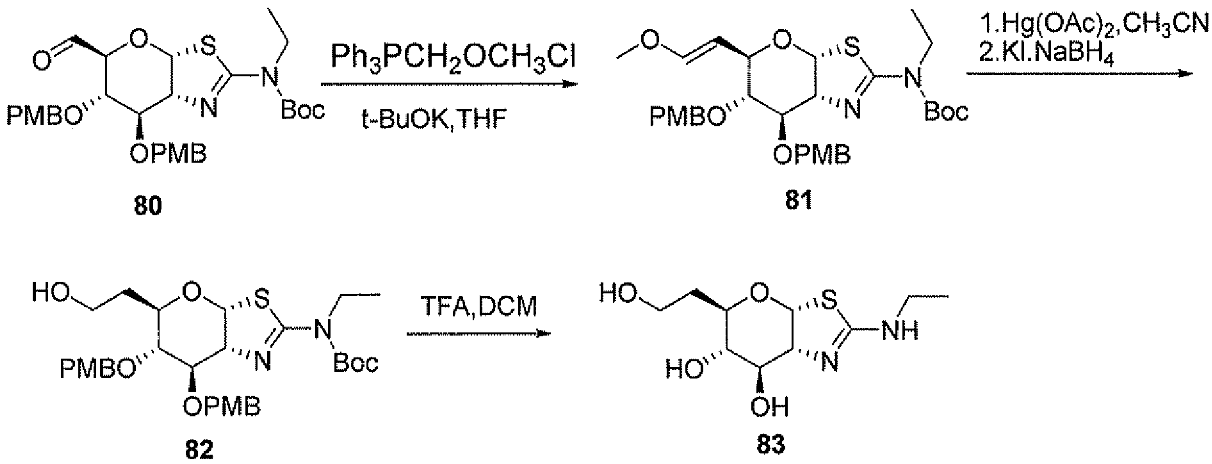


80

81

82

83

**Step 1**

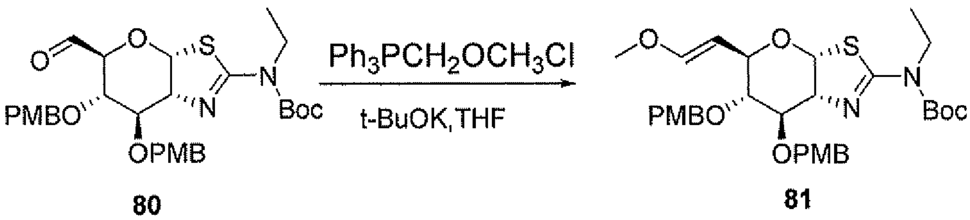


80

81

**tert-Butyl (3aR,5R,6R,7R,7aR)-6,7-bis(4-methoxybenzyloxy)-5-((E)-2-methoxyvinyl)-5,6,7,7a-tetrahydro-3aH-pyrano[3,2-d]thiazol-2-yl(ethyl)carbamate (81):** A solution of $Ph_3PCH_2OCH_3Cl$ (613 mg, 1.79 mmol) in THF (20 mL) was treated with potassium t-butoxide (191 mg, 1.71 mmol) at 0 °C in a nitrogen atmosphere for 30 min, and followed by addition of **80** (500 mg, 0.85 mmol) in THF (5 mL). The resulting solution was stirred for 3 hours at 25 °C, and then quenched by saturated aqueous $NH_4Cl$ (20 mL), extracted with dichloromethane (3 x 20 mL). The combined organic layers were washed with brine (2 x 20 mL), dried over anhydrous magnesium sulfate, and concentrated under vacuum to give a residue, which was purified by a silica gel column, eluting with 30 % ethyl acetate in petroleum ether to give compound **81** as a light yellow solid (200 mg, 38 %). (ES, *m/z*) $[M+H]^+$ 615.3.

**Step 2**

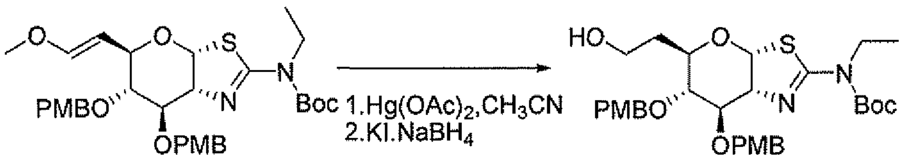


81 82

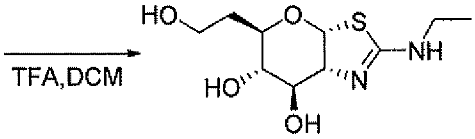


83

**(3aR,5R,6S,7R,7aR)-2-(Ethylamino)-5-(2-hydroxyethyl)-5,6,7,7a-tetrahydro-3aH-pyrano[3,2-d]thiazole-6,7-diol** A solution of compound **81** (200 mg, 0.33 mmol) in $CH_3CN$ (10 mL) and water (10 mL) was treated with $Hg(OAc)_2$ (128 mg, 0.40 mmol) for 4 hours at -5 °C, and followed by addition of KI (277 mg, 1.67 mmol) and $NaBH_4$ (50.67 mg, 1.33 mmol) sequentially. The resulting mixture was stirred overnight at 0 °C, and then quenched by saturated aqueous $NH_4Cl$ (20 mL), extracted with dichloromethane (3 x 20 mL). The combined organic layers were washed with brine (2 x 20 mL), dried over anhydrous magnesium sulfate, and condensed to give crude compound **82** as a yellow foam, which was dissolved into dichloromethane (10 mL) and treated with TFA (1 mL) overnight at room temperature. Removal of volatiles provided a residue, which was purified by Prep-HPLC with the following conditions [(Prep-HPLC): Column, 19*150 mm; mobile phase, WATER with 0.03 % $NH_3H_2O$ and $CH_3CN$ (10 % $CH_3CN$ up to 45 % in 10 min); Detector, 220 nm.] to give the title compound **83** (**Example 144**) as a yellow solid (8.1 mg, 28 %). (ES, *m/z*) $[M+H]^+$ 263.1; $^1$HNMR (300 MHz, $D_2O$) δ 6.18 - 6.20 (d, *J* = 6.3 Hz, 1H), 4.06 - 4.10 (t, *J*= 5.4 Hz, 1H), 3,87 - 3.91 (t, *J* = 3.9 Hz, 1H), 3.50 - 3.64 (m, 3H), 3.38 - 3.42 (m, 1H), 3.10 - 3.22 (m, 2H), 1.88 - 1.99 (s, 1H), 1.65 - 1.69 (m, 1H), 0.96 - 1.05 (t, *J* = 6,9 Hz, 3H).

The following Example 145 was prepared in a similar fashion to Example 144.

**Example 145**

| Example | structure | Name | MH+ |
|---|---|---|---|
| 145 | 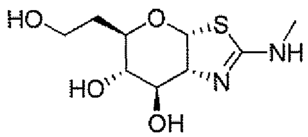 | (3aR,5R,6S,7R,7aR)-5-(2-hydroxyethyl)-2-(methylamino)-5,6,7,7a-tetrahydro-3aH-pyrano[3,2-d]thiazole-6,7-diol | 249.1 |

**Example 146**

**(3aR,5S,6S,7R,7aR)-2-(Dimethylamino)-5-(2-fluoropropan-2-yl)-5,6,7,7a-tetrahydro-3aH-pyrano[3,2-d]thiazole-6,7-diol**

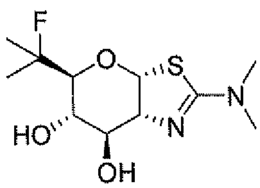

Scheme XVII

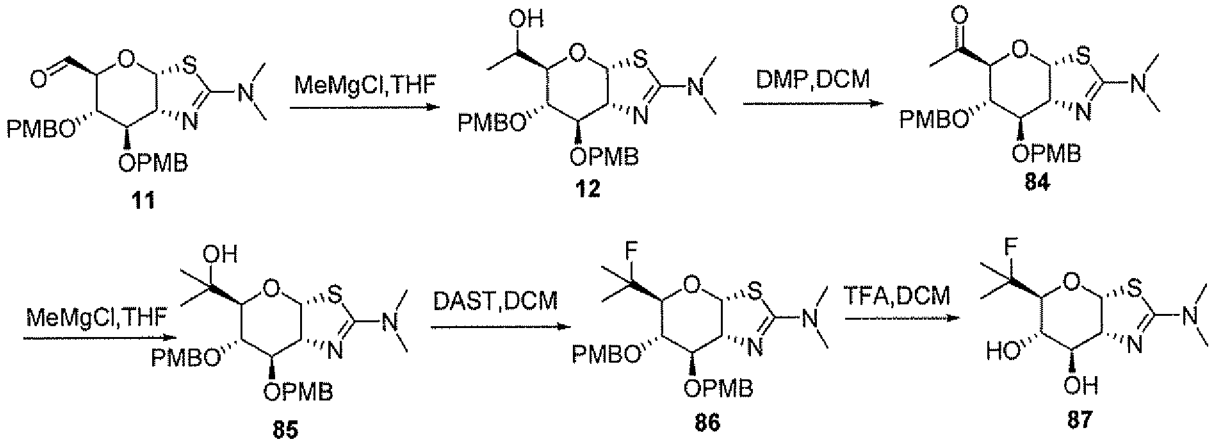


Step 1

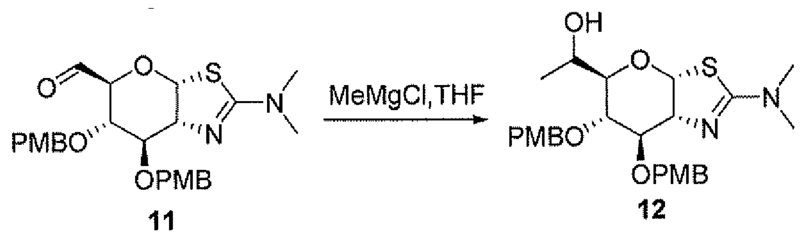


**(S)-1-((3aR,5R,6S,7R,7aR)-2-(Dimethylamino)-6,7-bis(4-methoxybenzyloxy)-5,6,7,7a-tetrahydro-3aH-pyrano[3,2-d]thiazol-5-yl)ethanol (12):** A solution of **11** (600 mg, 1.23 mmol) in THF (10 mL) was treated with methylmagnesium chlorine (0.82 mL, 3M in THF) for 2 hours at room temperature, and then quenched with saturated aqueous $NH_4Cl$ (20 mL), extracted with ethyl acetate (3 x 20 mL). The combined organic layers were dried over anhydrous magnesium sulfate and concentrated under vacuum to give a residue, which was purified by silica gel column, eluting with 1 - 2 % methanol in dichloromethane to give compound **(12)** as a yellow syrup (500 mg, 81 %, diastereomers' ratio is ~ 1:4). (ES, *m/z*): $[M+H]^+$ 503.0. $^1H$ NMR (300 MHz, $CDCl_3$) δ 7.23 7.35 (m, 4H), 6.85 - 6.92 (m, 4H), 6.30 - 6.32 (d, *J* = 6.6 Hz, 1H), 4.52 - 4.67 (m, 4H), 4.33 - 4.37 (m, 2H), 3.81 - 3.84 (m, 8H), 3.35 - 3.50 (m, 1H), 2.99 - 3.02 (m, 6H), 1.11 - 1.21 (m, 3H).

Step 2

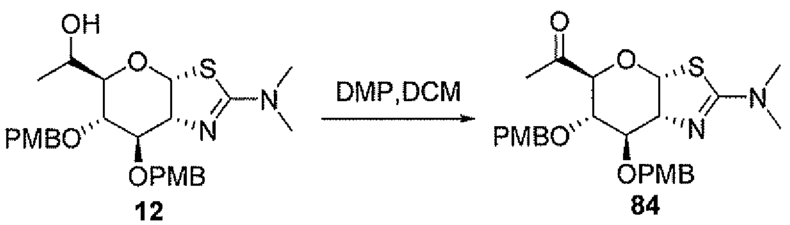


**1-((3aR,5S,6S,7R,7aR)-2-(Dimethylamino)-6,7-bis(4-methoxybenzyloxy)-5,6,7,7a-tetrahydro-3aH-pyrano[3,2-d]thiazol-5-yl)ethanone (84):** A solution of **12** (450 mg, 0.90 mmol) in dichloromethane (20 mL) was treated with Dess-Martin reagent (760 mg, 1.80 mmol) for 2 hours at room temperature, and then quenched by saturated aqueous $Na_2S_2O_3$ (20 mL) and saturated aqueous $NaHCO_3$ (20 mL), extracted with dichloromethane (2 x 20 mL). The combined organic layers were dried over anhydrous sodium sulfate and concentrated under reduced pressure to provide a residue, which was purified by silica gel column, eluting with 2 - 3 % methanol in dichloromethane to give compound **(84)** as a yellow syrup (400 mg, 88 %). (ES, *m/z*): $[M+H]^+$ 501.0. $^1H$ NMR (300 MHz, $CDCl_3$) $\delta$ 7.25 - 7.30 (m, 4H), 6.83 - 6.90 (m, 4H), 6.29 - 6.31 (d, *J* = 6.0 Hz, 1 H), 4.26 - 4.64 (m, 6H), 3.81 - 3.98 (m, 8H), 2.96 - 3.02 (m, 6H), 2.17 (s, 3H).

**Step 3**

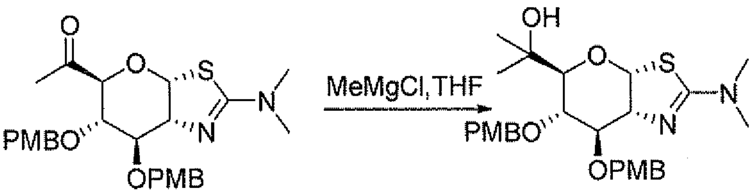


**84** **85**

**2-((3aR,5S,6S,7R,7aR)-2-(Dimethylamino)-6,7-bis(4-methoxybenzyloxy)-5,6,7,7a-tetrahydro-3aH-pyrano[3,2-d]thiazol-5-yl)propan-2-ol (85):** A solution of compound **84** (100 mg, 0.2 mmol) in THF (10 mL) was treated with methylmagnesium chlorine (0.5 mL, 3M in THF) for 2 hours at room temperature, then quenched with saturated aqueous $NH_4Cl$ (20 mL), extracted with ethyl acetate (3 x 20 mL). The combined organic layers were dried over anhydrous magnesium sulfate, concentrated under vacuum to give a residue, which was purified by silica gel column, eluting with 1 - 2 % methanol in dichloromethane to give compound **(85)** as a yellow syrup (100 mg, 97 %). (ES, *m/z*): $[M+H]^+$ 517.0. $^1H$ NMR (300 MHz, $CDCl_3$) $\delta$ 7.22 - 7.36 (m, 4H), 6.83 - 6.92 (m, 4H), 6.32 - 6.34 (d, *J* = 6.9 Hz, 1H), 4.25 - 4.70 (m, 7H), 3.81 (s, 6H), 3.33 - 3.36 (d, *J*= 8.7 Hz, 1H), 2.98 (s, 6H), 1.18 (s, 6H).

**Step 4**

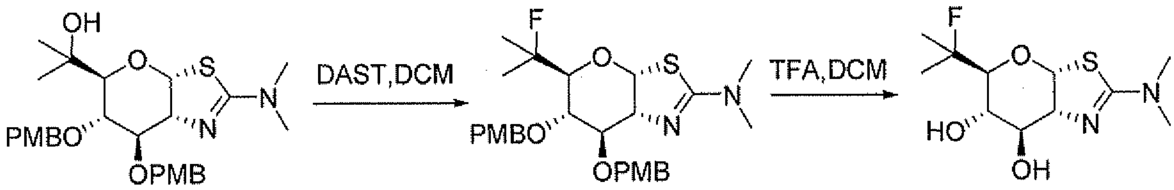


**85** **86** **87**

**(3aR,5S,6S,7R,7aR)-2-(Dimethylammo)-5-(2-ftuoropropan-2-yl)-5,6,7,7a-tetrahydro-3aH-pyrano[3,2-d]thiazole-6,7-diol (87):** A solution of **85** (100 mg, 0.19 mmol) in dichloromethane (10 mL) was treated with DAST (156 mg, 0.97 mmol) at -78 °C for 30 min. The reaction mixture was allowed to warm up to room temperature gradually. After stirring for another 30 min, the reaction was quenched with saturated aqueous $Na_2CO_3$ (10 mL) and extracted with dichloromethane (3 x 10 mL). The combined organic layers were dried over anhydrous $MgSO_4$ and concentrated under reduced pressure to give crude compound **86** as a yellow oil (100 mg), which was dissolved into dichloromethane (10 mL) and treated with TFA (1 mL) for 1 hour at room temperature. Removal of volatiles gave a residue, which was purified by Prep-HPLC with the following conditions [(Agilent 1200 Detecl Prep-HPLC): Column, SunFire Prep C18; mobile phase, water with 0.03% ammonia and $CH_3CN$; Detector, UV220nm] to afford the title compound **87** (**Example 146**) as a white solid (34.8 mg, 65 %). (ES, *m/z*): $[M+H]^+$ 279.0; $^1H$ NMR (300 MHz, $D_2O$) $\delta$ 6.21 - 6.23 (d, *J* = 6.6 Hz, 1H), 4.33 - 4.36 (t, *J* = 6.3 Hz, 1H), 4.14 - 4.16 (t, *J* = 2.7 Hz, 1H), 3.78 - 3.81 (m, 1H), 3.30 - 3.39 (m, 1H), 2.91 (s, 6H), 1.34 (s, 3H) 1.27 (s, 3H).

**Example 147**

**(3aR,5S,6S,7R,7 aR)-5-(1,1-Difluoroethyl)-2-(propylamino)-5,6,7,7a-tetrahydro-3aH-pyrano[3,2-d]thiazole-6,7-diol**

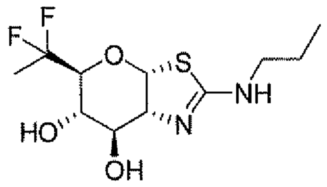

SCHEME XVIII

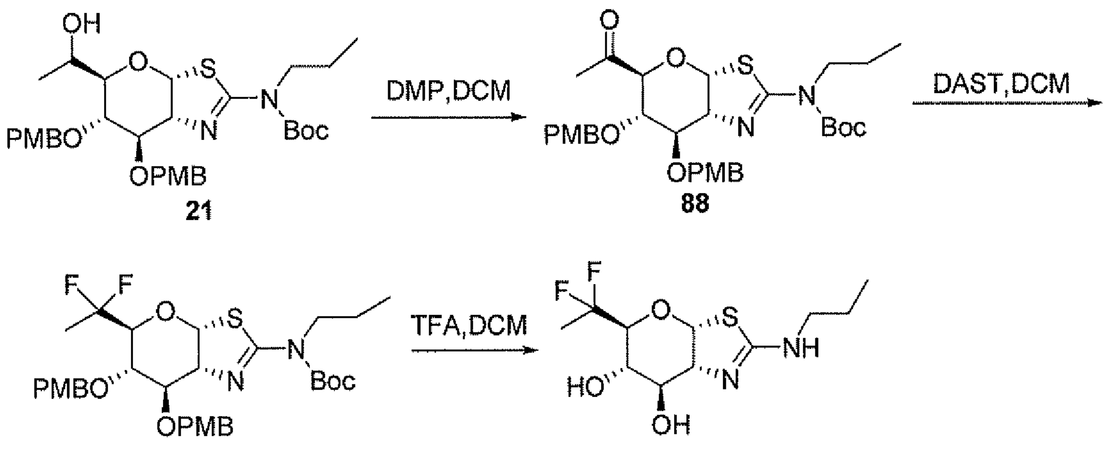


21 88 89 90

**Step 1**

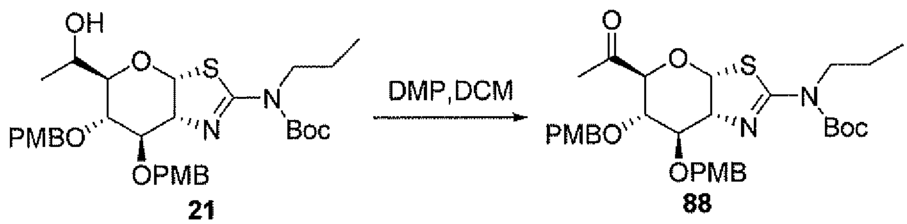


21 88

**tert-Butyl (3aR,5S,6S,7R,7aR)-5-acetyl-6,7-bis(4-methoxybenzyloxy)-5,6,7,7a-tetrahydro-3aH-pyrano[3,2-d]thiazol-2-yl(propyl)carbamate (88):** A solution of **21** (450 mg, 0.72 mmol) in dichloromethane (10 mL) was treated with Dess-martin reagent (780 mg, 1.83 mmol) for 1 hour at room temperature, then quenched by saturated aqueous $Na_2S_2O_3$ (20 mL) and saturated aqueous $NaHCO_3$ aqueous (20 mL), extracted with dichloromethane (2 x 20 mL). The combined organic layers were dried over anhydrous sodium sulfate and concentrated under reduced pressure to provide a residue, which was purified by 30 % ethyl acetate in petroleum ether to give compound **88** as light yellow oil (210 mg, 47 %). (ES, *mlz*): [M+HI$^+$ 615 .0. $^1$H NMR (300 MHz, $CDCl_3$) δ 7.21 - 7.31 (m, 4H), 6.84 - 6.92 (m, 4H), 6.05 - 6.07 (d, *J* = 6.9 Hz, 1H), 4.26 - 4.64 (m, 6H), 3.87 - 3.92 (m, 2H), 3.80 (s, 6H), 3.70 - 3.79 (m, 2H), 2.21 (s, 3H), 1.60 - 1.70 (m, 2H), 1.25 - 1.30 (t, *J* = 7.5 Hz, 3H),

**Step 2**

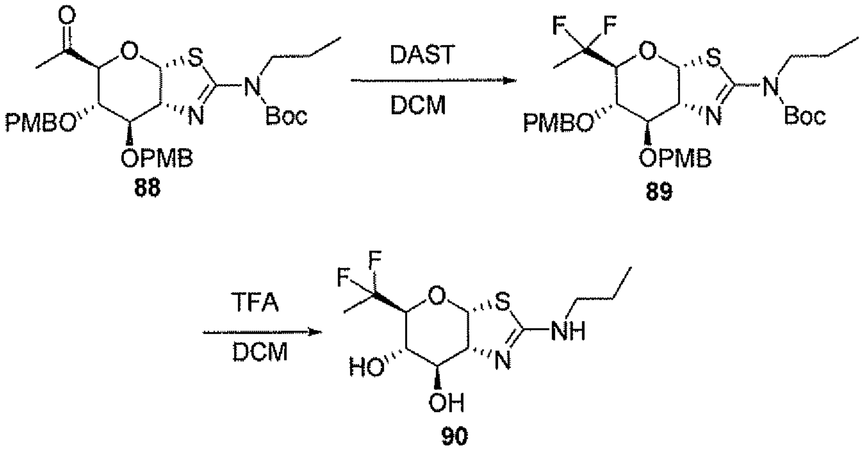


88 89 90

**(3aR,5S,6S,7R,7aR)-5-(1,1-Difluoroethyl)-2-(propylamino)-5,6,7,7a-tetrahydro-3aH-pyrano[3,2-d]thiazole-6,7-diol (90):** A solution **of 88** (200 mg, 0.33 mmol) in dichloromethane (10 mL) was treated with DAST (262 mg, 1.63 mmol) overnight at room temperature, then quenched by saturated aqueous $Na_2CO_3$ (10 mL), extracted with dichloromethane (3 x 10 mL). The combined organic layers were concentrated under vacuum to give crude compound **89** as a yellow foam (100 mg), which was dissolved into dichloromethane) (5 mL) and treated with TFA (0.5 mL) for 3 hours at room temperature. Removal of volatiles gave a residue, which was purified by Prep-HPLC with the following conditions [(Agilent 1200 prep HPLC): Column, SunFire Prep C18,19 * 50 mm 5 um; mobile phase, $H_2O$ with 0.03 % $NH_4OH$ and $CH_3CN$ (10% $CH_3CN$ up to 45 % in 10 min); Detector, UV 220 nm] to give the title compound **90** (**Example 147**) as a yellow solid (21.8 mg, 21.8 %). (ES, *m/z*) $[M+H]^+$ 297.0; $^1H$ NMR (300 MHz, $D_2O$) δ 6.20 - 6.22 (d, *J* = 6.6 Hz, 1H), 4.33 - 4.36 (t, *J* = 4.2 Hz, 1H), 4.15 - 4.18 (t, *J* = 3.0 Hz, 1H), 3.93 - 3.97 (m, 1H), 3.56 - 3.66 (m, 1H), 3.10 - 3.14 (t, *J* = 6.9 Hz, 2H), 1.43 -1.68 (m, 5H), 0.81 - 0.86 (t, *J*= 7.5 Hz, 3H).

The following compound was prepared in a manner analogous to Example 147.

**Example 148**

(3aR,5S,6S,7R,7aR)-5-(1,1 -difluoroethyl)-2-(methylamino)-5,6,7,7a-tetrahydro-3aH-pyrano[3,2-d]thiazole-6,7-diol

| Example | structure | Name | MH+ |
|---|---|---|---|
| 148 | 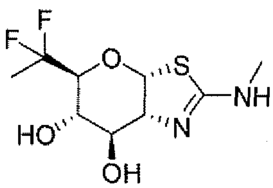 | (3aR,5S,6S,7R,7aR)-5-(1,1-difluoroethyl)-2-(methylamino)-5,6,7,7a-tetrahydro-3aH-pyrano[3,2-d]thiazole-6,7-diol | 269.0 |

**Examples 149 & 150**

**(3aR,5S,6S,7R,7aR)-5-((S)-2,2-difluoro-1-hydroxyethyl)-2-(dimethylamino)-5,6,7,7a-tetrahydro-3aH-pyrano[3,2-d]thiazole-6,7-diol (Example 149) & (3aR,5S,6S,7R,7aR)-5-((R)-2,2-difluoro-1-hydroxyethyl)-2-(dimethylamino)-5,6,7,7a-tetrahydro-3aH-pyrano[3,2-d]thiazole-6,7-diol (Example 150):**

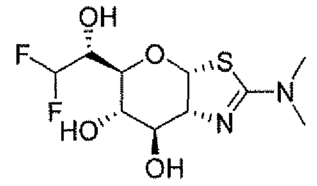
Example 149

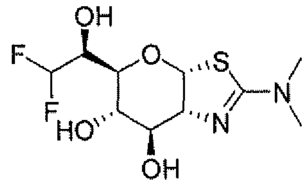
Example 150

SCHEME XIX

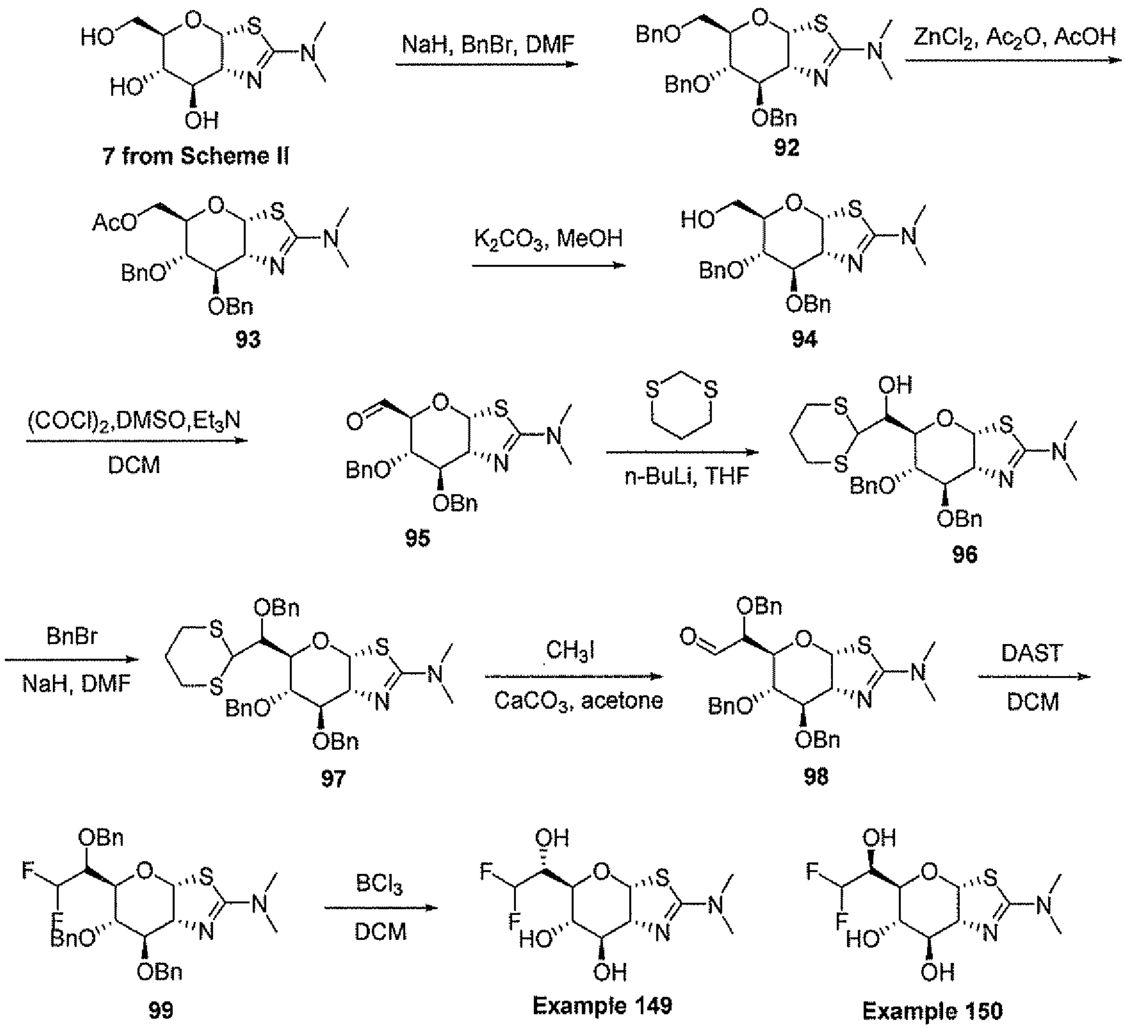

7 from Scheme II
92
93
94
95
96
97
98
99
Example 149
Example 150

**Step 1.**

**(3aR,5R,6S,7R,7aR)-6,7-bis(benzyloxy)-5-(benzyloxymethyl)-N,N-dimethyl-5,6,7,7a-tetrahydro-3aH-pyrano[3,2-d]thiazol-2-amine (92):** A solution of 7 (100 g, 0.4 mol) in DMF (600 mL) was treated with NaH (110 g, 3.2 mol, 70% dispersed by mineral oil) at 0°C for 30 min, followed by the addition of BnBr (410 g, 2.4 mol) dropwise. After kept additional 2 hours at room temperature, the mixture was poured into ice-water (1.5 kg) slowly and extracted with ethyl acetate (3 x 500 mL). The organic layers were combined, washed with brine (3 x 300 mL), dried over anhydrous sodium sulfate and concentrated under reduced pressure to give a residue, which was purified by a silica gel column, eluted with 10 % - 30 % ethyl acetate in petroleum ether to afford **92** (166 g, 80%) as a yellow oil; (ES, *m/z*) $[M+H]^+$ 519.0; $^1H$ NMR (300 MHz, $CDCl_3$) $\delta$ 7.26 - 7.48 (m, 15H), 6.40 (d, *J* = 6.6 Hz, 1H), 4.54 - 4.86 (m, 6H), 4.41 (d, *J* = 4.8 Hz, 1H), 4,17 - 4.18 (m, 1H), 3.60 - 3.79 (m, 4H), 3.12 (s, 6H).

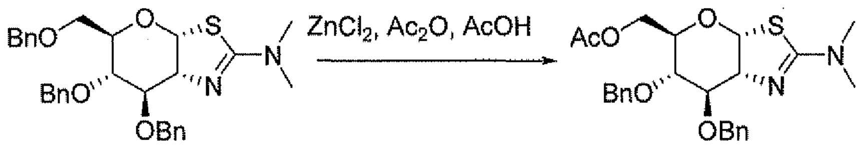


92 93

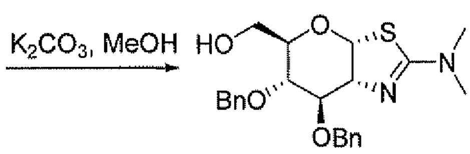


94

**Step 2.**

**((3aR,5R,6S,7R,7aR)-6,7-bis(benzyloxy)-2-(dimethylamino)-5,6,7,7a-tetrahydro-3aH-pyrano[3,2-d]thiazol-5-yl)methanol (94):** To a solution of **92** (166 g, 0.3 mol) in $Ac_2O$ (1 L) and AcOH (100 mL) was added anhydrous $ZnCl_2$ (353 g, 2,6 mol) at 0°C. After kept additional 2 hours at room temperature, the reaction was poured into ice-cold $H_2O$ (1.5 kg) slowly and extracted with dichloromethane (3 x 500 mL). The organic layers combined, washed with brine (3 x 200 mL) and dried over anhydrous sodium sulfate. After filtration, volatiles were distilled out by high vacuum to give crude **93** (160g, (ES, *m*/*z*) $[M+H]^+$ 471.0) as a brown oil. A solution of the above crude **93** in methanol (1 L) was treated with $K_2CO_3$ (18 g, 0.13 mol) at 30°C for 8 hours, after filtration, the solvent was distilled out under vacuum to give a residue, which was purified by a silica gel column with 20 % - 30% ethyl acetate in petroleum ether to afford the title compound **(94)** (108 g, 79% 2 steps) as a yellow syrup; (ES, *m*/*z*) $[M+H]^+$ 429.0; $^1$H NMR (300 MHz, $CDCl_3$) δ 7.26 - 7.43 (m, 10H), 6.29 (d, *J* = 6.6 Hz, 1H), 4.54 - 4.81 (m, 4H), 4.41 (d, *J* = 4.8 Hz, 1H), 4.17 - 4.18 (m, 1H), 3.57 - 3,79 (m, 4H), 3.02 (s, 6H).

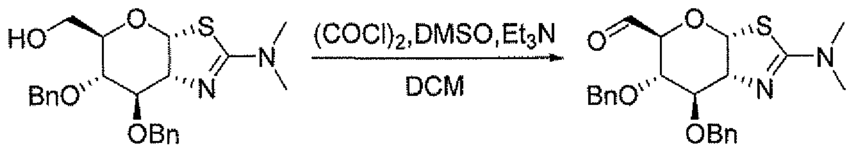


95

**Step 3.**

**(3aR,5S,6S,7R,7aR)-6,7-Bis(benzyloxy)-2-(dimethylamino)-5,6,7,7a-tetrahydro-3aH-pyrano[3,2-d]thiazole-5-carbaldehyde (95).** A solution of DMSO (14.6 g, 187 mmol) in dichloromethane (300 mL) was treated with oxalyl dichloride (17.7 g, 140 mmol) at -78 °C for 1 hour, then a solution of ((3aR,5R,6S,7R,7aR)-6,7-bis(benzyloxy)-2-(dimethylamino)-5,6,7,7a-tetrahydro-3aH-pyrano[3,2-d]thiazol-5-yl)methanol (10 g, 23 mmol) in dichloromethane (30 mL) was added slowly. The resulted solution was kept for 4 hours at -20 °C, followed by the addition of triethylamine (28.3 g, 280 mnnol) at -78 °C. After additional 1 hour at -50 °C., the reaction was quenched by water (300 mL) and extracted with dichloromethane (2x200 mL). The organic layers combined, washed with brine (3x100 mL) and dried over anhydrous sodium sulfate. After filtration, the filtrates were concentrated under vacuum to give a residue, which was purified by a silica gel column, eluted with 20 % ethyl acetate in dichloromethane to give compound **95** as a yellow syrup (8.1 g, 80 %). (ES, *m*/*z*): $[M+H]^+$ 426.7; $^1$H NMR (300 MHz, $CDCl_3$) δ 9.62 (s, 1H), 7.28 - 7.39 (m, 10H), 6.27 - 6.34 (m, 1H), 4.41 - 4.84 (m, 6H), 4.10 - 4.17 (m, 1H), 3.86 - 3.94 (m, 1H), 3.02 (s, 6H).

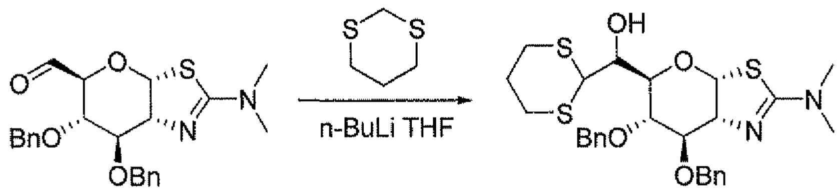


95 96

Step 4.

**(R)-((3aR,5R,6S,7R,7aR)-6,7-bis(benzyloxy)-2-(dimethylamino)-5,6,7,7a-tetrahydro-3aH-pyrano[3,2-d]thiazol-5-yl)(1,3-dithian-2-yl)methanol (96).** A solution of 1,3-dithiane (14.1 g, 117 mmol) in THF (100 mL) was treated with n-BuLi (44.9 mL, 112 mmol, 2.5M in hexane) at 0 °C for 1 hour, followed by the addition of a solution of the above aldehyde **95** in THF (20 mL) at -50°C. After kept additional 1 hour at 0°C, the reaction was quenched by saturated aqueous $NH_4Cl$ (150 mL) and extracted with ethyl acetate (3x80 mL). The combined organic layer was dried over anhydrous sodium sulfate, filtered and concentrated under reduced pressure to give a residue, which was purified by a silica gel column, eluted with 20% - 50% ethyl acetate in petroleum to afford the title compound as a yellow syrup (6.4 g, 62%,two epimers' ratio is 1:1 by $^1H$ NMR); (ES, *m/z)* $[M+H]^+$ 547.0; $^1H$ NMR (300 MHz, $CDCl_3$) $\delta$ 7.28 - 7.44 (m, 10H), 6.33 (d, *J*= 6.6 Hz, 1H), 4.63 - 4.84 (m, 4H), 4.52 - 4.58 (m, 1H), 4.12 - 4.15 (m, 1H), 3.98 - 4.05 (m, 3H), 3.82 - 3.85 (m, 1H), 2.99 (s, 6H), 2.80 - 2.87 (m, 1H), 2.61 - 2.68 (m, 3H), 1.65 - 1,69 (m, 2H).

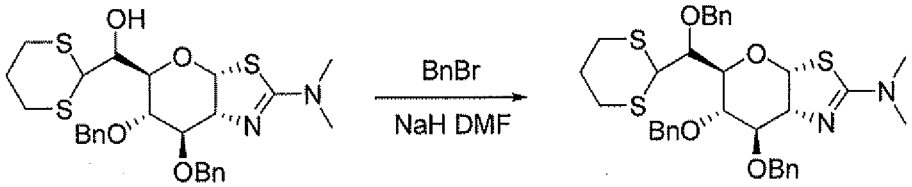


96 97

Step 5.

**(3aR,5S,6S,7R,7aR)-6,7-bis(benzyloxy)-5-((R)-benzyloxy(1,3-dithian-2-yl)methyl)-N,N-dimethyl-5,6,7,7a-tetrahydro-3aH-pyrano[3,2-d]thiazol-2-amine (97)**. A solution of **96** (3.3 g, 6 mmol) in DMF (40 mL) was treated with sodium hydride (830 mg, 24 mmol, 70% mineral oil dispersed) for 30 min at room temperature, followed by the addition of (bromomethyl)benzene (2.1 g, 12 mmol). After additional 1 hour at room temperature, the reaction was quenched by water (100 mL) and extracted with ethyl acetate (3x40 mL). The combined organic layer was washed with brine (5x30 mL), dried over anhydrous sodium sulfate, filtered and concentrated under reduced pressure to give a residue, which was purified by a silica gel column, eluted with 10% - 30% ethyl acetate in petroleum ether to afford **97** as a brown syrup (2.8 g, 73%); (ES, *m*/*z*) $[M+H]^+$ 637.1; $^1H$ NMR (300 MHz, $CDCl_3$) $\delta$ 7.24 - 7.41 (m, 15H), 6.32 - 6.34 (d, *J* = 6.3 Hz, 1H), 4.65 - 4.82 (m, 5H), 4.37 - 4.39 (m, 3H), 4.05 - 4.25 (m, 3H), 3.76 - 3.89 (m, 1H), 3.02 (s, 6H), 2.77 - 2.92 (m, 4H), 1.64 - 1.68 (m, 2H).

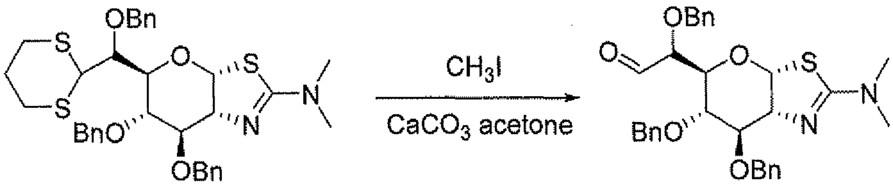


97 98

Step 6.

**(R)-2-(benzyloxy)-2-((3aR,5S,6S,7R,7aR)-6,7-bis(benzyloxy)-2-(dimethylamino)-5,6,7,7a-tetrahydro-3aH-pyrano[3,2-d]thiazol-5-yl)acetaldehyde (98).** To a solution of **97** (3 g, 4.7 mmol) in acetone (16 mL) and water (4 mL) was added $CaCO_3$ (4.7 g, 47 mmol) and $CH_3I$ (13.2 g, 94 mmol). After kept 20 hours at 50°C, the resulting solution

was diluted with dichloromethane (30 mL), filtered and extracted with dichloromethane (2 x 30 mL). The combined organic layer was dried over anhydrous sodium sulfate and concentrated under reduced pressure to afford crude **98,** which was used in next step directly; (ES, *m/z*) $[M+H]^+$ 547.0.

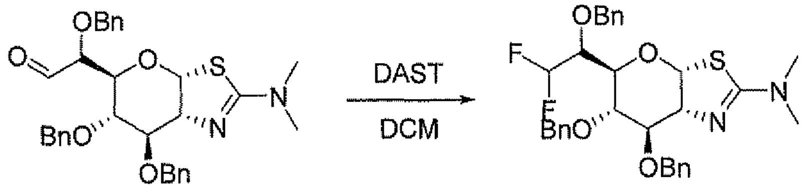


**98** **99**

**Step 7.**

**(3aR,5S,6S,7R,7aR)-6,7-bis(benzyloxy)-5-((R)-1-(benzyloxy)-2,2-difluoroethyl)-N,N-dimethyl-5,6,7,7a-tetrahydro-3aH-pyrano[3,2-d]thiazol-2-amine (99).** To a solution of the above crude **98** in dichloromethane (20 mL) was added DAST (2.9 g, 18 mmol) at -78°C under nitrogen atmosphere. After additional 2 hours at 0 °C, the reaction was quenched with water (10 mL) and neutralized by saturated aqueous sodium carbonate. The resulting solution was extracted with dichloromethane) (3x20 mL) and the combined organic layer was washed with brine (20 mL), dried over anhydrous sodium sulfate. After filtration, the filtrates were concentrated under reduced pressure to give a residue, which was purified by a silica gel column, eluted with 10% - 50% ethyl acetate in petroleum ether to afford 99 as a yellow syrup (1.1 g, 41%); (ES, *m/z*) $[M+H]^+$ 568.9; $^1$H NMR (300 MHz, $CDCl_3$) δ 7.31 - 7.46 (m, 15H), 6.34 (d, *J*= 6.6 Hz, 1H), 5.61 - 5.95 (m, 1H), 4.64 - 4.79 (m, 5H), 4.64 - 4.79 (m, 1 H), 4.09 - 4.23 (m, 4H), 3.74 - 3.82 (m, 1H), 3.02 (s, 6H).

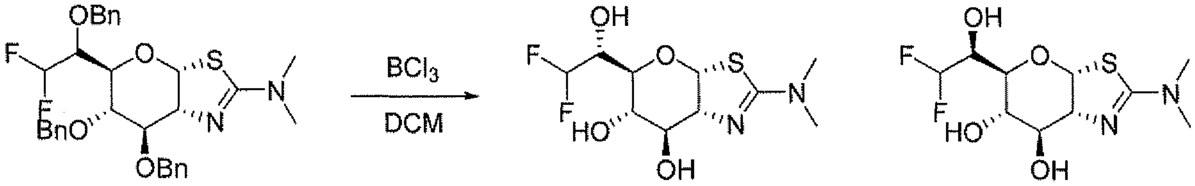


**99** **Example 149** **Example 150**

**Step 8.**

**(3aR,5S,6S,7R,7aR)-5-((S)-2,2-difluoro-1-hydroxyethyl)-2-(dimethylamino)-5,6,7,7a-tetrahydro-3aH-pyrano[3,2-d]thiazole-6,7-diol (Example 149) & (3aR,5S,6S,7R,7aR)-5-((R)-2,2-difluoro-1-hydroxyethyl)-2-(dimethylamino)-5,6,7,7a-tetrahydro-3aH-pyrano[3,2-d]thiazole-6,7-diol (Example 150).** A solution of **99** (600 mg, 1 mmol) in dichloromethane) (20 mL) was treated with $BCl_3$ (10 mL, 10 mmol, 1 M in dichloromethane) for 1 hour at -78 °C, then quenched with methanol (20 mL). Volatiles were distilled out to give a residue, which was dissolved into methanol (5 mL) and neutralized with Con. $NH_4OH$ (2 ml, 26% aqueous solution). After concentration, the crude product was purified by a silica gel column, eluted with 10 % methanol in dichloromethane to give a mixture of the two epimers; the two epimers were separated by Prep-HPLC with the following conditions: (Agilent Prep 1200 Detecl): Column, SunFire Prep C18; mobile phase, Water with 0.05% ammonia and $CH_3CN$, 5% $CH_3CN$ up to 40% in 8 min; Detector, 220nm) to afford (3aR,5S,6S,7R,7aR)-5-((S)-2,2-difluoro-1-hydroxyethyl)-2-(dimethylamino)-5,6,7,7a-tetrahydro-3aH-pyrano[3,2-d]thiazole-6,7-diol as a light yellow solid (14.4 mg, 5%), faster eluting isomer; (ES, *m/z*) $[M+H]^+$ 299.0, $^1$H NMR (300 MHz, $D_2O$) δ 6.19 (d, *J* = 6.6 Hz, 1H), 5,71 - 6.09 (m, 1 H), 4.15 (t, *J* = 6.0 Hz, 1H), 3.89 - 3.99 (m, 2H), 3.78 - 3.82 (m, 1H), 3.37 (t, *J* = 8.7 Hz, 1H), 2.89 (s, 6H); and (3aR,5S,6S,7R,7aR)-5-((R)-2,2-difluoro-1-hydroxyethyl)-2-(dimethylamino)-5,6,7,7a-tetrahydro-3aH-pyrano[3,2-d]thiazole-6,7-diol as a light yellow solid (53.9 mg, 17.4%), slower eluting isomer; (ES, *m/z*) $[M+H]^+$ 299.0, $^1$H NMR (300 MHz, $D_2O$) δ 6.20 (d, *J* = 6.3 Hz, 1H), 5.62 - 6.00 (m, 1H), 4.07 (t, *J* = 6.0 Hz, 1H), 3.87 - 3.96 (m, 2H), 3.72 - 3.77 (m, 1H), 3.62 - 3.65 (m, 1H), 2.88 (s, 6H).

**Examples 151 & 152**

**(3aR,5R,6S,7R,7aR)-5-((S)-1,2-dihydroxyethyl)-2-(dimethylamino)-5,6,7,7a-tetrabydro-3aH-pyrano[3,2-d]thiazole-6,7-diol (Example 151) & (3aR,5R,6S,7R,7aR)-5-((R)-1,2-dihydroxyethyl)-2-(dimethylamino)-5,6,7,7a-tetrahydro-3aH-pyrano[3,2-d]thiazole-6,7-diol (Example 152):**

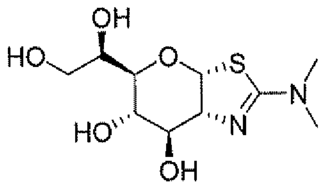

**Example 151**

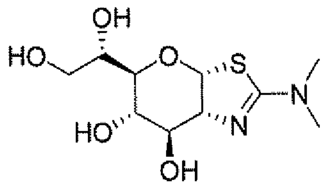

**Example 152**

Scheme XX

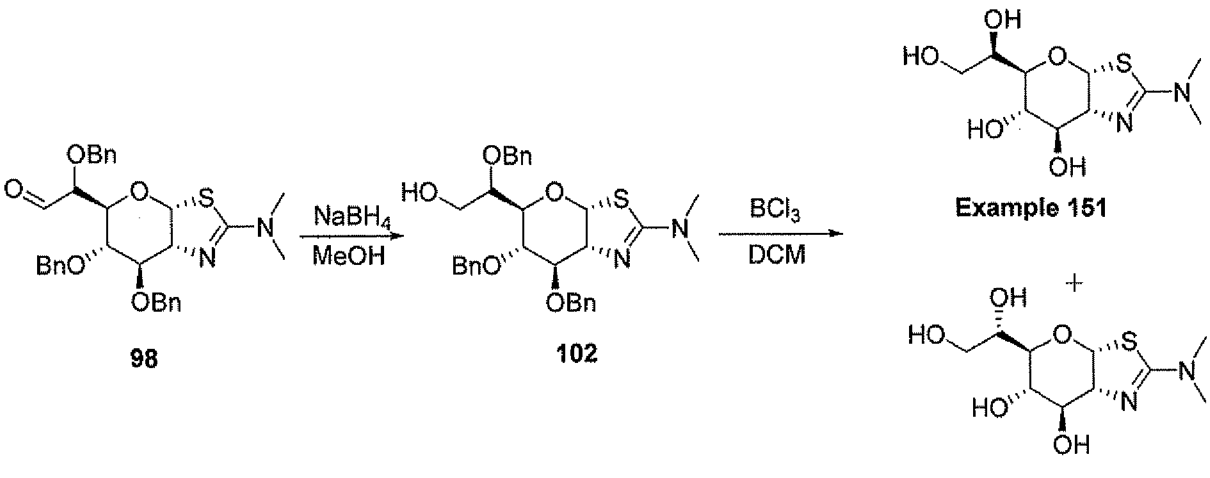


**98** **102** **Example 151** + **Example 152**

**Step 1**

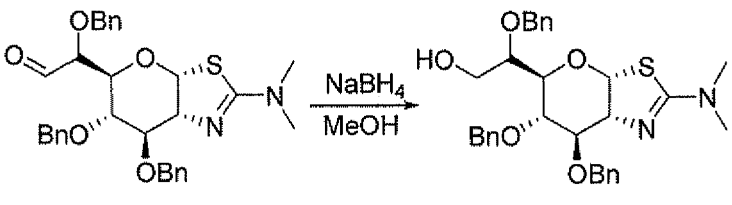


**98** **102**

**(S)-2-(benzyloxy)-2-((3aR,5R,6S,7R,7aR)-6,7-bis(benzyloxy)-2-(dimethylamino)-5,6,7,7a-tetrahydro-3aH-pyrano[3,2-d]thiazol-5-yl)ethanol (102):** A solution of **98** (1.5 g, 2.7 mmol) (*Prepared according to the synthesis of **Example 149 and 150***), ***step 6***) was treated with $NaBH_4$ (504 mg, 13 mmol) for 1 hour at 0°C. The resulting solution was diluted with $H_2O$ (60 mL) and extracted with ethyl acetate (3x30 mL). The combined organic layer was dried over anhydrous sodium sulfate, filtered and concentrated to give a residue, which was purified by silica gel column, eluted with 20% - 50% ethyl acetate in petroleum ether to give **102** as brown syrup (1.2 g, 80%); (ES, *m/z*): $[M+H]^+$ 549.1; $^1H$ NMR (300 MHz, $CDCl_3$) $\delta$ 7.22 - 7.45 (m, 15H), 6.35 (d, *J* = 6.9 Hz, 1H), 4.61 - 4.80 (m, 4H), 4.47 - 4.53(m, 2H), 4.25 - 4.36 (m, 1H), 4.08 - 4.12 (m, 1H), 3.89 - 3.92 (m, 1H), 3.46 - 3.78 (m, 4H), 3.01 (s, 6H).

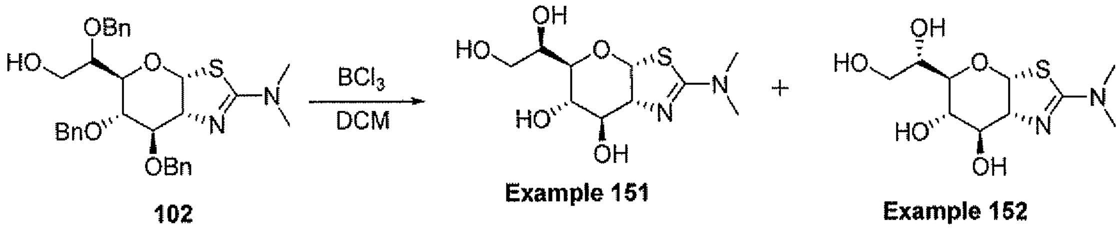


102

Example 151

Example 152

**(3aR,5R,6S,7R,7aR)-5-((S)-1,2-dihydroxyethyl)-2-(dimethylamino)-5,6,7,7a-tetrahydro-3aH-pyrano[3,2-d]thiazole-6,7-diol (Example 152) & (3aR,5R,6S,7R,7aR)-5-((R)-1,2-dihydroxyethyl)-2-(dimethylamino)-5,6,7,7a-tetrahydro-3aH-pyrano[3,2-dlthiazole-6,7-diol (Example 151):** A solution of **102** (500 mg, 0.9 mmol) in dichloromethane (20 mL) was treated with $BCl_3$ (9 ml, 9 mmol, 1 M in dichloromethane) at -60 °C under nitrogen for 30 min. The reaction was then quenched with methanol (20 mL). Volatiles were distilled out to give a residue, which was dissolved into methanol (5 mL) and neutralized with Con. $NH_4OH$ (2 ml, 26% aqueous solution). After concentration, the crude product was purified by a silica gel column, eluted with 10 % methanol in dichloromethane to give a mixture of the two epimers; the two epimers were separated by Prep-HPLC with the following conditions: Agilent Prep 1200 Detecl): Column, SunFire Prep C 18; mobile phase, Water with 0,05% ammonia and $CH_3CN$; 5% $CH_3CN$ up to 60% in 8 min; Detector, 220nm) to afford (3aR,5R,6S,7R,7aR)-5-((R)-1,2-dihydroxyethyl)-2-(dimethylamino)-5,6,7,7a-tetrahydro-3aH-pyrano[3,2-d]thiazole-6,7-diol as a white solid (23.1 mg, 9%), Faster eluting isomer; (ES, *m/z):* $[M+H]^+$ 279.0; $^1H$ NMR (300 MHz, $D_2O$) δ 6.17 (d, *J* = 6.3 Hz, 1H), 4.13 (t, *J* = 6.0 Hz, 1H), 3.95 (t, *J* = 4.5 Hz, 1H), 3.78 - 3.83 (m, 1 H), 3.61 - 3.71 (m, 2H), 3.50 - 3.54 (m, 2H), 2.88 (s, 6H); and (3aR,5R,6S,7R,7aR)-5-((S)-1,2-dihydroxyethyl)-2-(dimethylamino)-5,6,7,7a-tetrahydro-3aH-pyrano[3,2-d]thiazole-6,7-diol as a white solid (37.5 mg, 13%), Slower eluting isomer; (ES, *m/z*)*:* $[M+H]^+$ 279.0; $^1H$ NMR (300 MHz, $D_2O$) δ 6.18 (d, *J* = 6.3 Hz, 1H), 4.05 (t, *J* = 6.0 Hz, 1H), 3.90 (t, *J* = 5.1 Hz, 1H), 3.75 - 3.81 (m, 1H), 3.65 - 3.69 (m, 1H), 3.44 - 3.53 (m, 3H), 2.87 (s, 6H).

**Examples 153 & 154**

**(3aR,5S,6S,7R,7aR)-2-(dimethylamino)-5-((S)-2-fluoro-1-hydroxyethyl)-5,6,7,7a-tetrahydro-3aH-pyrano[3,2-d]thiazole-6,7-diol (Example 153) & (3aR,5S,6S,7R,7aR)-2-(dimethylamino)-5-((R)-2-fluoro-1-hydroxyethyl)-5,6,7,7a-tetrahydro-3aH-pyrano[3,2-d]thiazole-6,7-diol (Example 154):**

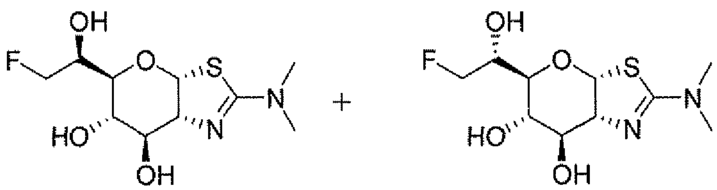

Example 153

Example 154

Scheme XXI

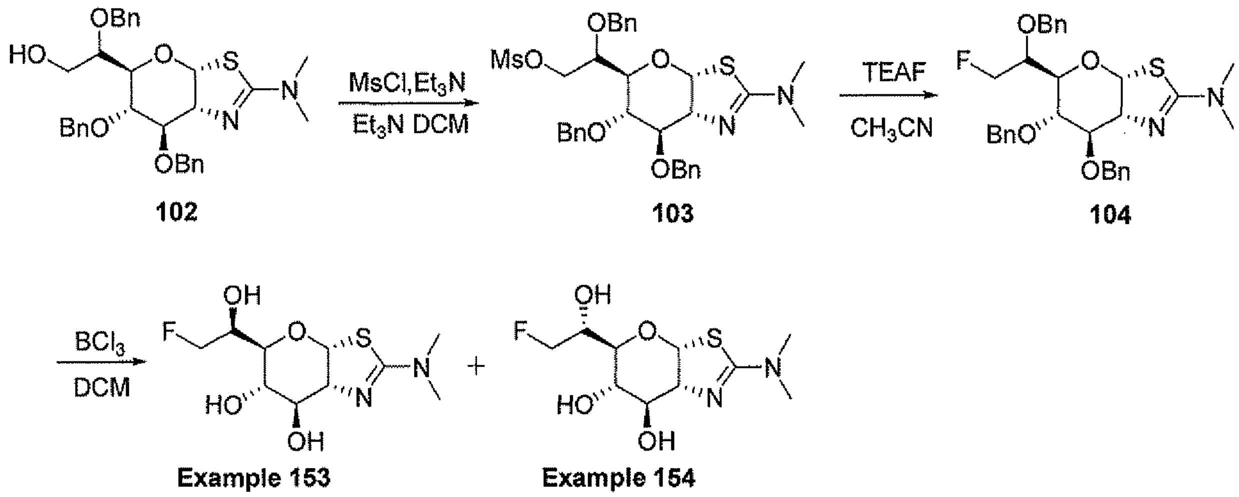


102 103 104

Example 153 Example 154

**Step 1**

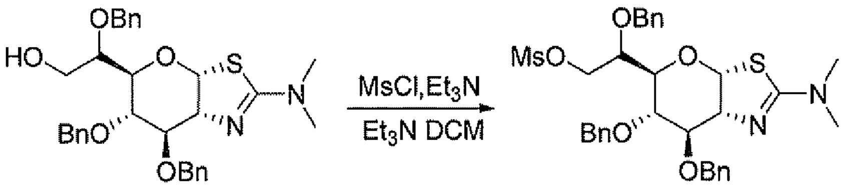


102 103

**(S)-2-(benzyloxy)-2-((3aR,5R,6S,7R,7aR)-6,7-bis(benzyloxy)-2-(dimethylamino)-5,6,7,7a-tetrahydro-3aH-pyrano[3,2-d]thiazol-5-yl)ethyl methanesulfonate (103):**

To a solution of **102** (1.2 g, 2.2 mmol) (*Prepared according to the synthesis of* **Examples 151 and 152, *step 1*)** in dichloromethane (30 mL) was added TEA (664 mg, 6.6 mmol) and MsCl (499 mg, 4.4 mmol) at 0°C. After kept 2 hours at room temperature, the reaction was quenched by water (100 mL) and extracted with dichloromethane (3x30 mL). The combined organic layer was washed with brine (20 mL) and dried over anhydrous sodium sulfate. After filtration, the filtrates were concentrated to give a residue, which was purified by silica gel column with 10% - 30% ethyl acetate in petroleum ether to give the title compound (1.1 g, 80%) as brown syrup; (ES, *m/z*): $[M+H]^+$ 627.0; $^1H$ NMR (300 MHz, $CDCl_3$) δ 7.22 - 7.45 (m, 15H), 6,35 (d, *J* = 6.3 Hz, 1H), 5.03 - 5.15 (m, 2H), 4.61 - 4.80 (m, 4H), 4.47 - 4.53 (m, 2H), 4.25 - 4.36 (m, 1H), 4.08 - 4.12 (m, 1H), 3.89 - 3.92 (m, 1H), 3.46 - 3.78 (m, 2H), 3.06 (s, 6H), 3.01 (s, 3H).

**Step 2**

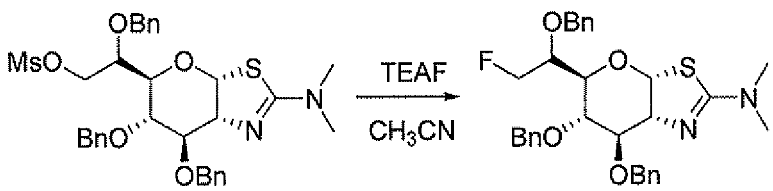


103 104

**(3aR,5S,bS,7R,7aR)-6,7-bis(benzyloxy)-5-((R)-1-(benzyloxy)-2-fluoroethyl)-N,N-dimethyl-5,6,7,7a-tetrahydro-3aH-pyrano[3,2-d]thiazol-2-amine (104):** A solution of **103** (1 g, 1.6 mmol) in $CH_3CN$ (20 mL) was treated with TEAF (2.38 g, 16 mmol) for 20 hours at reflux, then cooled to room temperature and poured into water (100 mL), extracted with dichloromethane (3x50 mL). The combined organic layer was washed with brine (2x50 mL), dried over anhydrous sodium sulfate and concentrated under reduced pressure to give a residue, which was purified by silica gel column with 20% - 50% ethyl acetate in petroleum ether to afford the title compound (400 mg, 46%) as light yellow syrup; (ES, *m/z*): $[M+H]^+$ 551.1; $^1H$ NMR (300 MHz, $CDCl_3$) δ 7.28 - 7.44 (m, 15H), 6.38 (d, *J* = 6.3 Hz, 1H), 4.61 - 4.80 (m, 4H), 4.47 - 4.53(m, 2H), 4.27 - 4.37 (m, 1H), 4.08 - 4.12 (m, 1H), 3.91 - 3.95 (m, 1H), 3.52 - 3.78 (m, 4H), 3.12 (s, 6H).

Step 3

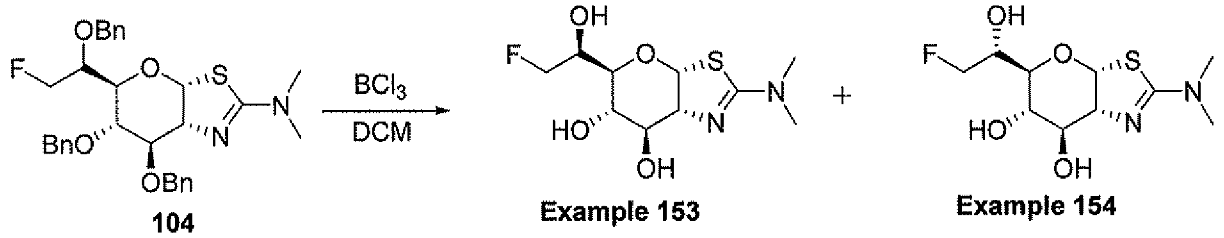


104 Example 153 Example 154

**(3aR,5S,6S,7R,7aR)-2-(dimethylamino)-5-((S)-2-fluoro-1-hydroxyethyl)-5,6,7,7a-tetrahydro-3aH-pyrano[3,2-d]thiazole-6,7-diol (Example 153) & (3aR,5S,6S,7R,7aR)-2-(dimethylamino)-5-((R)-2-fluoro-1-hydroxyethyl)-5,6,7,7a-tetrahydro-3aH-pyrano[3,2-d]thiazole-6,7-diol (Example 154):** A solution of **104** (400 mg, 0.7 mmol) in dichloromethane (15 mL) was treated with $BCl_3$ (7 mL, 7 mmol, 1M in dichloromethane) for 30 min at -60°C, then quenched with methanol (20 mL). Volatiles were distilled out to give a residue, which was dissolved into methanol (5 mL) and neutralized with Con. $NH_4OH$ (2 ml, 26% aqueous solution). After concentration, the crude product was purified by a silica gel column, eluted with 10 % methanol in dichloromethane to give a mixture of the two epimers; the two epimers were separated by Prep-HPLC with the following conditions: (Agilent Prep 1200 Detecl): Column, SunFire Prep C18; mobile phase, Water with 0.05% ammonia and $CH_3CN$; 5% $CH_3CN$ up to 100% in 8 min Detector, 220nm to afford (3aR,SS,6S,7R,7aR)-2-(dimethylamino)-5-((S)-2-fluoro-1-hydroxyethyl)-5,6,7,7a-tetrahydro-3aH-pyrano[3,2-d]thiazole-6,7-diol as a white solid (46.3 mg, 23%), faster eluting isomer; (ES, *m/z*): $[M+H]^+$ 281.0; $^1H$ NMR (300 MHz, $D_2O$) δ 6.17 (d, *J* = 6.6 Hz, 1H), 4.29 - 4.69 (m, 2H), 3.95 - 4.15(m, 3H), 3.68 - 3.72 (m, 1H), 3.54 - 3.58 (m, 1H), 2.88 (s, 3H), 2.87 (s, 3H); and (3aR,5S,6S,7R,7aR)-2-(dimethylamino)-5-((R)-2-fluoro-1-hydroxyethyl)-5,6,7,7a-tetrahydro-3aH-pyrano[3,2-d]thiazole-6,7-diol as a white solid (64 mg, 31%), slower eluting isomer; (ES, *m/z*): $[M+H]^+$ 281.0; $^1H$ NMR (300 MHz, $D_2O$) δ 6.17 (d, *J* = 6.3 Hz, 1H), 4.44 - 4.57 (m, 1H), 4.28 - 4.39 (m, 1H), 3.92 - 4.12 (m, 3H), 3.67 - 3.72 (m, 1H), 3.27 - 3.49 (m, 1H), 2.89 (s, 3H), 2.87 (s, 3H).

**Example 155**

**(3aR,5R,6S,7R,7aR)-2-(dimethylamino)-5-(2-fluoroethyl)-5,6,7,7a-tetrahydro-3aH-pyrano[3,2-d]thiazole-6,7-diol :**

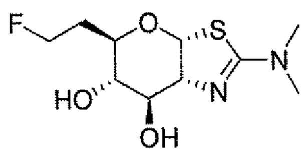

Example 155

Scheme XXII

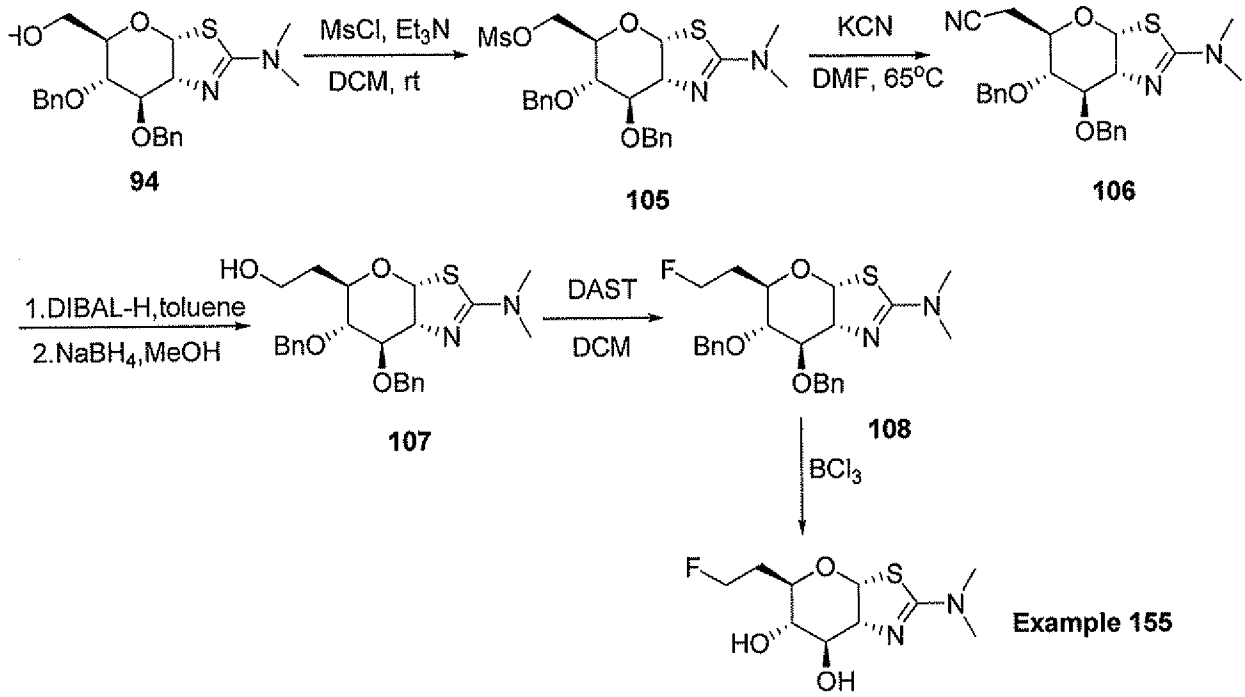


94 105 106 107 108

Example 155

**Step 1**

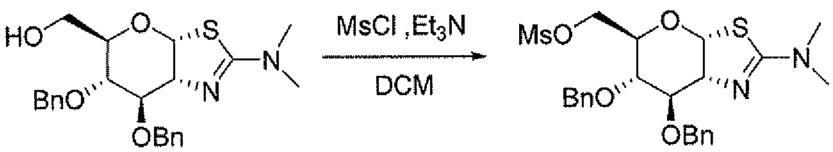


94 105

**((3aR,5R,6S,7R,7aR)-6,7-bis(benzyloxy)-2-(dimethylamino)-5,6,7,7a-tetrahydro-3aH-pyrano[3,2-d]thiazol-5-yl)methyl methanesulfonate (105).** To a solution of ((3aR,5R,6S,7R,7aR)-6,7-bis(benzyloxy)-2-(dimethylamino)-5,6,7,7a-tetrahydro-3aH-pyrano[3,2-d]thiazol-5-yl)methanol (**94**) (8 g, 18 mmol) (*Prepared according to the synthesis of* ***Example 149, step 2***) in dichloromethane (50 mL) was added methanesulfonyl chloride (4.3 g, 37 mmol) and triethylamine (3.8 g, 37 mmol) at 0 °C. After kept 2 hours at 25 °C, the reaction was quenched by water (50 mL) and extracted with dichloromethane (3x50 mL). The combined organic layer was washed with brine (2x30 mL), dried over anhydrous sodium sulfate, filtered and concentrated under reduced pressure to give a residue, which was purified by a silica gel column, eluted with 20% - 30% ethyl acetate in petroleum ether to afford the title compound as a brown syrup (8.5 g, 90%); (ES, *m*/*z*) $[M+H]^+$ 507.0; $^1$HNMR (300 MHz, $CDCl_3$) δ 7.28 - 7.48 (m, 10H), 6.38 (d, *J* = 6.6 Hz, 1H), 5.04 - 5.08 (m, 1H), 4.72 - 4.86 (m, 4H), 4.19 - 4.24 (m, 3H), 3.70 - 3.83 (m, 1H), 3.56 - 3.58 (m, 1H), 3.19 (s, 3H), 2.94 (s, 6H).

**Step 2**

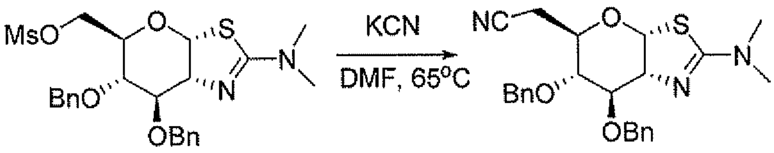


105 106

**2-((3aR,5R,6R,7R,7aR)-6,7-bis(benzyloxy)-2-(dimethylamino)-5,6,7,7a-tetrahydro-3aH-pyrano[3,2-d]thiazol-5-yl)acetonitrile (106).** A mixture of **105** (8.5 g, 17 mmol), KCN (2.7 g, 42 mmol) and KI (282 mg, 1.7 mmol) in DMF (80 mL) was heated to 65°C for 2 hours, then quenched by water (200 mL) and extracted with ethyl acetate (5x40 mL). The combined organic layer was washed with brine (4x30 mL), dried over anhydrous sodium sulfate and concentrated under reduced pressure to give a residue, which was purified by a silica gel column, eluted with 10% - 30% ethyl acetate in petroleum ether to afford the title compound as a yellow syrup (4.3 g, 55%); (ES, *m/z*) $[M+H]^+$ 438.0; $^1$HNMR (300 MHz, $CDCl_3$) δ 7.25 - 7.44 (m, 10H), 6.26 (d, *J* = 6.6 Hz, 1H), 4.57 - 4.84 (m, 3H), 4.54 - 4.56 (m, 1H), 4.35 - 4.39 (m, 1H), 4.23 - 4.26 (m, 1H), 3.70 - 3.82 (m, 1H), 3.54 - 3.55 (m, 1 H), 3.01 (s, 6H), 2.49 - 2.66 (m, 2H).

**Step 3**

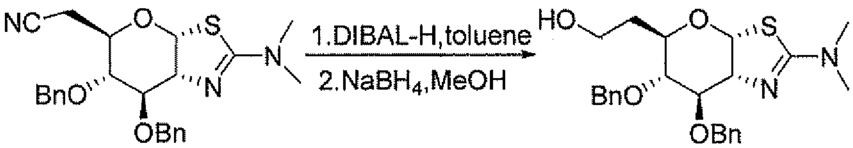


106 107

**2-((3aR,5R,6R,7R,7aR)-6,7-bis(benzyloxy)-2-(dimethylamino)-5,6,7,7a-tetrahydro-3aH-pyrano[3,2-d]thiazol-5-yl)ethanol (107).** A solution of **106** (400 mg, 0.9 mmol) in toluene (20 mL) was treated with DIBAL-H (2.3 mL, 2.3 mmol, 1M in toluene) for 1 hour at -25°C. Then the reaction was quenched with ice water (50 mL), extracted with ethyl acetate (3x40 mL). The combined organic layer was washed with brine (2x30 mL), dried over anhydrous sodium sulfate and concentrated under reduced pressure to afford crude aldehyde, which was dissolved into methanol (20 mL) and treated with $NaBH_4$ (105 mg, 2.7 mmol) for 1 hour at 25 °C. Then the reaction was quenched by water (50 mL) and extracted with ethyl acetate (3x30 mL). The combined organic layer was dried over anhydrous sodium sulfate and concentrated under reduced pressure to give a residue, which was purified by a silica gel column, eluted with 20% - 40% ethyl acetate in petroleum ether to afford **107** as a yellow syrup (270 mg, 67%); (ES, *m/z*) $[M+H]^+$ 443.0; $^1$HNMR (300 MHz, $CDCl_3$) δ 7.28 - 7.45 (m, 10H), 6.27 (d, *J* = 6.3 Hz, 1H), 4.65 - 4.81 (m, 4H), 4.29 - 4.37 (m, 2H), 3.66 - 3.75 (m, 3H), 3.51 - 3.55 (m, 1H), 3.01 (s, 6H), 1.93 - 1.99 (m, 2H).

**Step 4**

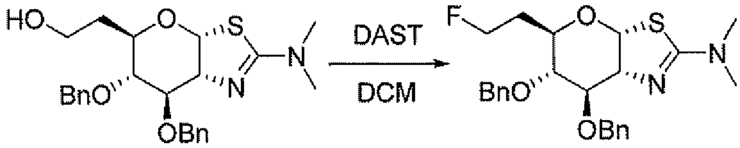


107 108

**(3aR,5R,6R,7R,7aR)-6,7-bis(benzyloxy)-5-(2-fluoraethyl)-N,N-dimethyl-5,6,7,7a-tetrahydro-3aH-pyrano[3,2-d]thiazol-2-amine (108).** A solution of **107** (250 mg, 0.6 mmol) in dichloromethane (20 mL) was treated with DAST (550 mg, 3.4 mmol) for 2 hours at 0 °C. Then the reaction was quenched by saturated aqueous $NaHCO_3$ (30 mL) and the aqueous layer was extracted with dichloromethane (2x20 mL). The combined organic layer was washed by brine (20 mL), dried over anhydrous sodium sulfate and concentrated under reduced pressure to give a residue, which was purified

by a silica gel column, eluted with 3% - 5% methanol in dichloromethane) to afford **108** as a white solid (135 mg, 45%); (ES, *m/z*) $[M+H]^+$ 445.0; $^1$HNMR (300 MHz, $CDCl_3$) δ 7.32 - 7.45 (m, 10H), 6.26 (d, *J* = 6.9 Hz, 1H), 4.57 - 4.82 (m, 5H), 4.40 - 4.56 (m, 3H), 3.64 - 3.71 (m, 1H), 3.47 - 3.49 (m, 1H), 3.01 (s, 6H), 2.06 - 2.25 (m, 1H), 1.70 - 1.82 (m, 1H).

**Step 5**

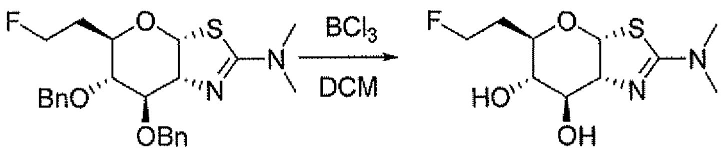


**108** **Example 155**

**(3aR,5R,6S,7R,7aR)-2-(dimethylamino)-5-(2-fluoroethyl)-5,6,7,7a-tetrahydro-3aH-pyrano[3,2-d]thiazole-6,7-diol.** A solution of **108** (300 mg, 0.7 mmol) in dichloromethane (20 mL) was treated with $BCl_3$ (7 mL, 7 mmol, 1 M in dichloromethane) for 2 hours at -60°C, then quenched by the addition of methanol (10 mL). Volatiles were distilled out under vacuum to give a residue, which was dissolved into methanol (5 mL) and neutralized by Con. $NH_4OH$ (2 ml, 26% aqueous solution). After concentration, the crude product was purified by a silica gel column, eluted with 10 % methanol in dichloromethane to give the title compound as a white solid (35 mg, 20%); (ES, *m/z*) $[M+H]^+$ 265.0; $^1$HNMR (300 MHz, $D_2O$) δ 6.16 (d, *J* = 6.3 Hz, 1H), 4.60 (t, *J* = 4.8 Hz, 1H), 4.45 (t, *J*= 4.8 Hz, 1H), 4.10 (t, *J* = 6.0 Hz, 1H), 3.92 (t, *J* = 4.8 Hz, 1H), 3.62 - 3.66 (m, 1H), 3.42 - 3.60 (m, 1H), 2.90 (s, 6H), 2.06 - 2.20 (m, 1H), 1.67 - 1.88 (m, 1H).

**Example 156**

**(3aR,5R,6S,7R,7aR)-5-(2,2-difluoroethyl)-2-(dimethylamino)-5,6,7,7a-tetrahydro-3aH-pyrano[3,2-d]thiazole-6,7-diol :**

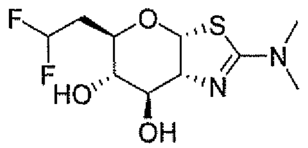

**Example 156**

Scheme XXIII

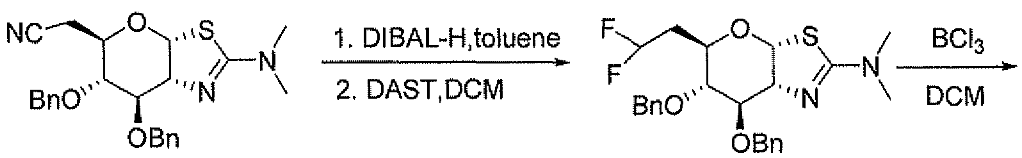


**106** **109**

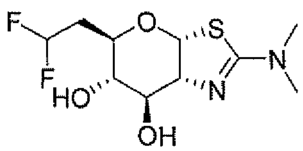

**Example 156**

**Step 1**

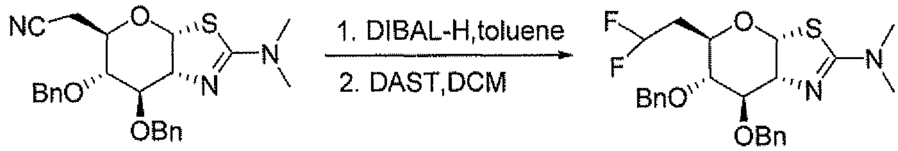


106 109

**(3aR,5R,6R,7R,7aR)-6,7-bis(benzyloxy)-5-(2,2-difluoroethyl)-N,N-dimethyl-5,6,7,7a-tetrahydro-3aH-pyrano[3,2-d]thiazol-2-amine (109).** A solution of **106** (400 mg, 0.9 mmol) (*Prepared according to the synthesis of Example 155, step 2)* in toluene (20 mL) was treated with DIBAL-H (2.3 mL, 2.3 mmol, 1M in toluene) for 1 hour at -25°C. Then the reaction was quenched with ice water (50 mL), extracted with ethyl acetate (3x40 mL). The combined organic layers were washed with brine (2x30 mL), dried over anhydrous sodium sulfate and concentrated under reduced pressure to afford crude aldehyde, which was dissolved into dichloromethane (20 mL) and treated with DAST (370 mg, 2.3 mmol) at - 78°C under nitrogen for 30 min. After additional 2 hours at 0 °C, the reaction was quenched with saturated aqueous sodium carbonate (10 mL) and the aqueous layer was extracted with dichloromethane) (3x20 mL). The combined organic layer was washed with brine (20 mL) and dried over anhydrous sodium sulfate. After filtration, the filtrates were concentrated under reduced pressure to give a residue, which was purified by a silica gel column, eluted with 10% - 40% ethyl acetate in petroleum ether to afford the title compound as a yellow syrup (110 mg, 48%); (ES, *m/z*) $[M+H]^+$ 463.0; $^1$HNMR (300 MHz, $CDCl_3$) $\delta$ 7.28 - 7.43 (m, 10H), 6.25 (d, *J* = 6.6 Hz, 1H), 5.71 - 6.09 (m, 1H), 4.59 - 4.81 (m, 4H), 4.25 - 4.37 (m, 2H), 3.67 - 3.74 (m, 1H), 3.44 - 3.47 (m, 1H), 3.03 (s, 6H), 2.16 - 2.24 (m, 1H), 1.88 - 2.08 (m, 1H).

**Step 2**

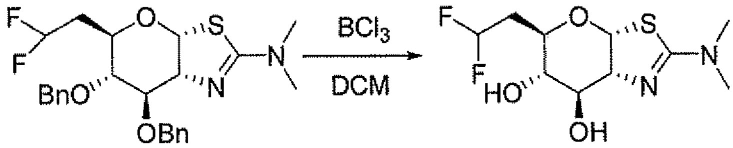


109 **Example 156**

**(3aR,5R,6S,7R,7aR)-5-(2,2-difluoroethyl)-2-(dimethylamino)-5,6,7,7a-tetrahydro-3aH-pyrano[3,2-d]thiazole-6,7-diol.** A solution of **109** (270 mg, 0.6 mmol) in dichloromethane (20 mL) was treated with $BCl_3$ (6 mL, 6 mmol, 1M in dichloromethane) for 2 hours at -60°C, then the reaction was quenched with methanol (20 mL). Volatiles were distilled out under vacuum to give a residue, which was dissolved into methanol (5 mL) and neutralized by Con. $NH_4OH$ (2 ml, 26% aqueous solution). After concentration, the crude product was purified by a silica gel column, eluted with 10 % methanol in dichloromethane to give the title compound as a white solid (41 mg, 25%); (ES, *m/z*) $[M+H]^+$ 283.0; $^1$HNMR (300 MHz, $D_2O$) $\delta$ 6.17 (d, *J* = 6.3 Hz, 1 H), 5.77 - 6.14 (m, 1H), 4.12 (t, *J* = 6.0 Hz, 1H), 3.93 (t, *J* = 5.1 Hz, 1H), 3.68 - 3.73 (m, 1H), 3.43 - 3.48 (m, 1H), 2.91 (s, 6H), 1.99 - 2.35 (m, 2H).

**Example 157**

**(3aR,5R,6S,7R,7aR)-2-(dimethylamino)-5-(2,2,2-trifluoroethyl)-5,6,7,7a-tetrahydro-3aH-pyrano[3,2-d]thiazole-6,7-diol :**

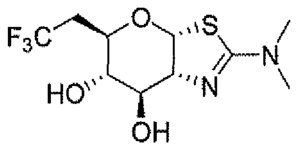

**Example 157**

Scheme XXIV

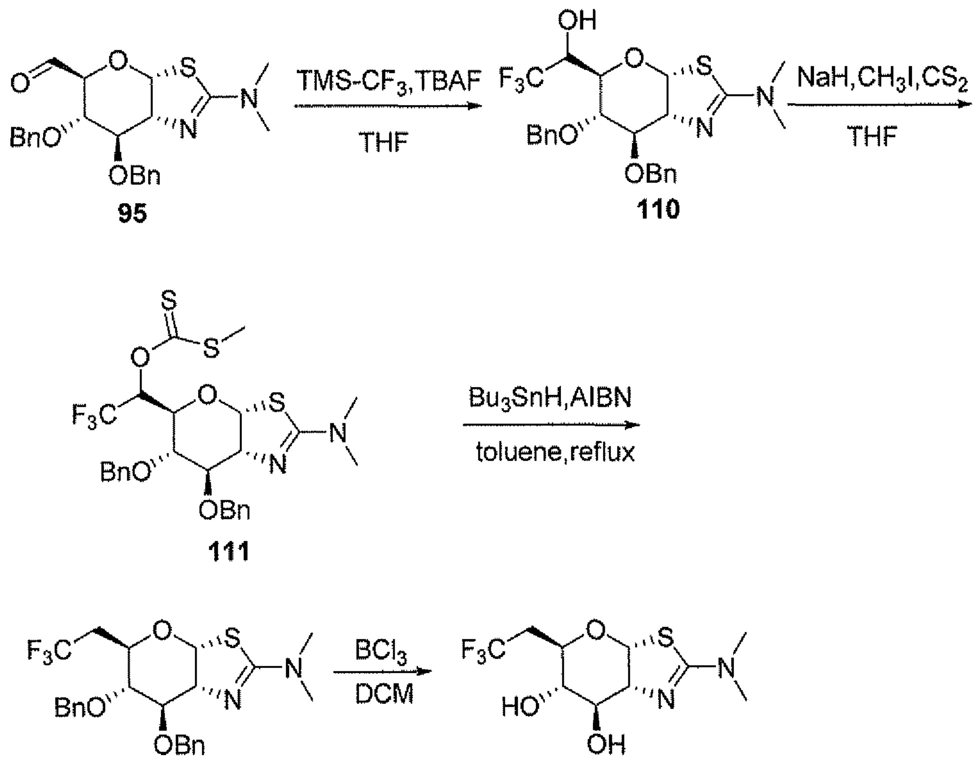


95 110 111 112 Example 157

**Step 1**

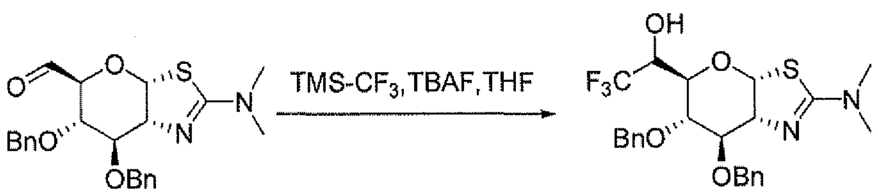


95 110

**(R)-1-((3aR,5R,6S,7R,7aR)-6,7-bis(benzyloxy)-2-(dimethylamino)-5,6,7,7a-tetrahydro-3aH-pyrano[3,2-d]thiazol-5-yl)-2,2,2-trifluoroethanol (110):** A mixture of the aldehyde (**95**) (3.0g, 7 mmol) (*Prepared according to the synthesis of Examples 149 step 3*) TBAF (1.06 g, 3 mmol) and 4A molecular sieves (2.0 g) in THF (60 mL) was stirred for 30min followed by the addition of $CF_3SiMe_3$ (5.8 g, 40mmol) at - 30°C. The mixture was kept overnight at room temperature, additional TBAF (20 g, 63 mmol)) was added. After stirred additional 1 hour, the reaction was quenched by brine (40 mL) and extracted with ethyl acetate (3x50 mL). The organic layers combined, washed with brine (3x50 mL), dried over anhydrous sodium sulfate and concentrated under reduced pressure to give a residue, which was purified by a silica gel column, eluted with 5% - 25% ethyl acetate in petroleum ether to afford **110** as a light yellow syrup (1.7g, 49%). (ES,

*m/z*): $[M+H]^+$ 497.7; $^1$H NMR (300 MHz, $CDCl_3$) δ 7.31 - 7.40 (m, 10H), 6.29 (d, *J* = 6.6 Hz, 1H), 4.57 - 4.78 (m, 4H), 4.30 - 4.43 (m, 2H), 3.80 - 4.17 (m, 4H), 3.01 (s, 6H).

**Step 2**

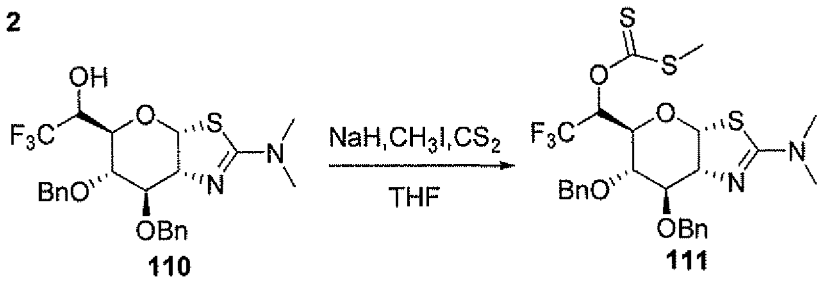


**110** **111**

**O-(R)-1-((3aR,5S,6S,7R,7aR)-6,7-bis(benzyloxy)-2-(dimethylamino)-5,6,7,7a-tetrahydro-3aH-pyrano[3,2-d]thiazol-5-yl)-2,2,2-trifluoroethyl S-methyl carbonodithioate (111):** A solution of compound **110** (1.5 g, 3 mmol), $CS_2$ (8 mL) and $CH_3I$ (8 mL) in tetrahydrofuran (50 mL) was treated with sodium hydride (242 mg, 6 mmol, 60% mineral oil dispersed) at 0 - 5 °C for 1.5 hours, then the reaction was quenched by the addition of water (50 mL) and extracted with dichloromethane (3x40 mL). The organic layers were combined, washed with brine (2x30 mL) and dried over magnesium sulfate. After filtration, the filtrates were concentrated under vacuum to give a residue, which was purified by a silica gel column, eluted with 15% - 30 % ethyl acetate in petroleum ether to give compound **111** as a yellow syrup (1.4 g, 79%). (ES, *m/z*): $[M+H]^+$ 586.7; $^1$HNMR ($CD_3Cl$, 300 MHz) δ 7.29 - 7.39 (m, 10H), 6.62 - 6.65 (m, 0.5H), 6.29 - 6.48 (m, 0.5H), 4.51 - 4.78 (m, 4H), 4.25 - 4.32 (m, 2H), 4.03 - 4.15 (m, 2H), 3.01 (s, 6H), 2.56 (s, 3H).

**Step 3**

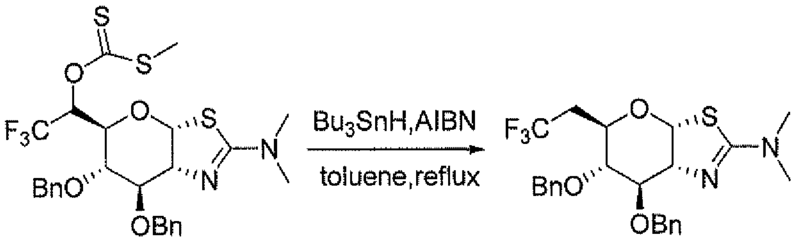


**111** **112**

**(3aR,5R,6R,7R,7aR)-6,7-bis(benzyloxy)-N,N-dimethyl-5-(2,2,2-trifluoroethyl)-5,6,7,7a-tetrahydro-3aH-pyrano[3,2-d]thiazol-2-amine (112):** To a solution of compound **111** (1.4 g, 2 mmol) in toluene (40 mL) was added tributylstannane (3.5 g, 12 mmol) and AIBN (20 mg, 0.1 mmol) at room temperature. The resulting solution was kept at 100°C for 1 hour, then cooled to room temperature and quenched by water (50 mL). The organic layer was separated and the aqueous layer was extracted with ethyl acetate (2x30mL). The combined organic layers were dried over magnesium sulfate. After filtration, the filtrates were concentrated under vacuum to give a residue, which was purified by a silica gel column, eluted with 10% - 20 % ethyl acetate in petroleum ether to give compound **112** as a light yellow syrup (1.0 g, 87%). (ES, *m/z):* $[M+H]^+$ 480.7; $^1$H NMR ($CD_3Cl$, 300 MHz) δ 7.29 - 7.43 (m, 10H), 6.23 (d, *J* = 6.6 Hz, 1H), 4.55 - 4.83 (m, 4H), 4.25 - 4.36 (m, 2H), 3.80 - 3.84 (m, 1H), 3.39 - 3.42 (m, 1H), 3.02 (s, 6H), 2.35 - 2.40 (m, 1H), 2.13 - 2.21 (m, 1H).

**Step 4**

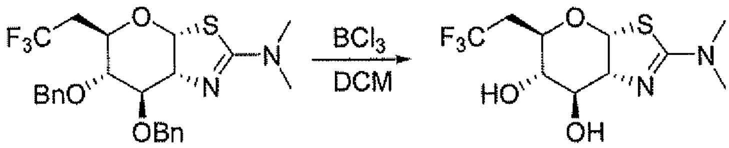


112

Example 157

**(3aR,5R,6S,7R,7aR)-2-(dimethylamino)-5-(2,2,2-trifluoroethyl)-5,6,7,7a-tetrahydro-3aH-pyrano[3,2-d]thiazole-6,7-diol:** A solution of compound **112** (300 mg, 0.6 mmol) in dichloromethane (10 mL) was added $BCl_3$ (3 mL, 3 mmol, 1M in dichloromethane) at -78°C. The resulting solution was kept at -20°C for 1 hour, then quenched by the addition of methanol (10 mL). Volatiles were distilled out under vacuum to give a residue, which was dissolved into methanol (5 mL) and neutralized by Con. $NH_4OH$ (2 ml, 26% aqueous solution). After concentration, the crude product was purified by a silica gel column, eluted with 10 % methanol in dichloromethane to give the title compound as a white solid (94.2 mg, 49%). (ES, *m/z*): $[M+H]^+$ 300.9; $^1$H NMR ($D_2O$, 300 MHz) δ 6.16 (d, *J* = 6.3 Hz, 1H), 4.11 - 4.15 (m, 1H), 3.93 - 3.97 (m, 1 H), 3.77 - 3.83 (m, 1H), 3.44 - 3.48 (m, 1H), 2.91 (s, 6H), 2.52 - 2.68 (m, 1H), 2,37 - 2.49 (m, 1H).

**Example 158 & 159**

**(3aR,5S,6S,7R,7aR)-2-(methylamino)-5-((S)-1,1,1-trifluoro-2-hydroxypropan-2-yl)-5,6,7,7a-tetrahydro-3aH-pyrano[3,2-d]thiazole-6,7-diol & (3aR,5S,6S,7R,7aR)-2-(methylamino)-5-(1,1,1-trifluoro-2-hydroxypropan-2-yl)-5,6,7,7a-tetrahydro-3aH-pyrano[3,2-d]thiazole-6,7-diol :**

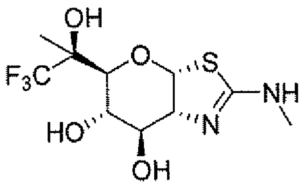

&

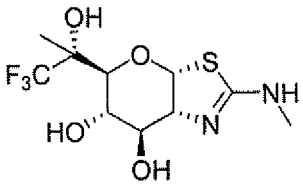

Example 158

Example 159

Scheme XXV

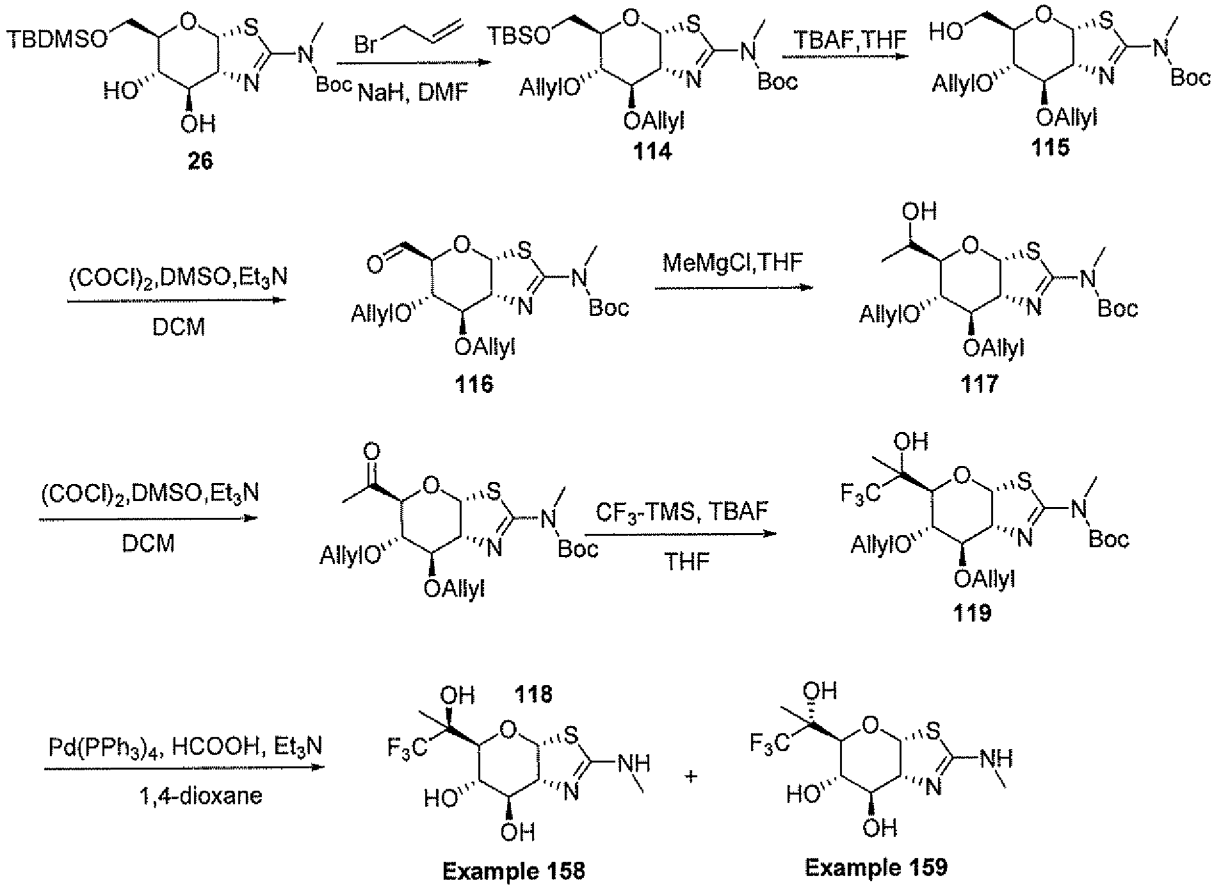


**Step 1**

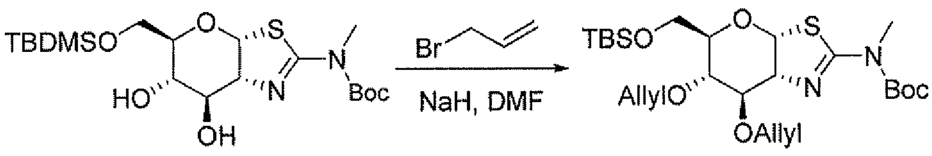


26

114

**tert-butyl (3aR,5R,6S,7R,7aR)-6,7-bis(allyloxy)-5-((tert-butyldimethylsilyloxy)methyl)-5,6,7,7a-tetrahydro-3aH-pyrano[3,2-djthiazol-2-yl(methyl)carbamate (114):** A solution of **26** (35 g, 78 mmol) (*Prepared according to the synthesis ofExample 3, step 4*) in DMF (250 mL) was treated with NaH (70%, 8 g, 233 mmol) at 5°C for 30 min, then Allyl-Br (28 g, 233 mmol) was added slowly. After stirring for 1.5 hours at 15°C., the reaction was quenched by $H_2O$ (300 mL), extracted with ethyl acetate (3x200 mL). The organic layers were collected, washed with brine (5x100 mL), dried over anhydrous sodium sulfate and concentrated under reduced pressure to give a residue, which was purified by a silica gel column, eluted with 5 % ~ 10 % ethyl acetate in petroleum ether to afford the title compound (32 g, 78 %) as a yellow oil; (ES, m/z) $[M+H]^+$ 529.1; $^1H$ NMR (300 MHz, $CDCl_3$) $\delta$ 6.07 (d, $J$ = 6.3 Hz, 1H), 5.85 - 6.05 (m, 2H), 5.15 - 5.38 (m, 4H), 4.22 - 4.30 (m, 4H), 4.02 - 4.07 (m, 2H), 3.76 - 3.78 (m, 2H), 3.60 - 3.62 (m, 1H), 3.48 - 3.52 (m, 1H), 3.31 (s, 3H), 1.55 (s, 9H), 0.93 (s, 9H), 0.08 (s, 6H).

**Step 2**

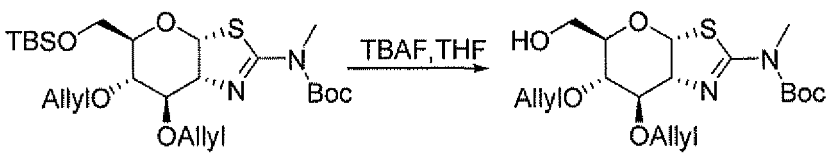


114 115

**tert-butyl (3aR,5R,6S,7R,7aR)-6,7-bis(allyloxy)-5-(hydroxymethyl)-5,6,7,7a-tetrahydro-3aH-pyrano[3,2-d]thiazol-2-yl(methyl)carbamate (115):** A solution of **114** (104 g, 197 mmol) in THF (700 mL) was treated with TBAF (77 g, 296 mmol) at 20 °C for 6 hours, then the reaction was quenched by water (500 mL), extracted with ethyl acetate (5x300 mL). The organic layers combined, washed with brine (2x150 mL), dried over anhydrous sodium sulfate and concentrated under reduced pressure to give a residue, which was purified by a silica gel column, eluted with 5% ~ 30% ethyl acetate in petroleum ether to afford the title compound (72 g, 88%) as a yellow oil; (ES, *m/z*) $[M+H]^+$ 415.0; $^1H$ NMR (300 MHz, $CDCl_3$) δ 6.07 (d, *J* = 6.9 Hz, 1H), 5.94 - 6.01 (m, 2H), 5.17 - 5.3 (m, 4H), 4.02 - 4.45 (m, 6H), 3.74 - 3.85 (m, 1H), 3.51 - 3.71 (m, 2H), 3.55 - 3.68 (m, 1H), 3.33 (s, 3H), 1.55 (s, 9H).

Step 3

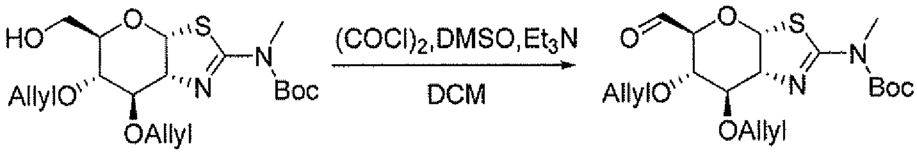


115 116

**tert-butyl (3aR,5S,6S,7R,7aR)-6,7-bis(allyloxy)-5-formyl-5,6,7,7a-tetrahydro-3aH-pyrano[3,2-d]thiazol-2-yl(methyl)carbamate** (116): A solution of DMSO (45 g, 580 mmol) in dichloromethane (450 mL) was treated with oxalyl dichloride (55 g, 435 mmol) at -78 °C for 1 hour, then a solution of 115 (30 g, 72 mmol) in dichloromethane (100 mL) was added slowly. The resulted solution was stirred for 4 hours at -20 °C followed by the addition of triethylamine (73 g, 725 mmol) at -78 °C. After stirring for additional 1 hour at - 20 °C, the reaction was quenched by water (600 mL), extracted with dichloromethane (2x300 mL). The organic layers combined, washed with brine (2x150 mL), dried over anhydrous sodium sulfate and concentrated under reduced pressure to afford crude 116 as a yellow syrup, used for next step without further purification; (ES, *m/z*) $[M+H]^+$ 413.0.

**Step 4**

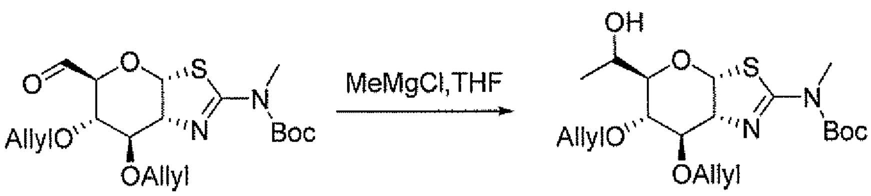


116 117

**tert-butyl (3aR,5R,6S,7R,7aR)-6,7-bis(allyloxy)-5-((S)-1-hydroxyethyl)-5,6,7,7a-tetrahydro-3aH-pyrano[3,2-d]thiazol-2-yl(methyl)carbamate (117):** A solution of crude **116** in anhydrous THF (300 mL) was treated with methylmagnesium chlorine (48 mL, 3M in THF, 145 mmol) at 10 °C overnight, then quenched with saturated aqueous $NH_4Cl$ (300 mL), extracted with ethyl acetate (3x200 mL). The organic layers combined, washed with brine (2x100 mL), dried over anhydrous magnesium sulfate and concentrated under reduced pressure to give a residue, which was purified by silica gel column, eluted with 3% ~ 25% ethyl acetate in petroleum ether to afford **117** (18 g, 58% 2 steps) as a yellow syrup; $[M+H]^+$ 429.1; $^1H$ NMR (300 MHz, $CDCl_3$) δ 5.97 - 6.11 (m, 1H), 5.87 - 5.95 (m, 2H), 5.21 - 5.36 (m, 4H), 4.21 - 4.45

(m, 4H), 3.68 - 4.05 (m, 3H), 3.65 - 3.68 (m, 1H), 3.32 (d, *J*= 4.2 Hz, 3H), 3.21 - 3.22 (m, 1H), 1.54 - 1.56 (s, 9H), 1.27 (s, 3H).

**Step 5**

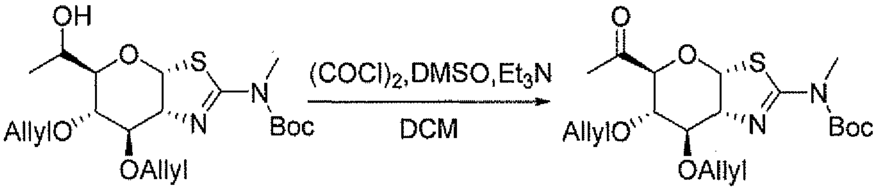


**117** **118**

**tert-butyl (3aR,5S,6S,7R,7aR)-5-acetyl-6,7-bis(allyloxy)-5,6,7,7a-tetrahydro-3aH-pyrano[3,2-d]thiazol-2-yl(methyl)carbamate (118):** A solution of DMSO (52 g, 672 mmol) in dichloromethane (500 mL) was treated with oxalyl dichloride (64 g, 505 mmol) at -78 °C for 1 hour, then a solution of **117** (36 g, 84 mmol) in dichloromethane (150 mL) was added. The resulted solution was stirred for 4 hours at -20 °C followed by the addition of triethylamine (85 g, 841 mmol) at -78 °C. After stirring at - 20°C for additional 1 hour, the reaction was quenched with water (600 mL), extracted with dichloromethane (2x300 mL). The organic layers combined, washed with brine (2x200 mL), dried over anhydrous sodium sulfate and concentrated under reduced pressure to give a residue, which was purified by silica gel column, eluted with 3% ~ 20% ethyl acetate in petroleum ether to afford **118** (26 g, 73%) as a yellow syrup; $[M+H]^+$ 427.0; $^1H$ NMR (300 MHz, $CDCl_3$) δ 6.02 (d, *J* = 6.3 Hz, 1H), 5.82 - 5.99 (m, 2H), 5.17 - 5.35 (m, 4H), 4.20 - 4.29 (m, 1H), 4.03 - 4.29 (m, 5H), 3,71 - 3.77 (m, 2H), 3.25 (s, 3H), 2.14 (s, 3H), 1.52 (s, 9H).

**Step 6**

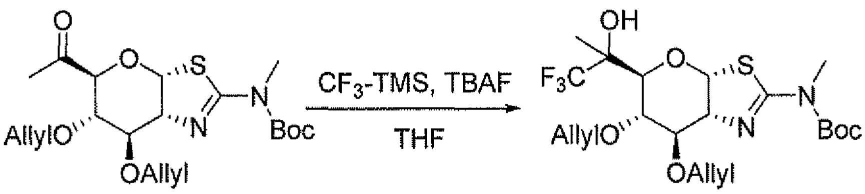


**118** **119**

**tert-butyl (3aR,5S,6S,7R,7aR)-6,7-bis(allyloxy)-5-((S)-1,1,1-trifluoro-2-hydroxypropan-2-yl)-5,6,7,7a-tetrahydro-3aH-pyrano[3,2-d]thiazol-2-yl(methyl)carbamate (119):** A mixture of TBAF (3.7 g, 14 mmol) and 4Å molecule sieves in THF (200 mL) was stirred for 30 min at 0°C followed by the addition of a solution of **118** (15 g, 35 mmol)) and TM5-$CF_3$ (20 g, 141 mmol) in THF (80 mL). After stirring for additional 12 hours at 25°C, additional TBAF (14 g, 54 mmol) was added, and the mixture was stirred for 1 hour. The reaction was quenched by brine (200 mL), extracted with ethyl acetate (3x150 mL), the organic layers combined, washed with brine (3x100 mL), dried over anhydrous sodium sulfate and concentrated under reduced pressure to give a residue, which was purified by a silica gel column, eluted with 5% ~ 25% ethyl acetate in petroleum ether to afford **119** (12 g, 69%) as a yellow oil; (ES, *m/z*) $[M+H]^+$ 497.1. $^1H$ NMR (300 MHz, $CDCl_3$) δ 6.12 - 6.18 (m, 1H), 5.86 - 6.00 (m, 2H), 5.19 - 5.37 (m, 4H), 4.02 - 4.45 (m, 6H), 3.92 - 3.96 (m, 1H), 3.62 - 3.64 (m, 1H), 3.25 (d, *J* = 3.6 Hz, 3H), 1.54 - 1.56 (m, 9H), 1.27 - 1.38 (m, 3H).

**Step 7**

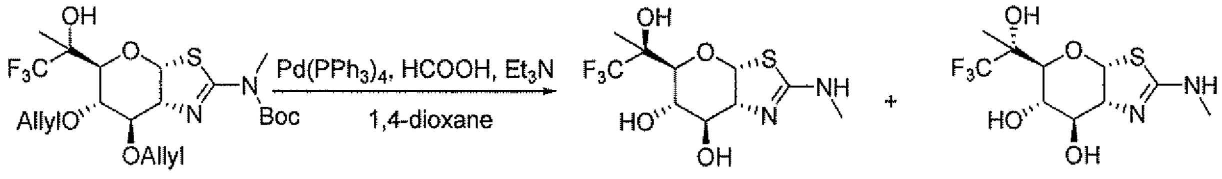


119 Example 158 Example 159

**(3aR,5S,6S,7R,7aR)-2-(methylamino)-5-((S)-1,1,1-trifluoro-2-hydroxypropan-2-yl)-5,6,7,7a-tetrahydro-3aH-pyrano[3,2-d]thiazole-6,7-diol & (3aR,5S,6S,7R,7aR)-2-(methylamino)-5-(1,1,1-trifluoro-2-hydroxypropan-2-yl)-5,6,7,7a-tetrahydro-3aH-pyrano[3,2-d]thiazole-6,7-diol:** To a solution of **119** (4.5 g, 9 mmol) in 1,4-dioxane (50 mL) was added $Pd(PPh_3)_4$ (2.1 g, 1.8 mmol), $Et_3N$ (2.3 g, 22.7 mmol) and HCOOH (0.8 g, 18 mmol) at 25°C under $N_2$ atmosphere. After 20 min at 60 °C, additional HCOOH (6.3 g, 136 mmol) was added and the mixture was stirred for additional 12 hours at 60 °C, then quenched by $H_2O$ (60 mL), extracted with dichloromethane (2x40 mL) to remove the organic impurities. The pH value of aqueous phase was adjusted to 7-8 by saturated aqueous $NaHCO_3$, then concentrated under reduced pressure to give a residue, which was purified by a silica gel column, eluted with 2% ~ 10% methanol in dichloromethane to give the mixture of the two epimer. Further separation by Prep HPLC [(Agilent 1200 prep HPLC): Column, Sun Fire Prep C18*50mm 5um; mobile phase, WATER with $CH_3CN$ (5% $CH_3CN$ up to 40% in 10 min); Detector, UV, 220nm] to afford (3aR,5S,6S,7R,7aR)-2-(methylamino)-5-((S)-1,1,1-trifluoro-2-hydroxypropan-2-yl)-5,6,7,7a-tetrahydro-3aH-pyrano[3,2-d]thiazole-6,7-diol as white solid (730 mg, 25.4%), Faster eluting isomer. (ES, *m/z*); 317.0; $^1$HNMR (300 MHz, $D_2O$) $\delta$ 6.27 (d, *J*= 6.9 Hz, 1H), 4.38 - 4.41 (m, 1H), 4.22 - 4.23 (m, 1H), 3.94 - 3.97 (d, *J* = 8.4 Hz, 1H), 3.56 - 3.60 (d, *J*= 8.4 Hz, 1H), 2.77 (s, 3H), 1.34 (s, 3H); and (3aR,5S,6S,7R,7aR)-2-(methylamino)-5-(1,1,1 -trifluoro-2-hydroxypropan-2-yl)-5,6,7,7a-tetrahydro-3aH-pyrano[3,2-d]thiazole-6,7-diol as white solid (810 mg, 28.2%), Slower eluting isomer. (ES, *m/z*): 317.0; $^1$HNMR (300 MHz, $D_2O$) $\delta$ 6.26 (d, *J* = 6.9 Hz, 1H), 4.37 - 4.39 (m, 1H), 4.18 - 4.20 (m, 1H), 3.96 - 3.99 (m, 1H), 3.60 - 3.62 (d, *J* = 8.4 Hz, 1H), 2.77 (s, 3H), 1.33 (s, 3H).\

**Example 160 and 161**

**(3aR,5S,6S,7R,7aR)-2-(ethylamino)-5-((S)-2,2,2-trifluoro-1-hydroxyethyl)-5,6,7,7a-tetrahydro-3aH-pyrano[3,2-d]thiazole-6,7-diol and (3aR,5S,6S,7R,7aR)-2-(ethylamino)-5-((R)-2,2,2-trifluoro-1-hydroxyethyl)-5,6,7,7a-tetrahydro-3aH-pyrano[3,2-d]thiazole-6,7-diol :**

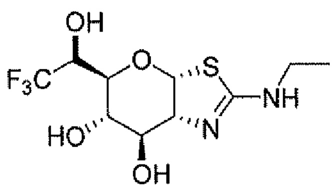

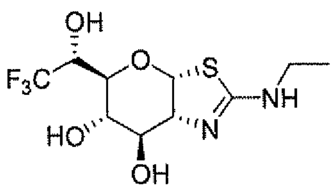

Example 160 Example 161

Scheme XXVI

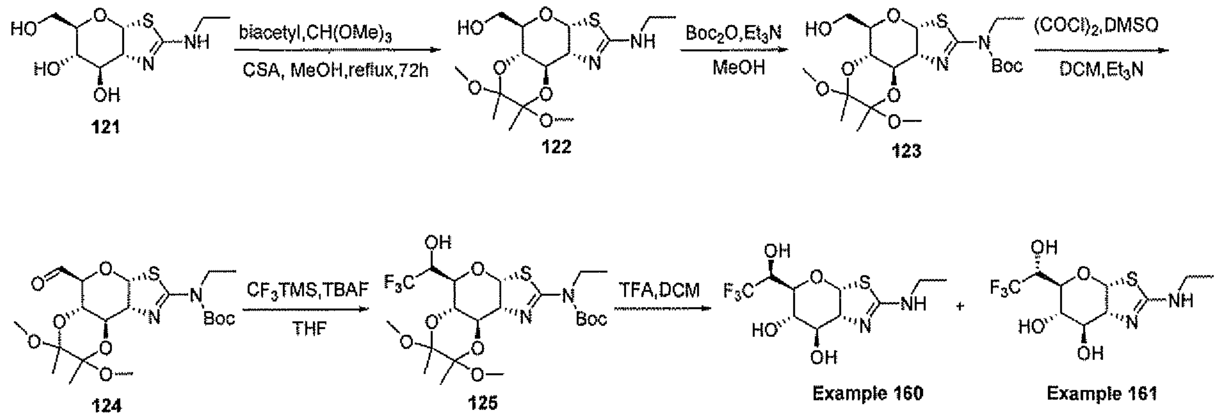

121 122 123 124 125 Example 160 Example 161

**Step 1-2**

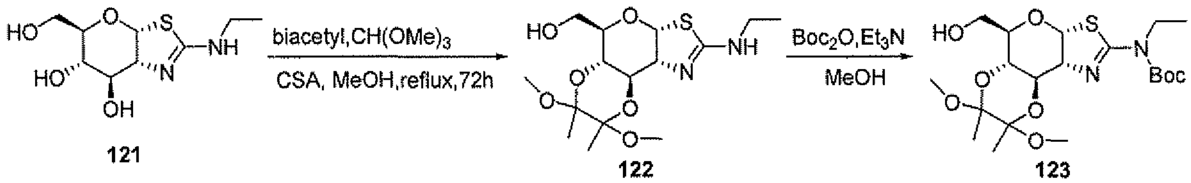

121 122 123

**tert-butyl N-ethyl-N-[(1R,2R,6R,8R,9S)-8-(hydroxymethyl)-11,12-dimethoxy-11,12-dimethyl-7,10,13-trioxa-5-thia-3-azatricyclo[7.4.0.0[2,6]]tridee-3-en-4-yl]carbamate (123):** *D*-(+)-10-Camphorsulfonic acid (112 g, 0.48 mol) was added to a solution of (3aR,5R,6S,7R,7aR)-2-(ethylamino)-5-(hydroxymethyl)-5,6,7,7a-tetrahydro-3aH-**pyrano[3,2-d]thiazole-6,7-diol (121)** (ref. Yuzwa, et al, Nature Chemical Biology, 2008, 4, 483 and WO 2008/025170, published March 6, 2008) (100 g, 0.4 mol), biacetyl (173 g, 2 mol) and trimethyl orthoformate (427 g, 4 mol) in anhydrous methanol (2 L). The mixture was heated at reflux for 72 hours, and followed by addition of triethylamine (53 g, 0.53 mol) at room temperature. The mixture containing crude **122** was treated with Boc anhydride (170 g, 0.78 mol) and stirred overnight at room temperature. Removal of volatiles gave a residue, which was dissolved into dichloromethane (1 L), washed with brine (5x100 mL), dried over anhydrous sodium sulphate and concentrated. The crude material was purified by a silica gel column, eluted with 10 % - 20 % ethyl acetate in petroleum ether to give **123** as a brown syrup (92 g, 49 %) as a 1:1 mixture of two isomers based on $^{1}H$ NMR. (ES, *m/z*) $[M+H]^+$ 463.0; $^{1}H$ NMR (300 MHz, $CDCl_3$) δ 6.11 (m, 2H), 4.26 - 4.29 (m, 1 H), 3.74 - 4.09 (m, 15H), 3.39 (s, 3H), 3.31 (s, 3H), 3.27 (s, 3H), 3,24 (s, 3H), 1.52 (s, 9H), 1.45 (s, 3H), 1.42 (s, 3H), 1.39 (s, 3H), 1.36 (s, 3H), 1.13 - 1.19 (m, 6H).

**Step 3**

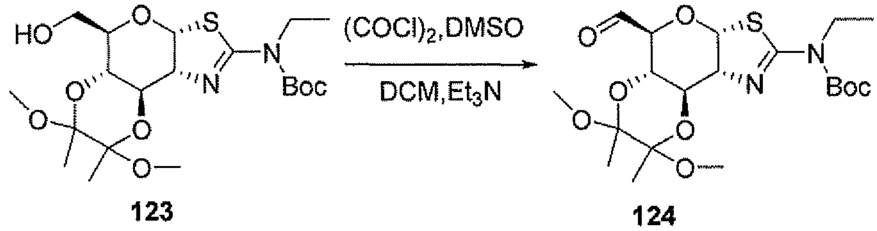

123 124

**tert-bntyl N-ethyl-N-[(1R,2R,6R,8S,9S)-8-formyl-11,12-dimethoxy-11,12-dimethyl-7,10,13-trioxa-5-thia-3-azatricyclo[7.4.0.0[2,6]]tridec-3-en-4-yl]carbamate (124):** A solution of DMSO (40 g, 0.52 mol) in anhydrous dichloromethane (1.5 L) was treated with oxallyl dichloride (49 g, 0.39 mol) at -78 °C for 1 hour, followed by addition of **123** (30 g, 65 mmol). The resulting solution was stirred for 4 hours at -30 °C and then cooled down to - 78 °C again at which time triethylamine (86 g, 0.85 mol) was added. After 1 hour at -50 °C, the reaction mixture was quenched by $H_2O$ (500 mL), washed with brine (3x100 mL), dried over anhydrous sodium sulfate and concentrated under vacuum to give a residue, which was purified by a short silica gel column, eluted with 30 % ethyl acetate in petroleum ether to give **124** as a brown syrup (18 g, 60 %). (ES, *m/z*) $[M+H]^+$ 461.0; $^1H$ NMR (300 MHz, $CDCl_3$) δ 8.93 (s, 1H), 8.90 (s, 1H), 6.15 - 6.18 (m, 2H), 4.41 - 4.45 (m, 1H), 4.28 - 4.30 (m, 1H), 3.76 - 4.09 (m, 13H), 3.38 (s, 3H), 3.32 (s, 3H), 3.28 (s, 3H), 3.26 (s, 3H), 1.58 (s, 9H), 1.47 (s, 3H), 1.43 (s, 3H), 1.39 (s, 3H), 1.37 (s, 3H), 1.14 - 1.21 (m, 6H).

**Step 4-5**

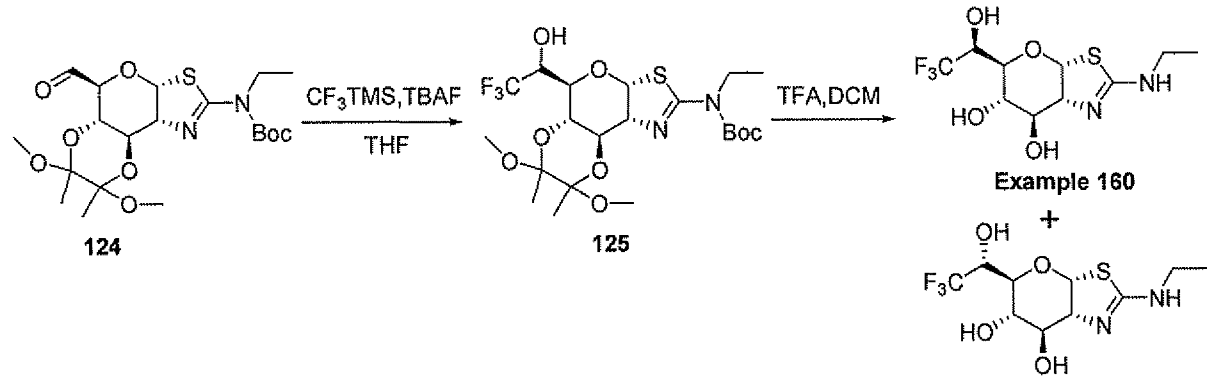


124 125 Example 160 + Example 161

**(3aR,5S,6S,7R,7aR)-2-(ethylamino)-5-((R)-2,2,2-trifluoro-1-hydroxyethyl)-5,6,7,7a-tetrahydro-3aH-pyrano[3,2-d]thiazole-6,7-diol** : A solution of **124** (21 g, 45 mmol) and $CF_3TMS$ (41 g, 290 mmol) in THF (100 mL) was added to a mixture of TBAF (6.6 g, 25 mmol) and 4Å molecular sieves in THF (400 mL) at 0 °C. The mixture was stirred at room temperature overnight before addition of additional TBAF (26.4 g, 100 mmol). After an additional 1 hour, a filtration was performed and the filtrates were concentrated to give a residue, which was dissolved into dichloromethane (500 mL), washed with brine (3x100 mL), dried over anhydrous $Na_2SO_4$ and concentrated to give crude **125** as a brown syrup (four isomers based on TLC and LCMS), which was treated with TFA (100 ml, 90 % in dichloromethane, v/v) overnight at room temperature. Removal of volatiles gave a residue, which was dissolved into methanol (50 mL) and neutralized with concentrated $NH_4OH$ (10 mL). After concentration under reduced pressure, the crude product was purified by a silica gel column, eluted with 1 % - 10 % methanol in dichloromethane to give **Example 160** (3aR,5S,6S,7R,7aR)-2-(ethylamino)-5-((S)-2,2,2-trifluoro-1-hydroxyethyl)-5,6,7,7a-tetrahydro-3aH-pyrano[3,2-d]thiazole-6,7-diol as a white solid (2.41 g, 16 %, faster eluting isomer by HPLC). (ES, *m/z*):$[M+H]^+$ 317.0; $^1H$ NMR (300 MHz, $D_2O$) 6.18 (d, *J* = 6.6 Hz, 1H), 4.21 - 4.30 (m, 2H), 4.08 (t, *J* = 3.6 Hz, 1H), 3.88 - 3.93 (m, 1H), 3.69 - 3.74 (m, 1H), 3.13 - 3.21 (m, 2H), 1.07 (d, *J*= 7.2 Hz, 3H); and **Example 161** (3aR,5S,6S,7R,7aR)-2-(ethylamino)-5-((R)-2,2,2-trifluoro-1-hydroxyethyl)-5,6,7,7a-tetrahydro-3aH-pyrano[3,2-d]thiazole-6,7-diol (2.48 g, 17%, slower eluting isomer by HPLC). (ES, *m/z*) $[M+H]^+$ 317.0; $^1H$ NMR (300 MHz, $D_2O$) 6.21 (d, *J* = 6.3 Hz, 1H), 4.23 - 4.31 (m, 1H), 4.09 (t, *J* = 3.0 Hz, 1H), 3.97 - 4.00 (m, 1H), 3.72 - 3.77 (m, 2H), 3.16 (q, *J* = 7.2 Hz, 2H), 1.05 (d, *J* = 7.2 Hz, 3H).

**Examples 162 and 163**

**(3aR,5R,6S,7R,7aR)-5-((R)-1-hydroxyethyl)-2-(2-hydroxyethylamino)-5,6,7,7a-tetrahydro-3aH-pyrano[3,2-d]thiazole-6,7-diol & (3aR,5R,6S,7R,7aR)-5-((S)-1-hydroxyethyl)-2-(2-hydroxyethylamino)-5,6,7,7a-tetrahydro-3aH-pyrano[3,2-d]thiazole-6,7-diol :**

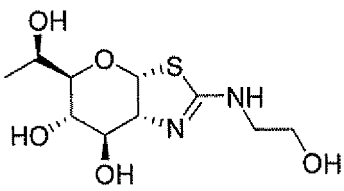

**Example 162**

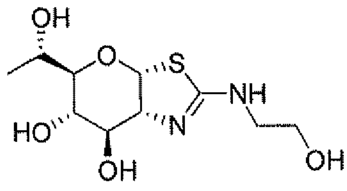

**Example 163**

Scheme XXVII

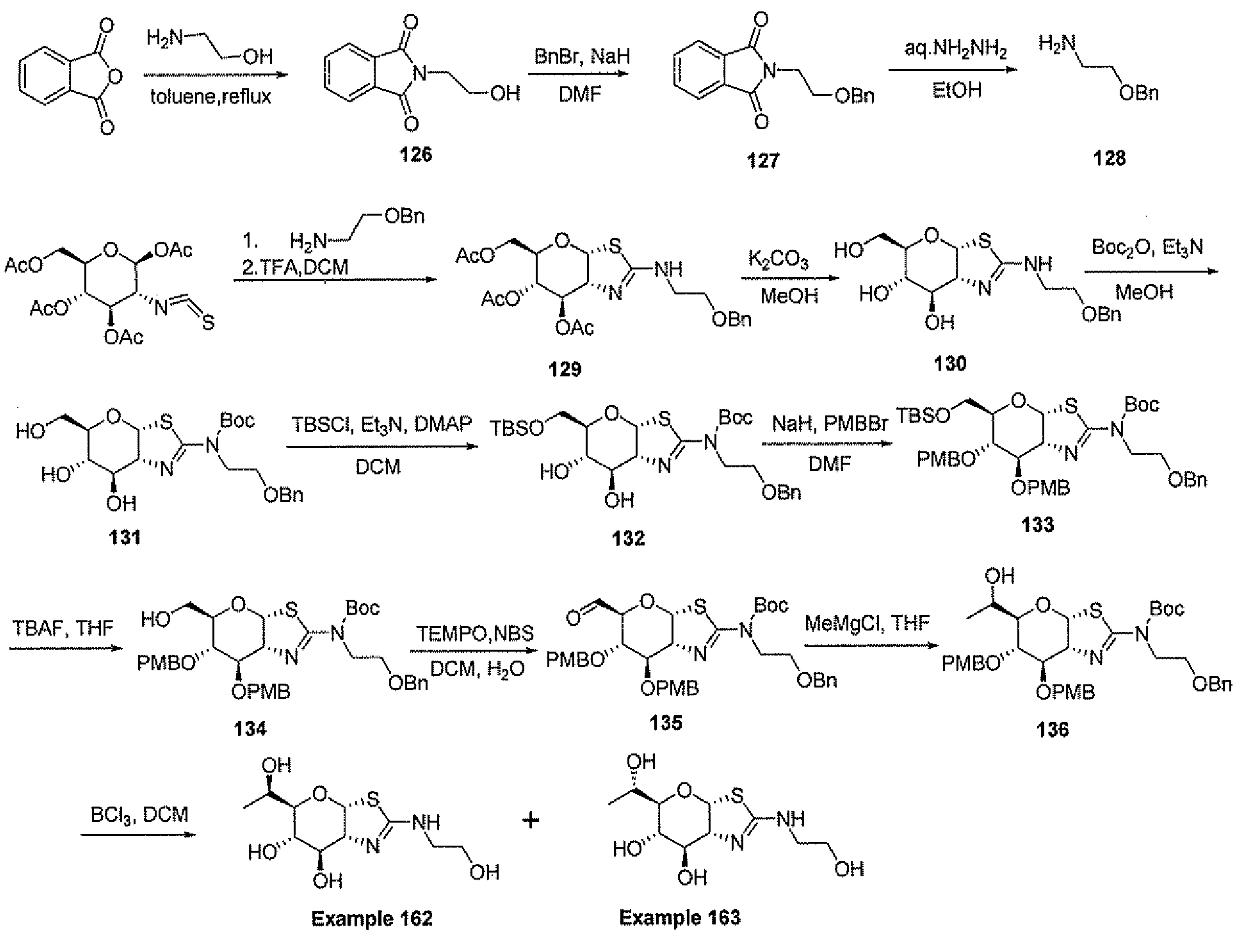


**Step 1**

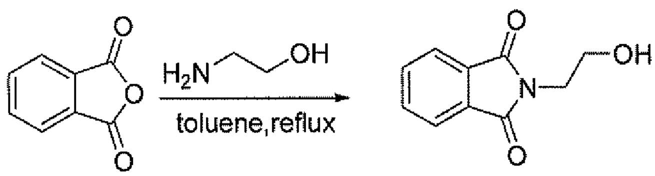


**126**

**2-(2-hydroxyethyl)isoindoline-1,3-dione (126):** A solution of 2-aminoethanol (41 g, 0.67 mol) and isobenzofuran-1,3-dione (100 g, 0.52 mol) in toluene (300 mL) was refluxed for 12 hours. Volatiles were distilled out to give a residue,

which was purified by recrystallization from chloroform/hexane (v/v, 1:6) to afford **126** as a white solid (110 g, 85 %). (ES, *m/z*) $[M+H]^+$ 192.0; $^1H$ NMR (300 MHz, $CDCl_3$) δ 7.84 - 7.90 (m, 2H), 7.71 - 7.77 (m, 2H), 3.87 - 3.93 (m, 4H), 2.10 (br, 1H).

**Step 2**

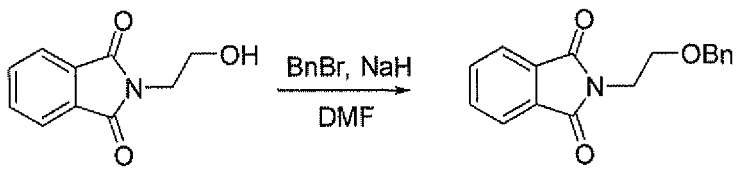


**126** **127**

**2-(2-(benzyloxy)ethyl)isoindoline-1,3-dione (127):** A solution of **126** (5 g, 26 mmol) in DMF (50 mL) was treated with sodium hydride (1.1 g, 32 mmol, 70 % dispersed in mineral oil) for 30 minutes at room temperature, followed by addition of BnBr (6.7 g, 39 mmol). After additional 1 hour, the reaction was quenched by water (200 mL) and extracted with ethyl acetate (4x50 mL). The combined organic layer was washed with brine (3x50 mL), dried over anhydrous sodium sulfate and concentrated under vacuum to give a residue, which was purified by a silica gel column, eluted with 10 % - 20 % ethyl acetate in petroleum ether to afford **127** as a white solid (4.8 g, 65 %).(ES, *m/z*) $[M+H]^+$ 282.0; $^1H$ NMR (300 MHz, $CDCl_3$) δ 7.85 - 7.88 (m, 2H), 7.71 - 7.74 (m, 2H), 7.25 - 7.31 (m, 5H), 4.55 (s, 2H), 3.95 (t, *J* = 6.0 Hz, 2H), 3.74 (t, *J* = 6.0 Hz, 2H).

**Step 3**

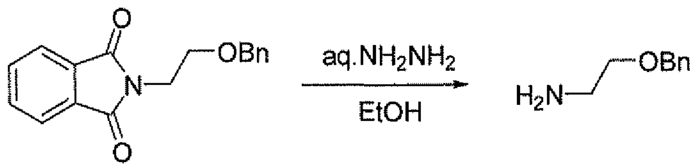


**127** **128**

**2-(benzyloxy)ethanamine (128):** A solution of **127** (10 g, 35 mmol) in ethanol (200 mL) was treated with hydrazine (84 g, 71 mmol, 80 % in water) at reflux for 12 hours. After a filtration, the filtrate was concentrated under vacuum to give a residue, which was purified by a silica gel column, eluted with 5 % methanol in dichloromethane to afford **128** as light yellow oil (4 g, 74 %). (ES, *m/z*) $[M+H]^+$ 152.0; $^1H$ NMR (300 MHz, $CDCl_3$) δ 7.28 - 7.40 (m, 5H), 4.56 (s, 2H), 3.54 (t, *J*= 5.4 Hz, 2H), 2.94 (t, *J* = 5.1 Hz, 2H).

**Step 4**

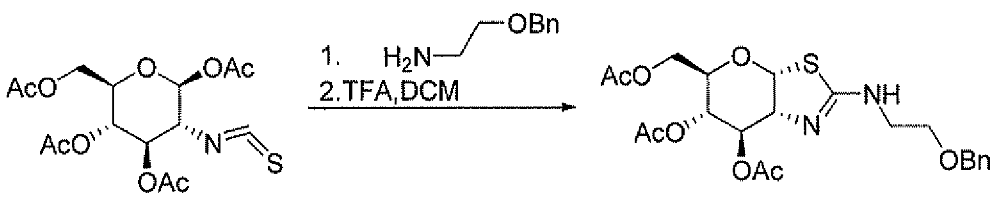


**5** **129**

**(3aR,5R,6S,7R,7aR)-5-(acetoxymethyl)-2-(2-(benzyloxy)ethylamino)-5,6,7,7a-tetrahydro-3aH-pyrano[3,2-d]thiazole-6,7-diyl diacetate (129):** To a solution of (2S,3R,4R,5S,6R)-6-(acetoxymethyl)-3-isothiocyanato-tetrahydro-2H-pyran-2,4,5-triyl triacetate (9.8 g, 25 mmol) in dichloromethane (100 mL) was added **128** (4 g, 26 mmol) at 0 °C. After stirred for 2 hours, trifluoroacetic acid (15.4 g, 159 mmol) was added. The resulting solution was stirred for 12 hours at room temperature, then quenched with ice-water (200 mL) and neutralized by the addition of $NaHCO_3$ (26 g, 318 mmol). The organic layer was separated and the aqueous layer was extracted with dichloromethane (3x100 mL). The combined organic layer was washed with brine (2x100 mL), dried over anhydrous sodium sulfate, and concentrated under vacuum to give a residue, which was purified by silica gel column, eluted with 10 % - 50 % ethyl acetate in petroleum ether to afford **129** as a white syrup (9.8 g, 77 %). (ES, *m/z*) $[M+H]^+$ 481.0; $^1H$ NMR (300 MHz, $CDCl_3$) δ 7.31 - 7.38 (m, 5H), 6.24 (d, *J* = 6.3 Hz, 1H), 5.41 - 5.43 (m, 1H), 4.94 - 4.98 (m, 1H), 4.55 (s, 2H), 4.34 - 4.37 (m, 1H), 4.14 - 4.16 (m, 3H), 3.83 - 3.87 (m, 1H), 3.64 (t, *J* = 5.4 Hz, 2H), 3.53 (t, *J* = 5.1 Hz, 2H), 2.12 (s, 3H), 2.10 (s, 3H), 2.09 (s, 3H).

**Step 5**

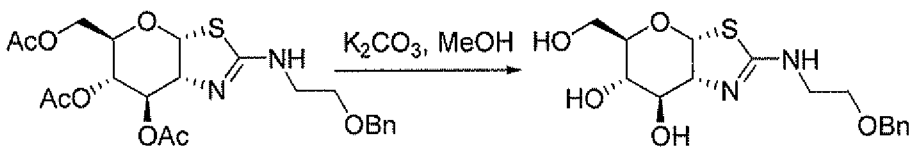


**129** **130**

**(3aR,5R,6S,7R,7aR)-2-(2-(benzyloxy)ethylamino)-5-(hydroxymethyl)-5,6,7,7a-tetrahydro-3aH-pyrano[3,2-d]thiazole-6,7-diol (130):** A solution of **129** (4.5 g, 9.4 mmol) in methanol (40 mL) was treated with potassium carbonate (260 mg, 2 mmol) for 3 hours at room temperature, then neutralized by the addition of acetic acid (0.5 mL). Volatiles were distilled out to give a residue, which was solidified from ethyl acetate. The solids were washed with ethyl acetate (3x20 mL) to give **130** as a light yellow solid (2.58 g, 80 %). (ES, *m/z*) $[M+H]^+$ 355.0; $^1H$ NMR (300 MHz, $D_2O$) δ 7.27 - 7.37 (m, 5H), 6.17 (d, *J* = 6.3 Hz, 1H), 4.48 (s, 2H), 4.34 - 4.37 (m, 1H), 4.03 - 4.07 (m, 1H), 3.88 - 3.91 (m, 1H), 3.58 - 3.67 (m, 2H), 3.31 - 3.55 (m, 5H).

**Step 6**

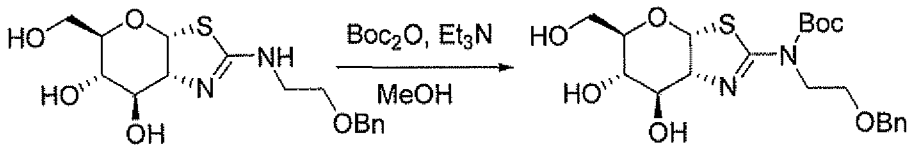


**130** **131**

**tert-butyl 2-(benzyloxy)ethyl((3aR,5R,6S,7R,7aR)-6,7-dihydroxy-5-(hydroxymethyl)-5,6,7,7a-tetrahydro-3aH-pyrano[3,2-d]thiazol-2-yl)carbamate (131):** To a solution of 130 in methanol (30 mL) was added $Boc_2O$ (3.1 g, 14 mmol) and triethylamine (1.9 g, 19 mmol). After stirred for 5 hours at room temperature, the resulting mixture was concentrated under vacuum to give a residue, which was purified by a silica gel column, eluted with 1 % - 5 % methanol in dichloromethane to afford **131** as a white solid (3.1 g, 73 %). (ES, *m/z*) $[M+H]^+$ 455.0; $^1H$ NMR (300 MHz, $CDCl_3$) δ 7.28 - 7.39 (m, 5H), 6.18 (d, *J* = 6.3 Hz, 1H), 4.49 (s, 2H), 4.34 - 4.37 (m, 1H), 4.03 - 4.07 (m, 1H), 3.89 - 3.93 (m, 1H), 3.59 - 3.67 (m, 2H), 3.32 - 3.57 (m, 5H), 1.58 (s, 9H).

**Step 7**

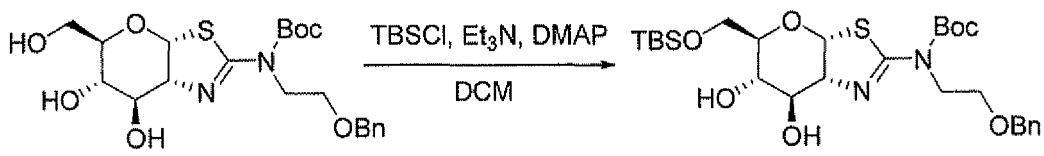


131 132

**tert-butyl 2-(benzyloxy)ethyl((3aR,5R,6S,7R,7aR)-5-((tert-butyldimethylsilyloxy)methyl)-6,7-dihydroxy-5,6,7,7a-tetrahydro-3aH-pyrano[3,2-d]thiazol-2-yl)carbamate (132):** A mixture of **131** (2.8 g, 6.2 mmol), DMAP (150 mg, 1.2 mmol), triethylamine (930 g, 9.2 mol) and tert-butylchlorodimethylsilane (1.1 g, 7.5 mmol) in dichloromethane (40 mL) was stirred for 12 hours at room temperature, then quenched with saturated aqueous $NaHCO_3$ solution (30 mL). The aqueous layer was extracted with dichloromethane (2x40 mL), and the combined organic layer was dried over anhydrous sodium sulfate and concentrated under vacuum. The crude product was purified by a silica gel column, eluted with 1 % - 3 % methanol in dichloromethane to afford **132** as a white solid (2.6 g, 75 %). (ES, *m/z*) $[M+H]^+$ 569.0; $^1H$ NMR (300 MHz, $CDCl_3$) $\delta$ 7.28 - 7.38 (m, 5H), 6.13 (d, *J* = 6.3 Hz, 1H), 4.49 (s, 2H), 4.34 - 4.36 (m, 1H), 4.06 - 4.08 (m, 1H), 3.83 - 3.90 (m, 1H), 3.59 - 3.67 (m, 2H), 3.30 - 3.55 (m, 5H), 1.58 (s, 9H), 0.98 (s, 9H), 0.15 (s, 3H), 0.08 (s, 3H).

**Step 8**

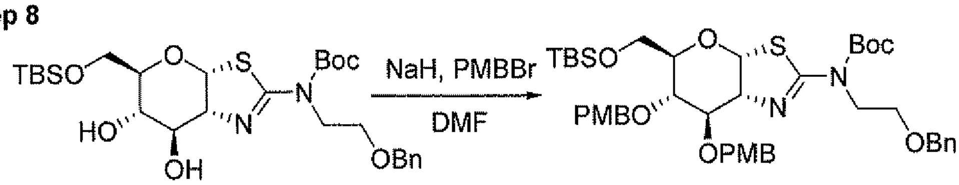


132 133

**tert-butyl 2-(benzyloxy)ethyl((3aR,5R,6S,7R,7aR)-5-((tert-butyldimethylsilyloxy)methyl)-6,7-bis(4-methoxybenzyloxy)-5,6,7,7a-tetrahydro-3aH-pyrano[3,2-d]thiazol-2-yl)carbamate (133):** A solution of **132** (12 g, 21 mmol) in DMF (60 mL) was treated with sodium hydride (2.9 g, 84 mmol, 70 % dispersed by mineral oil) for 30 minutes at 0 °C, followed by addition of PMBBr (25 g, 126 mmol). After additional 1.5 hours at 15 °C, the reaction was quenched with water (200 mL), and extracted with dichloromethane (3x100 mL). The combined organic layer was washed with brine (3x50 mL), dried over anhydrous sodium sulfate and concentrated under vacuum to give a residue, which was purified by a silica gel column, eluted with 5 % - 20 % ethyl acetate in petroleum ether to afford **133** as light yellow oil (8.5g, 50 %). (ES, *m/z*) $[M+H]^+$ 809.0; $^1H$ NMR (300 MHz, $CDCl_3$) $\delta$ 7.21 - 7.34 (m, 9H), 6.82 - 6.89 (m, 4H), 6.08 (d, *J* = 6.3 Hz, 1H), 4.52 - 4.57 (m, 4H), 4.50 (s, 2H), 4.33 - 4.35 (m, 1H), 4.05 - 4.06 (m, 1H), 3.84 - 3.91 (m, 1H), 3.81 (s, 3H), 3.80 (s, 3H), 3.58 - 3.66 (m, 2H), 3.31 - 3.54 (m, 5H), 1.56 (s, 9H), 0.96 (s, 9H), 0.14 (s, 3H), 0.07 (s, 3H).

Step 9

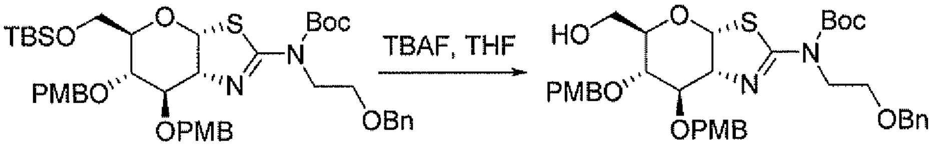


133 134

**tert-batyl-2-(benzyloxy)ethyl((3aR,5R,6S,7R,7aR)-5-(hydroxymethyl)-6,7-bis(4-methoxybenzyloxy)-5,6,7,7a-tetrahydro-3aH-pyrano[3,2-d]thiazol-2-yl)carbamate (134):** A solution of **133** (7.5 g, 9.3 mmol) in THF (30 mL) was treated with TBAF (4.9 g, 18,6 mmol) for 3 hours at room temperature. The reaction was quenched with water (100 mL) and extracted with ethyl acetate (3x50 mL). The combined organic layer was washed with brine (2x30 mL), dried over anhydrous sodium sulfate and concentrated under vacuum to give a residue, which was purified by a silica gel column, eluted with 3 % - 10 % ethyl acetate in petroleum ether to afford **134** as a light yellow syrup (5.8 g, 90 %). (ES, *m/z)* $[M+H]^+$ 695.0; $^1H$ NMR (300 MHz, $CDCl_3$) $\delta$ 7.21 - 7.36 (m, 9H), 6.80 - 6.85 (m, 4H), 6.09 (d, *J* = 6.3 Hz, 1H), 4.50 - 4.55 (m, 4H), 4.49 (s, 2H), 4.31 - 4.33 (m, 1H), 4.05 - 4.06 (m, 1H), 3.84 - 3.91 (m, 1H), 3.82 (s, 3H), 3.83 (s, 3H), 3.56

- 3.67 (m, 2H), 3.30 - 3.53 (m, 5H), 1.56 (s, 9H).

Step 10

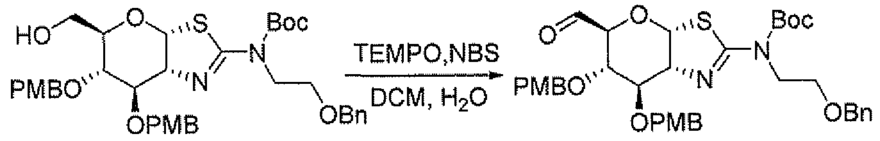


134 135

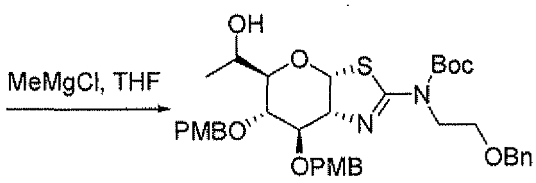


136

**tert-butyl 2-(benzyloxy)ethyl((3aR,5R,6S,7R,7aR)-5-((S)-1-hydroxyethyl)-6,7-bis(4-methoxybenzyloxy)-5,6,7,7a-tetrahydro-3aH-pyrano[3,2-d]thiazol-2-yl)carbamate (136):** To a solution of **134** (1.5 g, 2.2 mmol), $KHCO_3$ (970 mg, 9.7 mmol), TBAB (70 mg, 0.2 mmol), TEMPO (31 mg, 0.2 mmol) in dichloromethane (50 mL) and $H_2O$ (10 mL) was added NBS (423 mg, 2.4 mmol) at 0°C. After stirred for 30 minutes at room temperature, the reaction was diluted with water (50 mL) and the aqueous layer was extracted with dichloromethane (2x30 mL). The combined organic layers was dried over anhydrous sodium sulfate, and concentrated under vacuum to give crude **135**, which was dissolved into THF (20 mL). To the solution of **135** in THF was added MeMgBr (3 mL, 6 mmol, 2M in THF) at room temperature. After 1 hr, the reaction mixture was quenched with saturated aqueous $NH_4Cl$ solution (10 mL) and extracted with ethyl acetate (4x20 mL). The combined organic layer was washed with brine (2x20 mL), dried over anhydrous sodium sulfate and concentrated under vacuum. The crude residue was purified by a silica gel column, eluted with 10 % - 20 % ethyl acetate in petroleum ether to afford **136** (0.95 g, 63 %, two epimers, the ratio was 1:4 determined by $^1H$ NMR) as a light yellow syrup. (ES, *m/z*) $[M+H]^+$ 709.0; $^1H$ NMR (300 MHz, $CDCl_3$) δ 7.15 - 7.38 (m, 9H), 6.79 - 6.92 (m, 4H), 6.11 (m, 1H), 4.50 - 4.55 (m, 4H), 4.49 (s, 2H), 4.31 - 4.33 (m, 1H), 4.05 - 4.06 (m, 1H), 3.83 - 3.90 (m, 1H), 3.82 (s, 3H), 3.81 (s, 3H), 3.54 - 3.66 (m, 2H), 3.31 - 3.52 (m, 3H), 3.11 - 3.15 (m, 1H) 1.54 (s, 9H), 1.12 (d, *J* = 1.8 Hz, 3H).

**Step 11**

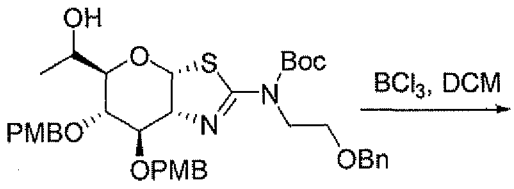


**136**

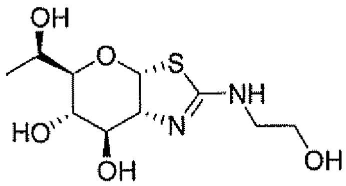

+

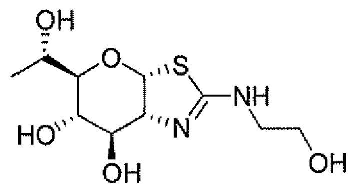

**Example 162** **Example 163**

**(3aR,5R,6S,7R,7aR)-5-((R)-1-hydroxyethyl)-2-(2-hydroxyethylamino)-5,6,7,7a-tetrahydro-3aH-pyrano[3,2-d]thiazole-6,7-diol and (3aR,5R,6S,7R,7aR)-5-((S)-1-hydro xyethyl)-2-(2-hydroxyethylamino)-5,6,7,7a-tetrahydro-3aH-pyrano[3,2-d]thiazole-6,7-diol:** A solution of **136** (1 g, 1.5 mmol) in dichloromethane (15 mL) was treated with $BCl_3$ (15 mL, 15 mmol, 1M in dichloromethane) at -78 °C for 3 hours, and then quenched by addition of methanol (10 mL). Volatiles were distilled out to give a residue, which was dissolved into methanol (5 mL) and neutralized with concentrated $NH_4OH$ (2 mL). After concentrated under reduced pressure, the crude product was purified by a silica gel column, eluted with 5 % - 20 % methanol in dichloromethane to give a mixture of the above two compounds. Separation by Prep-HPLC with the following conditions (Column, Sun fire prep. C18; mobile phase, water with 0.03 % $NH_4OH$ and $CH_3CN$ (10 % up to 45 % in time 10); Detector, UV 220nm) gave **Example 162** as a white solid (35 mg, 8.3 %, faster eluting isomer). (ES, *m/z*): $[M+H]^+$ 279.0; $^1H$ NMR (300 MHz, $D_2O$) δ 6.25 (d, *J* = 7.2 Hz, 1H), 4.16 - 4.17 (m, 1H), 3.97 - 3.99 (m, 2H), 3.55 - 3.59 (m, 3H), 3.51 - 3.52 (m, 1H), 3.28 - 3.29 (m, 2H), 1.08 (d, *J* = 6.6 Hz, 1H); and **Example 163** (200 mg, 47.7 %, slower eluting isomer). (ES, *m/z*):$[M+H]^+$ 279.0; $^1H$ NMR (300 MHz, $D_2O$) δ 6.24 (d, *J* = 6.3 Hz, 1H), 4.15 (t, *J*=5.7 Hz, 1H), 3.97 (t, *J* = 5.7 Hz, 1H), 3.84 - 3.87 (m, 1H), 3.57 - 3.63 (m, 3H), 3.21 - 3.30(m, 3H), 1.11 (d, *J* = 6.6 Hz,1H).

**Examples 164 and 165**

**(3aR,5S,6S,7R,7aR)-2-amino-5-[(1S)-2,2,2-trifluoro-1-hydroxyethyl]-3aH,5H,6H,7H,7aH-pyrano[3,2-d][1,3]thiazole-6,7-diol and (3aR,5S,6S,7R,7aR)-2-amino-5-[(1R)-2,2,2-trifluoro-1-hydroxyethyl]-3aH,5H,6H,7H,7aH-pyrano[3,2-d][1,3]thiazole-6,7-diol:**

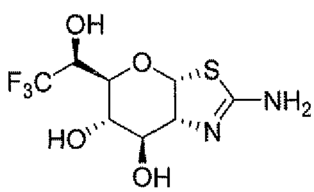

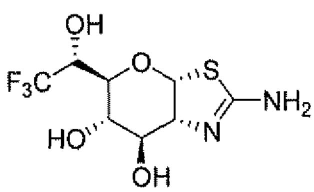

**Example 164** **Example 165**

Scheme XXVIII

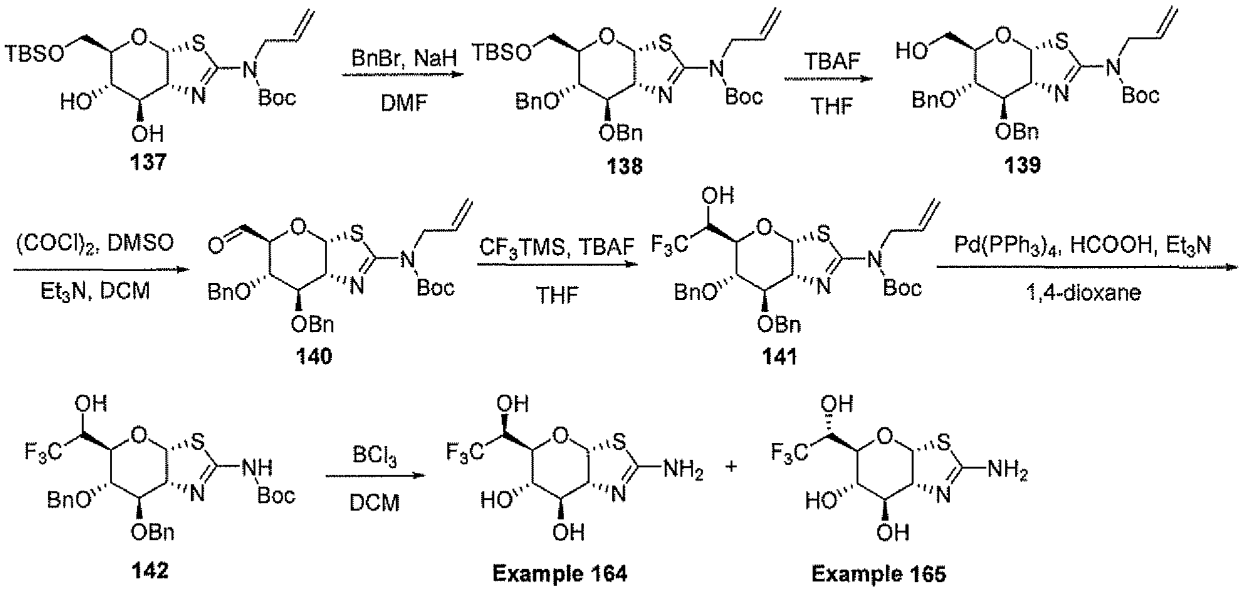


**Step 1**

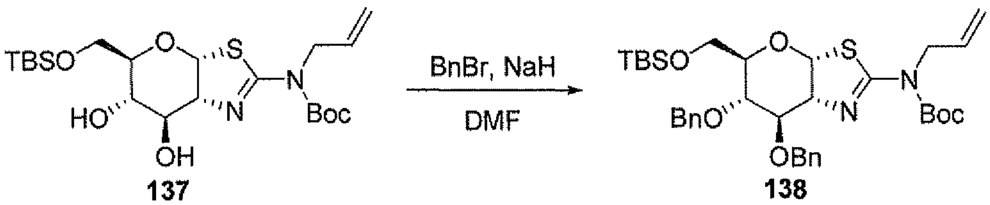


**tert-butyl N-[(3aR,5R,6S,7R,7aR)-6,7-bis(benzyloxy)-5-[[(tert-butyldimethylsilyl)oxy]methyl]-3aH,5H,6H,7H,7aH-pyrano[3,2-d][1,3]thiazol-2-yl]-N-(prop-2-en-1-yl)carbamate (138):** To a solution of tert-butyl N-[(3aR,5R,6S,7R,7aR)-5-[[(tert-butyldimethylsilyl)oxy]methyl]-6,7-dihydroxy-3aH,5H,6H,7H,7aH-pyrano[3,2-d][1,3]thiazol-2-yl]-N-(prop-2-en-1-yl)carbamate **137** (*prepared in a manner analogous to compound 17, Example 2 Steps 1 - 4*, except substituting allyl amine for propyl ylamine in step 1) (9 g, 19 mmol)) in DMF (80 mL) was added sodium hydride (3.1 g, 92 mmol, 70% dispersed by mineral oil) at 0 °C. After additional 30 min, benzyl bromide (5.2 mL, 48 mmol) was added. The resulting solution was stirred for 1 hour at 15 °C, then quenched by addition of saturated aqueous $NH_4Cl$ solution (200 mL) and extracted with dichloromethane (3x50 mL). The combined organic layer was washed with brine (3x50 mL), dried over anhydrous magnesium sulfate, and concentrated under vacuum. The residue was purified by flash column chromatography with 1% - 5% ethyl acetate in petroleum ether to afford **138** as a light yellow oil (9.5 g, 77%). (ES, *m/z*): $[M+H]^+$ 655.0; $^1H$ NMR (300 MHz, $CDCl_3$) $\delta$ 7.38 - 7.26 (m, 10 H), 6.09 (d, $J$ = 6.6 Hz, 1H), 5.89 - 5.75 (m, 1H), 5.14 - 5.00 (m, 2H), 4.80 - 4.56 (m, 3H), 4.45 - 4.32 (m, 3H), 4.16 (t, $J$ = 4.1 Hz, 1H), 4.05 - 3.45 (m, 5H), 1.50 (s, 9H), 0.88 (s, 9H), 0.04 (s, 6H).

**Step 2**

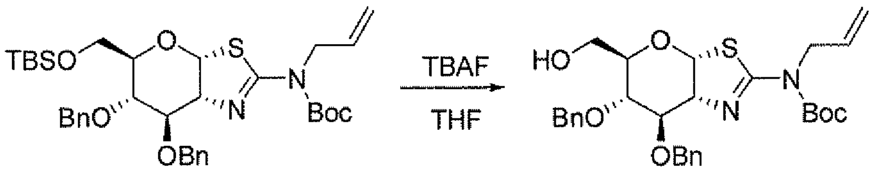


**138** **139**

**tert-butyl N-[(3aR,5R,6S,7R,7aR)-6,7-bis(benzyloxy)-5-(hydroxymethyl)-3aH,5H,6H,7H,7aH-pyrano[3,2-d][1,3]thiazol-2-yl]-N-(prop-2-en-1-yl)carbamate (139):** A solution of **138** (10 g, 15 mmol) in THF (50 mL) was treated with TBAF (8.5 g, 32 mmol) for 1.5 hours at room temperature. The reaction was then quenched by addition of water (100 mL) and extracted with ethyl acetate (3x50 mL). The combined organic layer was washed with brine (2x30 mL), dried over sodium sulfate, and concentrated under vacuum. The residue was purified by flash column chromatography with 5% - 10% ethyl acetate in petroleum ether to afford **139** as a light yellow syrup (6 g, 73%). (ES, *m/z*): $[M+H]^+$ 541.0; $^1H$ NMR (300 MHz, $CDCl_3$) $\delta$ 7.39 - 7.24 (m, 10 H), 6.08 (d, $J$ = 6.9 Hz, 1H), 5.92 - 5.75 (m, 1H), 5.13 - 4.99 (m, 2H), 4.76 - 4.64 (m, 2H), 4.55 (d, $J$ = 11.4 Hz, 1H), 4.43 - 4.34 (m, 3H), 4.28 - 4.26 (m, 1H), 3 .71 - 3.46 (m, 5H), 1.82 (t, $J$ = 6.5 Hz, 1H), 1.51 (s, 9H).

**Step 3**

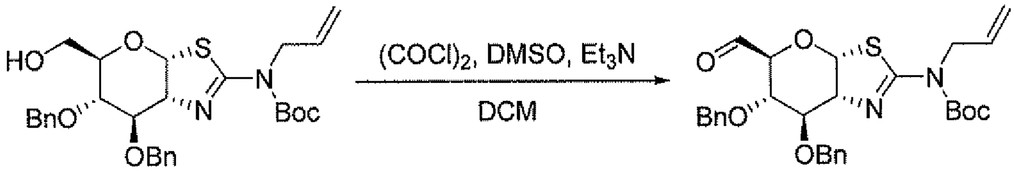


**139** **140**

**(3aR,5S,6S,7R,7aR)-6,7-bis(benzyloxy)-2-[methyl(prop-2-en-1-yl)aminol-3aH,5H,6H,7H,7aH-pyrano[3,2-d][1,3]thiazole-5-carbaldehyde (140):** A solution of DMSO (4.1 g, 52 mmol) in dry dichloromethane (70 mL) was treated with $(COCl)_2$ (4.9 g, 40 mmol) at -78 °C for 30 minutes, followed by addition of **139** (3 g, 6.6 mmol) in dichloromethane (10 mL) slowly. The resulting solution was stirred for 3 hours at -30 °C, then cooled down to -78 °C again and triethylamine (10 g, 90 mmol) was added. After additional 30 minutes at -50 °C, the resulting mixture was quenched by water (50 mL), and the aqueous layer was extracted with dichloromethane (3x30 mL). The combined organic layer was dried over sodium sulfate, and concentrated to afford crude the title compound as light yellow syrup, which was used in the next step without further purification.

**Step 4**

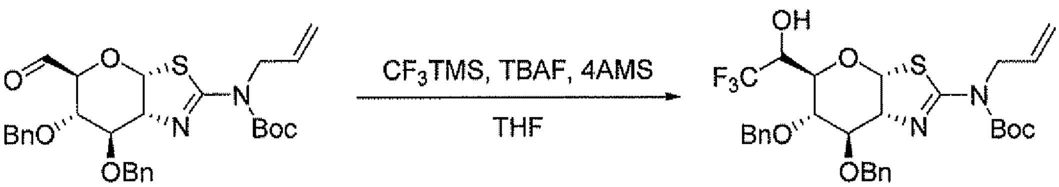


**140** **141**

**tert-butyl N-[(3aR,5R,6S,7R,7aR)-6,7-bis(benzyloxy)-5-(2,2,2-trifluoro-1-hydroxyethyl)-3aH,5H,6H,7H,7aH-pyrano[3,2-d][1,3]thiazol-2-yl]-N-(prop-2-en-1-yl)carbamate (141):** A mixture of TBAF (500 mg, 2 mmol) and 4Å molecular sieves (3 g) in THF (30 mL) was stirred for 30 minutes at 0 °C, followed by addition of a mixture of crude **140** and $CF_3TMS$ (3.7 g, 26 mmol) in THF (30 mL). The mixture was stirred for 2.5 hours at room temperature, followed by addition of additional TBAF (3 g, 11 mmol). After 1 hour, the reaction was then quenched by addition of water (20 mL). A filtration was performed and the filtrate was extracted with ethyl acetate (3x20 mL). The combined organic layer was washed with brine (2x20 mL), dried over magnesium sulfate, and concentrated. The residue was purified by flash column

chromatography with 10% - 20 % ethyl acetate in petroleum ether to afford the title compound as a light yellow oil (1.8 g, 53%, two isomers, ratio is 1:1 determined by $^1$H NMR). (ES, *m/z*): $[M+H]^+$609.0; $^1$H NMR (300 MHz, $CDCl_3$) δ 7.38 - 7.22 (m, 10H), 6.11 - 6.08 (m, 1H), 5.80 - 5.75 (m, 1H), 5.13 - 5.00 (m, 2H), 4.76 - 4.56 (m, 4H), 4.56 - 4.28 (m, 5H), 3.82 - 3.64 (m, 2H), 1.51 (s, 9H).

**Step 5**

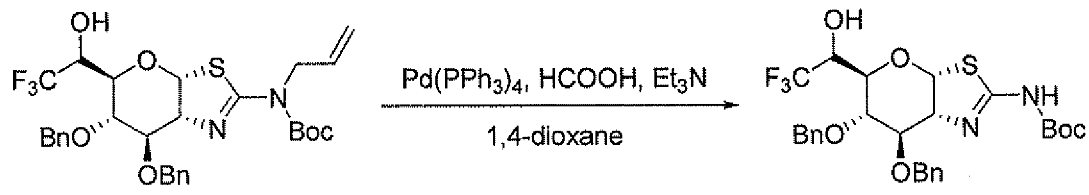


**141** **142**

**tert-butyl N-[(3aR,5R,6S,7R,7aR)-6,7-bis(benzyloxy)-5-(2,2,2-trifluoro-1-hydroxyethyl)-3aH,5H,6H,7H,7aH-pyrano[3,2-d][1,3]thiazol-2-yl]carbamate (142):**

To a solution of **141** (1.6 g, 2.6 mmol) in 1,4-dioxane (20 mL) was added $Pd(PPh_3)_4$ (760 mg, 0.6 mmol), triethylamine (664 mg, 6.5 mmol) and formic acid (242 mg, 5.2 mmol) at room temperature under nitrogen atmosphere. The resulting mixture was heated to 60 °C for 20 minutes, and followed by addition of additional formic acid (605 mg, 26 mmol). After stirred overnight at 60 °C, removal of volatiles gave a residue, which was dissolved into dichloromethane (50 mL) and neutralized with saturated aqueous $NaHCO_3$ solution (30 mL). The aqueous layer was extracted with dichloromethane (3x30 mL). The combined organic layer was dried over anhydrous sodium sulfate, and concentrated. The residue was purified by flash column chromatography with 20% - 30 % ethyl acetate in petroleum ether to afford **142** as colorless oil (1.1 g, 77%). (ES, *m/z):* $[M+H]^+$ 569.0; $^1$H NMR (300 MHz, $CDCl_3$) δ 7.34 - 7.23 (m, 10 H), 6.17 - 6.13 (m, 1 H), 4.62 - 4.46 (m, 2H), 4.43 - 4.00 (m, 4H), 3.91 - 3.73 (m, 3H), 1.52 (s, 9H).

**Step 6**

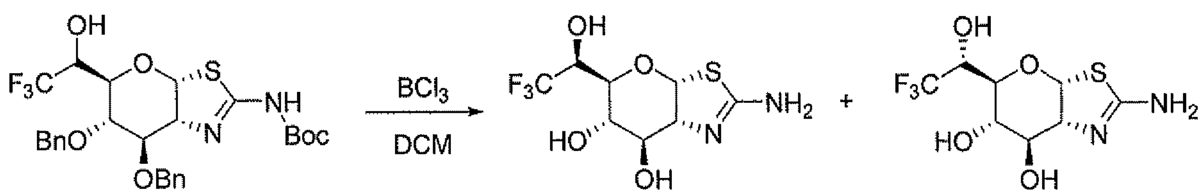


**142** **Example 164** **Example 165**

**(3aR,5S,6S,7R,7aR)-2-amino-5-[(1S)-2,2,2-trifluoro-1-hydroxyethyl]-3aH,5H,6H,7H,7aH-pyrano[3,2-d][1,3]thiazole-6,7-diol and (3aR,5S,6S,7R,7aR)-2-amino-5-[(1R)-2,2,2-trifluoro-1-hydroxyethyl]-3aH,5H,6H,7H,7aH-pyrano[3,2-d][1,3]thiazole-6,7-diol** A solution of **142** (1 g, 1.7 mmol) in dichloromethane (30 mL) was treated with $BCl_3$ (17 mL, 17 mmol, 1 M dichloromethane) at -78 °C for 4 hours. The reaction was quenched by addition of methanol (10 mL). Removal of volatiles gave a residue, which was dissolved into methanol (5 mL) and neutralized with concentrated $NH_4OH$ (2 mL). After concentrated under reduced pressure, the crude product was purified by a silica gel column, eluted with 5% - 20% methanol in dichloromethane to give a mixture of the above two compounds. Separation by Prep-HPLC with the following conditions (Column, Sun fire prep. C18; mobile phase, water with 0.03 % $NH_4OH$ and $CH_3CN$ (10 % up to 45 % in 10 min); Detector, UV 220nm) gave **(3aR,5S,6S,7R,7aR)-2-amino-5-[(1S)-2,2,2-trifluoro-1-hydroxyethyl]-3aH,5H,6H,7H,7aH-pyrano[3,2-d][1,3]thiazole-6,7-diol Example 164** as a light yellow solid (49 mg, 20%, faster eluting isomer); (ES, *m/z*): $[M+H]^+$ 289.0; $^1$H NMR (400 MHz, $D_2O$) δ 6.25 (d, *J* = 6.8 Hz, 1H), 4.29 - 4.26 (m, 2H), 4.07 (t, *J* = 4.0 Hz, 1H), 3.93 (dd, *J* = 8.4, 3.2 Hz, 1H), 3.73 (dd, *J* = 8.4, 4.8 Hz, 1H); and **(3aR,5S,6S,7R,7aR)-2-amino-5-[(1R)-2,2,2-trifluoro-1-hydroxyethyl]-3aH,5H,6H,7H,7aH-pyrano[3,2-d][1,3]thiazole-6,7-diol** as a light yellow solid (74 mg, 30%, slower eluting isomer); (ES, *m/z*): $[M+H]^+$ 289.0; $^1$HNMR (400 MHz, $D_2O$) δ 6.32 (d, *J* = 6.4 Hz, 1H), 4.31

(q, $J$ = 7.6 Hz, 1H), 4.15 (t, $J$ = 6.0 Hz, 1H), 4.00 (t, $J$ = 5.0 Hz, 1H), 3.83 - 3.76 (m, 2H).

The following compounds listed in Table were prepared by methods substantially as described above for the previous examples.

**Table**

| Example | Structure | Name | MH+ |
|---|---|---|---|
| 166 | 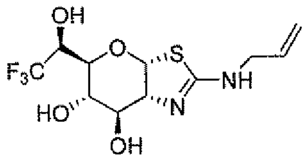 | (3aR,5S,6S,7R,7aR)-2-(allylamino)-5-((S)-2,2,2-trifluoro-1-hydroxyethyl)-5,6,7,7a-tetrahydro-3aH-pyrano[3,2-d]thiazole-6,7-diol (Faster eluting isomer by HPLC) | 328.9 |
| 167 | 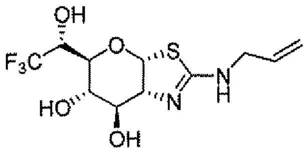 | (3aR,5S,6S,7R,7aR)-2-(allylamino)-5-((R)-2,2,2-trifluoro-1-hydroxyethyl)-5,6,7,7a-tetrahydro-3aH-pyrano[3,2-d]thiazole-6,7-diol (Slower eluting isomer by HPLC) | 328.9 |
| 168 | 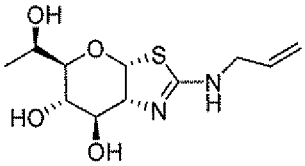 | (3aR,5R,6S,7R,7aR)-2-(allylamino)-5-((R)-1-hydroxyethyl)-5,6,7,7a-tetrahydro-3aH-pyrano[3,2-d]thiazole-6,7-diol (Faster eluting isomer by HPLC) | 275.0 |
| 169 | 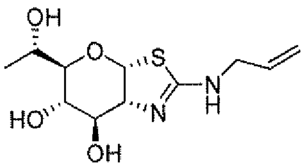 | (3aR,5R,6S,7R,7aR)-2-(allylamino)-5-((S)-1-hydroxyethyl)-5,6,7,7a-tetrahydro-3aH-pyrano[3,2-d]thiazole-6,7-diol (Slower eluting isomer by HPLC) | 275.0 |
| 170 | 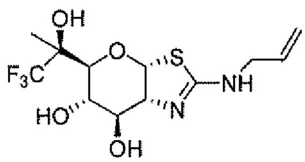 | (3aR,5S,6S,7R,7aR)-2-(allylamino)-5-((S)-1,1,1-trifluoro-2-hydroxypropan-2-yl)-5,6,7,7a-tetrahydro-3aH-pyrano[3,2-d]thiazole-6,7-diol (Faster eluting isomer by HPLC) | 343.0 |
| 171 | 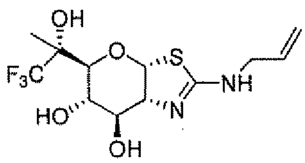 | (3aR,5S,6S,7R,7aR)-2-(allylamino)-5-(1,1,1-trifluoro-2-hydroxypropan-2-yl)-5,6,7,7a-tetrahydro-3aH-pyrano[3,2-d]thiazole-6,7-diol (Slower eluting isomer by HPLC) | 343.0 |
| 172 | 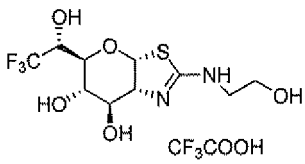 $CF_3COOH$ | (3aR,5S,6S,7R,7aR)-2-(2-hydroxyethylamino)-5-((R)-2,2,2-trifluoro-1-hydroxyethyl)-5,6,7,7a-tetrahydro-3aH-pyrano[3,2-d]thiazole-6,7-diol (Slower eluting isomer by HPLC)2,2,2-trifluoroacetate (Slower eluting isomer by HPLC) | 333.0 |
| 173 | 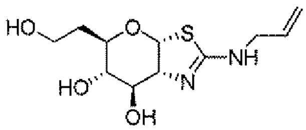 | (3aR,5R,6S,7R,7aR)-2-(allylamino)-5-(2-hydroxyethyl)-5,6,7,7a-tetrahydro-3aH-pyrano[3,2-d]thiazole-6,7-diol | 275.0 |
| 174 | 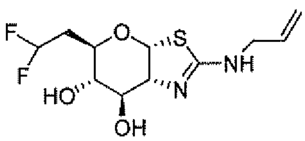 | (3aR,5R,6S,7R,7aR)-2-(allylamino)-5-(2,2-difluoroethyl)-5,6,7,7a-tetrahydro-3aH-pyrano[3,2-d]thiazole-6,7-diol | 295.0 |

**Example 175 and 176**

**(3aR,5S,6S,7R,7aR)-2-amino-5-[(2S)-1,1,1-trifluoro-2-hydroxypropan-2-yl]-3aH,5H,6H,7H,7aH-pyrano[3,2-d][1,3]thiazole-6,7-diol and (3aR,5S,6S,7R,7aR)-2-amino-5-[(2R)-1,1,1-trifluoro-2-hydroxypropan-2-yl]-3aH,5H,6H,7H,7aH-pyrano[3,2-d][1,3]thiazole-6,7-diol:**

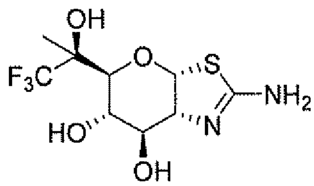

**Example 175**

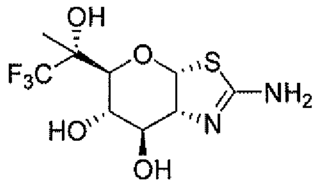

**Example 176**

Scheme XXIV

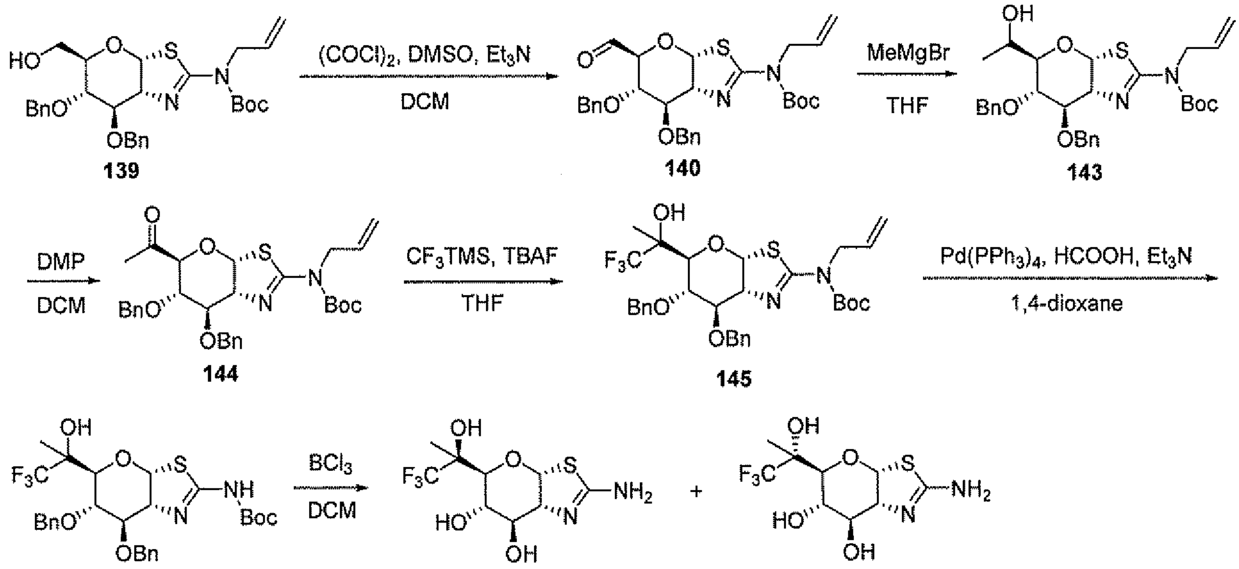


139, 140, 143, 144, 145, 146, Example 175, Example 176

**Step 1**

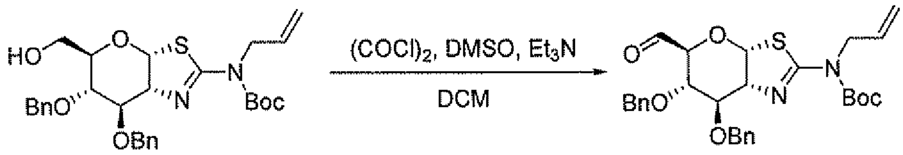


139 140

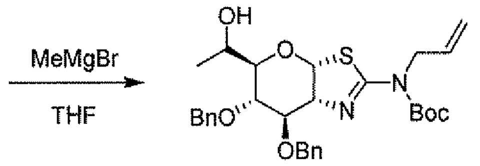


143

**tert-butyl N-[(3aR,5R,6S,7R,7aR)-6,7-bis(benzyloxy)-5-(1-hydroxyethyl)-3aH,5H,6H,7H,7aH-pyrano[3,2-d][1,3]thiazol-2-yl]-N-(prop-2-en-1-yl)carbamate (143):** A solution of DMSO (9.2 g, 118 mmol) in dry dichloromethane (70 mL) was treated with $(COCl)_2$ (11.6 g, 92 mmol) at -78 °C for 30 minutes, followed by addition of tert-butyl N-[(3aR,5R,6S,7R,7aR)-6,7-bis(benzyloxy)-5-(hydroxymethyl)-3aH,5H,6H,7H,7aH-pyrano[3,2-d][1,3]thiazol-2-yl]-N-(prop-2-en-1-yl)carbamate (8 g, 15 mmol) in dichloromethane (15 mL) slowly. The resulting solution was stirred for 3 hours at -30 °C, then cooled down to -78 °C again before addition of triethylamine (20 g, 180 mmol). After additional 30 minutes at -50 °C, the resulting mixture was quenched by water (50 mL), and the aqueous layer was extracted with dichloromethane (3x50 mL). The combined organic layer was dried over sodium sulfate, and concentrated to afford crude **140** as a light yellow syrup, which was dissolved into anhydrous THF (100 mL). The solution was treated with bromo(methyl)magnesium (20 ml, 40 mmol, 2M in THF) at 0 °C overnight. The reaction was then quenched with saturated aqueous $NH_4Cl$ solution (100 mL), and extracted with ethyl acetate (3x100 mL). The combined organic layer was washed with brine (2x50 mL), dried over anhydrous magnesium sulfate and concentrated under vacuum. The residue was purified by flash column chromatography with 10% - 20 % ethyl acetate in petroleum ether to afford **143** as a yellow syrup (7 g, 87%, two isomers, the ratio is 1:3.8 determined by $^1$HNMR). (ES, *m/z*): $[M+H]^+$ 555.0; $^1$H NMR (300 MHz, $CDCl_3$): 7.38 - 7.25 (m, 10H), 6.13 - 6.11 (m, 1H), 5.82 - 5.70 (m, 1H), 5.99 - 4.96 (m, 2H), 4.76 - 4.56 (m, 2H), 4.54 - 4.27 (m, 6H), 4.13 - 3.73 (m, 3H), 1.50 (s, 9H), 1.28 - 1.20 (m, 3H).

**Step 2**

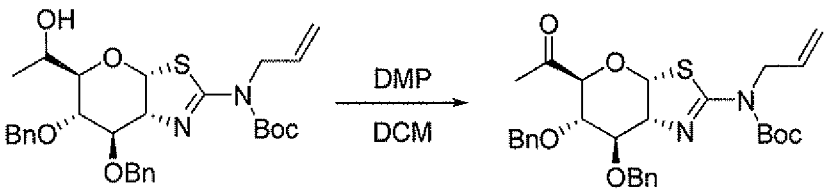


143 144

**tert-butyl N-[(3aR,5S,6S,7R,7aR)-5-acetyl-6,7-bis(benzyloxy)-3aH,5H,6H,7H,7aH-pyrano[3,2-d][1,3]thiazol-2-yl]-N-(prop-2-en-1-yl)carbamate (144):** To a solution of **143** (6 g, **11** mmol) in dichloromethane (120 mL) was added DMP (6.9 g, 16 mmol). The resulting solution was stirred for 1.5 hours at room temperature, then quenched by saturated aqueous sodium bicarbonate solution (80 mL), and extracted with dichloromethane (3x100 mL). The combined organic layer was washed with brine (2x50 mL), dried over anhydrous magnesium sulfate and concentrated under vacuum. The residue was purified by flash column chromatography with 5% - 10 % ethyl acetate in petroleum ether to afford **144** as a yellow syrup (4 g, 67%). (ES, *m/z*): $[M+H]^+$ 553.0; $^1$H NMR (300 MHz ,$CDCl_3$): 7.34 - 7.19 (m, 10 H), 6.10 (d, *J* = 6.9 Hz, 1H), 5.85 - 5.76 (m, 1H), 5.10 - 4.94 (m, 2H), 4.69 - 4.25 (m, 7H), 4.15 - 4.10 (m, 1H), 3.97 - 3.83 (m, 2H), 2.19 (s, 3H), 1.50 (s, 9H).

Step 3

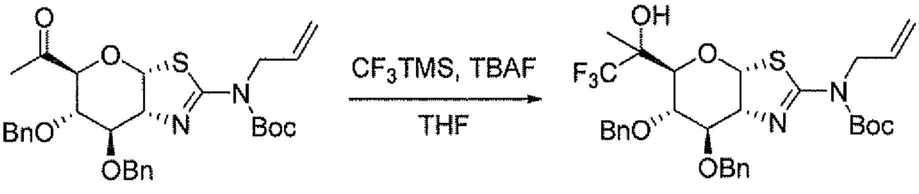


144 145

**tert-butyl N-[(3aR,5S,6S,7R,7aR)-6,7-bis(benzyloxy)-5-(1,1,1-trifluoro-2-hydroxypropan-2-yl)-3aH,5H,6H,7H,7aH-pyrano[3,2-d][1,3]thiazol-2-yl]-N-(prop-2-en-1-yl)carbamate (145):** A mixture of TBAF (1.1, 4.2 mmol) and 4Å molecular sieves (4 g) in THF (40 mL) was stirred for 30 minutes at 0 °C, followed by addition of a mixture of **144** (4.6 g, 8.3 mmol) and $CF_3TMS$ (5.9 g, 42 mmol) in THF (40 mL). The mixture was stirred for 3 hours at room temperature, followed by addition of additional TBAF (6 g, 22 mmol). After 1 hour, the reaction was then quenched by addition of water (80 mL). A filtration was performed and the filtrate was extracted with ethyl acetate (3x50 mL). The combined organic layer was washed with brine (2x30 mL), dried over magnesium sulfate, and concentrated. The residue was purified by flash column chromatography with 6% - 12% ethyl acetate in petroleum ether to afford the title compound as a brown syrup (3.5 g, 68%, two isomers, ratio is 1:1 determined by $^1H$ NMR). (ES, *m/z*): $[M+H]^+$ 623.0; $^1H$ NMR (300 MHz, $CDCl_3$): 7.37 - 7.23 (m, 10H), 6.13 - 6.07 (m, 1H), 5.76 - 5.74 (m, 1H), 5.05 - 4.92 (m, 2H), 4.74 - 4.67 (m, 2H), 4.47 - 4.25 (m, 7H), 3.93 - 3.88 (m, 1H), 1.50 (s, 9H), 1.28 - 1.23 (m, 3H).

Step 4

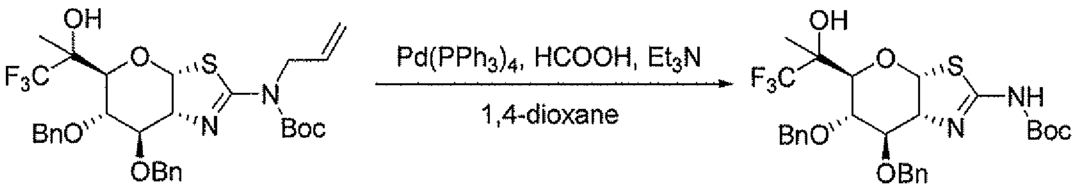


145 146

**tert-butyl (3aR,5S,6S,7R,7aR)-6,7-bis(benzyloxy)-5-((S)-1,1,1-trifluoro-2-hydroxypropan-2-yl)-5,6,7,7a-tetrahydro-3aH-pyrano [3,2-d] thiazol-2-ylcarbamate (146):** To a solution of **145** (1.7 g, 2.7 mmol) in 1,4-dioxane (50 mL) was added $Pd(PPh_3)_4$ (643 mg, 0.5 mmol), formic acid (260 mg, 5.6 mmol) and triethylamine (702 mg, 7.0 mmol) at room temperature under nitrogen atmosphere. The resulting mixture was heated to 60 °C, for 20 minutes, and followed by addition of additional formic acid (1.3 g, 28 mmol). After stirred overnight at 60 °C, removal of volatiles gave a residue, which was dissolved into dichloromethane (50 mL) and neutralized with saturated aqueous $NaHCO_3$ solution (30 mL). The aqueous layer was extracted with dichloromethane (3x30 mL). The combined organic layer was dried over anhydrous sodium sulfate, and concentrated. The residue was purified by flash column chromatography with 15% - 30% ethyl acetate in petroleum ether to afford the title compound as a orange syrup (1.4 g, 86%). (ES, *m/z):* $[M+H]^+$: 583.0; $^1H$ NMR (300 MHz, $CDCl_3$): 7.71 - 7.23 (m, 10H), 6.20 - 6.14 (m, 1H), 4.71 - 4.66 (m, 2H), 4.63 - 4.38 (m, 3H), 4.21 - 4.10 (m, 1H), 3 .93 - 3.92 (m, 1H), 3.79 - 3.66 (m, 1H), 1.50 (s, 9H), 1.38 - 1.08 (m, 3H).

Step 5

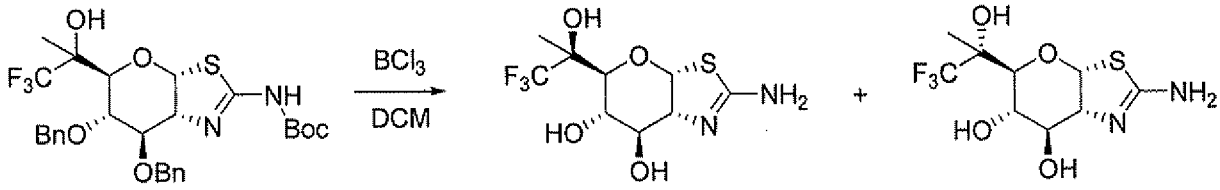


146 Example 175 Example 176

**(3aR,5S,6S,7R,7aR)-2-amino-5-[(2S)-1,1,1-trifluoro-2-hydroxypropan-2-yl]-3aH,5H,6H,7H,7aH-pyrano[3,2-d][1,3]thiazole-6,7-diol and (3aR,5S,6S,7R,7aR)-2-amino-5-[(2R)-1,1,1-trifluoro-2-hydroxypropan-2-yl]-3aH,5H,6H,7H,7aH-pyrano[3,2-d][1,3]thiazole-6,7-diol** : A solution of **146** (0.9 g, 1.5 mmol) in dichloromethane (20 mL) was treated with $BCl_3$ (15 mL, 15 mmol, 1M dichloromethane) at -78°C for 4 hours. The reaction was quenched by addition of methanol (10 mL). Removal of volatiles gave a residue, which was dissolved into methanol (5 mL) and neutralized with concentrated $NH_4OH$ (2 mL). After concentrated under reduced pressure, the crude product was purified by a silica gel column, eluted with 5% - 20% methanol in dichloromethane to give a mixture of the above two compounds. Separation by Prep-HPLC with the following conditions (Column, Sun fire prep. C18; mobile phase, water with 0.03 % $NH_4OH$ and $CH_3CN$ (15% up to 30% in 20 min); Detector, UV 220nm) gave **(3aR,5S,6S,7R,7aR)-2-amino-5-1(2S)-1,1,1-trifluoro-2-hydroxypropan-2-yl]-3aH,5H,6H,7H,7aH-pyrano[3,2-d][1,3]thiazole-6,7-diol** as a white solid (35.2 mg, 8%, faster eluting isomer). (ES, *m/z*): $[M+H]^+$: 303.0; $^1H$ NMR (300 MHz, $D_2O$): 6.27 (d, *J* = 6.9 Hz, 1H), 4.37 - 4.34 (m, 1H), 4.15 - 4.13 (m, 1H), 3.92 (d, *J* = 8.4 Hz, 1H), 3.56 (d, *J* = 8.4 Hz, 1H), 1.31 (s, 3H); and **(3aR,5S,6S,7R,7aR)-2-amino-5-[(2R)-1,1,1-trifluoro-2-hydroxypropan-2-yl]-3aH,5H,7H,7H,7aH-pyrano[3,2-d][1,3]thiazole-6,7-diol** as a white solid (35.1 mg, 8%, slower eluting isomer). (ES, *m/z):* $[M+H]^+$: 303.0; $^1H$ NMR (300 MHz, $D_2O$): 6.27 (d, *J* = 6.9 Hz, 1H), 4.38 - 4.35 (m, 1H), 4.16 - 4.14 (m, 1H), 3.96 (d, *J* = 8.1 Hz, 1 H), 3.58 (d, *J* = 8.4 Hz, 1H), 1.32 (s, 3H).

**Examples 177 and 178**

**(3aR,5R,6S,7R,7aR)-2-amino-5-vinyl-5,6,7,7a-tetrahydro-3aH-pyrano[3,2-d]thiazole-6,7-diol & (3aR,5R,6S,7R,7aR)-2-amino-5-ethyl-5,6,7,7a-tetrahydro-3aH-pyrano[3,2-d]thiazole-6,7-diol :**

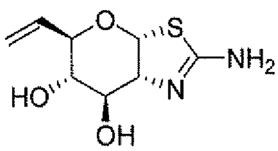

Example 177

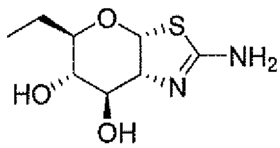

Example 178

Scheme XXX

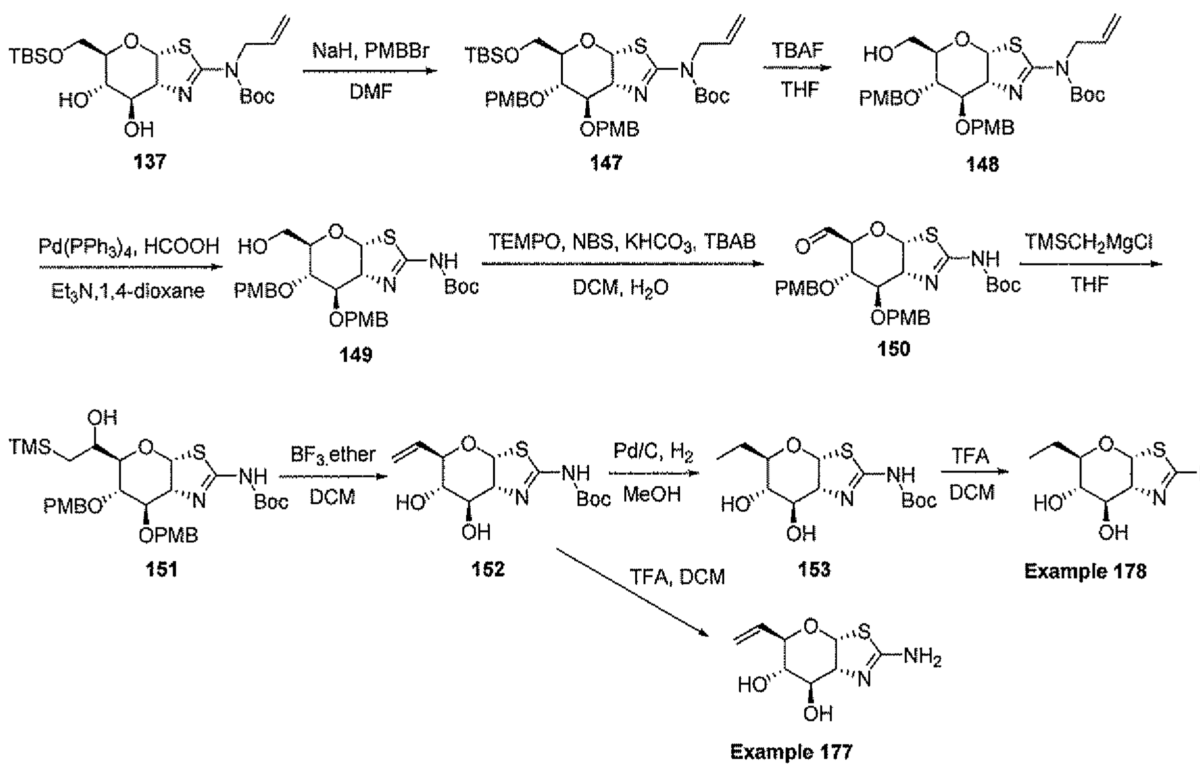


**Step 1**

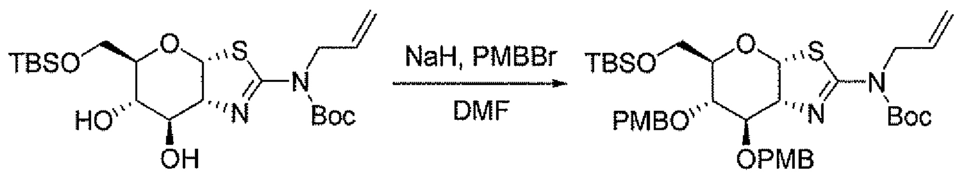


**137** **147**

**tert-butyl allyl((3aR,5R,6S,7R,7aR)-5-((tert-butyldimethylsilyloxy)methyl)-6,7-bis(4-methoxybenzyloxy)-5,6,7,7a-tetrahydro-3aH-pyrano[3,2-d]thiazol-2-yl)carbamate (147):** A solution of tert-butyl allyl((3aR,5R,6S,7R,7aR)-5-((tert-butyldimethylsilyloxy)methyl)-6,7-dihydroxy-5,6,7,7a-tetrahydro-3aH-pyrano[3,2-d]thiazol-2-yl)carbamate **137** (*prepared in a manner analogous to compound 17, Example 2 Steps 1 - 4*, except substituting allyl amine for propyl ylamine in step 1) (20 g, 42 mmol) in DMF (300 mL) was treated with sodium hydride (5.8 g, 168 mmol, 70% dispersed by mineral oil) for 30 minutes at 0 °C, followed by addition of PMBBr (34 g, 168 mmol). After additional 1.5 hours at 15 °C, the reaction was quenched with water (300 mL), and extracted with ethyl acetate (3x200 mL). The combined organic layer was washed with brine (3x150 mL), dried over anhydrous sodium sulfate and concentrated under vacuum. The residue was purified by flash column chromatography with 5% - 20% ethyl acetate in petroleum ether to afford the title compound as a light yellow oil (16 g, 53%). (ES, *m/z*): $[M+H]^+$ 715.0; $^1H$ NMR (300 MHz, $CDCl_3$) δ 7.36 - 7.33 (m, 2H), 7.24 - 7.21 (m, 2H), 6.92 - 6.85 (m, 4H), 6.10 (d, *J* = 6.6 Hz, 1H), 6.80 - 5.95 (m, 1H), 5.21 - 5.03 (m, 2H), 4.71 - 4.66 (m, 3H), 4.53 - 4.44 (m, 2H), 4.43 - 4.34 (m, 3H), 4.15 (t, *J* = 5.4 Hz, 1H), 3.91 - 3.84 (m, 1H), 3.82 (s, 3H), 3.81 (s, 3H), 3.78 - 3.74 (m, 2H), 1.53 (s, 9H), 0.09 (s, 9H), 0.06 (s, 6H).

**Step 2**

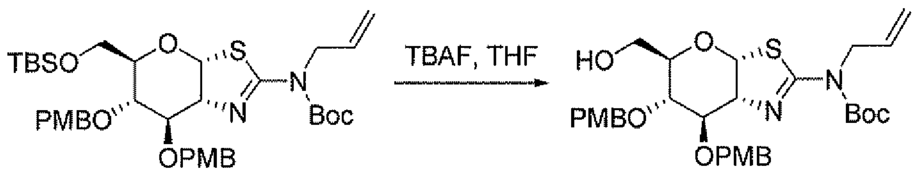


**147** **148**

**tert-butyl allyl((3aR,5R,6S,7R,7aR)-5-(hydroxymethyl)-6,7-bis(4-methoxybenzyloxy)-5,6,7,7a-tetrahydro-3aH-pyrano[3,2-d]thiazol-2-yl)carbamate (148):** A solution of **147** (15 g, 21 mmol) in THF (100 mL) was treated with TBAF (8.4 g, 32 mmol) for 3 hours at room temperature. The reaction was quenched with water (150 mL) and extracted with ethyl acetate (3x100 mL). The combined organic layer was washed with brine (2x50 mL), dried over anhydrous sodium sulfate and concentrated under vacuum. The residue was purified by flash column chromatography with 10% - 30% ethyl acetate in petroleum ether to afford **148** as a light yellow syrup (11.3 g, 90%). (ES, *m/z):* $[M+H]^+$ 601.0; $^1H$ NMR (300 MHz, $CDCl_3$) δ 7.36 - 7.33 (m, 2H), 7.21 - 7.19 (m, 2H), 6.91 - 6.82 (m, 4H), 6.09 (d, *J* = 6.6 Hz, 1H), 5.92 - 5.80 (m, 1H), 5.19 - 5.00 (m, 2H), 4.70 - 4.58 (m, 2H), 4.55 - 4.36 (m, 4H), 4.32 - 4.24 (m, 2H), 3.82 (s, 3H), 3.83 (s, 3H), 3.75 - 3.68 (m, 2H), 3.64 - 3.52 (m, 2H), 1.56 (s, 9H).

**Step 3**

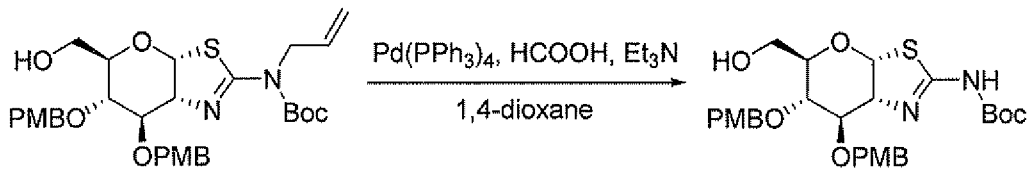


**148** **149**

**tert-butyl (3aR,5R,6S,7R,7aR)-5-(hydroxymethyl)-6,7-bis(4-methoxybenzyloxy)-5,6,7,7a-tetrahydro-3aH-pyrano[3,2-d]thiazol-2-ylcarbamate (149):** To a solution of **148** (10.5 g, 18 mmol) in 1,4-dioxane (100 mL) was added $Pd(PPh_3)_4$ (4.2 g, 3.6 mmol), $Et_3N$ (4.6 g, 45 mmol) and formic acid (4.9 g, 107 mmol) at room temperature under $N_2$ atmosphere. After 20 minutes at 60 °C, additional formic acid (24.5 g, 535 mmol) was added. The reaction was stirred at 60 °C for additional 3 hours. Removal of volatiles gave a residue, which was dissolved into dichloromethane (100 mL) and neutralized with saturated aqueous $NaHCO_3$ solution (30 mL). The aqueous layer was extracted with dichloromethane (3x50 mL). The combined organic layer was dried over anhydrous sodium sulfate, and concentrated under vacuum. The residue was purified by flash column chromatography with 10% - 50% ethyl acetate in petroleum ether to afford the title compound as a white syrup (6.4 g, 65%). (ES, *m/z*): $[M+H]^+$ 561.0; $^1H$ NMR (300 MHz, $CDCl_3$) δ 7.30 - 7.23 (m, 4H), 6.92 - 6.87 (m, 4H), 6.14 (d, *J* = 6.9 Hz, 1H), 4.56 - 4.52 (m, 3H), 4.36 - 4.32 (m, 1H), 4.25 - 4.19 (m, 1H), 4.13 - 4.07 (m, 1H), 3.82 - 3.80 (m, 1H), 3.79 (s, 3H), 3.78 (s, 3H), 3.76 - 3.51 (m, 3H), 1.54 (s, 9H).

**Step 4**

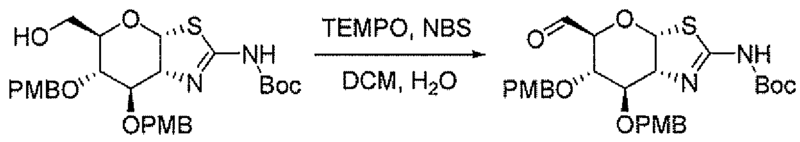


149 150

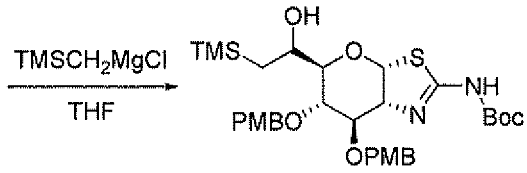


151

**tert-butyl (3aR,5R,6S,7R,7aR)-5-((R)-1-hydroxy-2-(trimethylsilyl)ethyl)-6,7-bis(4-methoxybenzyloxy)-5,6,7,7a-tetrahydro-3aH-pyrano[3,2-d]thiazol-2-ylcarbamate (51):**

To a mixture of 3 (2 g, 3.6 mmol), $KHCO_3$ (1.6 g, 16 mmol), TBAB (105 mg, 0.4 mmol), TEMPO (62 mg, 0.4 mmol) in dichloromethane (50 mL) and $H_2O$ (10 mL) was added NBS (695 mg, 3.9 mmol) at 0 °C. After stirred for 30 minutes at room temperature, the reaction was diluted with water (50 mL) and the aqueous layer was extracted with dichloromethane (2x40 mL). The combined organic layer was dried over anhydrous sodium sulfate, and concentrated under vacuum to give crude aldehyde 150, which was dissolved into THF (20 mL). The solution was treated with ((trimethylsilyl)methyl)magnesium chloride (7.2 mL, 7.2 mmol, 1M in ether). After 2 hours at 10 °C, the reaction was quenched with saturated aqueous $NH_4Cl$ solution (40 mL) and extracted with ethyl acetate (4x20 mL). The combined organic layer was washed with brine (2x30 mL), dried over anhydrous sodium sulfate and concentrated under vacuum. The residue was purified by flash column chromatography with 5% - 30% ethyl acetate in petroleum ether to afford **151** as a light yellow syrup (1.1 g, 48 %, two epimers, the ratio is 1:3 determined by $^1$HNMR). (ES, *m/z*): [M+H]l$^+$ 647.0; $^1$H NMR (300 MHz, $CDCl_3$) δ 7.31 - 7.19 (m, 4H), 6.93 - 6.85 (m, 4H), 6.23 - 6.19 (m, 1H), 4.62 - 4.50 (m, 4H), 4.47 - 4.43 (m, 3H), 3.85 - 3.83 (m, 1H), 3.81 (s, 3H), 3.80 (s, 3H), 3.25 - 3.21 (m, 1H), 1.54 (s, 9H), 0.89 - 0.87 (m, 1H), 0.74 - 0.72 (m, 1H), 0.05 (s, 3H), 0.04 (s, 3H), 0.03 (s, 3H).

**Step 5**

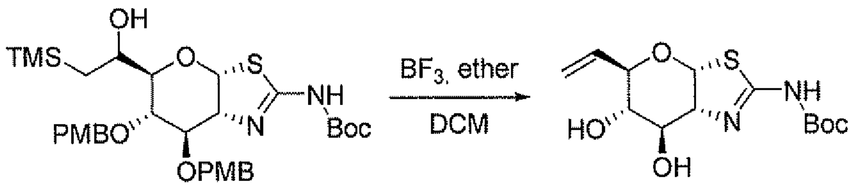


151 152

**tert-butyl (3aR,5R,6S,7R,7aR)-6,7-dihydroxy-5-vinyl-5,6,7,7a-tetrahydro-3aH-pyrano[3,2-d]thiazol-2-ylcarbamate (6):** A solution of **151** (900 mg, 1.4 mmol) in dichloromethane (20 mL) was treated with $BF_3$ (0.1 mL, 0.1 mmol, 1M in ether) for 20 minutes at 10 °C. Removal of volatiles gave a residue, which was dissolved into dichloromethane (20 mL) and neutralized with saturated aqueous $NaHCO_3$ solution (10 mL). The aqueous layer was extracted with dichloromethane (5x20 mL). The combined organic layer was dried over anhydrous sodium sulfate, and concentrated under vacuum. The residue was purified by flash column chromatography with 1% - 10% methanol in dichloromethane to afford **152** as a light yellow syrup (240 mg, 55%). (ES, *m/z):* [M+H]$^+$ 317.0; $^1$H NMR (300 MHz, $CDCl_3$) δ 6.11 (d, *J* = 6.6 Hz, 1H), 5.77 - 5.63 (m, 1H), 5.24 - 5.16 (m, 2H), 4.08 - 4.02 (m, 1H), 3.76 - 3.61 (m, 2H), 3.55 - 3.46 (m, 1H), 1.53 (s, 9H).

**Step 6**

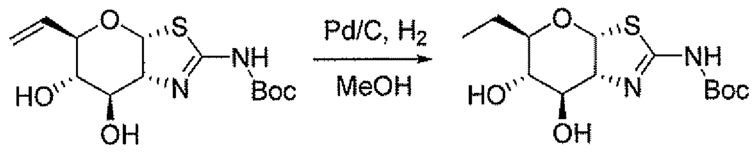


**152** **153**

**tert-butyl (3aR,5R,6S,7R,7aR)-5-ethyl-6,7-dihydroxy-5,6,7,7a-tetrahydro-3aH-pyrano[3,2-d]thiazol-2-ylcarbamate (7):** A mixture of **152** (160 mg, 0.5 mmol) and Pd/C (16 mg, 10% w/w) in methanol (20 mL) was stirred for 3 hours at room temperature under $H_2$ atmosphere (1 atm). After filtration, removal of solvents provided a residue, which was purified by flash column chromatography with 2% - 10% methanol in dichloromethane to afford **153** as a white syrup (130 mg, 81%). (ES, *m/z):* $[M+H]^+$ 319.0; $^1H$ NMR (300 MHz, $CDCl_3$) δ 6.07 (d, *J* = 6.3 Hz, 1H), 4.40 (t, *J* = 5.9 Hz, 1H), 4.17 (t, *J* = 6.6 Hz, 1H), 3.65 - 3.59 (m, 2H), 1.84 - 1.72 (m, 1H), 1.62 - 1.45 (m, 1H), 1.53 (s, 9H), 0.84 (t, *J* = 7.5 Hz, 3H).

**Step 7**

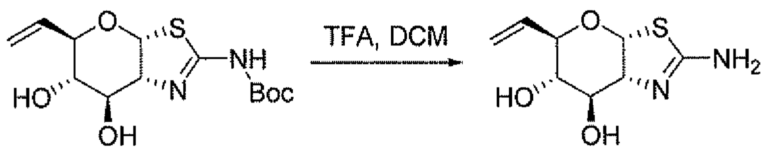


**152** **Example 177**

**(3aR,5R,6S,7R,7aR)-2-amino-5-vinyl-5,6,7,7a-tetrahydro-3aH-pyrano[3,2-d]thiazole-6,7-diol :** To solution of **152** (65 mg, 0.2 mmol) in dichloromethane (10 mL) was added TFA (1 mL). After 2 hours at room temperature, Removal of volatiles gave a residue, which was dissolved into methanol (3 mL) and neutralized with concentrated $NH_4OH$ (1 mL). After concentrated under reduced pressure, the crude product was purified by flash column chromatography with 5% - 30% methanol in dichloromethane to give (3aR,5R,6S,7R,7aR)-2-amino-5-vinyl-5,6,7,7a-tetrahydro-3aH-pyrano[3,2-d]thiazole-6,7-diol as a white solid (30 mg, 68 %). (ES, *m/z*): $[M+H]^+$ 217.0; $^1H$ NMR (300 MHz, $D_2O$) δ 6.24 (d, *J* = 6.3 Hz, 1 H), 5.87 - 5.75 (m, 1H), 5.35 - 5.26 (m, 2H), 4.07 (t, *J* = 6.3 Hz, 1H), 3.98 - 3.88 (m, 2H), 3.50 - 3.24 (m, 1H).

**Step 8**

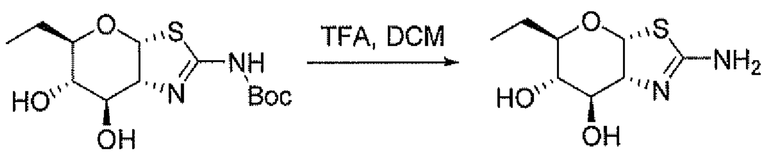


**153** **Example 178**

**(3aR,5R,6S,7R,7aR)-2-amino-5-ethyl-5,6,7,7a-tetrahydro-3aH-pyrano[3,2-d]thiazole-6,7-diol:** To a solution of **153** (70 mg, 0.2 mmol) in dichloromethane (10 mL) was added TFA (1 mL). After 2 hours at room temperature, removal of volatiles gave a residue, which was dissolved into methanol (3 mL) and neutralized with concentrated $NH_4OH$ (1 mL). After concentrated under reduced pressure, the crude product was purified by flash column chromatography with 5% - 30% methanol in dichloromethane to give (3aR,5R,6S,7R,7aR)-2-amino-5-ethyl-5,6,7,7a-tetrahydro-3aH-pyrano[3,2-d]thiazole-6,7-diol as a white solid (33 mg, 69 %). (ES, *m/z*): $[M+H]^+$ 219.0; $^1H$ NMR (300 MHz, $D_2O$) δ 6.18 (d, *J* = 6.6 Hz, 1H), 4.08 (t, *J* = 5.7 Hz, 1H), 3.89 (t, *J* = 4.5 Hz, 1H), 3.42 - 3.34 (m, 2H), 1.71 - 1.63 (m, 1H), 1.45 - 1.35 (m, 1H),

0.81 (t, *J* = 7.5 Hz, 3H).

**Examples 179 and 180**

**(3aR,5S,6S,7R,7aR)-2-(dimethylamino)-5-((R)-1-fluoroethyl)-5,6,7,7a-tetrahydro-3aH-pyrano[3,2-d]thiazole-6,7-diol & (3aR,5S,6S,7R,7aR)-2-(dimethylamino)-5-((S)-1-fluoroethyl)-5,6,7,7a-tetrahydro-3aH-pyrano[3,2-d]thiazole-6,7-diol :**

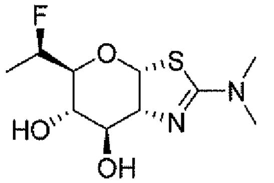

&

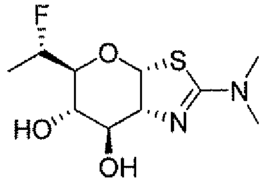

Scheme XXXI

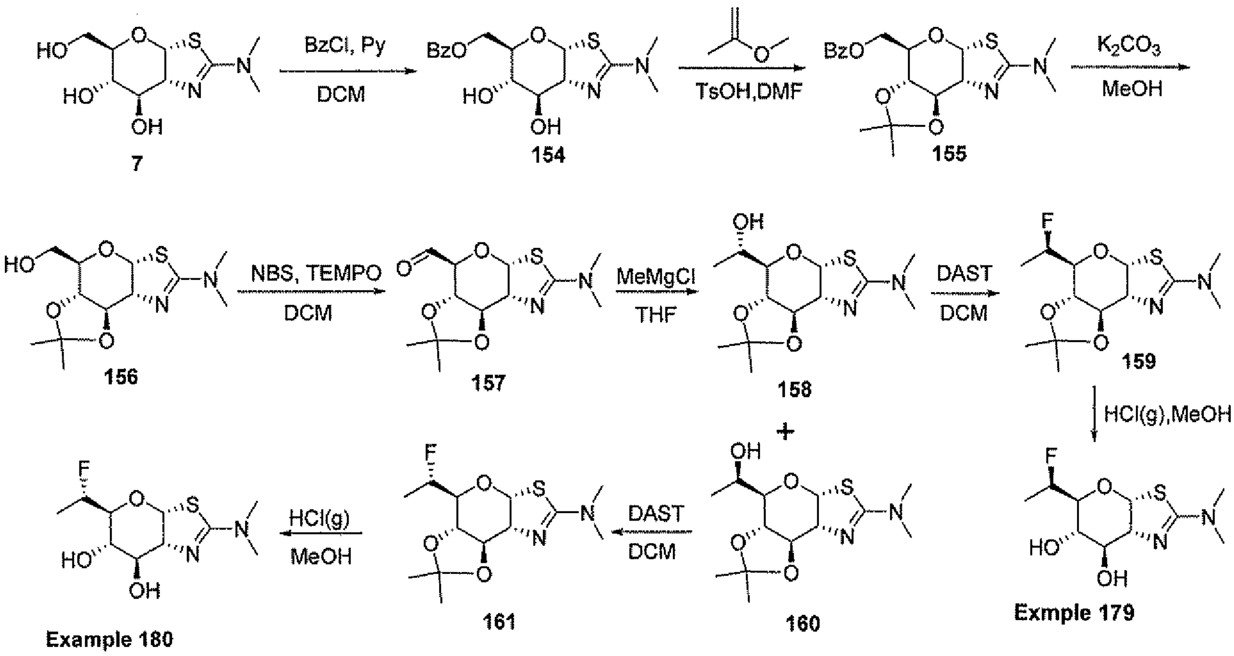


**Step 1**

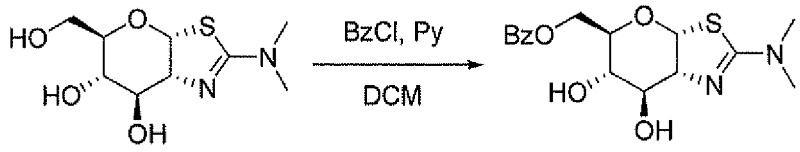


**154**

**((3aR,5R,6S,7R,7aR)-2-(dimethylamino)-6,7-dihydroxy-5,6,7,7a-tetrahydro-3aH-pyrano[3,2-d]thiazol-5-yl)methyl benzoate (154):** To a solution of (3aR,5R,6S,7R,7aR)-2-(dimethylamino)-5-(hydroxymethyl)-5,6,7,7a-tetrahydro-

3aH-pyrano[3,2-d]thiazole-6,7-diol (5 g, 20 mmol) in pyridine (150 mL) was added a solution of benzoyl chloride (3.4 g, 24 mmol) in dichloromethane (10 mL) at 0 °C within 10 minutes, and then stirred for 5 hours at 0 °C in an ice/salt bath. The reaction was quenched by the addition of saturated aqueous $NaHCO_3$ solution (100 mL), and extracted with dichloromethane (3x100 mL). The combined organic layer was washed with brine (2x20 mL), dried over sodium sulfate, filtered and concentrated under vacuum. The crude residue was purified by flash column chromatography with 2 % - 5 % methanol in dichloromethane to afford the title compound as an off-white solid (4.5 g, 63%). (ES, *m/z*): $[M+H]^+$ 353.0; $^1H$ NMR (300 MHz, $CD_3OD$) $\delta$ 8.07 - 8.01 (m, 2H), 7.99 - 7.40 (m, 3H), 6.43 (d, *J* = 6.6 Hz, 1H), 4.88 - 4.85 (m, 1H), 4.63 - 4.45 (m, 1H), 4.19 - 4.15 (m, 1H), 3.99 - 3.93 (m, 2H), 3.36 - 3.32 (m, 1H), 3.06 (s, 6H).

**Step 2**

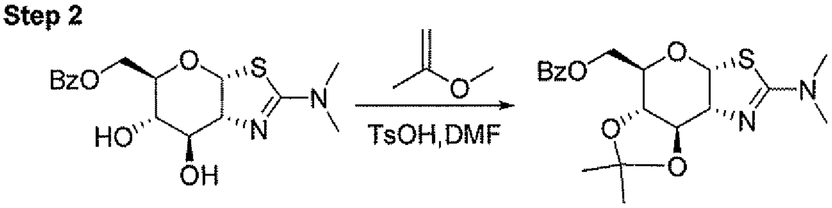


154 155

**[(1R,2R,6R,8R,9S)-4-(dimethylamino)-11,11-dimethyl-7,10,12-trioxa-5-thia-3-azatricyclo[7.3.0.0{2,6}] dodec-3-en-8-yl]methyl benzoate (155):** To a solution of **154** (3.0 g, 8.5 mmol) in DMF (20 mL) was added 4-methylbenzenesulfonic acid hydrate (3.2 g, 17 mmol) and 2-methoxyprop-1-ene (6.1 g, 85 mmol) sequentially. The resulting solution was stirred for 15 minutes at room temperature, before the reaction was quenched by the addition of saturated aqueous sodium bicarbonate solution (20 mL), which was then extracted with dichloromethane (3x20 mL). The combined organic layer was washed with brine (2x10 mL), dried over sodium sulfate, filtered and concentrated under vacuum. The crude residue was purified by flash column chromatography with 1 % - 10 % ethyl acetate in petroleum ether to afford **155** as an off-white solid (1.6 g, 48%). (ES, *m/z*): $[M+H]^+$ 393.0; $^1H$ NMR (300 MHz, $CDCl_3$) $\delta$ 8.08 - 8.05 (m, 2H), 7.63 - 7.45 (m, 3H), 6.32 - 6.30 (m, 1H), 4.60 - 4.52 (m, 2H), 4.41 - 4.31 (m, 2H), 3.97 - 3.91 (m, 1H), 3.70 - 3.63 (m, 1H), 3.55 - 3.50 (m, 1H), 3.39 - 3.30 (m, 1H), 3.10 (s, 6H), 1.50 (s, 6H).

**Step 3**

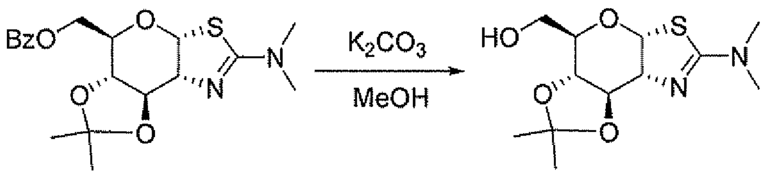


155 156

**[(1R,2R,6R,8R,9S)-4-(dimethylamino)-11,11-dimethyl-7,10,12-trioxa-5-thia-3-azatricyclo[7.3.0.0{2,6}] dodec-3-en-8-yl]methanol (156):** To a solution of **155** (4.0 g, 10 mmol) in methanol (30 mL) was added potassium carbonate (704 mg, 5.1 mmol). The resulting solution was stirred for 2 hours at room temperature, before the reaction was quenched by the addition of water (20 mL), which was then extracted with dichloromethane (3x20 mL). The combined organic layer was washed with brine (2x10 mL), dried over sodium sulfate, filtered and concentrated under vacuum. The crude residue was purified by flash column chromatography with 1 % -5 % methanol in dichloromethane to afford the title compound as a white solid (2.6 g, 88%). (ES, *m/z*): $[M+H]^+$ 289.0; $^1H$ NMR (300 MHz, $CDCl_3$) $\delta$ 6.34 (d, *J* = 6.0 Hz, 1H), 4.37 - 4.32 (m, 1H), 4.11 - 4.05 (m, 1H), 3.94 - 3.82 (m, 2H), 3.61 - 3.54 (m, 1H), 3.36 - 3.23 (m, 1H), 3.08 (s, 6H), 1.46 (s, 6H).

**Step4**

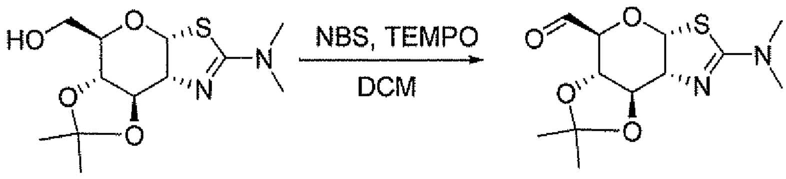


156 157

**(1R,2R,6R,8S,9S)-4-(dimethylamino)-11,11-dimethyl-7,10,12-trioxa-5-thia-3-azatricyclo[7.3.0.0{2,6}] dodec-3-ene-8-carbaldehyde (157):** To a solution of **156** (500 mg, 1.7 mmol) in dichloromethane (20 mL) and water (5 mL) was added 2,2,6,6-tetramethylpiperidinooxy (13 mg, 0.08 mmol), tetrabutylammonium bromide (27 mg, 0.08 mmol), potassium bicarbonate (788 mg, 7.9 mmol) and *N*-bromosuccinimide (342 mg, 1.9 mmol). The resulting solution was stirred for 30 minutes at room temperature, before the reaction was quenched by the addition of saturated aqueous sodium sulfite solution (10 mL), which was then extracted with dichloromethane (2x10 mL). The combined organic layer was washed with brine (2x10 mL), dried over sodium sulfate, filtered and concentrated under vacuum. The crude residue was purified by flash column chromatography with 10 % ethyl acetate in dichloromethane to afford **157** as an off-white solid (300 mg, 54%). (ES, *m/z*): $[M+H]^+$ 287.0; $^1$H NMR (300 MHz, $CDCl_3$) $\delta$ 9.82 (s, 1H), 6.34 (d, *J* = 6.0 Hz, 1H), 4.37 - 4.32 (m, 1H), 4.11 - 4.05 (m, 1H), 3.94 - 3.82 (m, 1H), 3.61 - 3.54 (m, 1H), 3.36 - 3.23 (m, 1H), 3.08 (s, 6H), 1.46 (s, 6H).

**Step 5**

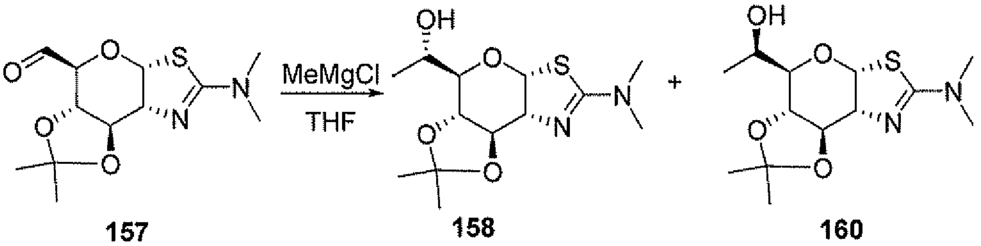


157 158 160

**(1S)-1-[(1R,2R,6R,8R,9S)-4-(dimethylamino)-11,11-dimethyl-7,10,12-trioxa-5-thia-3-azatricyclo [7.3.0.0{2,6}]dodec-3-en-8-yl]ethan-1-ol (5) & (1R)-1-[(1R,2R,6R,8R,9S)-4-(dimethylamino)-11,11-dimethyl-7,10,12-trioxa-5-thia-3-azatricyclo[7.3.0.0{2,6}]dodec-3-en-8-yl]ethan-1-ol (159):** To a stirred solution of **157** (1.0 g, 3.1 mmol) in THF (30 mL) was added MeMgCl (4.6 mL, 4.6 mmol, 1M solution in THF) at 0 °C. The resulting solution was stirred for 2 hours at room temperature, before the reaction was quenched by the addition of 10 % aqueous $NH_4Cl$ solution (10 mL), which was then extracted with ethyl acetate (3x20 mL). The combined organic layer was washed with brine (2x10 mL), dried over sodium sulfate, filtered and concentrated under vacuum. The crude residue was purified by chiral preparative HPLC with the following condition: (Chiralpak IC(SFC)2*25cm, 5umChiral-P(IC)002S09ICOOCJ-MI001, 220 nm, Mobile Phase: ethanol/hexane=1/1, run 14 minutes) to afford **158** as an off-white solid (230 mg, 23%, slower eluting isomer). (ES, *m/z*): $[M+H]^+$ 303.0; $^1$H NMR (300 MHz, $CDCl_3$) $\delta$ 6.16 (d, *J* = 5.7 Hz, 1H), 4.33 - 4.28 (m, 1H), 3.97 - 3.82 (m, 2H), 3.76 - 3.69 (m, 1H), 3.61 - 3.55 (m, 1H), 3.02 (s, 6H), 2.38 (s, 1H), 1.47 (s, 6H), 1.28 (d, *J* = 3.6 Hz, 3H); and **160** as an off-white solid (270 mg, 30%, faster eluting isomer). (ES, *m/z*): $[M+H]^+$ 303.0; $^1$H NMR (300 MHz, $CDCl_3$) $\delta$ 6.32 (d, *J* = 6.0 Hz, 1H), 4.30 - 4.26 (m, 1H), 4.06 - 4.01 (m, 1H), 3.88 - 3.73 (m, 2H), 3.71 - 3.63 (m, 1H), 3.02 (s, 6H), 2.31 (s, 1H), 1.47 (s, 6H), 1.30 - 1.22 (m, 3H).

**Step 6**

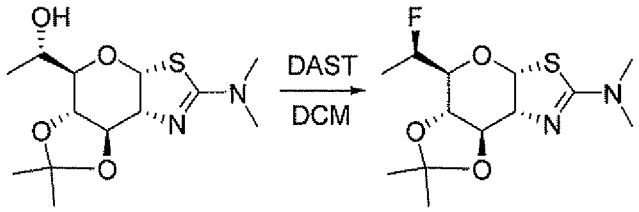


158 159

**(1R,2R,6R,8S,9S)-8-[(1R)-1-fluoroethyl]-N,N,11,11-tetramethyl-7,10,12-trioxa-5-thia-3-azatricyclo [7.3.0.0{2,6}]dodec-3-en-4-amine (159):** To a solution of **158** (230 mg, 1 mmol) in dichloromethane (10 mL) was treated with DAST (0.6 mL, 5.0 mmol) for 1 hour at -20 °C and additional 1 hour at room temperature. The reaction was quenched with saturated aqueous sodium bicarbonate solution (10 mL), which was then extracted with dichloromethane (2x10 mL). The combined organic layer was washed with brine (2x5 mL), dried over sodium sulfate, filtered and concentrated under vacuum. The crude residue was purified by flash column chromatography with 1 % - 5 % methanol in dichloromethane to afford the title compound as a white solid (160 mg, 34%). (ES, *m/z*): $[M+H]^+$ 305.0; $^1H$ NMR (300 MHz, $CDCl_3$) δ 6.16 (d, *J* = 5.7 Hz, 1H), 5.04 - 4.85 (m, 1H), 4.37 - 4.33 (m, 1H), 3.93 - 3.81 (m, 3H), 3.03 (s, 6H), 1.49 (s, 6H), 1.41 (dd, *J* = 6.6 Hz, 24.3 Hz, 3H).

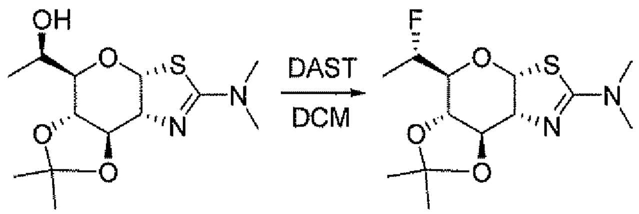


160 161

**(1R,2R,6R,8S,9S)-8-[(1S)-1-fluoroethyl]-N,N,11,11-tetramethyl-7,10,12-trioxa-5-thia-3-azatricyclo [7.3.0.0{2,6}]dodec-3-en-4-amine (161):** *Using the same procedure as Step 6*, **160** was converted to the title compound. Compound. **161** (160 mg, 30%) was obtained as white solid. (ES, *m/z*): $[M+H]^+$ 305.0; $^1H$ NMR (300 MHz, $CDCl_3$)δ 6.42 (d, *J* = 6,6 Hz, 1H), 4.95 - 4.76 (m, 1H), 4.62 - 4.56 (m, 1H), 4.11 - 4.00 (m, 1H), 3.89 - 3.85 (m, 1H), 3.15 (s, 6H), 1.49 (s, 6H), 1.43 - 1.41 (m, 3H).

**Step 7**

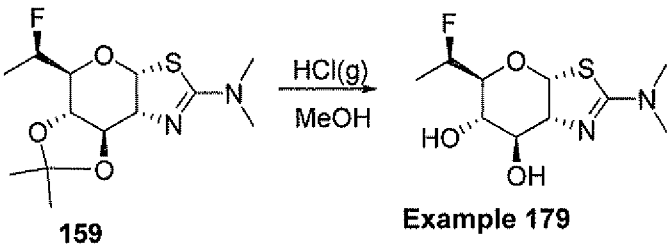


159 Example 179

**(3aR,5S,6S,7R,7aR)-2-(dimethylamino)-5-((R)-1-fluoroethyl)-5,6,7,7a-tetrahydro-3aH-pyrano[3,2-d]thiazole-6,7-diol:** A solution **of 159** (150 mg, 0.16 mmol) in methanol (10 mL) was treated with concentrated aqueous HCl (1 mL) for 1 hour at room temperature, then the resulting solution was concentrated under vacuum to give a residue, which was dissolved into methanol (5 mL) and neutralized with concentrated $NH_4OH$ (1 mL). After removal of all the solvent, the residue was purified by flash column chromatography with 10 % methanol in dichloromethane to afford the title compound as a white solid (50.1 mg, 38%). (ES, *m/z*): $[M+H]^+$ 265.0; $^1H$ NMR (300 MHz, $D_2O$) δ 6.23 (d, *J* = 6.6 Hz, 1H), 4.97 - 4.70 (m, 1H), 4.22 - 4.19 (m, 1H), 4.02 - 3.99 (m, 1H), 3.67 - 3.62 (m, 2H), 2.91 (s, 6H), 1.26 (dd, *J* = 6.6 Hz, 26.1 Hz, 3H).

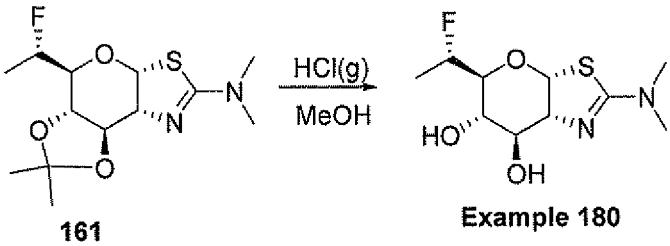


161

Example 180

**(3aR,5S,6S,7R,7aR)-2-(dimethylamino)-5-((S)-1-fluoroethyl)-5,6,7,7a-tetrahydro-3aH-pyrano[3,2-d]thiazole-6,7-diol Example 180.** Compound **161** was converted to the title compound *using the same procedure as described above in Step* 7. The title compound was obtained as a white solid (30 mg, 23%). (ES, *m/z*): $[M+H]^+$ 265.0; $^1H$ NMR (300 MHz, $D_2O$) $\delta$ 6.22 (d, *J* = 6.6 Hz, 1H), 4.96 - 4.73 (m, 1H), 4.15 - 4.11 (m, 1H), 3.97 - 3.93 (m, 1H), 3.69 - 3.65 (m, 1H), 3.46 - 3.33 (m, 1H), 2.90 (s, 6H), 1.26 (dd, *J* = 6.6 Hz, 26.1 Hz, 3H).

| Example | Structure | Name | MH+ |
|---|---|---|---|
| 181 | 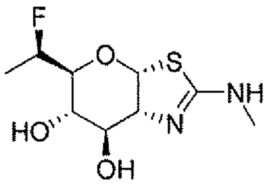 | (3aR,5S,6S,7R,7aR)-5-((R)-1-fluoroethyl)-2-(methylamino)-5,6,7,7a-tetrahydro-3aH-pyrano[3,2-d]thiazole-6,7-diol (Faster eluting isomer by HPLC) | 251.0 |
| 182 | 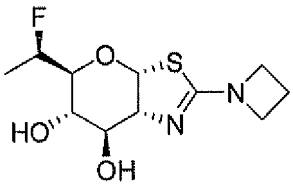 | (3aR,5S,6S,7R,7aR)-2-(azetidin-1-yl)-5-((R)-1-fluoroethyl)-5,6,7,7a-tetrahydro-3aH-pyrano[3,2-d]thiazole-6,7-diol (Faster eluting isomer by HPLC) | 276.9 |
| 183 | 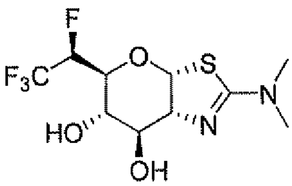 | (3aR,5S,6S,7R,7aR)-2-(dimethylamino)-5-((S)-1,2,2,2-tetrafluoroethyl)-5,6,7,7a-tetrahydro-3aH-pyrano[3,2-d]thiazole-6,7-diol (Faster eluting isomer by HPLC) | 318.9 |
| 184 | 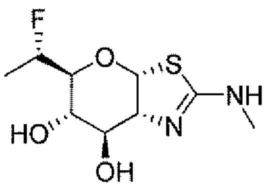 | (3aR,5S,6S,7R,7aR)-5-((S)-1-fluoroethyl)-2-(methylamino)-5,6,7,7a-tetrahydro-3aH-pyrano[3,2-d]thiazole-6,7-diol (Slower eluting isomer by HPLC) | 251.0 |
| 185 | 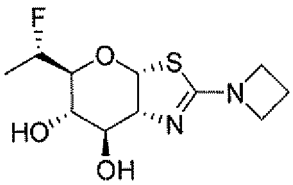 | (3aR,5S,6S,7R,7aR)-2-(azetidin-1-yl)-5-((S)-1-fluoroethyl)-5,6,7,7a-tetrahydro-3aH-pyrano[3,2-d]thiazole-6,7-diol (Slower eluting isomer by HPLC) | 276.9 |
| 186 | 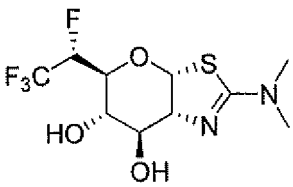 | (3aR,5S,6S,7R,7aR)-2-(dimethylamino)-5-((R)-1,2,2,2-tetrafluoroethyl)-5,6,7,7a-tetrahydro-3aH-pyrano[3,2-d]thiazole-6,7-diol (Slower eluting isomer by HPLC) | 318.9 |

(continued)

| Example | Structure | Name | MH+ |
|---|---|---|---|
| 187 | 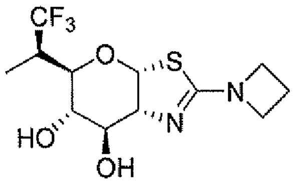 | (3aR,5R,6S,7R,7aR)-2-(azetidin-1-yl)-5-((R)-1,1,1-trifluoropropan-2-yl)-5,6,7,7a-tetrahydro-3aH-pyrano[3,2-d]thiazole-6,7-diol (Faster eluting isomer by HPLC) | 327.1 |
| 188 | 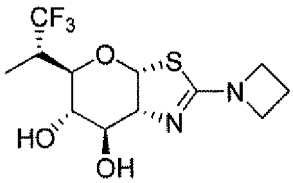 | (3aR,5R,6S,7R,7aR)-2-(azetidin-1-yl)-5-((S)-1,1,1-trifluoropropan-2-yl)-5,6,7,7a-tetrahydro-3aH-pyrano[3,2-d]thiazole-6,7-diol (Slower eluting isomer by HPLC) | 327.1 |
| 189 | 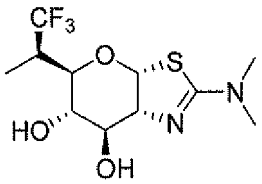 | (3aR,5R,6S,7R,7aR)-2-(dimethylamino)-5-((R)-1,1,1-trifluoropropan-2-yl)-5,6,7,7a-tetrahydro-3aH-pyrano[3,2-d]thiazole-6,7-diol (Faster eluting isomer by HPLC) | 314.9 |
| 190 | 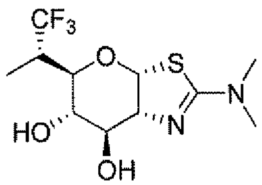 | (3aR,5R,6S,7R,7aR)-2-(dimethylamino)-5-((S)-1,1,1-trifluoropropan-2-yl)-5,6,7,7a-tetrahydro-3aH-pyrano[3,2-d]thiazole-6,7-diol (Slower eluting isomer by HPLC) | 314.9 |
| 191 | 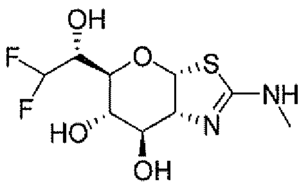 | (3aR,5S,6S,7R,7aR)-5-((R)-2,2-difluoro-1-hydroxyethyl)-2-(methylamino)-5,6,7,7a-tetrahydro-3aH-pyrano[3,2-d]thiazole-6,7-diol | 285.0 |
| 192 | 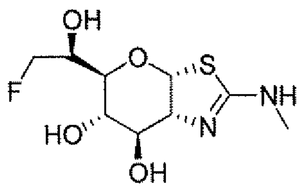 | (3aR,5S,6S,7R,7aR)-5-((S)-2-fluoro-1-hydroxyethyl)-2-(methylamino)-5,6,7,7a-tetrahydro-3aH-pyrano[3,2-d]thiazole-6,7-diol (Faster eluting isomer by HPLC) | 267.0 |
| 193 | 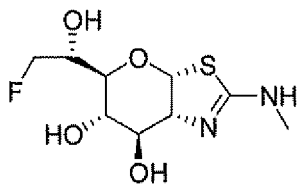 | (3aR,5S,6S,7R,7aR)-5-((R)-2-fluoro-1-hydroxyethyl)-2-(methylamino)-5,6,7,7a-tetrahydro-3aH-pyrano[3,2-d]thiazole-6,7-diol (Slower eluting isomer by HPLC) | 267.0 |
| 194 | 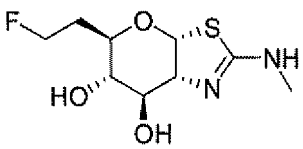 | (3aR,5R,6S,7R,7aR)-5-(2-fluoroethyl)-2-(methylamino)-5,6,7,7a-tetrahydro-3aH-pyrano[3,2-d]thiazole-6,7-diol | 251.0 |

(continued)

| Example | Structure | Name | MH+ |
|---|---|---|---|
| 195 | 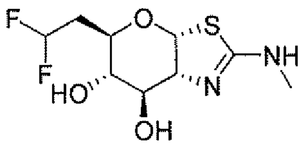 | (3aR,5R,6S,7R,7aR)-5-(2,2-difluoroethyl)-2-(methylamino)-5,6,7,7a-tetrahydro-3aH-pyrano[3,2-d]thiazole-6,7-diol | 269.0 |
| 196 | 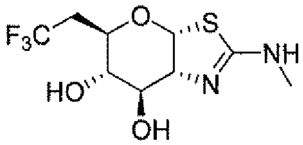 | (3aR,5R,6S,7R,7aR)-2-(methylamino)-5-(2,2,2-trifluoroethyl)-5,6,7,7a-tetrahydro-3aH-pyrano[3,2-d]thiazole-6,7-diol | 287.0 |
| 197 | 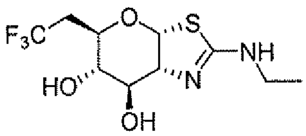 | (3aR,5R,6S,7R,7aR)-2-(ethylamino)-5-(2,2,2-trifluoroethyl)-5,6,7,7a-tetrahydro-3aH-pyrano[3,2-d]thiazole-6,7-diol | 301.0 |
| 198 | 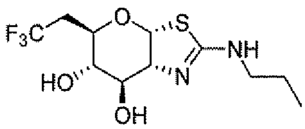 | (3aR,5R,6S,7R,7aR)-2-(propylamino)-5-(2,2,2-trifluoroethyl)-5,6,7,7a-tetrahydro-3aH-pyrano[3,2-d]thiazole-6,7-diol | 315.0 |
| 199 | 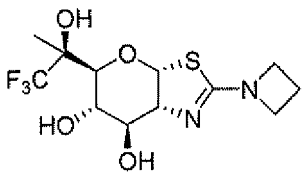 | (3aR,5S,6S,7R,7aR)-2-(azetidin-1-yl)-5-((S)-1,1,1-trifluoro-2-hydroxypropan-2-yl)-5,6,7,7a-tetrahydro-3aH-pyrano[3,2-d]thiazole-6,7-diol (Faster eluting isomer by HPLC) | 343.0 |
| 200 | 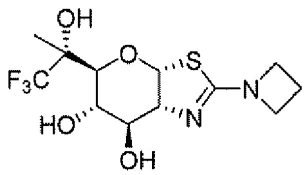 | (3aR,5S,6S,7R,7aR)-2-(azetidin-1-yl)-5-((R)1,1,1-trifluoro-2-hydroxypropan-2-yl)-5,6,7,7a-tetrahydro-3aH-pyrano[3,2-d]thiazole-6,7-diol (Slower eluting isomer by HPLC) | 343.0 |
| 201 | 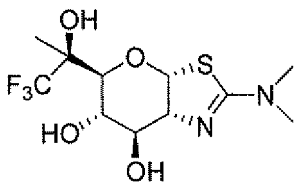 | (3aR,5S,6S,7R,7aR)-2-(dimethylamino)-5-((S)-1,1,1-trifluoro-2-hydroxypropan-2-yl)-5,6,7,7a-tetrahydro-3aH-pyrano[3,2-d]thiazole-6,7-diol (Faster eluting isomer by HPLC) | 330.9 |
| 202 | 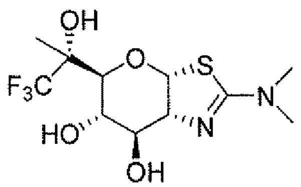 | (3aR,5S,6S,7R,7aR)-2-(dimethylamino)-5-((R) 1,1,1-trifluoro-2-hydroxypropan-2-yl)-5,6,7,7a-tetrahydro-3aH-pyrano[3,2-d]thiazole-6,7-diol (Slower eluting isomer by HPLC) | 331.0 |
| 203 | 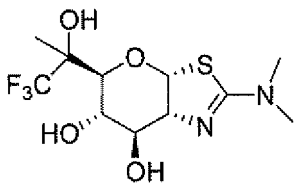 | (3aR,5S,6S,7R,7aR)-2-(dimethylamino)-5-((S)-1,1,1-trifluoro-2-hydroxypropan-2-yl)-5,6,7,7a-tetrahydro-3aH-pyrano[3,2-d]thiazole-6,7-diol (two epimers ratio 1:1) | 330.9 |

(continued)

| Example | Structure | Name | MH+ |
|---|---|---|---|
| 204 | 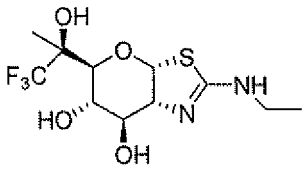 | (3aR,5S,6S,7R,7aR)-2-(ethylamino)-5-((S)-1,1,1-trifluoro-2-hydroxypropan-2-yl)-5,6,7,7a-tetrahydro-3 aH-pyrano [3,2-d]thiazole-6,7-diol (Faster eluting isomer by HPLC) | 331.0 |
| 205 | 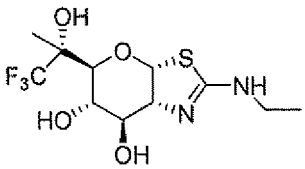 | (3aR,5S,6S,7R,7aR)-2-(ethylamino)-5-((R) 1,1,1-trifluoro-2-hydroxypropan-2-yl)-5,6,7,7a-tetrahydro-3aH-pyrano[3,2-d]thiazole-6,7-diol (Slower eluting isomer by HPLC) | 331.0 |
| 206 | 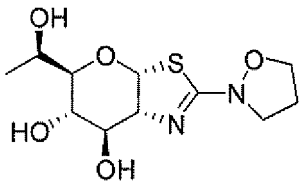 | (3aR,5R,6S,7R,7aR)-5-((R)-1-hydroxyethyl)-2-(isoxazolidin-2-yl)-5,6,7,7a-tetrahydro-3aH-pyrano[3,2-d]thiazole-6,7-diol(Faster eluting isomer by HPLC) | 291.0 |
| 207 | 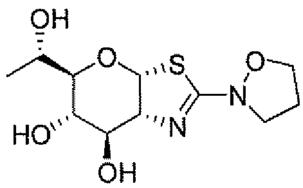 | (3aR,5R,6S,7R,7aR)-5-((S)-1-hydroxyethyl)-2-(isoxazolidin-2-yl)-5,6,7,7a-tetrahydro-3aH-pyrano[3,2-d]thiazole-6,7-diol (Slower eluting isomer by HPLC) | 291.0 |
| 208 | 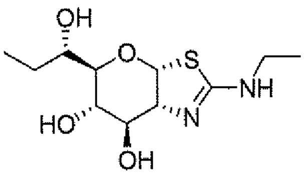 | (3aR,5R,6S,7R,7aR)-2-(ethylamino)-5-(S)-1-hydroxypropyl)-5,6,7,7a-tetrahydro-3aH-pyrano[3,2-d]thiazole-6,7-diol (Slower eluting isomer by HPLC) | 277.1 |
| 209 | 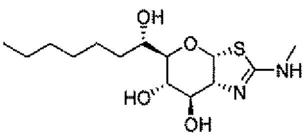 | (3aR,5R,6S,7R,7aR)-5-((S)-1-hydroxyheptyl)-2-(methylamino)-5,6,7,7a-tetrahydro-3aH-pyrano[3,2-d]thiazole-6,7-diol (Slower eluting isomer by HPLC) | 319.0 |
| 210 | 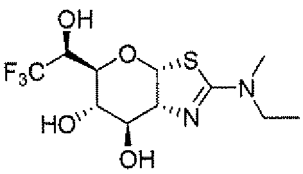 | (3aR,5S,6S,7R,7aR)-2-(ethyl(methyl)amino)-5-((S)-2,2,2-trifluoro-1-hydroxyethyl)-5,6,7,7a-tetrahydro-3aH-pyrano[3,2-d]thiazole-6,7-diol (Faster eluting isomer by HPLC) | 331.0 |
| 211 | 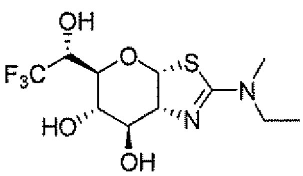 | (3aR,5S,6S,7R,7aR)-2-(ethyl(methyl)amino)-5-((R)-2,2,2-trifluoro-1-hydroxyethyl)-5,6,7,7a-tetrahydro-3aH-pyrano[3,2-d]thiazole-6,7-diol (Slower eluting isomer by HPLC) | 330.9 |

(continued)

| Example | Structure | Name | MH+ |
|---|---|---|---|
| 212 | 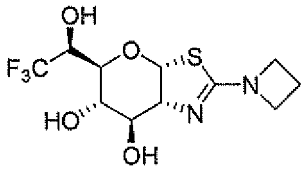 | (3aR,5S,6S,7R,7aR)-2-(azetidin-1-yl)-5-((S)-2,2,2-trifluoro-1-hydroxyethyl)-5,6,7,7a-tetrahydro-3aH-pyrano[3,2-d]thiazole-6,7-diol (Faster eluting isomer by HPLC) | 329.0 |
| 213 | 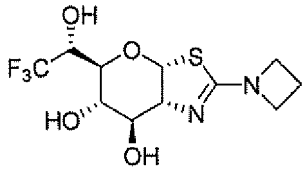 | (3aR,5S,6S,7R,7aR)-2-(azetidin-1-yl)-5-((R)-2,2,2-trifluoro-1-hydroxyethyl)-5,6,7,7a-tetrahydro-3aH-pyrano[3,2-d]thiazole-6,7-diol (Slower eluting isomer by HPLC) | 329.0 |
| 214 | 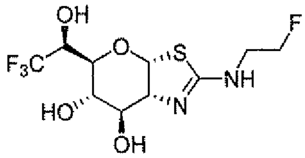 | (3aR,5S,6S,7R,7aR)-2-(2-fluoroethylamino)-5-((R)-2,2,2-trifluoro-1-hydroxyethyl)-5,6,7,7a-tetrahydro-3aH-pyrano[3,2-d]thiazole-6,7-diol (Faster eluting isomer by HPLC) | 335.0 |
| 215 | 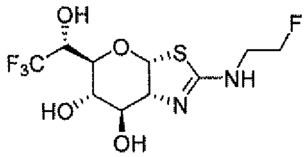 | (3aR,5S,6S,7R,7aR)-2-(2-fluoroethylamino)-5-((S)-2,2,2-trifluoro-1-hydroxyethyl)-5,6,7,7a-tetrahydro-3aH-pyrano[3,2-d]thiazole-6,7-diol | 335.0 |
| 216 | 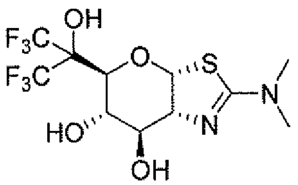 | (3aR,5S,6S,7R,7aR)-2-(dimethylamino)-5-(1,1,1,3,3,3-hexafluoro-2-hydroxypropan-2-yl)-5,6,7,7a-tetrahydro-3aH-pyrano[3,2-d]thiazole-6,7-diol | 384.9 |
| 217 | 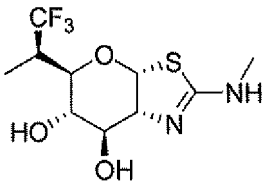 | (3aR,5R,6S,7R,7aR)-2-(methylamino)-5-((R)-1,1,1-trifluoropropan-2-yl)-5,6,7,7a-tetrahydro-3aH-pyrano[3,2-d]thiazole-6,7-diol (Faster eluting isomer by HPLC) | 301.2 |
| 218 | 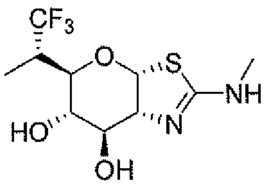 | (3aR,5R,6S,7R,7aR)-2-(methylamino)-5-((S)-1,1,1-trifluoroprapan-2-yl)-5,6,7,7a-tetrahydro-3aH-pyrano[3,2-d]thiazole-6,7-diol (Slower eluting isomer by HPLC) | 301.2 |

Biological Activity

Assay for determination of $K_I$ values for inhibition of O-GlcNAcase activity

Experimental procedure for kinetic analyses

Enzymatic reactions are carried out in a reaction containing 50mM $NaH_2PO_4$, 100 mM NaCl and 0.1% BSA (pH 7.0) using 2 mM 4-Methylumbelliferyl N-acetyl-β-D-glucosaminide dihydrate (Sigma M2133) dissolved in $ddH_2O$,

as a substrate. The amount of purified human O-GlcNAcase enzyme used in the reaction is 0.7 nM. Test compound of varying concentrations is added to the enzyme prior to initiation of the reaction. The reaction is performed at room temperature in a 96-well plate and is initiated with the addition of substrate. The production of fluorescent product is measured every 60 sec for 45 min with a Tecan Infinite M200 plate-reader with excitation at 355nM and emission detected at 460nM, with 4-Methylumbelliferone (Sigma M1381) used to produce a standard curve. The slope of product production is determined for each concentration of compound tested and plotted, using standard curve fitting algorithms for sigmoidal dose response curves. The values for a four parameter logistic curve fit of the data are determined.

Ki values are determined using the Cheng-Prusoff equation; the Km of O-GlcNacase for substrate is 0.2 mM.

Examples 1 to 218 were tested in the above described assay and exhibited $K_I$ values for inhibition of O-GlcNAcase in the range 0.1 nM - 10 μM.

Assay for determination of $K_I$ values for inhibition of β-hexosaminidase activity

Experimental procedure for kinetic analyses

Enzymatic reactions are carried out in a reaction containing 50mM $NaH_2PO_4$, 100 mM NaCl and 0.1% BSA (pH 7.0) using 2 mM 4-Methylumbelliferyl N-acctyl-β-D-glucosaminide dihydrate (Sigma M2133) dissolved in ddH2O, as a substrate. The amount of purified human β hexosaminidase enzyme used in the reaction is 24 nM. Test compound of varying concentrations is added to the enzyme prior to initiation of the reaction. The reaction is performed at room temperature in a 96-well plate and is initiated with the addition of substrate. The production of fluorescent product is measured every 60 sec for 45 min with a Tecan Infinite M200 plate-reader with excitation at 355nM and emission detected at 460nM, with 4-Methylumbelliferone (Sigma M1381) used to produce a standard curve. The slope of product production is determined for each concentration of compound tested and plotted, using standard curve fitting algorithms for sigmoidal dose response curves. The values for a four parameter logistic curve fit of the data are determined.

Ki values are determined using the Cheng-Prusoff equation..

When tested in this assay, many of the compounds described herein exhibit $K_I$ values for inhibition of β-hexosaminidase in the range 10 nM to greater than 100 μM.

The selectivity ratio for inhibition of O-GlcNAcase over β-hexosaminidase is defined here as:

$$K_I\ (\beta\text{-hexosaminidase})/K_I\ (\text{O-GlcNAcase})$$

In general, the compounds described herein exhibit a selectivity ratio in the range of about 10 to 100000. Thus, many compounds of the invention exhibit high selectivity for inhibition of O-GlcNAcase over β-hexosaminidase.

Assay for determination of cellular activity for compounds that inhibit O-GlcNAcase activity

Inhibition of O-GlcNAcase, which removes O-GlcNAc from cellular proteins, results in an increase in the level of O-GlcNAcylated protein in cells. An increase in O-GlcNAcylated protein can be measured by an antibody, such as RL-2, that binds to O-GlcNAcylated protein. The amount of O-GlcNAcylated protein:RL2 antibody interaction can be measured by enzyme linked immunosorbant assay (ELISA) procedures.

A variety of tissue culture cell lines, expressing endogenous levels of O-GlcNAcase, can be utilized; examples include rat PC-12, and human U-87, or SK-N-SH cells. Cells are plated in 96-well plates with approximately 10,000 cells / well. Compounds to be tested are dissolved in DMSO, either 2 or 10 mM stock solution, and then diluted with DMSO and water in a two-step process using a Tecan workstation. Cells are treated with diluted compounds for 24 hours (5.4 μL into 200 μL 1 well volume) to reach a final concentration of inhibitor desired to measure a compound concentration dependent response, typically, ten 3 fold dilution steps, starting at 10 μM are used to determine a concentration response curve. To prepare a cell lysate, the media from compound treated cells is removed, the cells are washed once with phosphate buffered saline (PBS) and then lysed for 5 minutes at room temperature in 50 μL of Phosphosafe reagent (Novagen Inc, Madison, WI) with protease inhibitors and PMSF. The cell lysate is collected and transferred to a new plate, which is then either coated to assay plates directly or frozen - 80°C until used in the ELISA procedure. If desired, the total protein concentration of samples is determined using 20 μL of the sample using the BCA method.

The ELISA portion of the assay is performed in a black Maxisorp 96-well plate that is coated overnight at 4°C with 100 μL /well of the cell lysate (1:10 dilution of the lysate with PBS containing protease inhibitors, phosphatase inhibitors, and PMSF. The following day the wells are washed 3 times with 300 μL /well of Wash buffer (Tris-buffered saline with 0.1% Tween 20). The wells are blocked with 100 μL /well Blocking buffer (Tris buffered saline w/0.05% Tween 20 and 2.5% Bovine serum albumin). Each well is then washed two times with 300 ul/well of wash buffer. The anti O-GlcNAc antibody RL-2 (Abcam, Cambridge, MA), diluted 1:1000 in blocking buffer, is added at 100 ul/well. The plate is sealed and incubated at 37°C for 2 hr with gentle shaking. The wells are then washed 3-times with 300 ul/well wash buffer. To detect the amount of RL-2 bound horseradish peroxidase (HRP) conjugated goat anti-mouse secondary antibody (diluted 1:3000 in blocking buffer) is added at100 μL /well. The plate is incubated for 60 min at 37°C with gentle shaking. Each wells is then washed 3-times with 300 ul/well wash buffer. The detection reagent is added, 100 μL /well of Amplex Ultra RED reagent (prepared by adding 30 μL of 10 mM Amplex Ultra Red stock solution to 10 ml PBS with 18 μL 3% hydrogen peroxide, $H_2O_2$). The detection reaction is incubated for 15 minutes at room temperature and then read with excitation at 530 nm and emission at 590 nm.

The amount of O-GlcNAcylated protein, as detected by the ELISA assay, is plotted for each concentration of test compound using standard using standard curve fitting algorithms for sigmoidal dose response curves. The values for a four parameter logistic curve fit of the data are determined, with the inflection point of the curve being the potency value for the test compound.

Representative data from the binding and cell-based assays described above are shown in the following table. Certain compounds of the invention exhibited superior potency in one or more of these assays as compared to compounds disclosed in WO 2006/092049 and WO 2008/025170.

| Example # | Cell-based ELISA EC50 (nM) | Fluorescence-based hOGA Ki (nM) |
|---|---|---|
| **1** | 2.37 | 0.17 |
| **2** | 1.34 | 0.05 |
| **3** | 16.69 | 0.90 |
| **4** | ND | 3.1 |
| **8** | 15.1 | 0.68 |
| **11** | 0.8 | 0.3 |
| **13** | ND | 0.4 |
| **25** | 9.01 | 0.70 |
| **27** | 3.23 | 0.12 |
| **40** | 139.00 | 7.95 |
| **42** | 8.75 | 1.03 |
| **47** | 187.20 | 6.40 |
| **51** | 22.22 | 5.1 |
| **53** | 9.26 | 0.7 |
| **57** | 5.90 | 0.33 |
| **61** | 25.31 | 2.46 |
| **63** | 2.57 | 0.22 |
| **65** | 2.7 | 0.45 |
| **67** | 17.13 | 1.25 |
| **69** | 134.10 | 9.36 |
| **71** | 48.83 | 9.12 |
| **76** | 3.63 | 0.18 |
| **77** | 195.80 | 16.28 |
| **80** | 5.31 | 0.5 |
| **82** | 97.17 | 5.84 |
| **91** | 11.85 | 0.53 |
| **95** | 1.05 | 0.16 |
| **96** | 4.55 | 0.19 |

(continued)

| Example # | Cell-based ELISA EC50 (nM) | Fluorescence-based hOGA Ki (nM) |
|---|---|---|
| **97** | 2.79 | 0.17 |
| **98** | 15.28 | 1.43 |
| **104** | 17.50 | 3.63 |
| **105** | 14.74 | 3.4 |
| **141** | 62.7 | 0.82 |
| **159** | 27.6 | 1.25 |
| **181** | 11.3 | 0.8 |
| **191** | 20.7 | 0.14 |
| **203** | 38.2 | 3.16 |
| **211** | 19.5 | 0.44 |
| **215** | 67.5 | 1.8 |

## REFERENCES

1. C. R. Torres, G. W. Hart, J Biol Chem 1984, 259, 3308.
2. R. S. Haltiwanger, G. D. Holt, G. W. Hart, J Biol Chem 1990, 265, 2563.
3. L. K. Kreppel, M. A. Blomberg, G. W. Hart, J Biol Chem 1997, 272, 9308.
4. W. A. Lubas, D. W. Frank, M. Krause, J. A. Hanover, J Biol Chem 1997, 272,9316.
5. W. A. Lubas, J. A. Hanover, J Biol Chem 2000, 275, 10983.
6. D. L. Dong, G. W. Hart, J Biol Chem 1994, 269, 19321.
7. Y. Gao, L. Wells, F. I. Comer, G. J. Parker, G. W. Hart, J Biol Chem 2001, 276, 9838.
8. E. P. Roquemore, M. R. Chevrier, R. J. Cotter, G. W. Hart, Biochemistry 1996, 35, 3578.
9. S. P. Jackson, R. Tjian, Cell 1988, 55, 125.
10. W. G. Kelly, M. E. Dahmus, G. W. Hart, J Biol Chem 1993, 268, 10416.
11. M. D. Roos, K. Su, J. R. Baker, J. E. Kudlow, Mol Cell Biol 1997, 17, 6472.
12. N. Lamarre-Vincent, L. C. Hsieh-Wilson, JAm Chem Soc 2003, 125, 6612.
13. F. Zhang, K. Su, X. Yang, D. B. Bowe, A. J. Paterson, J. E. Kudlow, Cell 2003, 115, 715.
14. K. Vosseller, L. Wells, M. D. Lane, G. W. Hart, Proc Natl Acad Sci USA 2002, 99, 5313.
15. W. A. Lubas, M. Smith, C. M. Starr, J. A. Hanover, Biochemistry 1995, 34, 1686.
16. L. S. Griffith, B. Schmitz, Biochem Biophys Res Commun 1995, 213, 424.
17. R. N. Cole, G. W. Hart, J Neurochem 1999, 73, 418.
18. I. Braidman, M. Carroll, N. Dance, D. Robinson, Biochem J 1974, 143, 295.
19. R. Ueno, C. S. Yuan, Biochim Biophys Acta 1991, 1074, 79.
20. C. Toleman, A. J. Paterson, T. R. Whisenhunt, J. E. Kudlow, J Biol Chem 2004.
21. F. Liu, K. Iqbal, I. Grundke-Iqbal, G. W. Hart, C. X. Gong, Proc Natl Acad Sci U S A 2004, 101, 10804.
22. T. Y. Chou, G. W. Hart, Adv Exp Med Biol 2001, 491, 413.
23. M. Goedert, M. G. Spillantini, N. J. Cairns, R. A. Crowther, Neuron 1992, 8, 159.
24. M. Goedert, M. G. Spillantini, R. Jakes, D. Rutherford, R. A. Crowther, Neuron 1989, 3, 519.
25. E. Kopke, Y. C. Tung, S. Shaikh, A. C. Alonso, K. Iqbal, I. Grundke-Iqbal, J Biol Chem 1993, 268, 24374.
26. H. Ksiezak-Reding, W. K. Liu, S. H. Yen, Brain Res 1992, 597, 209.
27. B. Henrissat, A. Bairoch, Biochem J 1996, 316 (Pt 2), 695.
28. B. Henrissat, A. Bairoch, Biochem J 1993, 293 (Pt 3), 781.
29. C. X. Gong, F. Liu, I. Grundke-Iqbal, K. Iqbal, J Neural Transm 2005, 112, 813.
30. K. Iqbal, C. Alonso Adel, E. El-Akkad, C. X. Gong, N. Haque, S. Khatoon, I. Tsujio, I. Grundke-Iqbal, J Neural Transm Suppl 2002, 309.
31. K. Iqbal, C. Alonso Adel, E. El-Akkad, C. X. Gong, N. Haque, S. Khatoon, J. J. Pei, H. Tanimukai, I. Tsujio, et al., J Mol Neurosci 2003, 20, 425.

32. W. Noble, E. Planel, C. Zehr, V. Olm, J. Meyerson, F. Suleman, K. Gaynor, L. Wang, J. LaFrancois, et al., Proc Natl Acad Sci U S A 2005, 102, 6990.
33. S. Le Corre, H. W. Klafki, N. Plesnila, G. Hubinger, A. Obermeier, H. Sahagun, B. Monse, P. Seneci, J. Lewis, et al., Proc Natl Acad Sci U S A 2006, 103, 9673.
34. S. J. Liu, J. Y. Zhang, H. L. Li, Z. Y. Fang, Q. Wang, H. M. Deng, C. X. Gong, I. Grundke-Iqbal, K. Iqbal, et al., J Biol Chem 2004, 279, 50078.
35. G. Li, H. Yin, J. Kuret, J Biol Chem 2004, 279, 15938.
36. T. Y. Chou, G. W. Hart, C. V. Dang, J Biol Chem 1995, 270, 18961.
37. X. Cheng, G. W. Hart, J Biol Chem 2001, 276, 10570.
38. X. Cheng, R. N. Cole, J. Zaia, G. W. Hart, Biochemistry 2000, 39, 11609.
39. L. S. Griffith, B. Schmitz, Eur J Biochem 1999, 262, 824.
40. K. Kamemura, G. W. Hart, Prog Nucleic Acid Res Mol Biol 2003, 73, 107.
41. L. Wells, L. K. Kreppel, F. I. Comer, B. E. Wadzinski, G. W. Hart, J Biol Chem 2004, 279, 38466.
42. L. Bertram, D. Blacker, K. Mullin, D. Keeney, J. Jones, S. Basu, S. Yhu, M. G. McInnis, R. C. Go, et al., Science 2000, 290, 2302.
43. S. Hoyer, D. Blum-Degen, H. G. Bernstein, S. Engelsberger, J. Humrich, S. Laufer, D. Muschner, A. Thalheimer, A. Turk, et al., Journal of Neural Transmission 1998, 105, 423.
44. C. X. Gong, F. Liu, I. Grundke-Iqbal, K. Iqbal, Journal of Alzheimers Disease 2006, 9, 1.
45. W. J. Jagust, J. P. Seab, R. H. Huesman, P. E. Valk, C. A. Mathis, B. R. Reed, P. G. Coxson, T. F. Budinger, Journal of Cerebral Blood Flow and Metabolism 1991, 11, 323.
46. S. Hoyer, Experimental Gerontology 2000, 35, 1363.
47. S. Hoyer, in Frontiers in Clinical Neuroscience: Neurodegeneration and Neuroprotection, Vol. 541, 2004, pp. 135.
48. R. N. Kalaria, S. I. Harik, Journal of Neurochemistry 1989, 53, 1083.
49. I. A. Simpson, K. R. Chundu, T. Davieshill, W. G. Honer, P. Davies, Annals of Neurology 1994, 35, 546.
50. S. M. de la Monte, J. R. Wands, Journal of Alzheimers Disease 2005, 7, 45.
51. X. W. Zhu, G. Perry, M. A. Smith, journal of Alzheimers Disease 2005, 7, 81.
52. J. C. de la Torre, Neurological Research 2004, 26, 517.
53. S. Marshall, W. T. Garvey, R. R. Traxinger, Faseb J 1991, 5, 3031.
54. S. P. Iyer, Y. Akimoto, G. W. Hart, J Biol Chem 2003, 278, 5399.
55. K. Brickley, M. J. Smith, M. Beck, F. A. Stephenson, J Biol Chem 2005, 280, 14723.
56. S. Knapp, C. H. Yang, T. Haimawitz, Tetrahedron Letters 2002, 43, 7101.
57. S. P. Iyer, G. W. Hart, J Biol Chem 2003, 278, 24608.
58. M. Jinek, J. Rehwinkel, B. D. Lazarus, E. Izaurralde, J. A. Hanover, E. Conti, Nat Struct Mol Biol 2004, 11, 1001.
59. K. Kamemura, B. K. Hayes, F. I. Comer, G. W. Hart, J Biol Chem 2002, 277, 19229.
60. Y. Deng, B. Li, F. Liu, K. Iqbal, I. Grundke-Iqbal, R. Brandt, C.-X. Gong, FASEB J. 2007, fj.07.
61. L. F. Lau, J. B. Schachter, P. A. Seymour, M. A. Sanner, Curr Top Med Chem 2002, 2, 395.
62. M. P. Mazanetz, P. M. Fischer, Nature Reviews Drug Discovery 2007, 6, 464.
63. S. A. Yuzwa, M. S. Macauley, J. E. Heinonen, X. Shan, R. J. Dennis, Y. He, G. E. Whitworth, K. A. Stubbs, E. J. McEachern, et al., Nat Chem Biol 2008, 4, 483.
64. P. Bounelis, J. Liu, Y. Pang, J. C. Chatham, R. B. Marchase, Shock 2004, 21 170 Suppl. 2, 58.
65. N. Fulop, V. Champattanachal, R. B. Marchase, J. C. Chatham, Circulation Research 2005, 97, E28.
66. J. Liu, R. B. Marchase, J. C. Chatham, Faseb Journal 2006, 20, A317.
67. R. Marchase, P. Bounelis, J. Chatham, I. Chaudry, Y. Pang, *PCT Int. Appl.* WO 2006016904 2006.
68. N. Fulop, P. P. Wang, R. B. Marchase, J. C. Chatham, Journal of Molecular and Cellular Cardiology 2004, 37, 286.
69. N. Fulop, P. P. Wang, R. B. Marchase, J. C. Chatham, Faseb Journal 2005, 19, A689.
70. J. Liu, R. B. Marchase, J. C. Chatham, Journal of Molecular and Cellular Cardiology 2007, 42, 177.
71. L. G. Not, C. A. Brocks, N. Fulop, R. B. Marchase, J. C. Chatham, Faseb Journal 2006, 20, A1471.
72. S. L. Yang, L. Y. Zou, P. Bounelis, I. Chaudry, J. C. Chatham, R. B. Marchase, Shock 2006, 25, 600.
73. L. Y. Zou, S. L. Yang, P. Bounelis, I. H. Chaudry, J. C. Chatham, R. B. Marchase, Faseb Journal 2005, 19, A1224.
74. R. B. Marchase, J. Liu, L. Y. Zou, V. Champattanachai, Y. Pang, N. Fulop, P. P. Wang, S. L. Yang, P, Bounelis, et al., Circulation 2004, 110, 1099.
75. J. Liu, Y. Pang, T. Chang, P. Bounelis, J. C. Chatham, R. B. Marchase, Journal of Molecular and Cellular Cardiology 2006, 40, 303.
76. J. Liu, J. C. Chatham, R. B. Marchase, Faseb Journal 2005, 19, A691.
77. T. Nagy, V. Champattanachai, R. B. Marchase, J. C. Chatham, American journal of Physiology-Cell Physiology 2006, 290, C57.
78. N. Fulop, R. B. Marchase, J. C. Chatham, Cardiovascular Research 2007, 73, 288.
79. T. Lefebvre, C. Guinez, V. Dehennaut, O. Beseme-Dekeyser, W. Morelle, J. C. Michalski, Expert Review of

Proteomics 2005, 2, 265.
80. L. Wells, K. Vosseller, G. W. Hart, Science 2001, 291, 2376.
81. J. A. Hanover, FASEB J 2001, 15, 1865.
82. D. A. McClain, W. A. Lubas, R. C. Cooksey, M. Hazel, G. J. Parker, D. C. Love, J. A. Hanover, Proc Natl A cad Sci U S A 2002, 99, 10695.
83. P. J. Yao, P. D. Coleman, J Neurosci 1998, 18, 2399.
84. W. H. Yang, J. E. Kim, H. W. Nam, J. W. Ju, H. S. Kim, Y. S. Kim, J. W. Cho, Nature Cell Biology 2006, 8, 1074.
85. B. Triggs-Raine, D. J. Mahuran, R. A. Gravel, Adv Genet 2001, 44, 199.
86. D. Zhou, J. Mattner, C. Cantu Iii, N. Schrantz, N. Yin, Y. Gao, Y. Sagiv, K. Hudspeth, Y. Wu, et al., Science 2004.
87. G. Legler, E. Lullau, E. Kappes, F. Kastenholz, Biochim Biophys Acta 1991, 1080, 89.
88. M. Horsch, L. Hoesch, A. Vasella, D. M. Rast, Eur J Biochem 1991, 197, 815.
89. J. Liu, A. R. Shikhman, M. K. Lotz, C. H. Wong, Chem Biol 2001, 8, 701.
90. S. Knapp, D. J. Vocadlo, Z. N. Gao, B. Kirk, J. P. Lou, S. G. Withers, J. Am. Chem. Soc. 1996, 118, 6804.
91. V. H. Lillelund, H. H. Jensen, X. Liang, M. Bols, Chem Rev 2002, 102, 515.
92. R. J. Konrad, I. Mikolaenko, J. F. Tolar, K. Liu, J. E. Kudlow, Biochem J 2001, 356, 31.
93. K. Liu, A. J. Paterson, F. Zhang, J. McAndrew, K. Fukuchi, J. M. Wyss, L. Peng, Y. Hu, J. E. Kudlow, J Neurochem 2004, 89,1044.
94. G. Parker, R. Taylor, D. Jones, D. McClain, J Biol Chem 2004, 279, 20636.
95. E. B. Arias, J. Kim, G. D. Cartee, Diabetes 2004, 53, 921.
96. A. Junod, A. E. Lambert, L. Orci, R. Pictet, A. E. Gonet, A. E. Renold, Proc Soc Exp Biol Med 1967, 126, 201.
97. R. A. Bennett, A. E. Pegg, Cancer Res 1981, 41, 2786.
98. K. D. Kroncke, K. Fehsel, A. Sommer, M. L. Rodriguez, V. Kolb-Bachofen, Biol Chem Hoppe Seyler 1995, 376, 179.
99. H. Yamamoto, Y. Uchigata, H. Okamoto, Nature 1981, 294, 284.
100. K. Yamada, K. Nonaka, T. Hanafusa, A. Miyazaki, H. Toyoshima, S. Tarui, Diabetes 1982, 31, 749.
101. V. Burkart, Z. Q. Wang, J. Radons, B. Heller, Z. Herceg, L. Stingl, E. F. Wagner, H. Kolb, Nat Med 1999, 5, 314.
102. M. D. Roos, W. Xie, K. Su, J. A. Clark, X. Yang, E. Chin, A. J. Paterson, J. E. Kudlow, Proc Assoc Am Physicians 1998, 110, 422.
103. Y. Gao, G. J. Parker, G. W. Hart, Arch Biochem Biophys 2000, 383, 296.
104. R. Okuyama, M. Yachi, Biochem Biophys Res Commun 2001, 287, 366.
105. N. E. Zachara, N. O'Donnell, W. D. Cheung, J. J. Mercer, J. D. Marth, G. W. Hart, J Biol Chem 2004, 279, 30133.
106. J. A. Hanover, Z. Lai, G. Lee, W. A. Lubas, S. M. Sato, Arch Biochem Biophys 1999, 362, 38.
107. K. Liu, A. J. Paterson, R. J. Konrad, A. F. Parlow, S. Jimi, M. Roh, E. Chin, Jr., J. E. Kudlow, Mol Cell Endocrinol 2002, 194, 135.
108. M. S. Macauley, G. E. Whitworth, A. W. Debowski, D. Chin, D. J. Vocadlo, J Biol Chem 2005, 280, 25313.
109. B. L. Mark, D. J. Vocadlo, S. Knapp, B. L. Triggs-Raine, S. G. Withers, M. N. James, J Biol Chem 2001, 276, 10330.
110. R. S. Haltiwanger, K. Grove, G. A. Philipsberg, J Biol Chem 1998, 273, 3611.
111. D. J. Miller, X. Gong, B. D. Shur, Development 1993, 118, 1279.
112. L. Y. Zou, S. L. Yang, S. H. Hu, I. H. Chaudry, R. B. Marchase, J. C. Chatham, Shock 2007, 27, 402.
113. J. B. Huang, A. J. Clark, H. R. Petty, Cellular Immunology 2007, 245, 1.
114. U. J. G. Conference, in US/Japan Glyco 2004 Conference, Honolulu, Hawaii, 2004.
115. L. Y. Zou, S. L. Yang, S. H. Hu, I. H. Chaudry, R. B. Marchase, J. C. Chatham, Faseb Journal 2006, 20, A1471.
116. V. Champattanachai, R. B. Marchase, J. C. Chatham, American Journal of Physiology-Cell Physiology 2007, 292, C178.
117. V. Champattanachai, R. B. Marchase, J. C. Chatham, American Journal of Physiology-Cell Physiology 2008, 294, C1509.
118. I. Khlistunova, M. Pickhardt, J. Biernat, Y. P. Wang, E. M. Mandelkow, E. Mandelkow, Current Alzheimer Research 2007, 4, 544.
119. P. Friedhoff, A. Schneider, E. M. Mandelkow, E. Mandelkow, Biochemistry 1998, 37, 10223.
120. M. Pickhardt, Z. Gazova, M. von Bergen, I. Khlistunova, Y. P. Wang, A. Hascher, E. M. Mandelkow, J. Biernat, E. Mandelkow, Journal of Biological Chemistry 2005, 280, 3628.
121. P. H. Liang, W. C. Cheng, Y. L. Lee, H. P. Yu, Y. T. Wu, Y. L. Lin, C. H. Wong, Chembiochem 2006, 7, 165.
122. J. J. Liu, M. M. D. Numa, H. T. Liu, S. J. Huang, P. Sears, A. R. Shikhman, C. H. Wong, Journal of Organic Chemistry 2004, 69, 6273.
123. Y. Takaoka, T. Kajimoto, C. H. Wong, Journal of Organic Chemistry 1993, 58, 4809.
124. T. M. Wrodnigg, A. E. Stutz, S. G. Withers, Tetrahedron Letters 1997, 38, 5463.

## Claims

1. A compound of Formula (I) or a pharmaceutically acceptable salt thereof:

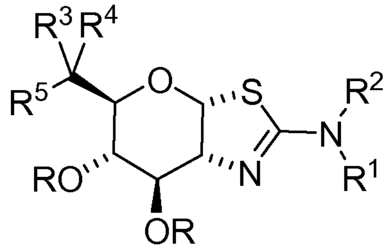

(I)

wherein:

each R is independently H or $C(O)CH_3$;
$R^1$ and $R^2$ are independently selected from the group consisting of: H, $C_{1-6}$alkyl, $C_{2-6}$alkenyl, $C_{1-6}$alkoxy, $-(CH_2)_n$-cyclopropyl and $-(CH_2)_n$-cyclobutyl wherein n is 0, 1,2, 3 or 4; or $R^1$ and $R^2$ may be joined together with the nitrogen atom to which they are attached to form azetidine, pyrrolidine, piperidine or isoxazolidine, said $C_{1-6}$alkyl, $C_{2-6}$alkenyl, $C_{1-6}$alkoxy, $-(CH_2)_n$-cyclopropyl, $-(CH_2)_n$-cyclobutyl, azetidine, pyrrolidine, piperidine and isoxazolidine optionally mono-substituted with fluoro, hydroxy or methyl;
R3 is selected from the group consisting of: $C_{1-8}$alkyl, $C_{2-8}$alkenyl, $C_{2-8}$alkynyl, $C_{3-6}$cycloalkyl, aryl and heteroaryl, each optionally mono-substituted with fluoro or OH;
$R^4$ is selected from the group consisting of: H, F, $C_{1-8}$alkyl, $C_{2-8}$alkenyl and $C_{2-8}$alkynyl, each, apart from H or F, optionally mono-substituted with fluoro or OH; or $R^3$ and $R^4$ and the carbon atom to which they are attached may join together to form vinyl or a 3 to 7-membered carbocyclic or heterocyclic ring, said 3 to 7-membered carbocyclic or heterocyclic ring optionally containing a double bond and optionally mono-substituted with fluoro or OH;

or a compound of Formula (Ia) or a pharmaceutically acceptable salt thereof:

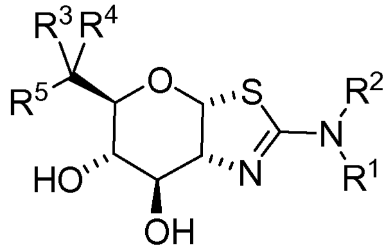

(Ia)

wherein:

$R^1$ and $R^2$ are independently selected from the group consisting of: H, $C_{1-6}$alkyl and cyclopropylmethyl; or $R^1$ and $R^2$ may be joined together with the nitrogen atom to which they are attached to form azetidine or pyrrolidine, said $C_{1-6}$alkyl, cyclopropylmethyl, azetidine or pyrrolidine optionally substituted with 1 to 3 substituents selected from fluoro and methyl;
R3 is selected from the group consisting of: $C_{1-8}$alkyl, $C_{2-8}$alkenyl, $C_{2-8}$alkynyl and $C_{3-6}$cycloalkyl, each optionally substituted with 1 to 3 substituents selected from fluoro and OH; and
$R^4$ is selected from the group consisting of: H, F, $C_{1-8}$alkyl, $C_{2-8}$alkenyl and $C_{2-8}$alkynyl, each excluding hydrogen optionally substituted with 1 to 3 substituents selected from fluoro and OH; or $R^3$ and $R^4$ and the carbon atom to which they are attached may join together to form a 5-membered carbocyclic ring optionally containing a double bond and optionally substituted with 1 to 3 substituents selected from fluoro and OH;

R5 is selected from H, F, OH and $OC(O)CH_3$;

with the proviso that when $R^4$ is F then $R^5$ is other than OH and $OC(O)CH_3$.

2. The compound of claim 1 of Formula (I) wherein $R^1$ and $R^2$ are independently selected from the group consisting of: H, $C_{1-6}$alkyl, $C_{1-6}$alkoxy, $-(CH_2)_n$-cyclopropyl and $-(CH_2)_n$-cyclobutyl wherein n is 0, 1, 2, 3 or 4; or $R^1$ and $R^2$ may be joined together with the nitrogen atom to which they are attached to form azetidine, pyrrolidine or piperidine, said $C_{1-6}$alkyl, $C_{1-6}$alkoxy, $-(CH_2)_n$-cyclopropyl, $-(CH_2)_n$-cyclobutyl, azetidine, pyrrolidine and piperidine optionally mono-substituted with fluoro or methyl.

3. The compound according to Claim 2 wherein each R is H.

4. The compound according to claim 3 wherein:

$R^1$ and $R^2$ are independently $C_{1-4}$alkyl;
R3 is $C_{1-6}$alkyl;
R4 is selected from the group consisting of: H and $C_{1-6}$alkyl; and
R5 is OH.

5. The compound according to Claim 4 wherein:

$R^1$ and $R^2$ are independently methyl or ethyl;
R3 is methyl or ethyl; and
$R^4$ is selected from the group consisting of: H, methyl and ethyl.

6. The compound according to Claim 3 wherein $R^3$ and $R^4$ and the carbon atom to which they are attached may join together to form a 3 to 7-membered carbocyclic or heterocyclic ring, said 3 to 7-membered carbocyclic or heterocyclic ring optionally containing a double bond and optionally mono-substituted with fluoro or OH.

7. The compound according to Claim 1 of Formula (Ia), wherein all variables are as defined in claim 1

8. The compound according to Claim 3 wherein $R^3$ is $CF_3$ and $R^5$ is OH.

9. A compound of claim 1 selected from the following group:

(3aR,5R,6S,7R,7aR)-2-(Dimethylamino)-5-((S)-1-hydroxyethyl)-5,6,7,7a-tetrahydro-3aH-pyrano[3,2-d]thiazole-6,7-diol;
(3aR,6S,7R,7aR)-5-((S)-1-hydroxyethyl)-2-(propylamino)-5,6,7,7a-tetrahydro-3aH-pyrano[3,2-d]thiazole-6,7-diol;
(3aR,5S,6S,7R,7aR)-5-(2-Hydroxypropan-2-yl)-2-(methylamino)-5,6,7,7a-tetrahydro-3aH-pyrano[3,2-d]thiazole-6,7-diol;
(3aR,5S,6S,7R,7aR)-2-(dimethylamino)-5-(2-hydroxypropan-2-yl)-5,6,7,7a-tetrahydro-3aH-pyrano[3,2-d]thiazole-6,7-diol;
(3aR,5R,6S,7R,7aR)-2-(Dimethylamino)-5-(2-hydroxyethyl)-5,6,7,7a-tetrahydro-3aH-pyrano[3,2-d]thiazole-6,7-diol;
(3aR,5R,6S,7R,7aR)-2-(Dimethylamino)-5-ethyl-5,6,7,7a-tetrahydro-3aH-pyrano[3,2-d]thiazole-6,7-diol;
(3aR,5R,6S,7R,7aR)-2-(dimethylamino)-5-vinyl-5,6,7,7a-tetrahydro-3aH-pyrano[3,2-d]thiazole-6,7-diol;
(3aR,5R,6S,7R,7aR)-5-Ethyl-2-(methylamino)-5,6,7,7a-tetrahydro-3aH-pyrano[3,2-d]thiazole-6,7-diol;
(3aR,5S,6S,7R,7aR)-2-(Dimethylamino)-5-((S)-1-fluoroethyl)-5,6,7,7a-tetrahydro-3aH-pyrano[3,2-d]thiazole-6,7-diol;
(3aR,5S,6S,7R,7aR)-2-(dimethylamino)-5-((S)-2,2,2-trifluoro-1-hydroxyethyl)-5,6,7,7a-tetrahydro-3aH-pyrano[3,2-d]thiazole-6,7-diol;
(3aR,5S,6S,7R,7aR)-2-(dimethylamino)-5-((R)-2,2,2-trifluoro-1-hydroxyethyl)-5,6,7,7a-tetrahydro-3aH-pyrano[3,2-d]thiazole-6,7-diol;
(3aR,5S,6S,7R,7aR)-5-(1,1-Difluoroethyl)-2-(dimethylamino)-5,6,7,7a-tetrahydro-3aH-pyrano[3,2-d]thiazole-6,7-diol;
(3aR,5R,6S,7R,7aR)-5-((S)-1-hydroxyethyl)-2-(methylamino)-5,6,7,7a-tetrahydro-3aH-pyrano[3,2-d]thiazole-6,7-diol;

(3aR,5R,6S,7R,7aR)-5-((R)-1-hydroxyethyl)-2-(methylamino)-5,6,7,7a-tetrahydro-3aH-pyrano[3,2-d]thiazole-6,7-diol;
(3aR,5R,6S,7R,7aR)-5-((S)-1-hydroxypropyl)-2-(methylamino)-5,6,7,7a-tetrahydro-3aH-pyrano[3,2-d]thiazole-6,7-diol;
(3aR,5R,6S,7R,7aR)-5-((R)-1-hydroxypropyl)-2-(methylamino)-5,6,7,7a-tetrahydro-3aH-pyrano[3,2-d]thiazole-6,7-diol;
(3aR,5R,6S,7R,7aR)-5-(1-hydroxy-2-methylpropyl)-2-(methylamino)-5,6,7,7a-tetrahydro-3aH-pyrano[3,2-d]thiazole-6,7-diol;
(3aR,5R,6S,7R,7aR)-5-(cyclopropyl(hydroxy)methyl)-2-(methylamino)-5,6,7,7a-tetrahydro-3aH-pyrano[3,2-d]thiazole-6,7-diol;
(3aR,5R,6S,7R,7aR)-5-(hydroxy(pyridin-3-yl)methyl)-2-(methylamino)-5,6,7,7a-tetrahydro-3aH-pyrano[3,2-d]thiazole-6,7-diol;
(3aR,5R,6S,7R,7aR)-5-(hydroxy(phenyl)methyl)-2-(methylamino)-5,6,7,7a-tetrahydro-3aH-pyrano[3,2-d]thiazole-6,7-diol;
(3aR,5R,6S,7R,7aR)-5-((R)-1-hydroxyallyl)-2-(methylamino)-5,6,7,7a-tetrahydro-3aH-pyrano[3,2-d]thiazole-6,7-diol;
(3aR,5R,6S,7R,7aR)-5-((S)-1-hydroxyallyl)-2-(methylamino)-5,6,7,7a-tetrahydro-3aH-pyrano[3,2-d]thiazole-6,7-diol;
(3aR,5R,6S,7R,7aR)-5-((R)-1-hydroxy-2-methylallyl)-2-(methylamino)-5,6,7,7a-tetrahydro-3aH-pyrano[3,2-d]thiazole-6,7-diol;
(3aR,5R,6S,7R,7aR)-5-((R)-1-hydroxybut-3-enyl)-2-(methylamino)-5,6,7,7a-tetrahydro-3aH-pyrano[3,2-d]thiazole-6,7-diol;
(3aR,5R,6S,7R,7aR)-5-((S)-1-hydroxybut-3-enyl)-2-(methylamino)-5,6,7,7a-tetrahydro-3aH-pyrano[3,2-d]thiazole-6,7-diol;
(3aR,5R,6S,7R,7aR)-2-(ethylamino)-5-((R)-1-hydroxyethyl)-5,6,7,7a-tetrahydro-3aH-pyrano[3,2-d]thiazole-6,7-diol;
(3aR,5R,6S,7R,7aR)-2-(ethylamino)-5-((S)-1-hydroxyethyl)-5,6,7,7a-tetrahydro-3aH-pyrano[3,2-d]thiazole-6,7-diol;
(3aR,5R,6S,7R,7aR)-5-((S)-cyclopropyl(hydroxy)methyl)-2-(ethylamino)-5,6,7,7a-tetrahydro-3aH-pyrano[3,2-d]thiazole-6,7-diol;
(3aR,5R,6S,7R,7aR)-2-(ethylamino)-5-(1-hydroxy-2-methylpropyl)-5,6,7,7a-tetrahydro-3aH-pyrano[3,2-d]thiazole-6,7-diol;
(3aR,5R,6S,7R,7aR)-2-(ethylamino)-5-(hydroxy(phenyl)methyl)-5,6,7,7a-tetrahydro-3aH-pyrano[3,2-d]thiazole-6,7-diol;
(3aR,5R,6S,7R,7aR)-2-(ethylamino)-5-((R)-1-hydroxyallyl)-5,6,7,7a-tetrahydro-3aH-pyrano[3,2-d]thiazole-6,7-diol;
(3aR,5R,6S,7R,7aR)-2-(ethylamino)-5-((S)-1-hydroxyallyl)-5,6,7,7a-tetrahydro-3aH-pyrano[3,2-d]thiazole-6,7-diol;
(3aR,5R,6S,7R,7aR)-2-(ethylamino)-5-(2-hydroxypropan-2-yl)-5,6,7,7a-tetrahydro-3aH-pyrano[3,2-d]thiazole-6,7-diol;
(3aR,5R,6S,7R,7aR)-2-(ethylamino)-5-((R)-1-hydroxy-2-methylallyl)-5,6,7,7a-tetrahydro-3aH-pyrano[3,2-d]thiazole-6,7-diol;
(3aR,5R,6S,7R,7aR)-2-(ethylamino)-5-((R)-1-hydroxybutyl)-5,6,7,7a-tetrahydro-3aH-pyrano[3,2-d]thiazole-6,7-diol;
(3aR,5R,6S,7R,7aR)-2-(ethylamino)-5-((S)-1-hydroxybutyl)-5,6,7,7a-tetrahydro-3aH-pyrano[3,2-d]thiazole-6,7-diol;
(3aR,5R,6S,7R,7aR)-2-(ethylamino)-5-((R)-1-hydroxypentyl)-5,6,7,7a-tetrahydro-3aH-pyrano[3,2-d]thiazole-6,7-diol;
(3aR,5R,6S,7R,7aR)-2-(ethylamino)-5-((S)-1-hydroxypentyl)-5,6,7,7a-tetrahydro-3aH-pyrano[3,2-d]thiazole-6,7-diol;
(3aR,5R,6S,7R,7aR)-2-(ethylamino)-5-((R)-1-hydroxybut-3-enyl)-5,6,7,7a-tetrahydro-3aH-pyrano[3,2-d]thiazole-6,7-diol;
(3aR,5R,6S,7R,7aR)-2-(ethylamino)-5-((S)-1-hydroxybut-3-enyl)-5,6,7,7a-tetrahydro-3aH-pyrano[3,2-d]thiazole-6,7-diol;
(3aR,5S,6S,7R,7aR)-5-(2-hydroxypropan-2-yl)-2-(propylamino)-5,6,7,7a-tetrahydro-3aH-pyrano[3,2-d]thiazole-6,7-diol;
(3aR,5R,6S,7R,7aR)-5-((S)-1-hydroxyallyl)-2-(propylamino)-5,6,7,7a-tetrahydro-3aH-pyrano[3,2-d]thiazole-6,7-diol;

(3aR,5R,6S,7R,7aR)-5-((R)-1-hydroxybutyl)-2-(propylamino)-5,6,7,7a-tetrahydro-3aH-pyrano[3,2-d]thiazole-6,7-diol;
(3aR,5R,6S,7R,7aR)-5-((S)-1-hydroxybutyl)-2-(propylamino)-5,6,7,7a-tetrahydro-3aH-pyrano[3,2-d]thiazole-6,7-diol;
(3aR,5R,6S,7R,7aR)-5-((R)-1-hydroxypentyl)-2-(propylamino)-5,6,7,7a-tetrahydro-3aH-pyrano[3,2-d]thiazole-6,7-diol;
(3aR,5R,6S,7R,7aR)-5-((S)-1-hydroxypentyl)-2-(propylamino)-5,6,7,7a-tetrahydro-3aH-pyrano[3,2-d]thiazole-6,7-diol;
(3aR,5R,6S,7R,7aR)-5-(1-hydroxypropyl)-2-(propylamino)-5,6,7,7a-tetrahydro-3aH-pyrano[3,2-d]thiazole-6,7-diol;
(3aR,5R,6S,7R,7aR)-5-((R)-1-hydroxybut-3-enyl)-2-(propylamino)-5,6,7,7a-tetrahydro-3aH-pyrano[3,2-d]thiazole-6,7-diol;
(3aR,5R,6S,7R,7aR)-5-((S)-1-hydroxybut-3-enyl)-2-(propylamino)-5,6,7,7a-tetrahydro-3aH-pyrano[3,2-d]thiazole-6,7-diol;
(3aR,5R,6S,7R,7aR)-5-((R)-1-hydroxyethyl)-2-(methoxy(methyl)amino)-5,6,7,7a-tetrahydro-3aH-pyrano[3,2-d]thiazole-6,7-diol;
(3aR,5R,6S,7R,7aR)-5-((S)-1-hydroxyethyl)-2-(methoxy(methyl)amino)-5,6,7,7a-tetrahydro-3aH-pyrano[3,2-d]thiazole-6,7-diol;
(3aR,5R,6S,7R,7aR)-2-(cyclopropylmethylamino)-5-((R)-1-hydroxyethyl)-5,6,7,7a-tetrahydro-3aH-pyrano[3,2-d]thiazole-6,7-diol;
(3aR,5R,6S,7R,7aR)-2-(cyclopropylmethylamino)-5-((S)-1-hydroxyethyl)-5,6,7,7a-tetrahydro-3aH-pyrano[3,2-d]thiazole-6,7-diol;
(3aR,5R,6S,7R,7aR)-2-(ethyl(methyl)amino)-5-((S)-1-hydroxybut-3-enyl)-5,6,7,7a-tetrahydro-3aH-pyrano[3,2-d]thiazole-6,7-diol;
(3aR,5R,6S,7R,7aR)-2-(ethyl(methyl)amino)-5-((R)-1-hydroxybut-3-enyl)-5,6,7,7a-tetrahydro-3aH-pyrano[3,2-d]thiazole-6,7-diol;
(3aR,5S,6S,7R,7aR)-2-(ethyl(methyl)amino)-5-(2-hydroxypropan-2-yl)-5,6,7,7a-tetrahydro-3aH-pyrano[3,2-d]thiazole-6,7-diol;
(3aR,5R,6S,7R,7aR)-2-(ethyl(methyl)amino)-5-(1-hydroxyethyl)-5,6,7,7a-tetrahydro-3aH-pyrano[3,2-d]thiazole-6,7-diol;
(3aR,5R,6S,7R,7aR)-2-(ethyl(methyl)amino)-5-((R)-1-hydroxypropyl)-5,6,7,7a-tetrahydro-3aH-pyrano[3,2-d]thiazole-6,7-diol;
(3aR,5R,6S,7R,7aR)-2-(ethyl(methyl)amino)-5-(1-hydroxypropyl)-5,6,7,7a-tetrahydro-3aH-pyrano[3,2-d]thiazole-6,7-diol;
(3aR,5S,6S,7R,7aR)-2-(ethyl(methyl)amino)-5-((R)-1-hydroxyallyl)-5,6,7,7a-tetrahydro-3aH-pyrano[3,2-d]thiazole-6,7-diol;
(3aR,5S,6S,7R,7aR)-2-(ethyl(methyl)amino)-5-(1-hydroxyallyl)-5,6,7,7a-tetrahydro-3aH-pyrano[3,2-d]thiazole-6,7-diol;
(3aR,5R,6S,7R,7aR)-2-(dimethylamino)-5-((S)-1-hydroxypropyl)-5,6,7,7a-tetrahydro-3aH-pyrano[3,2-d]thiazole-6,7-diol;
(3aR,5R,6S,7R,7aR)-2-(dimethylamino)-5-((S)-1-hydroxyallyl)-5,6,7,7a-tetrahydro-3aH-pyrano[3,2-d]thiazole-6,7-diol;
(3aR,5R,6S,7R,7aR)-2-(dimethylamino)-5-((R)-1-hydroxy-2-methylallyl)-5,6,7,7a-tetrahydro-3aH-pyrano[3,2-d]thiazole-6,7-diol;
(3aR,5R,6S,7R,7aR)-2-(dimethylamino)-5-((S)-1-hydroxy-2-methylallyl)-5,6,7,7a-tetrahydro-3aH-pyrano[3,2-d]thiazole-6,7-diol;
(3aR,5R,6S,7R,7aR)-2-(dimethylamino)-5-((R)-1-hydroxybut-3-enyl)-5,6,7,7a-tetrahydro-3aH-pyrano[3,2-d]thiazole-6,7-diol;
(3aR,5R,6S,7R,7aR)-2-(dimethylamino)-5-((S)-1-hydroxybut-3-enyl)-5,6,7,7a-tetrahydro-3aH-pyrano[3,2-d]thiazole-6,7-diol;
(3aR,5R,6S,7R,7aR)-2-(dimethylamino)-5-((R)-1-hydroxybutyl)-5,6,7,7a-tetrahydro-3aH-pyrano[3,2-d]thiazole-6,7-diol;
(3aR,5R,6S,7R,7aR)-2-(dimethylamino)-5-((S)-1-hydroxybutyl)-5,6,7,7a-tetrahydro-3aH-pyrano[3,2-d]thiazole-6,7-diol;
(3aR,5R,6S,7R,7aR)-2-(dimethylamino)-5-((R)-1-hydroxypentyl)-5,6,7,7a-tetrahydro-3aH-pyrano[3,2-d]thiazole-6,7-diol;
(3aR,5R,6S,7R,7aR)-2-(dimethylamino)-5-((S)-1-hydroxypentyl)-5,6,7,7a-tetrahydro-3aH-pyrano[3,2-d]thiazole-6,7-diol;

(3aR,5R,6S,7R,7aR)-2-(dimethylamino)-5-((S)-1-hydroxyhexyl)-5,6,7,7a-tetrahydro-3aH-pyrano[3,2-d]thiazole-6,7-diol;
(3aR,5R,6S,7R,7aR)-2-(dimethylamino)-5-((R)-1-hydroxyheptyl)-5,6,7,7a-tetrahydro-3aH-pyrano[3,2-d]thiazole-6,7-diol;
(3aR,5R,6S,7R,7aR)-2-(dimethylamino)-5-((S)-1-hydroxyheptyl)-5,6,7,7a-tetrahydro-3aH-pyrano[3,2-d]thiazole-6,7-diol;
(3aR,5R,6S,7R,7aR)-2-(azetidin-1-yl)-5-((R)-1-hydroxyethyl)-5,6,7,7a-tetrahydro-3aH-pyrano[3,2-d]thiazole-6,7-diol;
(3aR,5R,6S,7R,7aR)-2-(azetidin-1-yl)-5-((S)-1-hydroxyethyl)-5,6,7,7a-tetrahydro-3aH-pyrano[3,2-d]thiazole-6,7-diol;
(3aR,5S,6S,7R,7aR)-2-(azetidin-1-yl)-5-(2-hydroxypropan-2-yl)-5,6,7,7a-tetrahydro-3aH-pyrano[3,2-d]thiazole-6,7-diol;
(3aR,5R,6S,7R,7aR)-2-(azetidin-1-yl)-5-((S)-1-hydroxypropyl)-5,6,7,7a-tetrahydro-3aH-pyrano[3,2-d]thiazole-6,7-diol;
(3aR,5R,6S,7R,7aR)-2-(azetidin-1-yl)-5-((R)-1-hydroxyallyl)-5,6,7,7a-tetrahydro-3aH-pyrano[3,2-d]thiazole-6,7-diol;
(3aR,5R,6S,7R,7aR)-2-(azetidin-1-yl)-5-((S)-1-hydroxyallyl)-5,6,7,7a-tetrahydro-3aH-pyrano[3,2-d]thiazole-6,7-diol;
(3aR,5R,6S,7R,7aR)-2-(azetidin-1-yl)-5-((R)-1-hydroxybut-3-enyl)-5,6,7,7a-tetrahydro-3aH-pyrano[3,2-d]thiazole-6,7-diol;
(3aR,5R,6S,7R,7aR)-2-(azetidin-1-yl)-5-((S)-1-hydroxybut-3-enyl)-5,6,7,7a-tetrahydro-3aH-pyrano[3,2-d]thiazole-6,7-diol;
(3aR,5S,6S,7R,7aR)-5-(2-hydroxypropan-2-yl)-2-(methyl(propyl)amino)-5,6,7,7a-tetrahydro-3aH-pyrano[3,2-d]thiazole-6,7-diol;
(3aR,5R,6S,7R,7aR)-5-(1-hydroxypropyl)-2-(methyl(propyl)amino)-5,6,7,7a-tetrahydro-3aH-pyrano[3,2-d]thiazole-6,7-diol;
(3aR,5R,6S,7R,7aR)-5-((S)-1-hydroxyethyl)-2-(methyl(propyl)amino)-5,6,7,7a-tetrahydro-3aH-pyrano[3,2-d]thiazole-6,7-diol;
(3aR,5R,6S,7R,7aR)-5-((R)-1-hydroxyallyl)-2-(methyl(propyl)amino)-5,6,7,7a-tetrahydro-3aH-pyrano[3,2-d]thiazole-6,7-diol;
(3aR,5R,6S,7R,7aR)-5-((S)-1-hydroxyallyl)-2-(methyl(propyl)amino)-5,6,7,7a-tetrahydro-3aH-pyrano[3,2-d]thiazole-6,7-diol;
(3aR,5R,6S,7R,7aR)-5-((R)-1-hydroxybut-3-enyl)-2-(methyl(propyl)amino)-5,6,7,7a-tetrahydro-3aH-pyrano[3,2-d]thiazole-6,7-diol;
(3aR,5R,6S,7R,7aR)-5-((S)-1-hydroxybut-3-enyl)-2-(methyl(propyl)amino)-5,6,7,7a-tetrahydro-3aH-pyrano[3,2-d]thiazole-6,7-diol;
(3aR,5S,6S,7R,7aR)-5-(2-hydroxypropan-2-yl)-2-(pyrrolidin-1-yl)-5,6,7,7a-tetrahydro-3aH-pyrano[3,2-d]thiazole-6,7-diol;
(3aR,5R,6S,7R,7aR)-5-((S)-1-hydroxyethyl)-2-(pyrrolidin-1-yl)-5,6,7,7a-tetrahydro-3aH-pyrano[3,2-d]thiazole-6,7-diol;
(3aR,5R,6S,7R,7aR)-5-((R)-1-hydroxypropyl)-2-(pyrrolidin-1-yl)-5,6,7,7a-tetrahydro-3aH-pyrano[3,2-d]thiazole-6,7-diol;
(3aR,5R,6S,7R,7aR)-5-((S)-1-hydroxypropyl)-2-(pyrrolidin-1-yl)-5,6,7,7a-tetrahydro-3aH-pyrano[3,2-d]thiazole-6,7-diol;
(3aR,5R,6S,7R,7aR)-5-((S)-1-hydroxyallyl)-2-(pyrrolidin-1-yl)-5,6,7,7a-tetrahydro-3aH-pyrano[3,2-d]thiazole-6,7-diol;
(3aR,5S,6S,7R,7aR)-2-(methylamino)-5-((R)-2,2,2-trifluoro-1-hydroxyethyl)-5,6,7,7a-tetrahydro-3aH-pyrano[3,2-d]thiazole-6,7-diol;
(3aR,5S,6S,7R,7aR)-2-(propylamino)-5-((R)-2,2,2-trifluoro-1-hydroxyethyl)-5,6,7,7a-tetrahydro-3aH-pyrano[3,2-d]thiazole-6,7-diol;
(3aR,5S,6S,7R,7aR)-2-(methoxy(methyl)amino)-5-((R)-2,2,2-trifluoro-1-hydroxyethyl)-5,6,7,7a-tetrahydro-3aH-pyrano[3,2-d]thiazole-6,7-diol;
(3aR,5S,6S,7R,7aR)-2-(methylamino)-5-((S)-2,2,2-trifluoro-1-hydroxyethyl)-5,6,7,7a-tetrahydro-3aH-pyrano[3,2-d]thiazole-6,7-diol;
(3aR,5S,6S,7R,7aR)-2-(propylamino)-5-((S)-2,2,2-trifluoro-1-hydroxyethyl)-5,6,7,7a-tetrahydro-3aH-pyrano[3,2-d]thiazole-6,7-diol;
(3aR,5S,6S,7R,7aR)-2-(methoxy(methyl)amino)-5-((S)-2,2,2-trifluoro-1-hydroxyethyl)-5,6,7,7a-tetrahydro-3aH-pyrano[3,2-d]thiazole-6,7-diol;

(3aR,5S,6S,7R,7aR)-2-(ethylamino)-5-((S)-1-fluoroethyl)-5,6,7,7a-tetrahydro-3aH-pyrano[3,2-d]thiazole-6,7-diol;
(3aR,5R,6S,7R,7aR)-2-(dimethylamino)-5-((R)-1-hydroxyethyl)-5,6,7,7a-tetrahydro-3aH-pyrano[3,2-d]thiazole-6,7-diol;
(3aR,5R,6S,7R,7aR)-2-(ethylamino)-5-((S)-1-hydroxypropyl)-5,6,7,7a-tetrahydro-3aH-pyrano[3,2-d]thiazole-6,7-diol;
(3aR,5R,6S,7R,7aR)-2-(ethylamino)-5-((R)-1-hydroxypropyl)-5,6,7,7a-tetrahydro-3aH-pyrano[3,2-d]thiazole-6,7-diol;
(3aR,5S,6S,7R,7aR)-5-((S)-1-fluoroethyl)-2-(pyrrolidin-1-yl)-5,6,7,7a-tetrahydro-3aH-pyrano[3,2-d]thiazole-6,7-diol;
(3aR,5R,6S,7R,7aR)-5-((R)-1-hydroxybutyl)-2-(methylamino)-5,6,7,7a-tetrahydro-3aH-pyrano[3,2-d]thiazole-6,7-diol;
(3aR,5R,6S,7R,7aR)-5-((S)-1-hydroxybutyl)-2-(methylamino)-5,6,7,7a-tetrahydro-3aH-pyrano[3,2-d]thiazole-6,7-diol;
(3aR,5R,6S,7R,7aR)-5-((R)-1-hydroxypentyl)-2-(methylamino)-5,6,7,7a-tetrahydro-3aH-pyrano[3,2-d]thiazole-6,7-diol;
(3aR,5R,6S,7R,7aR)-5-((S)-1-hydroxypentyl)-2-(methylamino)-5,6,7,7a-tetrahydro-3aH-pyrano[3,2-d]thiazole-6,7-diol;
(3aR,5R,6S,7R,7aR)-5-((R)-1-hydroxyhexyl)-2-(methylamino)-5,6,7,7a-tetrahydropyrano[3,2-d]thiazole-6,7-diol;
(3aR,5R,6S,7R,7aR)-5-((S)-1-hydroxyhexyl)-2-(methylamino)-5,6,7,7a-tetrahydro-3aH-pyrano[3,2-d]thiazole-6,7-diol;
(3aR,5R,6S,7R,7aR)-5-((R)-1-hydroxyethyl)-2-(propylamino)-5,6,7,7a-tetrahydro-3aH-pyrano[3,2-d]thiazole-6,7-diol;
(3aR,5R,6S,7R,7aR)-2-(cyclopropylmethylamino)-5-((S)-1-hydroxy-2-methylallyl)-5,6,7,7a-tetrahydro-3aH-pyrano[3,2-d]thiazole-6,7-diol;
(3aR,5R,6S,7R,7aR)-2-(cyclopropylmethylamino)-5-((R)-1-hydroxypropyl)-5,6,7,7a-tetrahydro-3aH-pyrano[3,2-d]thiazole-6,7-diol;
(3aR,5R,6S,7R,7aR)-2-(cyclopropylmethylamino)-5-((S)-1-hydroxypropyl)-5,6,7,7a-tetrahydro-3aH-pyrano[3,2-d]thiazole-6,7-diol;
(3aR,5S,6S,7R,7aR)-2-(cyclopropylmethylamino)-5-((R)-1-hydroxybut-3-enyl)-5,6,7,7a-tetrahydro-3aH-pyrano[3,2-d]thiazole-6,7-diol;
(3aR,5S,6S,7R,7aR)-2-(cyclopropylmethylamino)-5-(1-hydroxybut-3-enyl)-5,6,7,7a-tetrahydro-3aH-pyrano[3,2-d]thiazole-6,7-diol;
(3aR,5R,6S,7R,7aR)-2-(cyclopropylmethylamino)-5-((S)-1-hydroxyallyl)-5,6,7,7a-tetrahydro-3aH-pyrano[3,2-d]thiazole-6,7-diol;
(3aR,5R,6S,7R,7aR)-2-(2-cyclopropylethylamino)-5-((R)-1-hydroxyethyl)-5,6,7,7a-tetrahydro-3aH-pyrano[3,2-d]thiazole-6,7-diol;
(3aR,5R,6S,7R,7aR)-2-(2-cyclopropylethylamino)-5-((S)-1-hydroxyethyl)-5,6,7,7a-tetrahydro-3aH-pyrano[3,2-d]thiazole-6,7-diol;
(3aR,5S,6S,7R,7aR)-2-(2-cyclopropylethylamino)-5-(2-hydroxypropan-2-yl)-5,6,7,7a-tetrahydro-3aH-pyrano[3,2-d]thiazole-6,7-diol;
(3aR,5R,6S,7R,7aR)-2-(ethyl(methyl)amino)-5-((R)-1-hydroxyethyl)-5,6,7,7a-tetrahydro-3aH-pyrano[3,2-d]thiazole-6,7-diol;
(3aR,5R,6S,7R,7aR)-2-(ethyl(methyl)amino)-5-((S)-1-hydroxybutyl)-5,6,7,7a-tetrahydro-3aH-pyrano[3,2-d]thiazole-6,7-diol;
(3aR,5R,6S,7R,7aR)-2-(ethyl(methyl)amino)-5-((S)-1-hydroxypentyl)-5,6,7,7a-tetrahydro-3aH-pyrano[3,2-d]thiazole-6,7-diol;
(3aR,5R,6S,7R,7aR)-2-(dimethylamino)-5-((R)-1-hydroxyhexyl)-5,6,7,7a-tetrahydro-3aH-pyrano[3,2-d]thiazole-6,7-diol;
(3aR,5R,6S,7R,7aR)-5-((S)-1-hydroxybutyl)-2-(pyrrolidin-1-yl)-5,6,7,7a-tetrahydro-3aH-pyrano[3,2-d]thiazole-6,7-diol;
(3aR,5R,6S,7R,7aR)-5-((S)-1-hydroxypentyl)-2-(pyrrolidin-1-yl)-5,6,7,7a-tetrahydro-3aH-pyrano[3,2-d]thiazole-6,7-diol;
(3aR,5S,6S,7R,7aR)-5-(1,1-difluoroethyl)-2-(methoxy(methyl)amino)-5,6,7,7a-tetrahydro-3aH-pyrano[3,2-d]thiazole-6,7-diol;
(3aR,5R,6S,7R,7aR)-5-ethyl-2-(ethyl(methyl)amino)-5,6,7,7a-tetrahydro-3aH-pyrano[3,2-d]thiazole-6,7-diol;
(3aR,5R,6S,7R,7aR)-2-(azetidin-1-yl)-5-ethyl-5,6,7,7a-tetrahydro-3aH-pyrano[3,2-d]thiazole-6,7-diol;

(3aR,5R,6S,7R,7aR)-2-(azetidin-1-yl)-5-vinyl-5,6,7,7a-tetrahydro-3aH-pyrano[3,2-d]thiazole-6,7-diol;
(3aR,5R,6S,7R,7aR)-5-ethyl-2-(propylamino)-5,6,7,7a-tetrahydro-3aH-pyrano[3,2-d]thiazole-6,7-diol;
(3aR,5R,6S,7R,7aR)-5-ethyl-2-(ethylamino)-5,6,7,7a-tetrahydro-3aH-pyrano[3,2-d]thiazole-6,7-diol;
(3aR,5R,6S,7R,7aR)-2-(ethylamino)-5-vinyl-5,6,7,7a-tetrahydro-3aH-pyrano[3,2-d]thiazole-6,7-diol;
(3aR,5S,6S,7R,7aR)-5-(1-hydroxycyclopentyl)-2-(methylamino)-5,6,7,7a-tetrahydro-3aH-pyrano[3,2-d]thiazole-6,7-diol;
(3aR,5S,6S,7R,7aR)-5-(1-hydroxycyclopent-3-enyl)-2-(methylamino)-5,6,7,7a-tetrahydro-3aH-pyrano[3,2-d]thiazole-6,7-diol;
(3aR,5S,6S,7R,7aR)-5-(2-fluoropropan-2-yl)-2-(methylamino)-5,6,7,7a-tetrahydro-3aH-pyrano[3,2-d]thiazole-6,7-diol;
(3aR,5R,6S,7R,7aR) -2- (methylamino)-5-(prop-1-en-2-yl)-5,6,7,7a-tetrahydro-3aH-pyrano[3,2-d]thiazole-6,7-diol;
(3aR,5R,6S,7R,7aR)-2-(2-Fluoroethylamino)-5-((S)-1-hydroxyethyl)-5,6,7,7a-tetrahydro-3aH-pyrano[3,2-d]thiazole-6,7-diol;
(3aR,5R,6S,7R,7aR)-2-(3-Fluoropropylamino)-5-((S)-1-hydroxyethyl)-5,6,7,7a-tetrahydro-3aH-pyrano[3,2-d]thiazole-6,7-diol;
(3aR,5R,6S,7R,7aR)-5-(3-Hydroxypropyl)-2-(methylamino)-5,6,7,7a-tetrahydro-3aH-pyrano[3,2-d]thiazole-6,7-diol;
(3aR,5R,6S,7R,7aR)-5-(2-hydroxyethyl)-2-(methylamino)-5,6,7,7a-tetrahydro-3aH-pyrano[3,2-d]thiazole-6,7-diol;
(3aR,5S,6S,7R,7aR)-5-(1,1-difluoroethyl)-2-(methylamino)-5,6,7,7a-tetrahydro-3aH-pyrano[3,2-d]thiazole-6,7-diol;
(3aR,5S,6S,7R,7aR)-5-((S)-2,2-difluoro-1-hydroxyethyl)-2-(dimethylamino)-5,6,7,7a-tetrahydro-3aH-pyrano[3,2-d]thiazole-6,7-diol;
(3aR,5S,6S,7R,7aR)-5-((R)-2,2-difluoro-1-hydroxyethyl)-2-(dimethylamino)-5,6,7,7a-tetrahydro-3aH-pyrano[3,2-d]thiazole-6,7-diol;
(3aR,5R,6S,7R,7aR)-5-((S)-1,2-dihydroxyethyl)-2-(dimethylamino)-5,6,7,7a-tetrahydro-3aH-pyrano[3,2-d]thiazole-6,7-diol;
(3aR,5R,6S,7R,7aR)-5-((R)-1,2-dihydroxyethyl)-2-(dimethylamino)-5,6,7,7a-tetrahydro-3aH-pyrano[3,2-d]thiazole-6,7-diol;
(3aR,5S,6S,7R,7aR)-2-(dimethylamino)-5-((S)-2-fluoro-1-hydroxyethyl)-5,6,7,7a-tetrahydro-3aH-pyrano[3,2-d]thiazole-6,7-diol;
(3aR,5S,6S,7R,7aR)-2-(dimethylamino)-5-((R)-2-fluoro-1-hydroxyethyl)-5,6,7,7a-tetrahydro-3aH-pyrano[3,2-d]thiazole-6,7-diol;
(3aR,5R,6S,7R,7aR)-2-(dimethylamino)-5-(2-fluoroethyl)-5,6,7,7a-tetrahydro-3aH-pyrano[3,2-d]thiazole-6,7-diol;
(3aR,5R,6S,7R,7aR)-5-(2,2-difluoroethyl)-2-(dimethylamino)-5,6,7,7a-tetrahydro-3aH-pyrano[3,2-d]thiazole-6,7-diol;
(3aR,5R,6S,7R,7aR)-2-(dimethylamino)-5-(2,2,2-trifluoroethyl)-5,6,7,7a-tetrahydro-3aH-pyrano[3,2-d]thiazole-6,7-diol;
(3aR,5S,6S,7R,7aR)-2-(methylamino)-5-((S)-1,1,1-trifluoro-2-hydroxypropan-2-yl)-5,6,7,7a-tetrahydro-3aH-pyrano[3,2-d]thiazole-6,7-diol;
(3aR,5S,6S,7R,7aR)-2-(methylamino)-5-(1,1,1-trifluoro-2-hydroxypropan-2-yl)-5,6,7,7a-tetrahydro-3aH-pyrano[3,2-d]thiazole-6,7-diol;
(3aR,5S,6S,7R,7aR)-2-(ethylamino)-5-((S)-2,2,2-trifluoro-1-hydroxyethyl)-5,6,7,7a-tetrahydro-3aH-pyrano[3,2-d]thiazole-6,7-diol;
(3aR,5S,6S,7R,7aR)-2-(ethylamino)-5-((R)-2,2,2-trifluoro-1-hydroxyethyl)-5,6,7,7a-tetrahydro-3aH-pyrano[3,2-d]thiazole-6,7-diol;
(3aR,5R,6S,7R,7aR)-5-((R)-1-hydroxyethyl)-2-(2-hydroxyethylamino)-5,6,7,7a-tetrahydro-3aH-pyrano[3,2-d]thiazole-6,7-diol;
(3aR,5R,6S,7R,7aR)-5-((S)-1-hydroxyethyl)-2-(2-hydroxyethylamino)-5,6,7,7a-tetrahydro-3aH-pyrano[3,2-d]thiazole-6,7-diol;
(3aR,5S,6S,7R,7aR)-2-amino-5-[(1S)-2,2,2-trifluoro-1-hydroxyethyl]-3aH,5H,6H,7H,7aH-pyrano[3,2-d][1,3]thiazole-6,7-diol;
(3aR,5S,6S,7R,7aR)-2-amino-5-[(1R)-2,2,2-trifluoro-1-hydroxyethyl]-3aH,5H,6H,7H,7aH-pyrano[3,2-d][1,3]thiazole-6,7-diol;
(3aR,5S,6S,7R,7aR)-2-(allylamino)-5-((S)-2,2,2-trifluoro-1-hydroxyethyl)-5,6,7,7a-tetrahydro-3aH-pyrano[3,2-d]thiazole-6,7-diol;

(3aR,S,6S,7R,7aR)-2-(allylamino)-5-((R)-2,2,2-trifluoro-1-hydroxyethyl)-5,6,7,7a-tetrahydro-3aH-pyrano[3,2-d]thiazole-6,7-diol;
(3aR,5R,6S,7R,7aR)-2-(allylamino)-5-((R)-1-hydroxyethyl)-5,6,7,7a-tetrahydro-3aH-pyrano[3,2-d]thiazole-6,7-diol;
(3aR,5R,6S,7R,7aR)-2-(allylamino)-5-((S)-1-hydroxyethyl)-5,6,7,7a-tetrahydro-3aH-pyrano[3,2-d]thiazole-6,7-diol;
(3aR,5S,6S,7R,7aR)-2-(allylamino)-5-((S)-1,1,1-trifluoro-2-hydroxypropan-2-yl)-5,6,7,7a-tetrahydro-3aH-pyrano[3,2-d]thiazole-6,7-diol;
(3aR,5S,6S,7R,7aR)-2-(allylamino)-5-(1,1,1-trifluoro-2-hydroxypropan-2-yl)-5,6,7,7a-tetrahydro-3aH-pyrano[3,2-d]thiazole-6,7-diol;
(3aR,5S,6S,7R,7aR)-2-(2-hydroxyethylamino)-5-((R)-2,2,2-trifluoro-1-hydroxyethyl)-5,6,7,7a-tetrahydro-3aH-pyrano[3,2-d]thiazole-6,7-diol;
(3aR,5R,6S,7R,7aR)-2-(allylamino)-5-(2-hydroxyethyl)-5,6,7,7a-tetrahydro-3aH-pyrano[3,2-d]thiazole-6,7-diol;
(3aR,5R,6S,7R,7aR)-2-(allylamino)-5-(2,2-difluoroethyl)-5,6,7,7a-tetrahydro-3aH-pyrano[3,2-d]thiazole-6,7-diol;
(3aR,5S,6S,7R,7aR)-2-amino-5-[(2S)-1,1,1-trifluoro-2-hydroxypropan-2-yl]-3aH,5H,6H,7H,7aH-pyrano[3,2-d][1,3]thiazole-6,7-diol;
(3aR,5S,6S,7R,7aR)-2-amino-5-[(2R)-1,1,1-trifluoro-2-hydroxypropan-2-yl]-3aH,5H,6H,7H,7aH-pyrano[3,2-d][1,3]thiazole-6,7-diol;
(3aR,5R,6S,7R,7aR)-2-amino-5-vinyl-5,6,7,7a-tetrahydro-3aH-pyrano[3,2-d]thiazole-6,7-diol;
(3aR,5R,6S,7R,7aR)-2-amino-5-ethyl-5,6,7,7a-tetrahydro-3aH-pyrano[3,2-d]thiazole-6,7-diol;
(3aR,5S,6S,7R,7aR)-2-(dimethylamino)-5-((R)-1-fluoroethyl)-5,6,7,7a-tetrahydro-3aH-pyrano[3,2-d]thiazole-6,7-diol;
(3aR,5S,6S,7R,7aR)-2-(dimethylamino)-5-((S)-1-fluoroethyl)-5,6,7,7a-tetrahydro-3aH-pyrano[3,2-d]thiazole-6,7-diol;
(3aR,5S,6S,7R,7aR)-5-((R)-1-fluoroethyl)-2-(methylamino)-5,6,7,7a-tetrahydro-3aH-pyrano[3,2-d]thiazole-6,7-diol;
(3aR,5S,6S,7R,7aR)-2-(azetidin-1-yl)-5-((R)-1-fluoroethyl)-5,6,7,7a-tetrahydro-3aH-pyrano[3,2-d]thiazole-6,7-diol;
(3aR,5S,6S,7R,7aR)-2-(dimethylamino)-5-((S)-1,2,2,2-tetrafluoroethyl)-5,6,7,7a-tetrahydro-3aH-pyrano[3,2-d]thiazole-6,7-diol;
(3aR,5S,6S,7R,7aR)-5-((S)-1-fluoroethyl)-2-(methylamino)-5,6,7,7a-tetrahydro-3aH-pyrano[3,2-d]thiazole-6,7-diol;
(3aR,5S,6S,7R,7aR)-2-(azetidin-1-yl)-5-((S)-l-fluoroethyl)-5,6,7,7a-tetrahydro-3aH-pyrano[3,2-d]thiazole-6,7-diol;
(3aR,5S,6S,7R,7aR)-2-(dimethylammo)-5-((R)-1,2,2,2-tetrafluoroethyl)-5,6,7,7a-tetrahydro-3aH-pyrano[3,2-d]thiazole-6,7-diol;
(3aR,5R,6S,7R,7aR)-2-(azetidin-1-yl)-5-((R)-1,1,1-trifluoropropan-2-yl)-5,6,7,7a-tetrahydro-3aH-pyrano[3,2-d]thiazole-6,7-diol;
(3aR,5R,6S,7R,7aR)-2-(azetidin-1-yl)-5-((S)-1,1,1-trifluoropropan-2-yl)-5,6,7,7a-tetrahydro-3aH-pyrano[3,2-d]thiazole-6,7-diol;
(3aR,5R,6S,7R,7aR)-2-(dimethylamino)-5-((R)-1,1,1-trifluoropropan-2-yl)-5,6,7,7a-tetrahydro-3aH-pyrano[3,2-d]thiazole-6,7-diol;
(3aR,5R,6S,7R,7aR)-2-(dimethylamino)-5-((S)-1,1,1-trifluoropropan-2-yl)-5,6,7,7a-tetrahydro-3aH-pyrano[3,2-d]thiazole-6,7-diol;
(3aR,5S,6S,7R,7aR)-5-((R)-2,2-difluoro-1-hydroxyethyl)-2-(methylamino)-5,6,7,7a-tetrahydro-3aH-pyrano[3,2-d]thiazole-6,7-diol;
(3aR,5S,6S,7R,7aR)-5-((S)-2-fluoro-1-hydroxyethyl)-2-(methylamino)-5,6,7,7a-tetrahydro-3aH-pyrano[3,2-d]thiazole-6,7-diol;
(3aR,5S,6S,7R,7aR)-5-((R)-2-fluoro-1-hydroxyethyl)-2-(methylamino)-5,6,7,7a-tetrahydro-3aH-pyrano[3,2-d]thiazole-6,7-diol;
(3aR,5R,6S,7R,7aR)-5-(2-fluoroethyl)-2-(methylamino)-5,6,7,7a-tetrahydro-3aH-pyrano[3,2-d]thiazole-6,7-diol;
(3aR,5R,6S,7R,7aR)-5-(2,2-difluoroethyl)-2-(methylamino)-5,6,7,7a-tetrahydro-3aH-pyrano[3,2-d]thiazole-6,7-diol;
(3aR,5R,6S,7R,7aR)-2-(methylamino)-5-(2,2,2-trifluoroethyl)-5,6,7,7a-tetrahydro-3aH-pyrano[3,2-d]thiazole-6,7-diol;

(3aR,5R,6S,7R,7aR)-2-(ethylamino)-5-(2,2,2-trifluoroethyl)-5,6,7,7a-tetrahydro-3aH-pyrano[3,2-d]thiazole-6,7-diol;
(3aR,5R,6S,7R,7aR)-2-(propylamino)-5-(2,2,2-trifluoroethyl)-5,6,7,7a-tetrahydro-3aH-pyrano[3,2-d]thiazole-6,7-diol;
(3aR,5S,6S,7R,7aR)-2-(azetidin-1-yl)-5-((S)-1,1,1-trifluoro-2-hydroxypropan-2-yl)-5,6,7,7a-tetrahydro-3aH-pyrano[3,2-d]thiazole-6,7-diol;
(3aR,5S,6S,7R,7aR)-2-(azetidin-1-yl)-5-((R) 1,1,1-trifluoro-2-hydroxypropan-2-yl)-5,6,7,7a-tetrahydro-3aH-pyrano[3,2-d]thiazole-6,7-diol;
(3aR,5S,6S,7R,7aR)-2-(dimethylamino)-5-((S)-1,1,1-trifluoro-2-hydroxypropan-2-yl)-5,6,7,7a-tetrahydro-3aH-pyrano[3,2-d]thiazole-6,7-diol;
(3aR,5S,6S,7R,7aR)-2-(dimethylamino)-5-((R)1,1,1-trifluoro-2-hydroxypropan-2-yl)-5,6,7,7a-tetrahydro-3aH-pyrano[3,2-d]thiazole-6,7-diol;
(3aR,5S,6S,7R,7aR)-2-(dimethylamino)-5-((S)-1,1,1-trifluoro-2-hydroxypropan-2-yl)-5,6,7,7a-tetrahydro-3aH-pyrano[3,2-d]thiazole-6,7-diol;
(3aR,5S,6S,7R,7aR)-2-(ethylamino)-5-((S)-1,1,1-trifluoro-2-hydroxypropan-2-yl)-5,6,7,7a-tetrahydro-3aH-pyrano[3,2-d]thiazole-6,7-diol;
(3aR,5S,6S,7R,7aR)-2-(ethylamino)-5-((R)1,1,1-trifluoro-2-hydroxypropan-2-yl)-5,6,7,7a-tetrahydro-3aH-pyrano[3,2-d]thiazole-6,7-diol;
(3aR,5R,6S,7R,7aR)-5-((R)-1-hydroxyethyl)-2-(isoxazolidin-2-yl)-5,6,7,7a-tetrahydro-3aH-pyrano[3,2-d]thiazole-6,7-diol;
(3aR,5R,6S,7R,7aR)-5-((S)-1-hydroxyethyl)-2-(isoxazolidin-2-yl)-5,6,7,7a-tetrahydro-3aH-pyrano[3,2-d]thiazole-6,7-diol;
(3aR,5R,6S,7R,7aR)-2-(ethylamino)-5-((S)-1-hydroxypropyl)-5,6,7,7a-tetrahydro-3aH-pyrano[3,2-d]thiazole-6,7-diol;
(3aR,5R,6S,7R,7aR)-5-((S)-1-hydroxyheptyl)-2-(methylamino)-5,6,7,7a-tetrahydro-3aH-pyrano[3,2-d]thiazole-6,7-diol;
(3aR,5S,6S,7R,7aR)-2-(ethyl(methyl)amino)-5-((S)-2,2,2-trifluoro-1-hydroxyethyl)-5,6,7,7a-tetrahydro-3aH-pyrano[3,2-d]thiazole-6,7-diol;
(3aR,5S,6S,7R,7aR)-2-(ethyl(methyl)amino)-5-((R)-2,2,2-trifluoro-1-hydroxyethyl)-5,6,7,7a-tetrahydro-3aH-pyrano[3,2-d]thiazole-6,7-diol;
(3aR,5S,6S,7R,7aR)-2-(azetidin-1-yl)-5-((S)-2,2,2-trifluoro-1-hydroxyethyl)-5,6,7,7a-tetrahydro-3aH-pyrano[3,2-d]thiazole-6,7-diol;
(3aR,5S,6S,7R,7aR)-2-(azetidin-1-yl)-5-((R)-2,2,2-trifluoro-1-hydroxyethyl)-5,6,7,7a-tetrahydro-3aH-pyrano[3,2-d]thiazole-6,7-diol;
(3aR,5S,6S,7R,7aR)-2-(2-fluoroethylamino)-5-((R)-2,2,2-trifluoro-1-hydroxyethyl)-5,6,7,7a-tetrahydro-3aH-pyrano[3,2-d]thiazole-6,7-diol;
(3aR,5S,6S,7R,7aR)-2-(2-fluoroethylamino)-5-((S)-2,2,2-trifluoro-1-hydroxyethyl)-5,6,7,7a-tetrahydro-3aH-pyrano[3,2-d]thiazole-6,7-diol;
(3aR,5S,6S,7R,7aR)-2-(dimethylamino)-5-(1,1,1,3,3,3-hexafluoro-2-hydroxypropan-2-yl)-5,6,7,7a-tetrahydro-3aH-pyrano[3,2-d]thiazole-6,7-diol;
(3aR,5R,6S,7R,7aR)-2-(methylamino)-5-((R)-1,1,1-trifluoropropan-2-yl)-5,6,7,7a-tetrahydro-3aH-pyrano[3,2-d]thiazole-6,7-diol; and
(3aR,5R,6S,7R,7aR)-2-(methylamino)-5-((S)-1,1,1-trifluoropropan-2-yl)-5,6,7,7a-tetrahydro-3aH-pyrano[3,2-d]thiazole-6,7-diol;

or a pharmaceutically acceptable salt of any of the foregoing compounds.

**10.** The compound according to Claim 1 wherein: each R is H, R5 is OH, $R^3$ is $C_{1-6}$alkyl, optionally mono-substituted with fluoro or hydroxy, and $R^4$ is H.

**11.** The compound according to Claim 1 wherein: each R is H, $R^5$ is H, $R^3$ is $C_{1-6}$alkyl, optionally mono-substituted with fluoro or hydroxy, and $R^4$ is H or $C_{1-6}$alkyl.

**12.** The compound according to Claim 1 wherein $R^1$ is $C_{1-6}$alkyl, optionally mono-substituted with hydroxy, and $R^2$ is H.

**13.** A pharmaceutical composition comprising a compound according to any of Claims 1 to 12 or a pharmaceutically acceptable salt thereof in combination with a pharmaceutically acceptable carrier.

**14.** A compound of any of claims 1-12 for use in treating a condition selected from one or more of the group consisting of an inflammatory disease, an allergy, asthma, allergic rhinitis, hypersensitivity lung diseases, hypersensitivity pneumonitis, eosinophilic pneumonias, delayed-type hypersensitivity, atherosclerosis, interstitial lung disease (ILD), idiopathic pulmonary fibrosis, ILD associated with rheumatoid arthritis, systemic lupus erythematosus, ankylosing spondylitis, systemic sclerosis, Sjogren's syndrome, polymyositis or dermatomyositis, systemic anaphylaxis or hypersensitivity response, drug allergy, insect sting allergy, autoimmune disease, rheumatoid arthritis, psoriatic arthritis, multiple sclerosis, Guillain-Barré syndrome, systemic lupus erythematosus, myastenia gravis, glomerulonephritis, autoimmune thyroiditis, graft rejection, allograft rejection, graft-versus-host disease, inflammatory bowel disease, Crohn's disease, ulcerative colitis, spondyloarthropathy, scleroderma, psoriasis, T-cell mediated psoriasis, inflammatory dermatosis, dermatitis, eczema, atopic dermatitis, allergic contact dermatitis, urticaria, vasculitis, necrotizing, cutaneous, and hypersensitivity vasculitis, eosinphilic myotis, eosiniphilic fasciitis, solid organ transplant rejection, heart transplant rejection, lung transplant rejection, liver transplant rejection, kidney transplant rejection, pancreas transplant rejection, kidney allograft, lung allograft, epilepsy, pain, fibromyalgia, stroke, neuroprotection.

**15.** A compound of any of claims 1-12 for use in treating a condition selected from the group consisting of a neurodegenerative disease, a tauopathy, cancer and stress.

**Patentansprüche**

**1.** Eine Verbindung der Formel (I) oder ein pharmazeutisch annehmbares Salz davon:

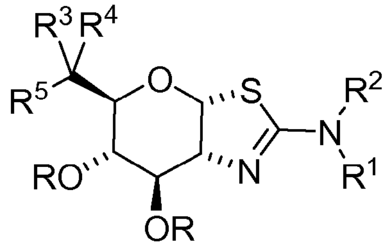

(I),

wobei:

jedes R unabhängig H oder $C(O)CH_3$ ist,
$R^1$ und $R^2$ unabhängig ausgewählt sind aus der Gruppe bestehend aus: H, $C_{1-6}$-Alkyl, $C_{2-6}$-Alkenyl, $C_{1-6}$-Alkoxy, $-(CH_2)_n$-Cyclopropyl und $-(CH_2)_n$-Cyclobutyl, wobei n 0, 1, 2, 3 oder 4 ist, oder $R^1$ und $R^2$ zusammen mit dem Stickstoffatom, an das sie gebunden sind, verbunden sein können unter Bildung von Azetidin, Pyrrolidin, Piperidin oder Isoxazolidin, wobei das $C_{1-6}$-Alkyl, $C_{2-6}$-Alkenyl, $C_{1-6}$-Alkoxy, $-(CH_2)_n$-Cyclopropyl, $-(CH_2)_n$-Cyclobutyl, Azetidin, Pyrrolidin, Piperidin und Isoxazolidin gegebenenfalls monosubstituiert sind mit Fluor, Hydroxy oder Methyl,
$R^3$ ausgewählt ist aus der Gruppe bestehend aus: $C_{1-8}$-Alkyl, $C_{2-8}$-Alkenyl, $C_{2-8}$-Alkinyl, $C_{3-6}$-Cycloalkyl, Aryl und Heteroaryl, wobei jedes davon gegebenenfalls monosubstituiert ist mit Fluor oder OH,
$R^4$ ausgewählt ist aus der Gruppe bestehend aus: H, F, $C_{1-8}$-Alkyl, $C_{2-8}$-Alkenyl und $C_{2-8}$-Alkinyl, wobei jedes davon, außer H oder F, gegebenenfalls monosubstituiert ist mit Fluor oder OH, oder $R^3$ und $R^4$ und das Kohlenstoffatom, an das sie gebunden sind, sich verbinden können unter Bildung von Vinyl oder einem 3- bis 7-gliedrigen carbocyclischen oder heterocyclischen Ring, wobei der 3- bis 7-gliedrige carbocyclische oder heterocyclische Ring gegebenenfalls eine Doppelbindung enthält und gegebenenfalls monosubstituiert sein kann mit Fluor oder OH,

oder ein Verbindung der Formel (Ia) oder ein pharmazeutisch annehmbares Salz davon:

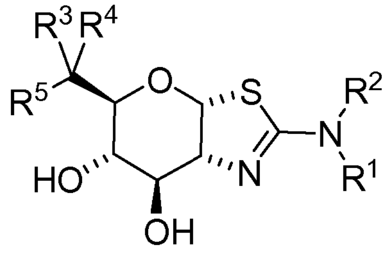

(Ia),

wobei:

$R^1$ und $R^2$ unabhängig ausgewählt sind aus der Gruppe bestehend aus: H, $C_{1-6}$-Alkyl und Cyclopropylmethyl, oder $R^1$ und $R^2$ zusammen mit dem Stickstoffatom, an das sie gebunden sind, verbunden sein können unter Bildung von Azetidin oder Pyrrolidin, wobei das $C_{1-6}$-Alkyl, Cyclopropylmethyl, Azetidin oder Pyrrolidin gegebenenfalls substituiert ist mit 1 bis 3 Substituenten, ausgewählt aus Fluor und Methyl,
$R^3$ ausgewählt ist aus der Gruppe bestehend aus: $C_{1-8}$-Alkyl, $C_{2-8}$-Alkenyl, $C_{2-8}$-Alkinyl und $C_{3-6}$-Cycloalkyl, wobei jedes davon gegebenenfalls substituiert ist mit 1 bis 3 Substituenten, ausgewählt aus Fluor und OH, und
$R^4$ ausgewählt ist aus der Gruppe bestehend aus: H, F, $C_{1-8}$-Alkyl, $C_{2-8}$-Alkenyl und $C_{2-8}$-Alkinyl, wobei jedes davon, außer Wasserstoff, gegebenenfalls substituiert ist mit 1 bis 3 Substituenten, ausgewählt aus Fluor und OH, oder $R^3$ und $R^4$ und das Kohlenstoffatom, an das sie gebunden sind, sich verbinden können unter Bildung eines 5-gliedrigen carbocyclischen Rings, der gegebenenfalls eine Doppelbindung enthält und gegebenenfalls substituiert ist mit 1 bis 3 Substituenten, ausgewählt aus Fluor und OH,
$R^5$ ausgewählt ist aus H, F, OH und $OC(O)CH_3$,

mit der Maßgabe, dass wenn $R^4$ F ist, $R^5$ dann anders als OH und $OC(O)CH_3$ ist.

**2.** Die Verbindung nach Anspruch 1 der Formel (I), wobei $R^1$ und $R^2$ unabhängig ausgewählt sind aus der Gruppe bestehend aus: H, $C_{1-6}$-Alkyl, $C_{1-6}$-Alkoxy, $-(CH_2)_n$-Cyclopropyl und $-(CH_2)_n$-Cyclobutyl, wobei n 0, 1, 2, 3, oder 4 ist, oder $R^1$ und $R^2$ mit dem Stickstoffatom, an das sie gebunden sind, verbunden sein können unter Bildung von Azetidin, Pyrrolidin oder Piperidin, wobei das $C_{1-6}$-Alkyl, $C_{1-6}$-Alkoxy, $-(CH_2)_n$-Cyclopropyl, $-(CH_2)_n$-Cyclobutyl, Azetidin, Pyrrolidin und Piperidin gegebenenfalls monosubstituiert sind mit Fluor oder Methyl.

**3.** Die Verbindung gemäß Anspruch 2, wobei jedes R H ist.

**4.** Die Verbindung gemäß Anspruch 3, wobei:

$R^1$ und $R^2$ unabhängig $C_{1-4}$-Alkyl sind,
$R^3$ $C_{1-6}$-Alkyl ist,
$R^4$ ausgewählt ist aus der Gruppe bestehend aus: H und $C_{1-6}$-Alkyl, und
$R^5$ OH ist.

**5.** Die Verbindung gemäß Anspruch 4, wobei:

$R^1$ und $R^2$ unabhängig Methyl oder Ethyl sind,
$R^3$ Methyl oder Ethyl ist und
$R^4$ ausgewählt ist aus der Gruppe, bestehend aus: H, Methyl und Ethyl.

**6.** Die Verbindung gemäß Anspruch 3, wobei $R^3$ und $R^4$ und das Kohlenstoffatom, an das sie gebunden sind, sich verbinden können unter Bildung eines 3- bis 7-gliedrigen carbocyclischen oder heterocyclischen Rings, wobei der 3- bis 7-gliedrige carbocyclische oder heterocyclische Ring gegebenenfalls eine Doppelbindung enthält und gegebenenfalls monosubstituiert ist mit Fluor oder OH.

**7.** Die Verbindung gemäß Anspruch 1 der Formel (Ia), wobei alle Variablen wie in Anspruch 1 definiert sind.

**8.** Die Verbindung gemäß Anspruch 3, wobei $R^3$ $CF_3$ ist und $R^5$ OH ist.

**9.** Eine Verbindung nach Anspruch 1, ausgewählt aus der folgenden Gruppe:

(3aR,5R,6S,7R,7aR)-2-(Dimethylamino)-5-((S)-1-hydroxyethyl)-5,6,7,7a-tetrahydro-3aH-pyrano[3,2-d]thiazol-6,7-diol,
(3aR,6S,7R,7aR)-5-((S)-1-Hydroxyethyl)-2-(propylamino)-5,6,7,7a-tetrahydro-3aH-pyrano[3,2-d]thiazol-6,7-diol,
(3aR,5S,6S,7R,7aR)-5-(2-Hydroxypropan-2-yl)-2-(methylamino)-5,6,7,7a-tetrahydro-3aH-pyrano[3,2-d]thiazol-6,7-diol,
(3aR,5S,6S,7R,7aR)-2-(Dimethylamino)-5-(2-hydroxypropan-2-yl)-5,6,7,7a-tetrahydro-3aH-pyrano[3,2-d]thiazol-6,7-diol,
(3aR,5R,6S,7R,7aR)-2-(Dimethylamino)-5-(2-hydroxyethyl)-5,6,7,7a-tetrahydro-3aH-pyrano[3,2-d]thiazol-6,7-diol,
(3aR,5R,6S,7R,7aR)-2-(Dimethylamino)-5-ethyl-5,6,7,7a-tetrahydro-3aH-pyrano[3,2-d]thiazol-6,7-diol,
(3aR,5R,6S,7R,7aR)-2-(Dimethylamino)-5-vinyl-5,6,7,7a-tetrahydro-3aH-pyrano[3,2-d]thiazol-6,7-diol,
(3aR,5R,6S,7R,7aR)-5-Ethyl-2-(methylamino)-5,6,7,7a-tetrahydro-3aH-pyrano[3,2-d]thiazol-6,7-diol,
(3aR,5S,6S,7R,7aR)-2-(Dimethylamino)-5-((S)-1-fluorethyl)-5,6,7,7a-tetrahydro-3aH-pyrano[3,2-d]thiazol-6,7-diol,
(3aR,5S,6S,7R,7aR)-2-(Dimethylamino)-5-((S)-2,2,2-trifluor-1-hydroxyethyl)-5,6,7,7a-tetrahydro-3aH-pyrano[3,2-d]thiazol-6,7-diol,
(3aR,5S,6S,7R,7aR)-2-(Dimethylamino)-5-((R)-2,2,2-trifluor-1-hydroxyethyl)-5,6,7,7a-tetrahydro-3aH-pyrano[3,2-d]thiazol-6,7-diol,
(3aR,5S,6S,7R,7aR)-5-(1,1-Difluorethyl)-2-(dimethylamino)-5,6,7,7a-tetrahydro-3aH-pyrano[3,2-d]thiazol-6,7-diol,
(3aR,5R,6S,7R,7aR)-5-((S)-1-Hydroxyethyl)-2-(methylamino)-5,6,7,7a-tetrahydro-3aH-pyrano[3,2-d]thiazol-6,7-diol,
(3aR,5R,6S,7R,7aR)-5-((R)-1-Hydroxyethyl)-2-(methylamino)-5,6,7,7a-tetrahydro-3aH-pyrano[3,2-d]thiazol-6,7-diol,
(3aR,5R,6S,7R,7aR)-5-((S)-1-Hydroxypropyl)-2-(methylamino)-5,6,7,7a-tetrahydro-3aH-pyrano[3,2-d]thiazol-6,7-diol,
(3aR,5R,6S,7R,7aR)-5-((R)-1-Hydroxypropyl)-2-(methylamino)-5,6,7,7a-tetrahydro-3aH-pyrano[3,2-d]thiazol-6,7-diol,
(3aR,5R,6S,7R,7aR)-5-(1-Hydroxy-2-methylpropyl)-2-(methylamino)-5,6,7,7a-tetrahydro-3aH-pyrano[3,2-d]thiazol-6,7-diol,
(3aR,5R,6S,7R,7aR)-5-(Cyclopropyl(hydroxy)methyl)-2-(methylamino)-5,6,7,7a-tetrahydro-3aH-pyrano[3,2-d]thiazol-6,7-diol,
(3aR,5R,6S,7R,7aR)-5-(Hydroxy(pyridin-3-yl)methyl)-2-(methylamino)-5,6,7,7a-tetrahydro-3aH-pyrano[3,2-d]thiazol-6,7-diol,
(3aR,5R,6S,7R,7aR)-5-(Hydroxy(phenyl)methyl)-2-(methylamino)-5,6,7,7a-tetrahydro-3aH-pyrano[3,2-d]thiazol-6,7-diol,
(3aR,5R,6S,7R,7aR)-5-((R)-1-Hydroxyallyl)-2-(methylamino)-5,6,7,7a-tetrahydro-3aH-pyrano[3,2-d]thiazol-6,7-diol,
(3aR,5R,6S,7R,7aR)-5-((S)-1-Hydroxyallyl)-2-(methylamino)-5,6,7,7a-tetrahydro-3aH-pyrano[3,2-d]thiazol-6,7-diol,
(3aR,5R,6S,7R,7aR)-5-((R)-1-Hydroxy-2-methylallyl)-2-(methylamino)-5,6,7,7a-tetrahydro-3aH-pyrano[3,2-d]thiazol-6,7-diol,
(3aR,5R,6S,7R,7aR)-5-((R)-1-Hydroxybut-3-enyl)-2-(methylamino)-5,6,7,7a-tetrahydro-3aH-pyrano[3,2-d]thiazol-6,7-diol,
(3aR,5R,6S,7R,7aR)-5-((S)-1-Hydroxybut-3-enyl)-2-(methylamino)-5,6,7,7a-tetrahydro-3aH-pyrano[3,2-d]thiazol-6,7-diol,
(3aR,5R,6S,7R,7aR)-2-(Ethylamino)-5-((R)-1-hydroxyethyl)-5,6,7,7a-tetrahydro-3aH-pyrano[3,2-d]thiazol-6,7-diol,
(3aR,5R,6S,7R,7aR)-2-(Ethylamino)-5-((S)-1-hydroxyethyl)-5,6,7,7a-tetrahydro-3aH-pyrano[3,2-d]thiazol-6,7-diol,
(3aR,5R,6S,7R,7aR)-5-((S)-Cyclopropyl(hydroxy)methyl)-2-(ethylamino)-5,6,7,7a-tetrahydro-3aH-pyrano[3,2-d]thiazol-6,7-diol,
(3aR,5R,6S,7R,7aR)-2-(Ethylamino)-5-(1-hydroxy-2-methylpropyl)-5,6,7,7a-tetrahydro-3aH-pyrano[3,2-d]thiazol-6,7-diol,
(3aR,5R,6S,7R,7aR)-2-(Ethylamino)-5-(hydroxy(phenyl)methyl)-5,6,7,7a-tetrahydro-3aH-pyrano[3,2-d]thiazol-6,7-diol,
(3aR,5R,6S,7R,7aR)-2-(Ethylamino)-5-((R)-1-hydroxyallyl)-5,6,7,7a-tetrahydro-3aH-pyrano[3,2-d]thiazol-6,7-diol,
(3aR,5R,6S,7R,7aR)-2-(Ethylamino)-5-((S)-1-hydroxyallyl)-5,6,7,7a-tetrahydro-3aH-pyrano[3,2-d]thiazol-6,7-diol,
(3aR,5R,6S,7R,7aR)-2-(Ethylamino)-5-(2-hydroxypropan-2-yl)-5,6,7,7a-tetrahydro-3aH-pyrano[3,2-d]thiazol-6,7-diol,
(3aR,5R,6S,7R,7aR)-2-(Ethylamino)-5-((R)-1-hydroxy-2-methylallyl)-5,6,7,7a-tetrahydro-3aH-pyrano[3,2-d]thiazol-6,7-diol,
(3aR,5R,6S,7R,7aR)-2-(Ethylamino)-5-((R)-1-hydroxybutyl)-5,6,7,7a-tetrahydro-3aH-pyrano[3,2-d]thiazol-6,7-diol,
(3aR,5R,6S,7R,7aR)-2-(Ethylamino)-5-((S)-1-hydroxybutyl)-5,6,7,7a-tetrahydro-3aH-pyrano[3,2-d]thiazol-6,7-diol,
(3aR,5R,6S,7R,7aR)-2-(Ethylamino)-5-((R)-1-hydroxypentyl)-5,6,7,7a-tetrahydro-3aH-pyrano[3,2-d]thiazol-6,7-diol,
(3aR,5R,6S,7R,7aR)-2-(Ethylamino)-5-((S)-1-hydroxypentyl)-5,6,7,7a-tetrahydro-3aH-pyrano[3,2-d]thiazol-6,7-diol,
(3aR,5R,6S,7R,7aR)-2-(Ethylamino)-5-((R)-1-hydroxybut-3-enyl)-5,6,7,7a-tetrahydro-3aH-pyrano[3,2-d]thiazol-6,7-diol,
(3aR,5R,6S,7R,7aR)-2-(Ethylamino)-5-((S)-1-hydroxybut-3-enyl)-5,6,7,7a-tetrahydro-3aH-pyrano[3,2-d]thiazol-6,7-diol,
(3aR,5S,6S,7R,7aR)-5-(2-Hydroxypropan-2-yl)-2-(propylamino)-5,6,7,7a-tetrahydro-3aH-pyrano[3,2-d]thiazol-6,7-diol,
(3aR,5R,6S,7R,7aR)-5-((S)-1-Hydroxyallyl)-2-(propylamino)-5,6,7,7a-tetrahydro-3aH-pyrano[3,2-d]thiazol-6,7-diol,
(3aR,5R,6S,7R,7aR)-5-((R)-1-Hydroxybutyl)-2-(propylamino)-5,6,7,7a-tetrahydro-3aH-pyrano[3,2-d]thiazol-6,7-diol,
(3aR,5R,6S,7R,7aR)-5-((S)-1-Hydroxybutyl)-2-(propylamino)-5,6,7,7a-tetrahydro-3aH-pyrano[3,2-d]thiazol-6,7-diol,
(3aR,5R,6S,7R,7aR)-5-((R)-1-Hydroxypentyl)-2-(propylamino)-5,6,7,7a-tetrahydro-3aH-pyrano[3,2-d]thiazol-6,7-diol,
(3aR,5R,6S,7R,7aR)-5-((S)-1-Hydroxypentyl)-2-(propylamino)-5,6,7,7a-tetrahydro-3aH-pyrano[3,2-d]thiazol-6,7-diol,
(3aR,5R,6S,7R,7aR)-5-(1-Hydroxypropyl)-2-(propylamino)-5,6,7,7a-tetrahydro-3aH-pyrano[3,2-d]thiazol-6,7-diol,
(3aR,5R,6S,7R,7aR)-5-((R)-1-Hydroxybut-3-enyl)-2-(propylamino)-5,6,7,7a-tetrahydro-3aH-pyrano[3,2-d]thiazol-6,7-diol,
(3aR,5R,6S,7R,7aR)-5-((S)-1-Hydroxybut-3-enyl)-2-(propylamino)-5,6,7,7a-tetrahydro-3aH-pyrano[3,2-d]thiazol-6,7-diol,
(3aR,5R,6S,7R,7aR)-5-((R)-1-Hydroxyethyl)-2-(methoxy(methyl)amino)-5,6,7,7a-tetrahydro-3aH-pyrano[3,2-d]thiazol-6,7-diol,
(3aR,5R,6S,7R,7aR)-5-((S)-1-Hydroxyethyl)-2-(methoxy(methyl)amino)-5,6,7,7a-tetrahydro-3aH-pyrano[3,2-d]thiazol-6,7-diol,
(3aR,5R,6S,7R,7aR)-2-(Cyclopropylmethylamino)-5-((R)-1-hydroxyethyl)-5,6,7,7a-tetrahydro-3aH-pyrano[3,2-d]thiazol-6,7-diol,
(3aR,5R,6S,7R,7aR)-2-(Cyclopropylmethylamino)-5-((S)-1-hydroxyethyl)-5,6,7,7a-tetrahydro-3aH-pyrano[3,2-d]thiazol-6,7-diol,
(3aR,5R,6S,7R,7aR)-2-(Ethyl(methyl)amino)-5-((S)-1-hydroxybut-3-enyl)-5,6,7,7a-tetrahydro-3aH-pyrano[3,2-d]thiazol-6,7-diol,
(3aR,5R,6S,7R,7aR)-2-(Ethyl(methyl)amino)-5-((R)-1-hydroxybut-3-enyl)-5,6,7,7a-tetrahydro-3aH-pyrano[3,2-d]thiazol-6,7-diol,
(3aR,5S,6S,7R,7aR)-2-(Ethyl(methyl)amino)-5-(2-hydroxypropan-2-yl)-5,6,7,7a-tetrahydro-3aH-pyrano[3,2-d]thiazol-6,7-diol,
(3aR,5R,6S,7R,7aR)-2-(Ethyl(methyl)amino)-5-(1-hydroxyethyl)-5,6,7,7a-tetrahydro-3aH-pyrano[3,2-d]thiazol-6,7-diol,
(3aR,5R,6S,7R,7aR)-2-(Ethyl(methyl)amino)-5-((R)-1-hydroxypropyl)-5,6,7,7a-tetrahydro-3aH-pyrano[3,2-d]thiazol-6,7-diol,
(3aR,5R,6S,7R,7aR)-2-(Ethyl(methyl)amino)-5-(1-hydroxypropyl)-5,6,7,7a-tetrahydro-3aH-pyrano[3,2-d]thiazol-6,7-diol,
(3aR,5S,6S,7R,7aR)-2-(Ethyl(methyl)amino)-5-((R)-1-hydroxyallyl)-5,6,7,7a-tetrahydro-3aH-pyrano[3,2-d]thiazol-6,7-diol,
(3aR,5S,6S,7R,7aR)-2-(Ethyl(methyl)amino)-5-(1-hydroxyallyl)-5,6,7,7a-tetrahydro-3aH-pyrano[3,2-d]thiazol-6,7-diol,
(3aR,5R,6S,7R,7aR)-2-(Dimethylamino)-5-((S)-1-hydroxypropyl)-5,6,7,7a-tetrahydro-3aH-pyrano[3,2-d]thiazol-6,7-diol,
(3aR,5R,6S,7R,7aR)-2-(Dimethylamino)-5-((S)-1-hydroxyallyl)-5,6,7,7a-tetrahydro-3aH-pyrano[3,2-d]thiazol-6,7-diol,
(3aR,5R,6S,7R,7aR)-2-(Dimethylamino)-5-((R)-1-hydroxy-2-methylallyl)-5,6,7,7a-tetrahydro-3aH-pyrano[3,2-d]thiazol-6,7-diol,
(3aR,5R,6S,7R,7aR)-2-(Dimethylamino)-5-((S)-1-hydroxy-2-methylallyl)-5,6,7,7a-tetrahydro-3aH-pyrano[3,2-d]thiazol-6,7-diol,
(3aR,5R,6S,7R,7aR)-2-(Dimethylamino)-5-((R)-1-hydroxybut-3-enyl)-5,6,7,7a-tetrahydro-3aH-pyrano[3,2-d]thiazol-6,7-diol,
(3aR,5R,6S,7R,7aR)-2-(Dimethylamino)-5-((S)-1-hydroxybut-3-enyl)-5,6,7,7a-tetrahydro-3aH-pyrano[3,2-d]thiazol-6,7-diol,
(3aR,5R,6S,7R,7aR)-2-(Dimethylamino)-5-((R)-1-hydroxybutyl)-5,6,7,7a-tetrahydro-3aH-pyrano[3,2-d]thiazol-6,7-diol,
(3aR,5R,6S,7R,7aR)-2-(Dimethylamino)-5-((S)-1-hydroxybutyl)-5,6,7,7a-tetrahydro-3aH-pyrano[3,2-d]thiazol-6,7-diol,
(3aR,5R,6S,7R,7aR)-2-(Dimethylamino)-5-((R)-1-hydroxypentyl)-5,6,7,7a-tetrahydro-3aH-pyrano[3,2-d]thiazol-6,7-diol,
(3aR,5R,6S,7R,7aR)-2-(Dimethylamino)-5-((S)-1-hydroxypentyl)-5,6,7,7a-tetrahydro-3aH-pyrano[3,2-d]thiazol-6,7-diol,
(3aR,5R,6S,7R,7aR)-2-(Dimethylamino)-5-((S)-1-hydroxyhexyl)-5,6,7,7a-tetrahydro-3aH-pyrano[3,2-d]thiazol-6,7-diol,
(3aR,5R,6S,7R,7aR)-2-(Dimethylamino)-5-((R)-1-hydroxyheptyl)-5,6,7,7a-tetrahydro-3aH-pyrano[3,2-d]thiazol-6,7-diol,
(3aR,5R,6S,7R,7aR)-2-(Dimethylamino)-5-((S)-1-hydroxyheptyl)-5,6,7,7a-tetrahydro-3aH-pyrano[3,2-d]thiazol-6,7-diol,
(3aR,5R,6S,7R,7aR)-2-(Azetidin-1-yl)-5-((R)-1-hydroxyethyl)-5,6,7,7a-tetrahydro-3aH-pyrano[3,2-d]thiazol-6,7-diol,
(3aR,5R,6S,7R,7aR)-2-(Azetidin-1-yl)-5-((S)-1-hydroxyethyl)-5,6,7,7a-tetrahydro-3aH-pyrano[3,2-d]thiazol-6,7-diol,
(3aR,5S,6S,7R,7aR)-2-(Azetidin-1-yl)-5-(2-hydroxypropan-2-yl)-5,6,7,7a-tetrahydro-3aH-pyrano[3,2-d]thiazol-6,7-diol,
(3aR,5R,6S,7R,7aR)-2-(Azetidin-1-yl)-5-((S)-1-hydroxypropyl)-5,6,7,7a-tetrahydro-3aH-pyrano[3,2-d]thiazol-6,7-diol,
(3aR,5R,6S,7R,7aR)-2-(Azetidin-1-yl)-5-((R)-1-hydroxyallyl)-5,6,7,7a-tetrahydro-3aH-pyrano[3,2-d]thiazol-6,7-diol,
(3aR,5R,6S,7R,7aR)-2-(Azetidin-1-yl)-5-((S)-1-hydroxyallyl)-5,6,7,7a-tetrahydro-3aH-pyrano[3,2-d]thiazol-6,7-diol,
(3aR,5R,6S,7R,7aR)-2-(Azetidin-1-yl)-5-((R)-1-hydroxybut-3-enyl)-5,6,7,7a-tetrahydro-3aH-pyrano[3,2-d]thiazol-6,7-diol,
(3aR,5R,6S,7R,7aR)-2-(Azetidin-1-yl)-5-((S)-1-hydroxybut-3-enyl)-5,6,7,7a-tetrahydro-3aH-pyrano[3,2-d]thiazol-6,7-diol,
(3aR,5S,6S,7R,7aR)-5-(2-Hydroxypropan-2-yl)-2-(methyl(propyl)amino)-5,6,7,7a-tetrahydro-3aH-pyrano[3,2-d]thiazol-6,7-diol,
(3aR,5R,6S,7R,7aR)-5-(1-Hydroxypropyl)-2-(methyl(propyl)amino)-5,6,7,7a-tetrahydro-3aH-pyrano[3,2-d]thiazol-6,7-diol,
(3aR,5R,6S,7R,7aR)-5-((S)-1-Hydroxyethyl)-2-(methyl(propyl)amino)-5,6,7,7a-tetrahydro-3aH-pyrano[3,2-d]thiazol-6,7-diol,
(3aR,5R,6S,7R,7aR)-5-((R)-1-Hydroxyallyl)-2-(methyl(propyl)amino)-5,6,7,7a-tetrahydro-3aH-pyrano[3,2-d]thiazol-6,7-diol,
(3aR,5R,6S,7R,7aR)-5-((S)-1-Hydroxyallyl)-2-(methyl(propyl)amino)-5,6,7,7a-tetrahydro-3aH-pyrano[3,2-d]thiazol-6,7-diol,
(3aR,5R,6S,7R,7aR)-5-((R)-1-Hydroxybut-3-enyl)-2-(methyl(propyl)amino)-5,6,7,7a-tetrahydro-3aH-pyrano[3,2-d]thiazol-6,7-diol,
(3aR,5R,6S,7R,7aR)-5-((S)-1-Hydroxybut-3-enyl)-2-(methyl(propyl)amino)-5,6,7,7a-tetrahydro-3aH-pyrano[3,2-d]thiazol-6,7-diol,
(3aR,5S,6S,7R,7aR)-5-(2-Hydroxypropan-2-yl)-2-(pyrrolidin-1-yl)-5,6,7,7a-tetrahydro-3aH-pyrano[3,2-d]thiazol-6,7-diol,
(3aR,5R,6S,7R,7aR)-5-((S)-1-Hydroxyethyl)-2-(pyrrolidin-1-yl)-5,6,7,7a-tetrahydro-3aH-pyrano[3,2-d]thiazol-6,7-diol,
(3aR,5R,6S,7R,7aR)-5-((R)-1-Hydroxypropyl)-2-(pyrrolidin-1-yl)-5,6,7,7a-tetrahydro-3aH-pyrano[3,2-d]thiazol-6,7-diol,
(3aR,5R,6S,7R,7aR)-5-((S)-1-Hydroxypropyl)-2-(pyrrolidin-1-yl)-5,6,7,7a-tetrahydro-3aH-pyrano[3,2-d]thiazol-6,7-diol,
(3aR,5R,6S,7R,7aR)-5-((S)-1-Hydroxyallyl)-2-(pyrrolidin-1-yl)-5,6,7,7a-tetrahydro-3aH-pyrano[3,2-d]thiazol-6,7-diol,
(3aR,5S,6S,7R,7aR)-2-(Methylamino)-5-((R)-2,2,2-trifluor-1-hydroxyethyl)-5,6,7,7a-tetrahydro-3aH-pyrano[3,2-d]thiazol-6,7-diol,
(3aR,5S,6S,7R,7aR)-2-(Propylamino)-5-((R)-2,2,2-trifluor-1-hydroxyethyl)-5,6,7,7a-tetrahydro-3aH-pyrano[3,2-d]thiazol-6,7-diol,
(3aR,5S,6S,7R,7aR)-2-(Methoxy(methyl)amino)-5-((R)-2,2,2-trifluor-1-hydroxyethyl)-5,6,7,7a-tetrahydro-3aH-pyrano[3,2-d]thiazol-6,7-diol,
(3aR,5S,6S,7R,7aR)-2-(Methylamino)-5-((S)-2,2,2-trifluor-1-hydroxyethyl)-5,6,7,7a-tetrahydro-3aH-pyrano[3,2-d]thiazol-6,7-diol,
(3aR,5S,6S,7R,7aR)-2-(Propylamino)-5-((S)-2,2,2-trifluor-1-hydroxyethyl)-5,6,7,7a-tetrahydro-3aH-pyrano[3,2-d]thiazol-6,7-diol,
(3aR,5S,6S,7R,7aR)-2-(Methoxy(methyl)amino)-5-((S)-2,2,2-trifluor-1-hydroxyethyl)-5,6,7,7a-tetrahydro-3aH-pyrano[3,2-d]thiazol-6,7-diol,
(3aR,5S,6S,7R,7aR)-2-(Ethylamino)-5-((S)-1-fluorethyl)-5,6,7,7a-tetrahydro-3aH-pyrano[3,2-d]thiazol-6,7-diol,
(3aR,5R,6S,7R,7aR)-2-(Dimethylamino)-5-((R)-1-hydroxyethyl)-5,6,7,7a-tetrahydro-3aH-pyrano[3,2-d]thiazol-6,7-diol,
(3aR,5R,6S,7R,7aR)-2-(Ethylamino)-5-((S)-1-hydroxypropyl)-5,6,7,7a-tetrahydro-3aH-pyrano[3,2-d]thiazol-6,7-diol,
(3aR,5R,6S,7R,7aR)-2-(Ethylamino)-5-((R)-1-hydroxypropyl)-5,6,7,7a-tetrahydro-3aH-pyrano[3,2-d]thiazol-6,7-diol,
(3aR,5S,6S,7R,7aR)-5-((S)-1-Fluorethyl)-2-(pyrrolidin-1-yl)-5,6,7,7a-tetrahydro-3aH-pyrano[3,2-d]thiazol-6,7-diol,
(3aR,5R,6S,7R,7aR)-5-((R)-1-Hydroxybutyl)-2-(methylamino)-5,6,7,7a-tetrahydro-3aH-pyrano[3,2-d]thiazol-6,7-diol,
(3aR,5R,6S,7R,7aR)-5-((S)-1-Hydroxybutyl)-2-(methylamino)-5,6,7,7a-tetrahydro-3aH-pyrano[3,2-d]thiazol-6,7-diol,
(3aR,5R,6S,7R,7aR)-5-((R)-1-Hydroxypentyl)-2-(methylamino)-5,6,7,7a-tetrahydro-3aH-pyrano[3,2-d]thiazol-6,7-diol,
(3aR,5R,6S,7R,7aR)-5-((S)-1-Hydroxypentyl)-2-(methylamino)-5,6,7,7a-tetrahydro-3aH-pyrano[3,2-d]thiazol-6,7-diol,
(3aR,5R,6S,7R,7aR)-5-((R)-1-Hydroxyhexyl)-2-(methylamino)-5,6,7,7a-tetrahydropyrano[3,2-d]thiazol-6,7-diol,
(3aR,5R,6S,7R,7aR)-5-((S)-1-Hydroxyhexyl)-2-(methylamino)-5,6,7,7a-tetrahydro-3aH-pyrano[3,2-d]thiazol-6,7-diol,
(3aR,5R,6S,7R,7aR)-5-((R)-1-Hydroxyethyl)-2-(propylamino)-5,6,7,7a-tetrahydro-3aH-pyrano[3,2-d]thiazol-6,7-diol,
(3aR,5R,6S,7R,7aR)-2-(Cyclopropylmethylamino)-5-((S)-1-hydroxy-2-methylallyl)-5,6,7,7a-tetrahydro-3aH-pyrano[3,2-d]thiazol-6,7-diol,
(3aR,5R,6S,7R,7aR)-2-(Cyclopropylmethylamino)-5-((R)-1-hydroxypropyl)-5,6,7,7a-tetrahydro-3aH-pyrano[3,2-d]thiazol-6,7-diol,
(3aR,5R,6S,7R,7aR)-2-(Cyclopropylmethylamino)-5-((S)-1-hydroxypropyl)-5,6,7,7a-tetrahydro-3aH-pyrano[3,2-d]thiazol-6,7-diol,
(3aR,5S,6S,7R,7aR)-2-(Cyclopropylmethylamino)-5-((R)-1-hydroxybut-3-enyl)-5,6,7,7a-tetrahydro-3aH-pyrano[3,2-d]thiazol-6,7-diol,
(3aR,5S,6S,7R,7aR)-2-(Cyclopropylmethylamino)-5-(1-hydroxybut-3-enyl)-5,6,7,7a-tetrahydro-3aH-pyrano[3,2-d]thiazol-6,7-diol,
(3aR,5R,6S,7R,7aR)-2-(Cyclopropylmethylamino)-5-((S)-1-hydroxyallyl)-5,6,7,7a-tetrahydro-3aH-pyrano[3,2-d]thiazol-6,7-diol,
(3aR,5R,6S,7R,7aR)-2-(2-Cyclopropylethylamino)-5-((R)-1-hydroxyethyl)-5,6,7,7a-tetrahydro-3aH-pyrano[3,2-d]thiazol-6,7-diol,
(3aR,5R,6S,7R,7aR)-2-(2-Cyclopropylethylamino)-5-((S)-1-hydroxyethyl)-5,6,7,7a-tetrahydro-3aH-pyrano[3,2-d]thiazol-6,7-diol,
(3aR,5S,6S,7R,7aR)-2-(2-Cyclopropylethylamino)-5-(2-hydroxypropan-2-yl)-5,6,7,7a-tetrahydro-3aH-pyrano[3,2-d]thiazol-6,7-diol,
(3aR,5R,6S,7R,7aR)-2-(Ethyl(methyl)amino)-5-((R)-1-hydroxyethyl)-5,6,7,7a-tetrahydro-3aH-pyrano[3,2-d]thiazol-6,7-diol,
(3aR,5R,6S,7R,7aR)-2-(Ethyl(methyl)amino)-5-((S)-1-hydroxybutyl)-5,6,7,7a-tetrahydro-3aH-pyrano[3,2-d]thiazol-6,7-diol,
(3aR,5R,6S,7R,7aR)-2-(Ethyl(methyl)amino)-5-((S)-1-hydroxypentyl)-5,6,7,7a-tetrahydro-3aH-pyrano[3,2-d]thiazol-6,7-diol,
(3aR,5R,6S,7R,7aR)-2-(Dimethylamino)-5-((R)-1-hydroxyhexyl)-5,6,7,7a-tetrahydro-3aH-pyrano[3,2-d]thiazol-6,7-diol,
(3aR,5R,6S,7R,7aR)-5-((S)-1-Hydroxybutyl)-2-(pyrrolidin-1-yl)-5,6,7,7a-tetrahydro-3aH-pyrano[3,2-d]thiazol-6,7-diol,
(3aR,5R,6S,7R,7aR)-5-((S)-1-Hydroxypentyl)-2-(pyrrolidin-1-yl)-5,6,7,7a-tetrahydro-3aH-pyrano[3,2-d]thiazol-6,7-diol,
(3aR,5S,6S,7R,7aR)-5-(1,1-Difluorethyl)-2-(methoxy(methyl)amino)-5,6,7,7a-tetrahydro-3aH-pyrano[3,2-d]thiazol-6,7-diol,
(3aR,5R,6S,7R,7aR)-5-Ethyl-2-(ethyl(methyl)amino)-5,6,7,7a-tetrahydro-3aH-pyrano[3,2-d]thiazol-6,7-diol,
(3aR,5R,6S,7R,7aR)-2-(Azetidin-1-yl)-5-ethyl-5,6,7,7a-tetrahydro-3aH-pyrano[3,2-d]thiazol-6,7-diol,
(3aR,5R,6S,7R,7aR)-2-(Azetidin-1-yl)-5-vinyl-5,6,7,7a-tetrahydro-3aH-pyrano[3,2-d]thiazol-6,7-diol,
(3aR,5R,6S,7R,7aR)-5-Ethyl-2-(propylamino)-5,6,7,7a-tetrahydro-3aH-pyrano[3,2-d]thiazol-6,7-diol,
(3aR,5R,6S,7R,7aR)-5-Ethyl-2-(ethylamino)-5,6,7,7a-tetrahydro-3aH-pyrano[3,2-d]thiazol-6,7-diol,
(3aR,5R,6S,7R,7aR)-2-(Ethylamino)-5-vinyl-5,6,7,7a-tetrahydro-3aH-pyrano[3,2-d]thiazol-6,7-diol,
(3aR,5S,6S,7R,7aR)-5-(1-Hydroxycyclopentyl)-2-(methylamino)-5,6,7,7a-tetrahydro-3aH-pyrano[3,2-d]thiazol-6,7-diol,
(3aR,5S,6S,7R,7aR)-5-(1-Hydroxycyclopent-3-enyl)-2-(methylamino)-5,6,7,7a-tetrahydro-3aH-pyrano[3,2-d]thiazol-6,7-diol,
(3aR,5S,6S,7R,7aR)-5-(2-Fluorpropan-2-yl)-2-(methylamino)-5,6,7,7a-tetrahydro-3aH-pyrano[3,2-d]thiazol-6,7-diol,
(3aR,5R,6S,7R,7aR)-2-(Methylamino)-5-(prop-1-en-2-yl)-5,6,7,7a-tetrahydro-3aH-pyrano[3,2-d]thiazol-6,7-diol,
(3aR,5R,6S,7R,7aR)-2-(2-Fluorethylamino)-5-((S)-1-hydroxyethyl)-5,6,7,7a-tetrahydro-3aH-pyrano[3,2-d]thiazol-6,7-diol,
(3aR,5R,6S,7R,7aR)-2-(3-Fluorpropylamino)-5-((S)-1-hydroxyethyl)-5,6,7,7a-tetrahydro-3aH-pyrano[3,2-d]thiazol-6,7-diol,
(3aR,5R,6S,7R,7aR)-5-(3-Hydroxypropyl)-2-(methylamino)-5,6,7,7a-tetrahydro-3aH-pyrano[3,2-d]thiazol-6,7-diol,
(3aR,5R,6S,7R,7aR)-5-(2-Hydroxyethyl)-2-(methylamino)-5,6,7,7a-tetrahydro-3aH-pyrano[3,2-d]thiazol-6,7-diol,
(3aR,5S,6S,7R,7aR)-5-(1,1-Difluorethyl)-2-(methylamino)-5,6,7,7a-tetrahydro-3aH-pyrano[3,2-d]thiazol-6,7-diol,
(3aR,5S,6S,7R,7aR)-5-((S)-2,2-Difluor-1-hydroxyethyl)-2-(dimethylamino)-5,6,7,7a-tetrahydro-3aH-pyrano[3,2-d]thiazol-6,7-diol,
(3aR,5S,6S,7R,7aR)-5-((R)-2,2-Difluor-1-hydroxyethyl)-2-(dimethylamino)-5,6,7,7a-tetrahydro-3aH-pyrano[3,2-d]thiazol-6,7-diol,
(3aR,5R,6S,7R,7aR)-5-((S)-1,2-Dihydroxyethyl)-2-(dimethylamino)-5,6,7,7a-tetrahydro-3aH-pyrano[3,2-d]thiazol-6,7-diol,
(3aR,5R,6S,7R,7aR)-5-((R)-1,2-Dihydroxyethyl)-2-(dimethylamino)-5,6,7,7a-tetrahydro-3aH-pyrano[3,2-d]thiazol-6,7-diol,
(3aR,5S,6S,7R,7aR)-2-(Dimethylamino)-5-((S)-2-fluor-1-hydroxyethyl)-5,6,7,7a-tetrahydro-3aH-pyrano[3,2-d]thiazol-6,7-diol,
(3aR,5S,6S,7R,7aR)-2-(Dimethylamino)-5-((R)-2-fluor-1-hydroxyethyl)-5,6,7,7a-tetrahydro-3aH-pyrano[3,2-d]thiazol-6,7-diol,
(3aR,5R,6S,7R,7aR)-2-(Dimethylamino)-5-(2-fluorethyl)-5,6,7,7a-tetrahydro-3aH-pyrano[3,2-d]thiazol-6,7-diol,
(3aR,5R,6S,7R,7aR)-5-(2,2-Difluorethyl)-2-(dimethylamino)-5,6,7,7a-tetrahydro-3aH-pyrano[3,2-d]thiazol-6,7-diol,
(3aR,5R,6S,7R,7aR)-2-(Dimethylamino)-5-(2,2,2-trifluorethyl)-5,6,7,7a-tetrahydro-3aH-pyrano[3,2-d]thiazol-6,7-diol,
(3aR,5S,6S,7R,7aR)-2-(Methylamino)-5-((S)-1,1,1-trifluor-2-hydroxypropan-2-yl)-5,6,7,7a-tetrahydro-3aH-pyrano[3,2-d]thiazol-6,7-diol,
(3aR,5S,6S,7R,7aR)-2-(Methylamino)-5-(1,1,1-trifluor-2-hydroxypropan-2-yl)-5,6,7,7a-tetrahydro-3aH-pyrano[3,2-d]thiazol-6,7-diol,
(3aR,5S,6S,7R,7aR)-2-(Ethylamino)-5-((S)-2,2,2-trifluor-1-hydroxyethyl)-5,6,7,7a-tetrahydro-3aH-pyrano[3,2-d]thiazol-6,7-diol,
(3aR,5S,6S,7R,7aR)-2-(Ethylamino)-5-((R)-2,2,2-trifluor-1-hydroxyethyl)-5,6,7,7a-tetrahydro-3aH-pyrano[3,2-d]thiazol-6,7-diol,
(3aR,5R,6S,7R,7aR)-5-((R)-1-Hydroxyethyl)-2-(2-hydroxyethylamino)-5,6,7,7a-tetrahydro-3aH-pyrano[3,2-d]thiazol-6,7-diol,
(3aR,5R,6S,7R,7aR)-5-((S)-1-Hydroxyethyl)-2-(2-hydroxyethylamino)-5,6,7,7a-tetrahydro-3aH-pyrano[3,2-d]thiazol-6,7-diol,
(3aR,5S,6S,7R,7aR)-2-Amino-5-[(1S)-2,2,2-trifluor-1-hydroxyethyl]-3aH,5H,6H,7H,7aH-pyrano[3,2-d][1,3]thiazol-6,7-diol,
(3aR,5S,6S,7R,7aR)-2-Amino-5-[(1 R)-2,2,2-trifluor-1-hydroxyethyl]-3aH,5H,6H,7H,7aH-pyrano[3,2-d][1,3]thiazol-6,7-diol,
(3aR,5S,6S,7R,7aR)-2-(Allylamino)-5-((S)-2,2,2-trifluor-1-hydroxyethyl)-5,6,7,7a-tetrahydro-3aH-pyrano[3,2-d]thiazol-6,7-diol,
(3aR,5S,6S,7R,7aR)-2-(Allylamino)-5-((R)-2,2,2-trifluor-1-hydroxyethyl)-5,6,7,7a-tetrahydro-3aH-pyrano[3,2-d]thiazol-6,7-diol,
(3aR,5R,6S,7R,7aR)-2-(Allylamino)-5-((R)-1-hydroxyethyl)-5,6,7,7a-tetrahydro-3aH-pyrano[3,2-d]thiazol-6,7-diol,
(3aR,5R,6S,7R,7aR)-2-(Allylamino)-5-((S)-1-hydroxyethyl)-5,6,7,7a-tetrahydro-3aH-pyrano[3,2-d]thiazol-6,7-diol,
(3aR,5S,6S,7R,7aR)-2-(Allylamino)-5-((S)-1,1,1-trifluor-2-hydroxypropan-2-yl)-5,6,7,7a-tetrahydro-3aH-pyrano[3,2-d]thiazol-6,7-diol,
(3aR,5S,6S,7R,7aR)-2-(Allylamino)-5-(1,1,1-trifluor-2-hydroxypropan-2-yl)-5,6,7,7a-tetrahydro-3aH-pyrano[3,2-d]thiazol-6,7-diol,
(3aR,5S,6S,7R,7aR)-2-(2-Hydroxyethylamino)-5-((R)-2,2,2-trifluor-1-hydroxyethyl)-5,6,7,7a-tetrahydro-3aH-pyrano[3,2-d]thiazol-6,7-diol,
(3aR,5R,6S,7R,7aR)-2-(Allylamino)-5-(2-hydroxyethyl)-5,6,7,7a-tetrahydro-3aH-pyrano[3,2-d]thiazol-6,7-diol,
(3aR,5R,6S,7R,7aR)-2-(Allylamino)-5-(2,2-difluorethyl)-5,6,7,7a-tetrahydro-3aH-pyrano[3,2-d]thiazol-6,7-diol,
(3aR,5S,6S,7R,7aR)-2-Amino-5-[(2S)-1,1,1-trifluor-2-hydroxypropan-2-yl]-3aH,5H,6H,7H,7aH-pyrano[3,2-d][1,3]thiazol-6,7-diol,
(3aR,5S,6S,7R,7aR)-2-Amino-5-[(2R)-1,1,1-trifluor-2-hydroxypropan-2-yl]-3aH,5H,6H,7H,7aH-pyrano[3,2-d][1,3]thiazol-6,7-diol,
(3aR,5R,6S,7R,7aR)-2-Amino-5-vinyl-5,6,7,7a-tetrahydro-3aH-pyrano[3,2-d]thiazol-6,7-diol,
(3aR,5R,6S,7R,7aR)-2-Amino-5-ethyl-5,6,7,7a-tetrahydro-3aH-pyrano[3,2-d]thiazol-6,7-diol,
(3aR,5S,6S,7R,7aR)-2-(Dimethylamino)-5-((R)-1-fluorethyl)-5,6,7,7a-tetrahydro-3aH-pyrano[3,2-d]thiazol-6,7-diol,
(3aR,5S,6S,7R,7aR)-2-(Dimethylamino)-5-((S)-1-fluorethyl)-5,6,7,7a-tetrahydro-3aH-pyrano[3,2-d]thiazol-6,7-diol,
(3aR,5S,6S,7R,7aR)-5-((R)-1-Fluorethyl)-2-(methylamino)-5,6,7,7a-tetrahydro-3aH-pyrano[3,2-d]thiazol-6,7-diol,
(3aR,5S,6S,7R,7aR)-2-(Azetidin-1-yl)-5-((R)-1-fluorethyl)-5,6,7,7a-tetrahydro-3aH-pyrano[3,2-d]thiazol-6,7-diol,
(3aR,5S,6S,7R,7aR)-2-(Dimethylamino)-5-((S)-1,2,2,2-tetrafluorethyl)-5,6,7,7a-tetrahydro-3aH-pyrano[3,2-d]thiazol-6,7-diol,
(3aR,5S,6S,7R,7aR)-5-((S)-1-Fluorethyl)-2-(methylamino)-5,6,7,7a-tetrahydro-3aH-pyrano[3,2-d]thiazol-6,7-diol,
(3aR,5S,6S,7R,7aR)-2-(Azetidin-1-yl)-5-((S)-1-fluorethyl)-5,6,7,7a-tetrahydro-3aH-pyrano[3,2-d]thiazol-6,7-diol,
(3aR,5S,6S,7R,7aR)-2-(Dimethylamino)-5-((R)-1,2,2,2-tetrafluorethyl)-5,6,7,7a-tetrahydro-3aH-pyrano[3,2-d]thiazol-6,7-diol,
(3aR,5R,6S,7R,7aR)-2-(Azetidin-1-yl)-5-((R)-1,1,1-trifluorpropan-2-yl)-5,6,7,7a-tetrahydro-3aH-pyrano[3,2-d]thiazol-6,7-diol,
(3aR,5R,6S,7R,7aR)-2-(Azetidin-1-yl)-5-((S)-1,1,1-trifluorpropan-2-yl)-5,6,7,7a-tetrahydro-3aH-pyrano[3,2-d]thiazol-6,7-diol,
(3aR,5R,6S,7R,7aR)-2-(Dimethylamino)-5-((R)-1,1,1-trifluorpropan-2-yl)-5,6,7,7a-tetrahydro-3aH-pyrano[3,2-d]thiazol-6,7-diol,
(3aR,5R,6S,7R,7aR)-2-(Dimethylamino)-5-((S)-1,1,1-trifluorpropan-2-yl)-5,6,7,7a-tetrahydro-3aH-pyrano[3,2-d]thiazol-6,7-diol,
(3aR,5S,6S,7R,7aR)-5-((R)-2,2-Difluor-1-hydroxyethyl)-2-(methylamino)-5,6,7,7a-tetrahydro-3aH-pyrano[3,2-d]thiazol-6,7-diol,
(3aR,5S,6S,7R,7aR)-5-((S)-2-Fluor-1-hydroxyethyl)-2-(methylamino)-5,6,7,7a-tetrahydro-3aH-pyrano[3,2-d]thiazol-6,7-diol,
(3aR,5S,6S,7R,7aR)-5-((R)-2-Fluor-1-hydroxyethyl)-2-(methylamino)-5,6,7,7a-tetrahydro-3aH-pyrano[3,2-d]thiazol-6,7-diol,
(3aR,5R,6S,7R,7aR)-5-(2-Fluorethyl)-2-(methylamino)-5,6,7,7a-tetrahydro-3aH-pyrano[3,2-d]thiazol-6,7-diol,
(3aR,5R,6S,7R,7aR)-5-(2,2-Difluorethyl)-2-(methylamino)-5,6,7,7a-tetrahydro-3aH-pyrano[3,2-d]thiazol-6,7-diol,
(3aR,5R,6S,7R,7aR)-2-(Methylamino)-5-(2,2,2-trifluorethyl)-5,6,7,7a-tetrahydro-3aH-pyrano[3,2-d]thiazol-6,7-diol,
(3aR,5R,6S,7R,7aR)-2-(Ethylamino)-5-(2,2,2-trifluorethyl)-5,6,7,7a-tetrahydro-3aH-pyrano[3,2-d]thiazol-6,7-diol,
(3aR,5R,6S,7R,7aR)-2-(Propylamino)-5-(2,2,2-trifluorethyl)-5,6,7,7a-tetrahydro-3aH-pyrano[3,2-d]thiazol-6,7-diol,
(3aR,5S,6S,7R,7aR)-2-(Azetidin-1-yl)-5-((S)-1,1,1-trifluor-2-hydroxypropan-2-yl)-5,6,7,7a-tetrahydro-3aH-pyrano[3,2-d]thiazol-6,7-diol,
(3aR,5S,6S,7R,7aR)-2-(Azetidin-1-yl)-5-((R)-1,1,1-trifluor-2-hydroxypropan-2-yl)-5,6,7,7a-tetrahydro-3aH-pyrano[3,2-d]thiazol-6,7-diol,
(3aR,5S,6S,7R,7aR)-2-(Dimethylamino)-5-((S)-1,1,1-trifluor-2-hydroxypropan-2-yl)-5,6,7,7a-tetrahydro-3aH-pyrano[3,2-d]thiazol-6,7-diol,
(3aR,5S,6S,7R,7aR)-2-(Dimethylamino)-5-((R)-1,1-trifluor-2-hydroxypropan-2-yl)-5,6,7,7a-tetrahydro-3aH-pyrano[3,2-d]thiazol-6,7-diol,
(3aR,5S,6S,7R,7aR)-2-(Dimethylamino)-5-((S)-1,1,1-trifluor-2-hydroxypropan-2-yl)-5,6,7,7a-tetrahydro-3aH-pyrano[3,2-d]thiazol-6,7-diol,
(3aR,5S,6S,7R,7aR)-2-(Ethylamino)-5-((S)-1,1,1-trifluor-2-hydroxypropan-2-yl)-5,6,7,7a-tetrahydro-3aH-pyrano[3,2-d]thiazol-6,7-diol,
(3aR,5S,6S,7R,7aR)-2-(Ethylamino)-5-((R)-1,1,1-trifluor-2-hydroxypropan-2-yl)-5,6,7,7a-tetrahydro-3aH-pyrano[3,2-d]thiazol-6,7-diol,
(3aR,5R,6S,7R,7aR)-5-((R)-1-Hydroxyethyl)-2-(isoxazolidin-2-yl)-5,6,7,7a-tetrahydro-3aH-pyrano[3,2-d]thiazol-6,7-diol,
(3aR,5R,6S,7R,7aR)-5-((S)-1-Hydroxyethyl)-2-(isoxazolidin-2-yl)-5,6,7,7a-tetrahydro-3aH-pyrano[3,2-d]thiazol-6,7-diol,
(3aR,5R,6S,7R,7aR)-2-(Ethylamino)-5-((S)-1-hydroxypropyl)-5,6,7,7a-tetrahydro-3aH-pyrano[3,2-d]thiazol-6,7-diol,
(3aR,5R,6S,7R,7aR)-5-((S)-1-Hydroxyheptyl)-2-(methylamino)-5,6,7,7a-tetrahydro-3aH-pyrano[3,2-d]thiazol-6,7-diol,
(3aR,5S,6S,7R,7aR)-2-(Ethyl(methyl)amino)-5-((S)-2,2,2-trifluor-1-hydroxyethyl)-5,6,7,7a-tetrahydro-3aH-pyrano[3,2-d]thiazol-6,7-diol,
(3aR,5S,6S,7R,7aR)-2-(Ethyl(methyl)amino)-5-((R)-2,2,2-trifluor-1-hydroxyethyl)-5,6,7,7a-tetrahydro-3aH-pyrano[3,2-d]thiazol-6,7-diol,
(3aR,5S,6S,7R,7aR)-2-(Azetidin-1-yl)-5-((S)-2,2,2-trifluor-1-hydroxyethyl)-5,6,7,7a-tetrahydro-3aH-pyrano[3,2-d]thiazol-6,7-diol,
(3aR,5S,6S,7R,7aR)-2-(Azetidin-1-yl)-5-((R)-2,2,2-trifluor-1-hydroxyethyl)-5,6,7,7a-tetrahydro-3aH-pyrano[3,2-d]thiazol-6,7-diol,
(3aR,5S,6S,7R,7aR)-2-(2-Fluorethylamino)-5-((R)-2,2,2-trifluor-1-hydroxyethyl)-5,6,7,7a-tetrahydro-3aH-pyrano[3,2-d]thiazol-6,7-diol,

(3aR,5S,6S,7R,7aR)-2-(2-Fluorethylamino)-5-((S)-2,2,2-trifluor-1-hydroxyethyl)-5,6,7,7a-tetrahydro-3aH-pyrano[3,2-d]thiazol-6,7-diol,
(3aR,5S,6S,7R,7aR)-2-(Dimethylamino)-5-(1,1,1,3,3,3-hexafluor-2-hydroxypropan-2-yl)-5,6,7,7a-tetrahydro-3aH-pyrano[3,2-d]thiazol-6,7-diol,
(3aR,5R,6S,7R,7aR)-2-(Methylamino)-5-((R)-1,1,1-trifluorpropan-2-yl)-5,6,7,7a-tetrahydro-3aH-pyrano[3,2-d]thiazol-6,7-diol und
(3aR,5R,6S,7R,7aR)-2-(Methylamino)-5-((S)-1,1,1-trifluorpropan-2-yl)-5,6,7,7a-tetrahydro-3aH-pyrano[3,2-d]thiazol-6,7-diol,

oder ein pharmazeutisch annehmbares Salz von einer der obigen Verbindungen.

**10.** Die Verbindung gemäß Anspruch 1, wobei: jedes R H ist, $R^5$ OH ist, $R^3$ $C_{1-6}$-Alkyl ist, gegebenenfalls monosubstituiert mit Fluor oder Hydroxy, und $R^4$ H ist.

**11.** Die Verbindung gemäß Anspruch 1, wobei: jedes R H ist, $R^5$ H ist, $R^3$ $C_{1-6}$-Alkyl ist, gegebenenfalls monosubstituiert mit Fluor oder Hydroxy, und $R^4$ H oder $C_{1-6}$-Alkyl ist.

**12.** Die Verbindung gemäß Anspruch 1, wobei $R^1$ $C_{1-6}$-Alkyl ist, gegebenenfalls monosubstituiert mit Hydroxy, und $R^2$ H ist.

**13.** Eine pharmazeutische Zusammensetzung, umfassend eine Verbindung gemäß einem der Ansprüche 1 bis 12 oder ein pharmazeutisch annehmbares Salz davon in Kombination mit einem pharmazeutisch annehmbaren Träger.

**14.** Eine Verbindung nach einem der Ansprüche 1-12 zur Verwendung bei der Behandlung eines Zustandes, ausgewählt aus einer oder mehreren der Gruppe, bestehend aus einer Entzündungserkrankung, einer Allergie, Asthma, allergischer Rhinitis, Lungenüberempfindlichkeitserkrankungen, hypersensitiver Pneumonitis, eosinophilen Pneumonien, Spättyphypersensitivität, Atherosklerose, interstitieller Lungenerkrankung (ILD), idiopathischer Lungenfibrose, ILD in Verbindung mit Gelenkrheumatismus, systemischem Lupus erythematodes, Spondylitis ankylosans, systemischer Sklerose, Sjögren-Syndrom, Polymyositis oder Dermatomyositis, systemischer Anaphylaxie oder Überempfindlichkeitsreaktion, Arzneimittelallergie, Insektenstichallergie, Autoimmunerkrankung, Gelenkrheumatismus, Psoriasisarthritis, multipler Sklerose, Guillain-Barré-Syndrom, systemischem Lupus erythematodes, Myastenia gravis, Glomerulonephritis, Autoimmunthyreopathie, Transplantatabstoßung, Allotransplantatabstoßung, Graft-versus-Host-Erkrankung, entzündlicher Darmerkrankung, Morbus Crohn, Colitis ulcerosa, Spondyloarthropathie, Sklerodermie, Psoriasis, T-Zell-vermittelter Psoriasis, entzündlicher Dermatose, Dermatitis, Ekzem, atopischer Dermatitis, allergischer Kontaktdermatitis, Urtikaria, Vaskulitis, nekrotisierender, kutaner und Hypersensitivitäts-Vaskulitis, eosinophiler Myositis, eosinophiler Fasziitis, Organtransplantatabstoßung, Herztransplantatabstoßung, Lungentransplantatabstoßung, Lebertransplantatabstoßung, Nierentransplantatabstoßung, Bauchspeicheldrüsentransplantatabtoßung, Nierenallotransplantat, Lungenallotransplantat, Epilepsie, Schmerz, Fibromyalgie, Schlaganfall, Neuroprotektion.

**15.** Eine Verbindung nach einem der Ansprüche 1-12 zur Verwendung bei der Behandlung eines Zustandes, ausgewählt aus der Gruppe bestehend aus einer neurodegenerativen Erkrankung, einer Tauopathie, Krebs und Stress.

## Revendications

**1.** Composé de Formule (I) ou un sel pharmaceutiquement acceptable de celui-ci:

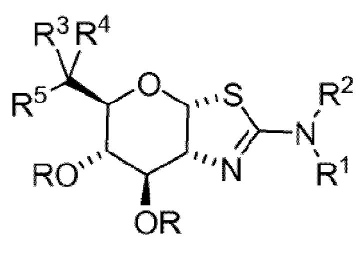

(I)

où:

chaque R est indépendamment H ou $C(O)CH_3$;

$R^1$ et $R^2$ sont indépendamment choisis dans le groupe consistant en: H, $C_{1-6}$alkyle, $C_{2-6}$alcényle, $C_{1-6}$alcoxy, $-(CH_2)_n$-cyclopropyle et $-(CH_2)_n$-cyclobutyle où n est 0, 1, 2, 3 ou 4; ou $R^1$ et $R^2$ peuvent être joints conjointement avec l'atome d'azote auquel ils sont attachés pour former azétidine, pyrrolidine, pipéridine ou isoxazolidine, lesdits $C_{1-6}$alkyle, $C_{2-6}$alcényle, $C_{1-6}$alcoxy, $-(CH_2)_n$-cyclopropyle, $-(CH_2)_n$-cyclobutyle, azétidine, pyrrolidine, pipéridine et isoxazolidine étant facultativement monosubstitués par fluoro, hydroxy ou méthyle;

$R^3$ est choisi dans le groupe consistant en: $C_{1-8}$alkyle, $C_{2-8}$alcényle, $C_{2-8}$alcynyle, $C_{3-6}$cycloalkyle, aryle et hétéroaryle, chacun facultativement monosubstitué par fluoro ou OH;

$R^4$ est choisi dans le groupe consistant en: H, F, $C_{1-8}$alkyle, $C_{1-8}$alcényle et $C_{2-8}$alcynyle, chacun, mis à part H ou F, facultativement monosubstitué par fluoro ou OH; ou $R^3$ et $R^4$ et l'atome de carbone auquel ils sont attachés peuvent se joindre ensemble pour former un vinyle ou un cycle carbocyclique ou hétérocyclique à 3 à 7 chaînons, ledit cycle carbocyclique ou hétérocyclique à 3 à 7 chaînons contenant facultativement une liaison double et étant facultativement monosubstitué par fluoro ou OH;

ou un composé de Formule (Ia) ou un sel pharmaceutiquement acceptable de celui-ci:

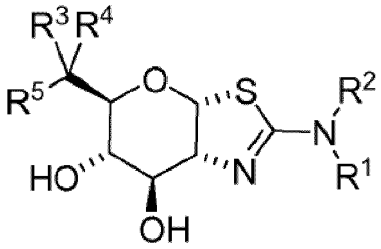

(Ia)

où:

$R^1$ et $R^2$ sont indépendamment choisis dans le groupe consistant en: H, $C_{1-6}$alkyle et cyclopropylméthyle; ou $R^1$ et $R^2$ peuvent être joints conjointement avec l'atome d'azote auquel ils sont attachés pour former azétidine ou pyrrolidine, ledit $C_{1-6}$alkyle, cyclopropylméthyle, azétidine ou pyrrolidine étant facultativement substitué par 1 à 3 substituants choisis parmi fluoro et méthyle;

$R^3$ est choisi dans le groupe consistant en: $C_{1-8}$alkyle, $C_{2-8}$alcényle, $C_{2-8}$alcynyle et $C_{3-6}$cycloalkyle, chacun facultativement substitué par 1 à 3 substituants choisis parmi fluoro et OH; et

$R^4$ est choisi dans le groupe consistant en: H, F, $C_{1-8}$alkyle, $C_{2-8}$alcényle et $C_{2-8}$alcynyle, chacun, à l'exclusion de l'hydrogène, étant facultativement substitué par 1 à 3 substituants choisis parmi fluoro et OH; ou $R^3$ et $R^4$ et l'atome de carbone auquel ils sont attachés peuvent se joindre ensemble pour former un cycle carbocyclique à 5 chaînons contenant facultativement une liaison double et étant facultativement substitué par 1 à 3 substituants choisis parmi fluoro et OH;

$R^5$ est choisi parmi H, F, OH et $OC(O)CH_3$;

avec pour condition que lorsque $R^4$ est F alors $R^5$ est autre que OH et $OC(O)CH_3$.

**2.** Composé selon la revendication 1 de Formule (I), dans lequel $R^1$ et $R^2$ sont indépendamment choisis dans le groupe consistant en: H, $C_{1-6}$alkyle, $C_{1-6}$alcoxy, $-(CH_2)_n$-cyclopropyle et $-(CH_2)_n$-cyclobutyle où n est 0, 1, 2, 3 ou 4; ou $R^1$ et $R^2$ peuvent être joints conjointement avec l'atome d'azote auquel ils sont attachés pour former azétidine, pyrrolidine ou pipéridine, lesdits $C_{1-6}$alkyle, $C_{1-6}$alcoxy, $-(CH_2)_n$-cyclopropyle, $-(CH_2)_n$-cyclobutyle, azétidine, pyrrolidine et pipéridine étant facultativement monosubstitués par fluoro ou méthyle.

**3.** Composé selon la revendication 2, dans lequel chaque R est H.

**4.** Composé selon la revendication 3, dans lequel:

$R^1$ et $R^2$ sont indépendamment $C_{1-4}$alkyle;

$R^3$ est $C_{1-6}$alkyle;
$R^4$ est choisi dans le groupe consistant en: H et $C_{1-6}$alkyle; et
$R^5$ est OH.

**5.** Composé selon la revendication 4, dans lequel:

$R^1$ et $R^2$ sont indépendamment méthyle ou éthyle;
$R^3$ est méthyle ou éthyle; et
$R^4$ est choisi dans le groupe consistant en: H, méthyle et éthyle.

**6.** Composé selon la revendication 3, dans lequel $R^3$ et $R^4$ et l'atome de carbone auquel ils sont attachés peuvent se joindre ensemble pour former un cycle carbocyclique ou hétérocyclique à 3 à 7 chaînons, ledit cycle carbocyclique ou hétérocyclique à 3 à 7 chaînons contenant facultativement une liaison double et étant facultativement monosubstitué par fluoro ou OH.

**7.** Composé selon la revendication 1 de Formule (Ia), dans lequel toutes les variables sont telles que définies dans la revendication 1.

**8.** Composé selon la revendication 3, dans lequel $R^3$ est $CF_3$ et $R^5$ est OH.

**9.** Composé selon la revendication 1, choisi dans le groupe suivant:

(3aR,5R,6S,7R,7aR)-2-(diméthylamino)-5-((S)-1-hydroxyéthyl)-5,6,7,7a-tétrahydro-3aH-pyrano[3,2-d]thiazole-6,7-diol;
(3aR,6S,7R,7aR)-5-((S)-1-hydroxyéthyl)-2-(propylamino)-5,6,7,7a-tétrahydro-3aH-pyrano[3,2-d]thiazole-6,7-diol;
(3aR,5S,6S,7R,7aR)-5-(2-hydroxypropan-2-yl)-2-(méthylamino)-5,6,7,7a-tétrahydro-3aH-pyrano[3,2-d]thiazole-6,7-diol;
(3aR,5S,6S,7R,7aR)-2-(diméthylamino)-5-(2-hydroxypropan-2-yl)-5,6,7,7a-tétrahydro-3aH-pyrano[3,2-d]thiazole-6,7-diol;
(3aR,5R,6S,7R,7aR)-2-(diméthylamino)-5-(2-hydroxyéthyl)-5,6,7,7a-tétrahydro-3aH-pyrano[3,2-d]thiazole-6,7-diol;
(3aR,5R,6S,7R,7aR)-2-(diméthylamino)-5-éthyl-5,6,7,7a-tétrahydro-3aH-pyrano [3,2-d]thiazole-6,7-diol;
(3aR,5R,6S,7R,7aR)-2-(diméthylamino)-5-vinyl-5,6,7,7a-tétrahydro-3aH-pyrano [3,2-d]thiazole-6,7-diol;
(3aR,5R,6S,7R,7aR)-5-éthyl-2-(méthylamino)-5,6,7,7a-tétrahydro-3aH-pyrano [3,2-d]thiazole-6,7-diol;
(3aR,5S,6S,7R,7aR)-2-(diméthylamino)-5-((S)-1-fluoroéthyl)-5,6,7,7a-tétrahydro-3aH-pyrano[3,2-d]thiazole-6,7-diol;
(3aR,5S,6S,7R,7aR)-2-(diméthylamino)-5-((S)-2,2,2-trifluoro-1-hydroxyéthyl)-5,6,7,7a-tétrahydro-3aH-pyrano[3,2-d]thiazole-6,7-diol;
(3aR,5S,6S,7R,7aR)-2-(diméthylamino)-5-((R)-2,2,2-trifluoro-1-hydroxyéthyl)-5,6,7,7a-tétrahydro-3aH-pyrano[3,2-d]thiazole-6,7-diol;
(3aR,5S,6S,7R,7aR)-5-(1,1-difluoroéthyl)-2-(diméthylamino)-5,6,7,7a-tétrahydro-3aH-pyrano[3,2-d]thiazole-6,7-diol;
(3aR,5R,6S,7R,7aR)-5-((S)-1-hydroxyéthyl)-2-(méthylamino)-5,6,7,7a-tétrahydro -3aH-pyrano[3,2-d]thiazole-6,7-diol;
(3aR,5R,6S,7R,7aR)-5-((R)-1-hydroxyéthyl)-2-(méthylamino)-5,6,7,7a-tétrahydro -3aH-pyrano[3,2-d]thiazole-6,7-diol;
(3aR,5R,6S,7R,7aR)-5-((S)-1-hydroxypropyl)-2-(méthylamino)-5,6,7,7a-tétrahydro-3aH-pyrano[3,2-d]thiazole-6,7-diol;
(3aR,5R,6S,7R,7aR)-5-((R)-1-hydroxypropyl)-2-(méthylamino)-5,6,7,7a-tétrahydro-3aH-pyrano[3,2-d]thiazole-6,7-diol;
(3aR,5R,6S,7R,7aR)-5-(1-hydroxy-2-méthylpropyl)-2-(méthylamino)-5,6,7,7a-tétrahydro-3aH-pyrano[3,2-d]thiazole-6,7-diol;
(3aR,5R,6S,7R,7aR)-5-(cyclopropyl(hydroxy)méthyl)-2-(méthylamino)-5,6,7,7a-tétrahydro-3aH-pyrano[3,2-d]thiazole-6,7-diol;
(3aR,5R,6S,7R,7aR)-5-(hydroxy(pyridin-3-yl)méthyl)-2-(méthylamino)-5,6,7,7a-tétrahydro-3aH-pyrano[3,2-d]thiazole-6,7-diol;
(3aR,5R,6S,7R,7aR)-5-(hydroxy(phényl)méthyl)-2-(méthylamino)-5,6,7,7a-tétrahydro-3aH-pyrano[3,2-d]thiazole-6,7-diol;
(3aR,5R,6S,7R,7aR)-5-((R)-1-hydroxyallyl)-2-(méthylamino)-5,6,7,7a-tétrahydro-3aH- pyrano[3,2-d]thiazole-6,7-diol;
(3aR,5R,6S,7R,7aR)-5-((S)-1-hydroxyallyl)-2-(méthylamino)-5,6,7,7a-tétrahydro-3aH- pyrano[3,2-d]thiazole-6,7-diol;
(3aR,5R,6S,7R,7aR)-5-((R)-1-hydroxy-2-méthylallyl)-2-(méthylamino)-5,6,7,7a-tétrahydro-3aH-pyrano[3,2-d]thiazole-6,7-diol;
(3aR,5R,6S,7R,7aR)-5-((R)-1-hydroxybut-3-ényl)-2-(méthylamino)-5,6,7,7a-tétrahydro-3aH-pyrano[3,2-d]thiazole-6,7-diol;
(3aR,5R,6S,7R,7aR)-5-((S)-1-hydroxybut-3-ényl)-2-(méthylamino)-5,6,7,7a-tétrahydro- 3aH-pyrano[3,2-d]thiazole-6,7-diol;
(3aR,5R,6S,7R,7aR)-2-(éthylamino)-5-((R)-1-hydroxyéthyl)-5,6,7,7a-tétrahydro-3aH- pyrano[3,2-d]thiazole-6,7-diol;
(3aR,5R,6S,7R,7aR)-2-(éthylamino)-5-((S)-1-hydroxyéthyl)-5,6,7,7a-tétrahydro-3aH- pyrano[3,2-d]thiazole-6,7-diol;
(3aR,5R,6S,7R,7aR)-5-((S)-cyclopropyl(hydroxy)méthyl)-2-(éthylamino)-5,6,7,7a - tétrahydro-3aH-pyrano[3,2-d]thiazole-6,7-diol;
(3aR,5R,6S,7R,7aR)-2-(éthylamino)-5-(1-hydroxy-2-méthylpropyl)-5,6,7,7a-tétrahydro- 3aH-pyrano[3,2-d]thiazole-6,7-diol;
(3aR,5R,6S,7R,7aR)-2-(éthylamino)-5-(hydroxy(phényl)méthyl)-5,6,7,7a-tétrahydro- 3aH-pyrano[3,2-d]thiazole-6,7-diol;
(3aR,5R,6S,7R,7aR)-2-(éthylamino)-5-((R)-1-hydroxyallyl)-5,6,7,7a-tétrahydro-3aH- pyrano[3,2-d]thiazole-6,7-diol;
(3aR,5R,6S,7R,7aR)-2-(éthylamino)-5-((S)-1-hydroxyallyl)-5,6,7,7a-tétrahydro-3aH- pyrano[3,2-d]thiazole-6,7-diol;
(3aR,5R,6S,7R,7aR)-2-(éthylamino)-5-(2-hydroxypropan-2-yl)-5,6,7,7a-tétrahydro-3aH- pyrano[3,2-d]thiazole-6,7-diol;
(3aR,5R,6S,7R,7aR)-2-(éthylamino)-5-((R)-1-hydroxy-2-méthylallyl)-5,6,7,7a-tétrahydro-3aH-pyrano[3,2-d]thiazole-6,7-diol;
(3aR,5R,6S,7R,7aR)-2-(éthylamino)-5-((R)-1-hydroxybutyl)-5,6,7,7a-tétrahydro-3aH- pyrano[3,2-d]thiazole-6,7-diol;
(3aR,5R,6S,7R,7aR)-2-(éthylamino)-5-((S)-1-hydroxybutyl)-5,6,7,7a-tétrahydro-3aH- pyrano[3,2-d]thiazole-6,7-diol;
(3aR,5R,6S,7R,7aR)-2-(éthylamino)-5-((R)-1-hydroxypentyl)-5,6,7,7a-tétrahydro-3aH-pyrano[3,2-d]thiazole-6,7-diol;
(3aR,5R,6S,7R,7aR)-2-(éthylamino)-5-((S)-1-hydroxypentyl)-5,6,7,7a-tétrahydro-3aH- pyrano[3,2-d]thiazole-6,7-diol;
(3aR,5R,6S,7R,7aR)-2-(éthylamino)-5-((R)-1-hydroxybut-3-ényl)-5,6,7,7a-tétrahydro-3aH-pyrano[3,2-d]thiazole-6,7-diol;
(3aR,5R,6S,7R,7aR)-2-(éthylamino)-5-((S)-1-hydroxybut-3-ényl)-5,6,7,7a-tétrahydro-3aH-pyrano[3,2-d]thiazole-6,7-diol; (3 aR,S S,6S,7R,7aR)-5-(2-hydroxypropan-2-yl)-2-(propylamino)-5,6,7,7a-tétrahydro-3aH-pyrano[3,2-d]thiazole-6,7-diol;
(3aR,5R,6S,7R,7aR)-5-((S)-1-hydroxyallyl)-2-(propylamino)-5,6,7,7a-tétrahydro-3aH-pyrano[3,2-d]thiazole-6,7-diol;
(3aR,5R,6S,7R,7aR)-5-((R)-1-hydroxybutyl)-2-(propylamino)-5,6,7,7a-tétrahydro -3aH-pyrano[3,2-d]thiazole-6,7-diol;
(3aR,5R,6S,7R,7aR)-5-((S)-1-hydroxybutyl)-2-(propylamino)-5,6,7,7a-tétrahydro-3aH-pyrano[3,2-d]thiazole-6,7-diol;
(3aR,5R,6S,7R,7aR)-5-((R)-1-hydroxypentyl)-2-(propylamino)-5,6,7,7a-tétrahydro-3aH-pyrano[3,2-d]thiazole-6,7-diol;
(3aR,5R,6S,7R,7aR)-5-((S)-1-hydroxypentyl)-2-(propylamino)-5,6,7,7a-tétrahydro-3aH-pyrano[3,2-d]thiazole-6,7-diol;
(3aR,5R,6S,7R,7aR)-5-(1-hydroxypropyl)-2-(propylamino)-5,6,7,7a-tétrahydro-3aH-pyrano[3,2-d]thiazole-6,7-diol;
(3aR,5R,6S,7R,7aR)-5-((R)-1-hydroxybut-3-ényl)-2-(propylamino)-5,6,7,7a-tétrahydro-3aH-pyrano[3,2-d]thiazole-6,7-diol;
(3aR,5R,6S,7R,7aR)-5-((S)-1-hydroxybut-3-ényl)-2-(propylamino)-5,6,7,7a-tétrahydro-3aH-pyrano[3,2-d]thiazole-6,7-diol;

(3aR,5R,6S,7R,7aR)-5-((R)-1-hydroxyéthyl)-2-(méthoxy(méthyl)amino)-5,6,7,7a-tétrahydro-3aH-pyrano[3,2-d]thiazole-6,7-diol;
(3aR,5R,6S,7R,7aR)-5-((S)-1-hydroxyéthyl)-2-(méthoxy(méthyl)amino)-5,6,7,7a-tétrahydro-3aH-pyrano[3,2-d]thiazole-6,7-diol;
(3aR,5R,6S,7R,7aR)-2-(cyclopropylméthylamino)-5-((R)-1-hydroxyéthyl)-5,6,7,7 a- tétrahydro-3aH-pyrano[3,2-d]thiazole-6,7-diol;
(3aR,5R,6S,7R,7aR)-2-(cyclopropylméthylamino)-5-((S)-1-hydroxyéthyl)-5,6,7,7 a- tétrahydro-3aH-pyrano[3,2-d]thiazole-6,7-diol;
(3aR,5R,6S,7R,7aR)-2-(éthyl(méthyl)amino)-5-((S)-1-hydroxybut-3-ényl)-5,6,7,7 a- tétrahydro-3aH-pyrano[3,2-d]thiazole-6,7-diol;
(3aR,5R,6S,7R,7aR)-2-(éthyl(méthyl)amino)-5-((R)-1-hydroxybut-3-ényl)-5,6,7,7 a- tétrahydro-3aH-pyrano[3,2-d]thiazole-6,7-diol;
(3aR,5S,6S,7R,7aR)-2-(éthyl(méthyl)amino)-5-(2-hydroxypropan-2-yl)-5,6,7,7a-tétrahydro-3aH-pyrano[3,2-d]thiazole-6,7-diol;
(3aR,5R,6S,7R,7aR)-2-(éthyl(méthyl)amino)-5-(1-hydroxyéthyl)-5,6,7,7a-tétrahydro-3aH-pyrano[3,2-d]thiazole-6,7-diol;
(3aR,5R,6S,7R,7aR)-2-(éthyl(méthyl)amino)-5-((R)-1-hydroxypropyl)-5,6,7,7a-tétrahydro-3aH-pyrano[3,2-d]thiazole-6,7-diol;
(3aR,5R,6S,7R,7aR)-2-(éthyl(méthyl)amino)-5-(1-hydroxypropyl)-5,6,7,7a-tétrahydro-3aH-pyrano[3,2-d]thiazole-6,7-diol;
(3aR,5S,6S,7R,7aR)-2-(éthyl(méthyl)amino)-5-((R)-1-hydroxyallyl)-5,6,7,7a-tétrahydro-3aH-pyrano[3,2-d]thiazole-6,7-diol;
(3aR,5S,6S,7R,7aR)-2-(éthyl(méthyl)amino)-5-(1-hydroxyallyl)-5,6,7,7a-tétrahydro-3aH-pyrano[3,2-d]thiazole-6,7-diol;
(3aR,5R,6S,7R,7aR)-2-(diméthylamino)-5-((S)-1-hydroxypropyl)-5,6,7,7a-tétrahydro-3aH-pyrano[3,2-d]thiazole-6,7-diol;
(3aR,5R,6S,7R,7aR)-2-(diméthylamino)-5-((S)-1-hydroxyallyl)-5,6,7,7a-tétrahydro-3aH-pyrano[3,2-d]thiazole-6,7-diol;
(3aR,5R,6S,7R,7aR)-2-(diméthylamino)-5-((R)-1-hydroxy-2-méthylallyl)-5,6,7,7a - tétrahydro-3aH-pyrano[3,2-d]thiazole-6,7-diol;
(3aR,5R,6S,7R,7aR)-2-(diméthylamino)-5-((S)-1-hydroxy-2-méthylallyl)-5,6,7,7a - tétrahydro-3aH-pyrano[3,2-d]thiazole-6,7-diol;
(3aR,5R,6S,7R,7aR)-2-(diméthylamino)-5-((R)-1-hydroxybut-3-ényl)-5,6,7,7a-tétrahydro-3aH-pyrano[3,2-d]thiazole-6,7-diol;
(3aR,5R,6S,7R,7aR)-2-(diméthylamino)-5-((S)-1-hydroxybut-3-ényl)-5,6,7,7a-tétrahydro-3aH-pyrano[3,2-d]thiazole-6,7-diol;
(3aR,5R,6S,7R,7aR)-2-(diméthylamino)-5-((R)-1-hydroxybutyl)-5,6,7,7a-tétrahydro-3aH-pyrano[3,2-d]thiazole-6,7-diol;
(3aR,5R,6S,7R,7aR)-2-(diméthylamino)-5-((S)-1-hydroxybutyl)-5,6,7,7a-tétrahydro-3aH-pyrano[3,2-d]thiazole-6,7-diol;
(3aR,5R,6S,7R,7aR)-2-(diméthylamino)-5-((R)-1-hydroxypentyl)-5,6,7,7a-tétrahydro-3aH-pyrano[3,2-d]thiazole-6,7-diol;
(3aR,5R,6S,7R,7aR)-2-(diméthylamino)-5-((S)-1-hydroxypentyl)-5,6,7,7a-tétrahydro-3aH-pyrano[3,2-d]thiazole-6,7-diol;
(3aR,5R,6S,7R,7aR)-2-(diméthylamino)-5-((S)-1-hydroxyhexyl)-5,6,7,7a-tétrahydro-3aH-pyrano[3,2-d]thiazole-6,7-diol;
(3aR,5R,6S,7R,7aR)-2-(diméthylamino)-5-((R)-1-hydroxyheptyl)-5,6,7,7a-tétrahydro-3aH-pyrano[3,2-d]thiazole-6,7-diol;
(3aR,5R,6S,7R,7aR)-2-(diméthylamino)-5-((S)-1-hydroxyheptyl)-5,6,7,7a-tétrahydro-3aH-pyrano[3,2-d]thiazole-6,7-diol;
(3aR,5R,6S,7R,7aR)-2-(azétidin-1-yl)-5-((R)-1-hydroxyéthyl)-5,6,7,7a-tétrahydro-3aH-pyrano[3,2-d]thiazole-6,7-diol;
(3aR,5R,6S,7R,7aR)-2-(azétidin-1-yl)-5-((S)-1-hydroxyéthyl)-5,6,7,7a-tétrahydro-3aH-pyrano[3,2-d]thiazole-6,7-diol;
(3aR,5S,6S,7R,7aR)-2-(azétidin-1-yl)-5-(2-hydroxypropan-2-yl)-5,6,7,7a-tétrahydro-3aH-pyrano[3,2-d]thiazole-6,7-diol;
(3aR,5R,6S,7R,7aR)-2-(azétidin-1-yl)-5-((S)-1-hydroxypropyl)-5,6,7,7a-tétrahydro-3aH-pyrano[3,2-d]thiazole-6,7-diol;

(3aR,5R,6S,7R,7aR)-2-(azétidin-1-yl)-5-((R)-1-hydroxyallyl)-5,6,7,7a-tétrahydro-3aH-pyrano[3,2-d]thiazole-6,7-diol;
(3aR,5R,6S,7R,7aR)-2-(azétidin-1-yl)-5-((S)-1-hydroxyallyl)-5,6,7,7a-tétrahydro-3aH-pyrano[3,2-d]thiazole-6,7-diol;
(3aR,5R,6S,7R,7aR)-2-(azétidin-1-yl)-5-((R)-1-hydroxybut-3-ényl)-5,6,7,7a-tétrahydro-3aH-pyrano[3,2-d]thiazole-6,7-diol;
(3aR,5R,6S,7R,7aR)-2-(azétidin-1-yl)-5-((S)-1-hydroxybut-3-ényl)-5,6,7,7a-tétrahydro-3aH-pyrano[3,2-d]thiazole-6,7-diol;
(3aR,5S,6S,7R,7aR)-5-(2-hydroxypropan-2-yl)-2-(méthyl(propyl)amino)-5,6,7,7a-tétrahydro-3aH-pyrano[3,2-d]thiazole-6,7-diol;
(3aR,5R,6S,7R,7aR)-5-(1-hydroxypropyl)-2-(méthyl(propyl)amino)-5,6,7,7a-tétrahydro-3aH-pyrano[3,2-d]thiazole-6,7-diol;
(3aR,5R,6S,7R,7aR)-5-((S)-1-hydroxyéthyl)-2-(méthyl(propyl)amino)-5,6,7,7a-tétrahydro-3aH-pyrano[3,2-d]thiazole-6,7-diol;
(3aR,5R,6S,7R,7aR)-5-((R)-1-hydroxyallyl)-2-(méthyl(propyl)amino)-5,6,7,7a-tétrahydro-3aH-pyrano[3,2-d]thiazole-6,7-diol;
(3aR,5R,6S,7R,7aR)-5-((S)-1-hydroxyallyl)-2-(méthyl(propyl)amino)-5,6,7,7a-tétrahydro-3aH-pyrano[3,2-d]thiazole-6,7-diol;
(3aR,5R,6S,7R,7aR)-5-((R)-1-hydroxybut-3-ényl)-2-(méthyl(propyl)amino)-5,6,7,7a-tétrahydro-3aH-pyrano[3,2-d]thiazole-6,7-diol;
(3aR,5R,6S,7R,7aR)-5-((S)-1-hydroxybut-3-ényl)-2-(méthyl(propyl)amino)-5,6,7,7a-tétrahydro-3aH-pyrano[3,2-d]thiazole-6,7-diol; (3 aR,S S,6S,7R,7aR)-5-(2-hydroxypropan-2-yl)-2-(pyrrolidin-1-yl)-5,6,7,7a-tétrahydro-3aH-pyrano[3,2-d]thiazole-6,7-diol;
(3aR,5R,6S,7R,7aR)-5-((S)-1-hydroxyéthyl)-2-(pyrrolidin-1-yl)-5,6,7,7a-tétrahydro-3aH-pyrano[3,2-d]thiazole-6,7-diol;
(3aR,5R,6S,7R,7aR)-5-((R)-1-hydroxypropyl)-2-(pyrrolidin-1-yl)-5,6,7,7a-tétrahydro-3aH-pyrano[3,2-d]thiazole-6,7-diol;
(3aR,5R,6S,7R,7aR)-5-((S)-1-hydroxypropyl)-2-(pyrrolidin-1-yl)-5,6,7,7a-tétrahydro-3aH-pyrano[3,2-d]thiazole-6,7-diol;
(3aR,5R,6S,7R,7aR)-5-((S)-1-hydroxyallyl)-2-(pyrrolidin-1-yl)-5,6,7,7a-tétrahydro-3aH-pyrano[3,2-d]thiazole-6,7-diol;
(3aR,5S,6S,7R,7aR)-2-(méthylamino)-5-((R)-2,2,2-trifluoro-1-hydroxyéthyl)-5,6,7,7a-tétrahydro-3aH-pyrano[3,2-d]thiazole-6,7-diol;
(3 aR,S S,6S,7R,7aR)-2-(propylamino)-5-((R)-2,2,2-trifluoro-1-hydroxyéthyl)-5,6,7,7a-tétrahydro-3aH-pyrano[3,2-d]thiazole-6,7-diol;
(3aR,5S,6S,7R,7aR)-2-(méthoxy(méthyl)amino)-5-((R)-2,2,2-trifluoro-1-hydroxy éthyl)-5,6,7,7a-tétrahydro-3aH-pyrano[3,2-d]thiazole-6,7-diol;
(3aR,5S,6S,7R,7aR)-2-(méthylamino)-5-((S)-2,2,2-trifluoro-1-hydroxyéthyl)-5,6,7,7a-tétrahydro-3aH-pyrano[3,2-d]thiazole-6,7-diol;
(3aR,5S,6S,7R,7aR)-2-(propylamino)-5-((S)-2,2,2-trifluoro-1-hydroxyéthyl)-5,6,7,7a-tétrahydro-3aH-pyrano[3,2-d]thiazole-6,7-diol;
(3aR,5S,6S,7R,7aR)-2-(méthoxy(méthyl)amino)-5-((S)-2,2,2-trifluoro-1-hydroxyéthyl)-5,6,7,7a-tétrahydro-3aH-pyrano[3,2-d]thiazole-6,7-diol;
(3aR,5S,6S,7R,7aR)-2-(éthylamino)-5-((S)-1-fluoroéthyl)-5,6,7,7a-tétrahydro-3aH - pyrano[3,2-d]thiazole-6,7-diol;
(3aR,5R,6S,7R,7aR)-2-(diméthylamino)-5-((R)-1-hydroxyéthyl)-5,6,7,7a-tétrahydro-3aH-pyrano[3,2-d]thiazole-6,7-diol;
(3aR,5R,6S,7R,7aR)-2-(éthylamino)-5-((S)-1-hydroxypropyl)-5,6,7,7a-tétrahydro-3aH-pyrano[3,2-d]thiazole-6,7-diol;
(3aR,5R,6S,7R,7aR)-2-(éthylamino)-5-((R)-1-hydroxypropyl)-5,6,7,7a-tétrahydro-3aH-pyrano[3,2-d]thiazole-6,7-diol;
(3aR,5S,6S,7R,7aR)-5-((S)-1-fluoroéthyl)-2-(pyrrolidin-1-yl)-5,6,7,7a-tétrahydro-3aH-pyrano[3,2-d]thiazole-6,7-diol;
(3aR,5R,6S,7R,7aR)-5-((R)-1-hydroxybutyl)-2-(méthylamino)-5,6,7,7a-tétrahydro -3aH-pyrano[3,2-d]thiazole-6,7-diol;
(3aR,5R,6S,7R,7aR)-5-((S)-1-hydroxybutyl)-2-(méthylamino)-5,6,7,7a-tétrahydro -3aH-pyrano[3,2-d]thiazole-6,7-diol;
(3aR,5R,6S,7R,7aR)-5-((R)-1-hydroxypentyl)-2-(méthylamino)-5,6,7,7a-tétrahydro-3aH-pyrano[3,2-d]thiazole-6,7-diol;
(3aR,5R,6S,7R,7aR)-5-((S)-1-hydroxypentyl)-2-(méthylamino)-5,6,7,7a-tétrahydro-3aH-pyrano[3,2-d]thiazole-6,7-diol;
(3aR,5R,6S,7R,7aR)-5-((R)-1-hydroxyhexyl)-2-(méthylamino)-5,6,7,7a-tétrahydro-pyrano[3,2-d]thiazole-6,7-diol;
(3aR,5R,6S,7R,7aR)-5-((S)-1-hydroxyhexyl)-2-(méthylamino)-5,6,7,7a-tétrahydro -3aH-pyrano[3,2-d]thiazole-6,7-diol;
(3aR,5R,6S,7R,7aR)-5-((R)-1-hydroxyéthyl)-2-(propylamino)-5,6,7,7a-tétrahydro-3aH-pyrano[3,2-d]thiazole-6,7-diol;
(3aR,5R,6S,7R,7aR)-2-(cyclopropylméthylamino)-5-((S)-1-hydroxy-2-méthylallyl)-5,6,7,7a-tétrahydro-3aH-pyrano[3,2-d]thiazole-6,7-diol;
(3aR,5R,6S,7R,7aR)-2-(cyclopropylméthylamino)-5-((R)-1-hydroxypropyl)-5,6,7,7a-tétrahydro-3aH-pyrano[3,2-d]thiazole-6,7-diol;
(3aR,5R,6S,7R,7aR)-2-(cyclopropylméthylamino)-5-((S)-1-hydroxypropyl)-5,6,7,7a-tétrahydro-3aH-pyrano[3,2-d]thiazole-6,7-diol;
(3aR,5S,6S,7R,7aR)-2-(cyclopropylméthylamino)-5-((R)-1-hydroxybut-3-ényl)-5,6,7,7a-tétrahydro-3aH-pyrano[3,2-d]thiazole-6,7-diol;
(3aR,5S,6S,7R,7aR)-2-(cyclopropylméthylamino)-5-(1-hydroxybut-3-ényl)-5,6,7,7a-tétra-hydro-3aH-pyrano[3,2-d]thiazole-6,7-diol;
(3aR,5R,6S,7R,7aR)-2-(cyclopropylméthylamino)-5-((S)-1-hydroxyallyl)-5,6,7,7a -tétra-hydro-3aH-pyrano[3,2-d]thiazole-6,7-diol;
(3aR,5R,6S,7R,7aR)-2-(2-cyclopropyléthylamino)-5-((R)-1-hydroxyéthyl)-5,6,7,7a-tétra-hydro-3aH-pyrano[3,2-d]thiazole-6,7-diol;
(3aR,5R,6S,7R,7aR)-2-(2-cyclopropyléthylamino)-5-((S)-1-hydroxyéthyl)-5,6,7,7a-tétra-hydro-3aH-pyrano[3,2-d]thiazole-6,7-diol;
(3aR,5S,6S,7R,7aR)-2-(2-cyclopropyléthylamino)-5-(2-hydroxypropan-2-yl)-5,6,7,7a-tétrahydro-3aH-pyrano[3,2-d]thiazole-6,7-diol;
(3aR,5R,6S,7R,7aR)-2-(éthyl(méthyl)amino)-5-((R)-1-hydroxyéthyl)-5,6,7,7a-tétrahydro-3aH-pyrano[3,2-d]thiazole-6,7-diol;
(3aR,5R,6S,7R,7aR)-2-(éthyl(méthyl)amino)-5-((S)-1-hydroxybutyl)-5,6,7,7a-tétrahydro-3aH-pyrano[3,2-d]thiazole-6,7-diol;
(3aR,5R,6S,7R,7aR)-2-(éthyl(méthyl)amino)-5-((S)-1-hydroxypentyl)-5,6,7,7a-tétra-hydro-3aH-pyrano[3,2-d]thiazole-6,7-diol;
(3aR,5R,6S,7R,7aR)-2-(diméthylamino)-5-((R)-1-hydroxyhexyl)-5,6,7,7a-tétrahydro-3aH-pyrano[3,2-d]thiazole-6,7-diol;
(3aR,5R,6S,7R,7aR)-5-((S)-1-hydroxybutyl)-2-(pyrrolidin-1-yl)-5,6,7,7a-tétrahydro-3aH-pyrano[3,2-d]thiazole-6,7-diol;
(3aR,5R,6S,7R,7aR)-5-((S)-1-hydroxypentyl)-2-(pyrrolidin-1-yl)-5,6,7,7a-tétrahydro-3aH-pyrano[3,2-d]thiazole-6,7-diol;
(3aR,5S,6S,7R,7aR)-5-(1,1-difluoroéthyl)-2-(méthoxy(méthyl)amino)-5,6,7,7a-tétra-hydro-3aH-pyrano[3,2-d]thiazole-6,7-diol;
(3aR,5R,6S,7R,7aR)-5-éthyl-2-(éthyl(méthyl)amino)-5,6,7,7a-tétrahydro-3aH-pyrano[3,2-d]thiazole-6,7-diol;
(3aR,5R,6S,7R,7aR)-2-(azétidin-1-yl)-5-éthyl-5,6,7,7a-tétrahydro-3aH-pyrano [3,2-d]thiazole-6,7-diol;
(3aR,5R,6S,7R,7aR)-2-(azétidin-1-yl)-5-vinyl-5,6,7,7a-tétrahydro-3aH-pyrano [3,2-d]thiazole-6,7-diol;
(3aR,5R,6S,7R,7aR)-5-éthyl-2-(propylamino)-5,6,7,7a-tétrahydro-3aH-pyrano [3,2-d]thiazole-6,7-diol;
(3aR,5R,6S,7R,7aR)-5-éthyl-2-(éthylamino)-5,6,7,7a-tétrahydro-3aH-pyrano [3,2-d]thiazole-6,7-diol;
(3aR,5R,6S,7R,7aR)-2-(éthylamino)-5-vinyl-5,6,7,7a-tétrahydro-3aH-pyrano [3,2-d]thiazole-6,7-diol;
(3aR,5S,6S,7R,7aR)-5-(1-hydroxycyclopentyl)-2-(méthylamino)-5,6,7,7a-tétrahydro-3aH-pyrano[3,2-d]thiazole-6,7-diol;
(3aR,5S,6S,7R,7aR)-5-(1-hydroxycyclopent-3-ényl)-2-(méthylamino)-5,6,7,7a-tétra-hydro-3aH-pyrano[3,2-d]thiazole-6,7-diol;
(3aR,5S,6S,7R,7aR)-5-(2-fluoropropan-2-yl)-2-(méthylamino)-5,6,7,7a-tétrahydro -3aH-pyrano[3,2-d]thiazole-6,7-diol;
(3aR,5R,6S,7R,7aR)-2-(méthylamino)-5-(prop-1-en-2-yl)-5,6,7,7a-tétrahydro-3aH - pyrano[3,2-d]thiazole-6,7-diol;
(3aR,5R,6S,7R,7aR)-2-(2-Fluoroéthylamino)-5-((S)-1-hydroxyéthyl)-5,6,7,7a-tétrahydro-3aH-pyrano[3,2-d]thiazole-6,7-diol;
(3aR,5R,6S,7R,7aR)-2-(3-Fluoropropylamino)-5-((S)-1-hydroxyéthyl)-5,6,7,7a-tétra-hydro-3aH-pyrano[3,2-

d]thiazole-6,7-diol;
(3aR,5R,6S,7R,7aR)-5-(3-Hydroxypropyl)-2-(méthylamino)-5,6,7,7a-tétrahydro-3aH-pyrano[3,2-d]thiazole-6,7-diol;
(3aR,5R,6S,7R,7aR)-5-(2-hydroxyéthyl)-2-(méthylamino)-5,6,7,7a-tétrahydro-3aH-pyrano[3,2-d]thiazole-6,7-diol;
(3aR,5S,6S,7R,7aR)-5-(1,1-difluoroéthyl)-2-(méthylamino)-5,6,7,7a-tétrahydro-3aH-pyrano[3,2-d]thiazole-6,7-diol;
(3aR,5S,6S,7R,7aR)-5-((S)-2,2-difluoro-1-hydroxyéthyl)-2-(diméthylamino)-5,6,7,7a-tétrahydro-3aH-pyrano[3,2-d]thiazole-6,7-diol;
(3aR,5S,6S,7R,7aR)-5-((R)-2,2-difluoro-1-hydroxyéthyl)-2-(diméthylamino)-5,6,7,7a-tétrahydro-3aH-pyrano[3,2-d]thiazole-6,7-diol;
(3aR,5R,6S,7R,7aR)-5-((S)-1,2-dihydroxyéthyl)-2-(diméthylamino)-5,6,7,7a-tétrahydro-3aH-pyrano[3,2-d]thiazole-6,7-diol;
(3aR,5R,6S,7R,7aR)-5-((R)-1,2-dihydroxyéthyl)-2-(diméthylamino)-5,6,7,7a-tétrahydro-3aH-pyrano[3,2-d]thiazole-6,7-diol;
(3aR,5S,6S,7R,7aR)-2-(diméthylamino)-5-((S)-2-fluoro-1-hydroxyéthyl)-5,6,7,7a-tétra-hydro-3aH-pyrano[3,2-d]thiazole-6,7-diol;
(3aR,5S,6S,7R,7aR)-2-(diméthylamino)-5-((R)-2-fluoro-1-hydroxyéthyl)-5,6,7,7a-tétra-hydro-3aH-pyrano[3,2-d]thiazole-6,7-diol;
(3aR,5R,6S,7R,7aR)-2-(diméthylamino)-5-(2-fluoroéthyl)-5,6,7,7a-tétrahydro-3aH-pyrano[3,2-d]thiazole-6,7-diol;
(3aR,5R,6S,7R,7aR)-5-(2,2-difluoroéthyl)-2-(diméthylamino)-5,6,7,7a-tétrahydro-3aH-pyrano[3,2-d]thiazole-6,7-diol;
(3aR,5R,6S,7R,7aR)-2-(diméthylamino)-5-(2,2,2-trifluoroéthyl)-5,6,7,7a-tétrahydro-3aH-pyrano[3,2-d]thiazole-6,7-diol;
(3aR,5S,6S,7R,7aR)-2-(méthylamino)-5-((S)-1,1,1-trifluoro-2-hydroxypropan-2-yl)-5,6,7,7a-tétrahydro-3aH-pyrano[3,2-d]thiazole-6,7-diol;
(3aR,5S,6S,7R,7aR)-2-(méthylamino)-5-(1,1,1-trifluoro-2-hydroxypropan-2-yl)-5,6,7,7a-tétrahydro-3aH-pyrano[3,2-d]thiazole-6,7-diol;
(3aR,5S,6S,7R,7aR)-2-(éthylamino)-5-((S)-2,2,2-trifluoro-1-hydroxyéthyl)-5,6,7,7a-tétrahydro-3aH-pyrano[3,2-d]thiazole-6,7-diol;
(3aR,5S,6S,7R,7aR)-2-(éthylamino)-5-((R)-2,2,2-trifluoro-1-hydroxyéthyl)-5,6,7,7a-tétrahydro-3aH-pyrano[3,2-d]thiazole-6,7-diol;
(3aR,5R,6S,7R,7aR)-5-((R)-1-hydroxyéthyl)-2-(2-hydroxyéthylamino)-5,6,7,7a-tétra-hydro-3aH-pyrano[3,2-d]thiazole-6,7-diol;
(3aR,5R,6S,7R,7aR)-5-((S)-1-hydroxyéthyl)-2-(2-hydroxyéthylamino)-5,6,7,7a-tétra-hydro-3aH-pyrano[3,2-d]thiazole-6,7-diol;
(3aR,5S,6S,7R,7aR)-2-amino-5-[(1S)-2,2,2-trifluoro-1-hydroxyéthyl]-3aH,5H,6H,7H,7aH-pyrano[3,2-d][1,3]thiazole-6,7-diol;
(3aR,5S,6S,7R,7aR)-2-amino-5-[(1R)-2,2,2-trifluoro-1-hydroxyéthyl]-3aH,5H,6H,7H,7aH-pyrano[3,2-d][1,3]thiazole-6,7-diol;
(3aR,5S,6S,7R,7aR)-2-(allylamino)-5-((S)-2,2,2-trifluoro-1-hydroxyéthyl)-5,6,7,7a-tétra-hydro-3aH-pyrano[3,2-d]thiazole-6,7-diol;
(3aR,5S,6S,7R,7aR)-2-(allylamino)-5-((R)-2,2,2-trifluoro-1-hydroxyéthyl)-5,6,7,7a-tétra-hydro-3aH-pyrano[3,2-d]thiazole-6,7-diol;
(3aR,5R,6S,7R,7aR)-2-(allylamino)-5-((R)-1-hydroxyéthyl)-5,6,7,7a-tétrahydro-3aH-pyrano[3,2-d]thiazole-6,7-diol;
(3aR,5R,6S,7R,7aR)-2-(allylamino)-5-((S)-1-hydroxyéthyl)-5,6,7,7a-tétrahydro-3aH-pyrano[3,2-d]thiazole-6,7-diol;
(3aR,5S,6S,7R,7aR)-2-(allylamino)-5-((S)-1,1,1-trifluoro-2-hydroxypropan-2-yl)-5,6,7,7a-tétrahydro-3aH-pyrano[3,2-d]thiazole-6,7-diol;
(3aR,5S,6S,7R,7aR)-2-(allylamino)-5-(1,1,1-trifluoro-2-hydroxypropan-2-yl)-5,6,7,7a-tétrahydro-3aH-pyrano[3,2-d]thiazole-6,7-diol;
(3aR,5S,6S,7R,7aR)-2-(2-hydroxyéthylamino)-5-((R)-2,2,2-trifluoro-1-hydroxyéthyl)-5,6,7,7a-tétrahydro-3aH-pyrano[3,2-d]thiazole-6,7-diol;
(3aR,5R,6S,7R,7aR)-2-(allylamino)-5-(2-hydroxyéthyl)-5,6,7,7a-tétrahydro-3aH-pyrano[3,2-d]thiazole-6,7-diol;
(3aR,5R,6S,7R,7aR)-2-(allylamino)-5-(2,2-difluoroéthyl)-5,6,7,7a-tétrahydro-3aH - pyrano[3,2-d]thiazole-6,7-diol;
(3aR,5S,6S,7R,7aR)-2-amino-5-[(2S)-1,1,1-trifluoro-2-hydroxypropan-2-yl]-3aH,5H,6H,7H,7aH-pyrano[3,2-d][1,3]thiazole-6,7-diol;
(3aR,5S,6S,7R,7aR)-2-amino-5-[(2R)-1,1,1-trifluoro-2-hydroxypropan-2-yl]-3aH,5H,6H,7H,7aH-pyrano[3,2-d][1,3]thiazole-6,7-diol;
(3aR,5R,6S,7R,7aR)-2-amino-5-vinyl-5,6,7,7a-tétrahydro-3aH-pyrano[3,2-d] thiazole-6,7-diol;
(3aR,5R,6S,7R,7aR)-2-amino-5-éthyl-5,6,7,7a-tétrahydro-3aH-pyrano[3,2-d] thiazole-6,7-diol;
(3aR,5S,6S,7R,7aR)-2-(diméthylamino)-5-((R)-1-fluoroéthyl)-5,6,7,7a-tétrahydro-3aH-pyrano[3,2-d]thiazole-6,7-diol;
(3aR,5S,6S,7R,7aR)-2-(diméthylamino)-5-((S)-1-fluoroéthyl)-5,6,7,7a-tétrahydro-3aH-pyrano[3,2-d]thiazole-6,7-diol;
(3aR,5S,6S,7R,7aR)-5-((R)-1-fluoroéthyl)-2-(méthylamino)-5,6,7,7a-tétrahydro-3aH-pyrano[3,2-d]thiazole-6,7-diol;
(3aR,5S,6S,7R,7aR)-2-(azétidin-1-yl)-5-((R)-1-fluoroéthyl)-5,6,7,7a-tétrahydro-3aH-pyrano[3,2-d]thiazole-6,7-diol;
(3aR,5S,6S,7R,7aR)-2-(diméthylamino)-5-((S)-1,2,2,2-tétrafluoroéthyl)-5,6,7,7a-tétra-hydro-3aH-pyrano[3,2-d]thiazole-6,7-diol;
(3aR,5S,6S,7R,7aR)-5-((S)-1-fluoroéthyl)-2-(méthylamino)-5,6,7,7a-tétrahydro-3aH-pyrano[3,2-d]thiazole-6,7-diol;
(3aR,5S,6S,7R,7aR)-2-(azétidin-1-yl)-5-((S)-1-fluoroéthyl)-5,6,7,7a-tétrahydro-3aH-pyrano[3,2-d]thiazole-6,7-diol;
(3aR,5S,6S,7R,7aR)-2-(diméthylamino)-5-((R)-1,2,2,2-tétrafluoroéthyl)-5,6,7,7a-tétra-hydro-3aH-pyrano[3,2-d]thiazole-6,7-diol;
(3aR,5R,6S,7R,7aR)-2-(azétidin-1-yl)-5-((R)-1,1,1-trifluoropropan-2-yl)-5,6,7,7a-tétra-hydro-3aH-pyrano[3,2-d]thiazole-6,7-diol;
(3aR,5R,6S,7R,7aR)-2-(azétidin-1-yl)-5-((S)-1,1,1-trifluoropropan-2-yl)-5,6,7,7a-tétra-hydro-3aH-pyrano[3,2-d]thiazole-6,7-diol;
(3aR,5R,6S,7R,7aR)-2-(diméthylamino)-5-((R)-1,1,1-trifluoropropan-2-yl)-5,6,7,7a-tétrahydro-3aH-pyrano[3,2-d]thiazole-6,7-diol;
(3aR,5R,6S,7R,7aR)-2-(diméthylamino)-5-((S)-1,1,1-trifluoropropan-2-yl)-5,6,7,7a-tétra-hydro-3aH-pyrano[3,2-d]thiazole-6,7-diol;
(3aR,5S,6S,7R,7aR)-5-((R)-2,2-difluoro-1-hydroxyéthyl)-2-(méthylamino)-5,6,7,7a-tétrahydro-3aH-pyrano[3,2-d]thiazole-6,7-diol;
(3aR,5S,6S,7R,7aR)-5-((S)-2-fluoro-1-hydroxyéthyl)-2-(méthylamino)-5,6,7,7a-tétra-hydro-3aH-pyrano[3,2-d]thiazole-6,7-diol;
(3aR,5S,6S,7R,7aR)-5-((R)-2-fluoro-1-hydroxyéthyl)-2-(méthylamino)-5,6,7,7a-tétra-hydro-3aH-pyrano[3,2-d]thiazole-6,7-diol;
(3aR,5R,6S,7R,7aR)-5-(2-fluoroéthyl)-2-(méthylamino)-5,6,7,7a-tétrahydro-3aH-pyrano[3,2-d]thiazole-6,7-diol;
(3aR,5R,6S,7R,7aR)-5-(2,2-difluoroéthyl)-2-(méthylamino)-5,6,7,7a-tétrahydro-3aH-pyrano[3,2-d]thiazole-6,7-diol;
(3aR,5R,6S,7R,7aR)-2-(méthylamino)-5-(2,2,2-trifluoroéthyl)-5,6,7,7a-tétrahydro-3aH-pyrano[3,2-d]thiazole-6,7-diol;
(3aR,5R,6S,7R,7aR)-2-(éthylamino)-5-(2,2,2-trifluoroéthyl)-5,6,7,7a-tétrahydro-3aH-pyrano[3,2-d]thiazole-6,7-diol;
(3aR,5R,6S,7R,7aR)-2-(propylamino)-5-(2,2,2-trifluoroéthyl)-5,6,7,7a-tétrahydro-3aH-pyrano[3,2-d]thiazole-6,7-diol;
(3aR,5S,6S,7R,7aR)-2-(azétidin-1-yl)-5-((S)-1,1,1-trifluoro-2-hydroxypropan-2-yl)-5,6,7,7a-tétrahydro-3aH-pyrano[3,2-d]thiazole-6,7-diol;
(3aR,5S,6S,7R,7aR)-2-(azétidin-1-yl)-5-((R) 1,1,1-trifluoro-2-hydroxypropan-2-yl) -5,6,7,7a-tétrahydro-3aH-pyrano[3,2-d]thiazole-6,7-diol;
(3aR,5S,6S,7R,7aR)-2-(diméthylamino)-5-((S)-1,1,1-trifluoro-2-hydroxypropan-2-yl)-5,6,7,7a-tétrahydro-3aH-pyrano[3,2-d]thiazole-6,7-diol;
(3aR,5S,6S,7R,7aR)-2-(diméthylamino)-5-((R)1,1,1-trifluoro-2-hydroxypropan-2-yl)-5,6,7,7a-tétrahydro-3aH-pyrano[3,2-d]thiazole-6,7-diol;
(3aR,5S,6S,7R,7aR)-2-(diméthylamino)-5-((S)-1,1,1-trifluoro-2-hydroxypropan-2-yl)-5,6,7,7a-tétrahydro-3aH-pyrano[3,2-d]thiazole-6,7-diol;
(3aR,5S,6S,7R,7aR)-2-(éthylamino)-5-((S)-1,1,1-trifluoro-2-hydroxypropan-2-yl)-5,6,7,7a-tétrahydro-3aH-pyrano[3,2-d]thiazole-6,7-diol;
(3aR,5S,6S,7R,7aR)-2-(éthylamino)-5-((R)1,1,1-trifluoro-2-hydroxypropan-2-yl)-5,6,7,7a-tétrahydro-3aH-pyrano[3,2-d]thiazole-6,7-diol;
(3aR,5R,6S,7R,7aR)-5-((R)-1-hydroxyéthyl)-2-(isoxazolidin-2-yl)-5,6,7,7a-tétrahydro-3aH-pyrano[3,2-d]thiazole-6,7-diol;
(3aR,5R,6S,7R,7aR)-5-((S)-1-hydroxyéthyl)-2-(isoxazolidin-2-yl)-5,6,7,7a-tétrahydro-3aH-pyrano[3,2-d]thiazole-6,7-diol;
(3aR,5R,6S,7R,7aR)-2-(éthylamino)-5-((S)-1-hydroxypropyl)-5,6,7,7a-tétrahydro-3aH-pyrano[3,2-d]thiazole-6,7-diol;
(3aR,5R,6S,7R,7aR)-5-((S)-1-hydroxyheptyl)-2-(méthylamino)-5,6,7,7a-tétrahydro-3aH-pyrano[3,2-d]thiazole-6,7-diol;
(3aR,5S,6S,7R,7aR)-2-(éthyl(méthyl)amino)-5-((S)-2,2,2-trifluoro-1-hydroxyéthyl)-5,6,7,7a-tétrahydro-3aH-pyrano[3,2-d]thiazole-6,7-diol;
(3aR,5S,6S,7R,7aR)-2-(éthyl(méthyl)amino)-5-((R)-2,2,2-trifluoro-1-hydroxyéthyl)-5,6,7,7a-tétrahydro-3aH-pyrano[3,2-d]thiazole-6,7-diol;
(3aR,5S,6S,7R,7aR)-2-(azétidin-1-yl)-5-((S)-2,2,2-trifluoro-1-hydroxyéthyl)-5,6,7,7a-tétrahydro-3aH-pyrano[3,2-d]thiazole-6,7-diol;
(3aR,5S,6S,7R,7aR)-2-(azétidin-1-yl)-5-((R)-2,2,2-trifluoro-1-hydroxyéthyl)-5,6,7,7a-tétrahydro-3aH-pyrano[3,2-d]thiazole-6,7-diol;
(3aR,5S,6S,7R,7aR)-2-(2-fluoroéthylamino)-5-((R)-2,2,2-trifluoro-1-hydroxyéthyl)-5,6,7,7a-tétrahydro-3aH-pyrano[3,2-d]thiazole-6,7-diol;
(3aR,5S,6S,7R,7aR)-2-(2-fluoroéthylamino)-5-((S)-2,2,2-trifluoro-1-hydroxyéthyl)-5,6,7,7a-tétrahydro-3aH-pyrano[3,2-d]thiazole-6,7-diol;
(3aR,5S,6S,7R,7aR)-2-(diméthylamino)-5-(1,1,1,3,3,3-hexafluoro-2-hydroxypropan-2-yl)-5,6,7,7a-tétrahydro-3aH-pyrano[3,2-d]thiazole-6,7-diol;
(3aR,5R,6S,7R,7aR)-2-(méthylamino)-5-((R)-1,1,1-trifluoropropan-2-yl)-5,6,7,7a-tétra-hydro-3aH-pyrano[3,2-d]thiazole-6,7-diol; et
(3aR,5R,6S,7R,7aR)-2-(méthylamino)-5-((S)-1,1,1-trifluoropropan-2-yl)-5,6,7,7a-tétra-hydro-3aH-pyrano[3,2-d]thiazole-6,7-diol;

ou un sel pharmaceutiquement acceptable de l'un quelconque des composés précités.

**10.** Composé selon la revendication 1, dans lequel: chaque R est H, $R^5$ est OH, $R^3$ est $C_{1-6}$alkyle, facultativement monosubstitué par fluoro ou hydroxy, et $R^4$ est H.

**11.** Composé selon la revendication 1, dans lequel: chaque R est H, $R^5$ est H, $R^3$ est $C_{1-6}$alkyle, facultativement monosubstitué par fluoro ou hydroxy, et $R^4$ est H ou $C_{1-6}$alkyle.

**12.** Composé selon la revendication 1, dans lequel $R^1$ est $C_{1-6}$alkyle, facultativement monosubstitué par hydroxy, et $R^2$ est H.

**13.** Composition pharmaceutique comprenant un composé selon l'une quelconque des revendications 1 à 12 ou un sel pharmaceutiquement acceptable de celui-ci, en combinaison avec un véhicule pharmaceutiquement acceptable.

**14.** Composé selon l'une quelconque des revendications 1 à 12 pour l'utilisation dans le traitement d'une affection choisie parmi une ou plusieurs du groupe constituant en une maladie inflammatoire, une allergie, un asthme, une rhinite allergique, des maladies pulmonaires d'hypersensibilité, une pneumonie d'hypersensibilité, des pneumonies éosinophiles, une hypersensibilité de type retardée, une athérosclérose, une maladie pulmonaire interstitielle (ILD), une fibrose pulmonaire idiopathique, une ILD associée à une polyarthrite rhumatoïde, un lupus érythémateux systémique, une spondylarthrite ankylosante, une sclérodermie systémique, un syndrome de Sjogren, une polymyosite ou une dermatomyosite, une anaphylaxie ou réponse d'hypersensibilité systémique, une allergie à substance médicamenteuse, une allergie à piqûre d'insecte, une maladie auto-immune, une polyarthrite rhumatoïde, une arthrite psoriasique, une sclérose en plaques, un syndrome de Guillain-Barré, un lupus érythémateux systémique, une myasthénie grave, une glomérulonéphrite, une thyroïdite auto-immune, un rejet de greffon, rejet d'allogreffe, une maladie greffon-contre-hôte, une maladie intestinale inflammatoire, une maladie de Crohn, une rectocolite ulcérative, une spondyloarthropathie, un scléroderme, un psoriasis, un psoriasis induit par les lymphocytes T, une dermatose inflammatoire, une dermatite, un eczéma, une dermatite atopique, une dermatite de contact allergique, un urticaire, une vasculite, une vasculite nécrosante, cutanée et d'hypersensibilité, une myosite éosinophile, une fasciite éosinophile, un rejet de transplantation d'organe solide, un rejet de transplantation cardiaque, un rejet de transplantation pulmonaire, un rejet de transplantation hépatique, un rejet de transplantation rénale, un rejet de transplantation pancréatique, une allogreffe rénale, une allogreffet pulmonaire, une épilepsie, une douleur, une fibromyalgie, une attaque, une neuroprotection.

**15.** Composé selon l'une quelconque des revendications 1 à 12 pour l'utilisation dans le traitement d'une affection choisie dans le groupe consistant en une maladie neurodégénérative, une tauopathie, un cancer et un stress.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CA 2006000300 W
- WO 2006092049 A
- CA 2007001554 W
- WO 2008025170 A
- CA 2009001087 W
- WO 2010012106 A
- CA 2009001088 W
- WO 2010012107 A
- CA 2009001302 W
- WO 2010037207 A
- WO 2013028715 A
- WO 2006016904 A


### Non-patent literature cited in the description


- **COUTINHO, P.M. ; HENRISSAT, B.** *Carbohydrate-Active Enzymes,* 1999, http://afmb.cnrs-mrs.fr/CAZY
- **ALFONSO GENNARO.** Remington: the Science & Practice of Pharmacy. Williams & Wilkins, 2000
- **D. J. SILVA.** *J. Org. Chem.,* 1999, vol. 64 (16), 5926-5929
- **M.V. GONZALEZ.** *Carbohydrate Research,* 1986, vol. 154, 49-62
- **YUZWA et al.** *Nature Chemical Biology,* 2008, vol. 4, 483
- **C. R. TORRES ; G. W. HART.** *J Biol Chem,* 1984, vol. 259, 3308
- **R. S. HALTIWANGER ; G. D. HOLT ; G. W. HART.** *J Biol Chem,* 1990, vol. 265, 2563
- **L. K. KREPPEL ; M. A. BLOMBERG ; G. W. HART.** *J Biol Chem,* 1997, vol. 272, 9308
- **W. A. LUBAS ; D. W. FRANK ; M. KRAUSE ; J. A. HANOVER.** *J Biol Chem,* 1997, vol. 272, 9316
- **W. A. LUBAS ; J. A. HANOVER.** *J Biol Chem,* 2000, vol. 275, 10983
- **D. L. DONG ; G. W. HART.** *J Biol Chem,* 1994, vol. 269, 19321
- **Y. GAO ; L. WELLS ; F. I. COMER ; G. J. PARKER ; G. W. HART.** *J Biol Chem,* 2001, vol. 276, 9838
- **E. P. ROQUEMORE ; M. R. CHEVRIER ; R. J. COTTER ; G. W. HART.** *Biochemistry,* 1996, vol. 35, 3578
- **S. P. JACKSON ; R. TJIAN.** *Cell,* 1988, vol. 55, 125
- **W. G. KELLY ; M. E. DAHMUS ; G. W. HART.** *J Biol Chem,* 1993, vol. 268, 10416
- **M. D. ROOS ; K. SU ; J. R. BAKER ; J. E. KUDLOW.** *Mol Cell Biol,* 1997, vol. 17, 6472
- **N. LAMARRE-VINCENT ; L. C. HSIEH-WILSON.** *JAm Chem Soc,* 2003, vol. 125, 6612
- **F. ZHANG ; K. SU ; X. YANG ; D. B. BOWE ; A. J. PATERSON ; J. E. KUDLOW.** *Cell,* 2003, vol. 115, 715
- **K. VOSSELLER ; L. WELLS ; M. D. LANE ; G. W. HART.** *Proc Natl Acad Sci USA,* 2002, vol. 99, 5313
- **W. A. LUBAS ; M. SMITH ; C. M. STARR ; J. A. HANOVER.** *Biochemistry,* 1995, vol. 34, 1686
- **L. S. GRIFFITH ; B. SCHMITZ.** *Biochem Biophys Res Commun,* 1995, vol. 213, 424
- **R. N. COLE ; G. W. HART.** *J Neurochem,* 1999, vol. 73, 418
- **I. BRAIDMAN ; M. CARROLL ; N. DANCE ; D. ROBINSON.** *Biochem J,* 1974, vol. 143, 295
- **R. UENO ; C. S. YUAN.** *Biochim Biophys Acta,* 1991, vol. 1074, 79
- **C. TOLEMAN ; A. J. PATERSON ; T. R. WHISENHUNT ; J. E. KUDLOW.** *J Biol Chem,* 2004
- **F. LIU ; K. IQBAL ; I. GRUNDKE-IQBAL ; G. W. HART ; C. X. GONG.** *Proc Natl Acad Sci U S A,* 2004, vol. 101, 10804
- **T. Y. CHOU ; G. W. HART.** *Adv Exp Med Biol,* 2001, vol. 491, 413
- **M. GOEDERT ; M. G. SPILLANTINI ; N. J. CAIRNS ; R. A. CROWTHER.** *Neuron,* 1992, vol. 8, 159
- **M. GOEDERT ; M. G. SPILLANTINI ; R. JAKES ; D. RUTHERFORD ; R. A. CROWTHER.** *Neuron,* 1989, vol. 3, 519
- **E. KOPKE ; Y. C. TUNG ; S. SHAIKH ; A. C. ALONSO ; K. IQBAL ; I. GRUNDKE-IQBAL.** *J Biol Chem,* 1993, vol. 268, 24374
- **H. KSIEZAK-REDING ; W. K. LIU ; S. H. YEN.** *Brain Res,* 1992, vol. 597, 209
- **B. HENRISSAT ; A. BAIROCH.** *Biochem J,* 1996, vol. 316, 695
- **B. HENRISSAT ; A. BAIROCH.** *Biochem J,* 1993, vol. 293, 781

- **C. X. GONG ; F. LIU ; I. GRUNDKE-IQBAL ; K. IQBAL.** *J Neural Transm,* 2005, vol. 112, 813
- **K. IQBAL ; C. ALONSO ADEL ; E. EL-AKKAD ; C. X. GONG ; N. HAQUE ; S. KHATOON ; I. TSUJIO ; I. GRUNDKE-IQBAL.** *J Neural Transm Suppl,* vol. 2002, 309
- **K. IQBAL ; C. ALONSO ADEL ; E. EL-AKKAD ; C. X. GONG ; N. HAQUE ; S. KHATOON ; J. J. PEI ; H. TANIMUKAI ; I. TSUJIO et al.** *J Mol Neurosci,* 2003, vol. 20, 425
- **W. NOBLE ; E. PLANEL ; C. ZEHR ; V. OLM ; J. MEYERSON ; F. SULEMAN ; K. GAYNOR ; L. WANG ; J. LAFRANCOIS et al.** *Proc Natl Acad Sci U S A,* 2005, vol. 102, 6990
- **S. LE CORRE ; H. W. KLAFKI ; N. PLESNILA ; G. HUBINGER ; A. OBERMEIER ; H. SAHAGUN ; B. MONSE ; P. SENECI ; J. LEWIS et al.** *Proc Natl Acad Sci U S A,* 2006, vol. 103, 9673
- **S. J. LIU ; J. Y. ZHANG ; H. L. LI ; Z. Y. FANG ; Q. WANG ; H. M. DENG ; C. X. GONG ; I. GRUNDKE-IQBAL ; K. IQBAL et al.** *J Biol Chem,* 2004, vol. 279, 50078
- **G. LI ; H. YIN ; J. KURET.** *J Biol Chem,* 2004, vol. 279, 15938
- **T. Y. CHOU ; G. W. HART ; C. V. DANG.** *J Biol Chem,* 1995, vol. 270, 18961
- **X. CHENG ; G. W. HART.** *J Biol Chem,* 2001, vol. 276, 10570
- **X. CHENG ; R. N. COLE ; J. ZAIA ; G. W. HART.** *Biochemistry,* 2000, vol. 39, 11609
- **L. S. GRIFFITH ; B. SCHMITZ.** *Eur J Biochem,* 1999, vol. 262, 824
- **K. KAMEMURA ; G. W. HART.** *Prog Nucleic Acid Res Mol Biol,* 2003, vol. 73, 107
- **L. WELLS ; L. K. KREPPEL ; F. I. COMER ; B. E. WADZINSKI ; G. W. HART.** *J Biol Chem,* 2004, vol. 279, 38466
- **L. BERTRAM ; D. BLACKER ; K. MULLIN ; D. KEENEY ; J. JONES ; S. BASU ; S. YHU ; M. G. MCINNIS ; R. C. GO et al.** *Science,* 2000, vol. 290, 2302
- **S. HOYER ; D. BLUM-DEGEN ; H. G. BERNSTEIN ; S. ENGELSBERGER ; J. HUMRICH ; S. LAUFER ; D. MUSCHNER ; A. THALHEIMER ; A. TURK et al.** *Journal of Neural Transmission,* 1998, vol. 105, 423
- **C. X. GONG ; F. LIU ; I. GRUNDKE-IQBAL ; K. IQBAL.** *Journal of Alzheimers Disease,* 2006, vol. 9, 1
- **W. J. JAGUST ; J. P. SEAB ; R. H. HUESMAN ; P. E. VALK ; C. A. MATHIS ; B. R. REED ; P. G. COXSON ; T. F. BUDINGER.** *Journal of Cerebral Blood Flow and Metabolism,* 1991, vol. 11, 323
- **S. HOYER.** *Experimental Gerontology,* 2000, vol. 35, 1363
- **S. HOYER.** *Frontiers in Clinical Neuroscience: Neurodegeneration and Neuroprotection,* 2004, vol. 541, 135
- **R. N. KALARIA ; S. I. HARIK.** *Journal of Neurochemistry,* 1989, vol. 53, 1083
- **I. A. SIMPSON ; K. R. CHUNDU ; T. DAVIESHILL ; W. G. HONER ; P. DAVIES.** *Annals of Neurology,* 1994, vol. 35, 546
- **S. M. DE LA MONTE ; J. R. WANDS.** *Journal of Alzheimers Disease,* 2005, vol. 7, 45
- **X. W. ZHU ; G. PERRY ; M. A. SMITH.** *journal of Alzheimers Disease,* 2005, vol. 7, 81
- **J. C. DE LA TORRE.** *Neurological Research,* 2004, vol. 26, 517
- **S. MARSHALL ; W. T. GARVEY ; R. R. TRAXINGER.** *Faseb J,* 1991, vol. 5, 3031
- **S. P. IYER ; Y. AKIMOTO ; G. W. HART.** *J Biol Chem,* 2003, vol. 278, 5399
- **K. BRICKLEY ; M. J. SMITH ; M. BECK ; F. A. STEPHENSON.** *J Biol Chem,* 2005, vol. 280, 14723
- **S. KNAPP ; C. H. YANG ; T. HAIMAWITZ.** *Tetrahedron Letters,* 2002, vol. 43, 7101
- **S. P. IYER ; G. W. HART.** *J Biol Chem,* 2003, vol. 278, 24608
- **M. JINEK ; J. REHWINKEL ; B. D. LAZARUS ; E. IZAURRALDE ; J. A. HANOVER ; E. CONTI.** *Nat Struct Mol Biol,* 2004, vol. 11, 1001
- **K. KAMEMURA ; B. K. HAYES ; F. I. COMER ; G. W. HART.** *J Biol Chem,* 2002, vol. 277, 19229
- **Y. DENG ; B. LI ; F. LIU ; K. IQBAL ; I. GRUNDKE-IQBAL ; R. BRANDT ; C.-X. GONG.** *FASEB J.,* 2007, 07
- **L. F. LAU ; J. B. SCHACHTER ; P. A. SEYMOUR ; M. A. SANNER.** *Curr Top Med Chem,* 2002, vol. 2, 395
- **M. P. MAZANETZ ; P. M. FISCHER.** *Nature Reviews Drug Discovery,* 2007, vol. 6, 464
- **S. A. YUZWA ; M. S. MACAULEY ; J. E. HEINONEN ; X. SHAN ; R. J. DENNIS ; Y. HE ; G. E. WHITWORTH ; K. A. STUBBS ; E. J. MCEACHERN et al.** *Nat Chem Biol,* 2008, vol. 4, 483
- **P. BOUNELIS ; J. LIU ; Y. PANG ; J. C. CHATHAM ; R. B. MARCHASE.** *Shock,* 2004, vol. 21 170 (2), 58
- **N. FULOP ; V. CHAMPATTANACHAL ; R. B. MARCHASE ; J. C. CHATHAM.** *Circulation Research,* 2005, vol. 97, E28
- **J. LIU ; R. B. MARCHASE ; J. C. CHATHAM.** *Faseb Journal,* 2006, vol. 20, A317
- **N. FULOP ; P. P. WANG ; R. B. MARCHASE ; J. C. CHATHAM.** *Journal of Molecular and Cellular Cardiology,* 2004, vol. 37, 286
- **N. FULOP ; P. P. WANG ; R. B. MARCHASE ; J. C. CHATHAM.** *Faseb Journal,* 2005, vol. 19, A689
- **J. LIU ; R. B. MARCHASE ; J. C. CHATHAM.** *Journal of Molecular and Cellular Cardiology,* 2007, vol. 42, 177

- **L. G. NOT ; C. A. BROCKS ; N. FULOP ; R. B. MARCHASE ; J. C. CHATHAM.** *Faseb Journal,* 2006, vol. 20, A1471
- **S. L. YANG ; L. Y. ZOU ; P. BOUNELIS ; I. CHAUDRY ; J. C. CHATHAM ; R. B. MARCHASE.** *Shock,* 2006, vol. 25, 600
- **L. Y. ZOU ; S. L. YANG ; P. BOUNELIS ; I. H. CHAUDRY ; J. C. CHATHAM ; R. B. MARCHASE.** *Faseb Journal,* 2005, vol. 19, A1224
- **R. B. MARCHASE ; J. LIU ; L. Y. ZOU ; V. CHAMPATTANACHAI ; Y. PANG ; N. FULOP ; P. P. WANG ; S. L. YANG ; P, BOUNELIS et al.** *Circulation,* 2004, vol. 110, 1099
- **J. LIU ; Y. PANG ; T. CHANG ; P. BOUNELIS ; J. C. CHATHAM ; R. B. MARCHASE.** *Journal of Molecular and Cellular Cardiology,* 2006, vol. 40, 303
- **J. LIU ; J. C. CHATHAM ; R. B. MARCHASE.** *Faseb Journal,* 2005, vol. 19, A691
- **T. NAGY ; V. CHAMPATTANACHAI ; R. B. MARCHASE ; J. C. CHATHAM.** *American journal of Physiology-Cell Physiology,* 2006, vol. 290, C57
- **N. FULOP ; R. B. MARCHASE ; J. C. CHATHAM.** *Cardiovascular Research,* 2007, vol. 73, 288
- **T. LEFEBVRE ; C. GUINEZ ; V. DEHENNAUT ; O. BESEME-DEKEYSER ; W. MORELLE ; J. C. MICHALSKI.** *Expert Review of Proteomics,* 2005, vol. 2, 265
- **L. WELLS ; K. VOSSELLER ; G. W. HART.** *Science,* 2001, vol. 291, 2376
- **J. A. HANOVER.** *FASEB J,* 2001, vol. 15, 1865
- **D. A. MCCLAIN ; W. A. LUBAS ; R. C. COOKSEY ; M. HAZEL ; G. J. PARKER ; D. C. LOVE ; J. A. HANOVER.** *Proc Natl A cad Sci U S A,* 2002, vol. 99, 10695
- **P. J. YAO ; P. D. COLEMAN.** *J Neurosci,* 1998, vol. 18, 2399
- **W. H. YANG ; J. E. KIM ; H. W. NAM ; J. W. JU ; H. S. KIM ; Y. S. KIM ; J. W. CHO.** *Nature Cell Biology,* 2006, vol. 8, 1074
- **B. TRIGGS-RAINE ; D. J. MAHURAN ; R. A. GRAVEL.** *Adv Genet,* 2001, vol. 44, 199
- **D. ZHOU ; J. MATTNER ; C. CANTU III ; N. SCHRANTZ ; N. YIN ; Y. GAO ; Y. SAGIV ; K. HUDSPETH ; Y. WU et al.** *Science,* 2004
- **G. LEGLER ; E. LULLAU ; E. KAPPES ; F. KASTENHOLZ.** *Biochim Biophys Acta,* 1991, vol. 1080, 89
- **M. HORSCH ; L. HOESCH ; A. VASELLA ; D. M. RAST.** *Eur J Biochem,* 1991, vol. 197, 815
- **J. LIU ; A. R. SHIKHMAN ; M. K. LOTZ ; C. H. WONG.** *Chem Biol,* 2001, vol. 8, 701
- **S. KNAPP ; D. J. VOCADLO ; Z. N. GAO ; B. KIRK ; J. P. LOU ; S. G. WITHERS.** *J. Am. Chem. Soc.,* 1996, vol. 118, 6804
- **V. H. LILLELUND ; H. H. JENSEN ; X. LIANG ; M. BOLS.** *Chem Rev,* 2002, vol. 102, 515
- **R. J. KONRAD ; I. MIKOLAENKO ; J. F. TOLAR ; K. LIU ; J. E. KUDLOW.** *Biochem J,* 2001, vol. 356, 31
- **K. LIU ; A. J. PATERSON ; F. ZHANG ; J. MCANDREW ; K. FUKUCHI ; J. M. WYSS ; L. PENG ; Y. HU ; J. E. KUDLOW.** *J Neurochem,* 2004, vol. 89, 1044
- **G. PARKER ; R. TAYLOR ; D. JONES ; D. MCCLAIN.** *J Biol Chem,* 2004, vol. 279, 20636
- **E. B. ARIAS ; J. KIM ; G. D. CARTEE.** *Diabetes,* 2004, vol. 53, 921
- **A. JUNOD ; A. E. LAMBERT ; L. ORCI ; R. PICTET ; A. E. GONET ; A. E. RENOLD.** *Proc Soc Exp Biol Med,* 1967, vol. 126, 201
- **R. A. BENNETT ; A. E. PEGG.** *Cancer Res,* 1981, vol. 41, 2786
- **K. D. KRONCKE ; K. FEHSEL ; A. SOMMER ; M. L. RODRIGUEZ ; V. KOLB-BACHOFEN.** *Biol Chem Hoppe Seyler,* 1995, vol. 376, 179
- **H. YAMAMOTO ; Y. UCHIGATA ; H. OKAMOTO.** *Nature,* 1981, vol. 294, 284
- **K. YAMADA ; K. NONAKA ; T. HANAFUSA ; A. MIYAZAKI ; H. TOYOSHIMA ; S. TARUI.** *Diabetes,* 1982, vol. 31, 749
- **V. BURKART ; Z. Q. WANG ; J. RADONS ; B. HELLER ; Z. HERCEG ; L. STINGL ; E. F. WAGNER ; H. KOLB.** *Nat Med,* 1999, vol. 5, 314
- **M. D. ROOS ; W. XIE ; K. SU ; J. A. CLARK ; X. YANG ; E. CHIN ; A. J. PATERSON ; J. E. KUDLOW.** *Proc Assoc Am Physicians,* 1998, vol. 110, 422
- **Y. GAO ; G. J. PARKER ; G. W. HART.** *Arch Biochem Biophys,* 2000, vol. 383, 296
- **R. OKUYAMA ; M. YACHI.** *Biochem Biophys Res Commun,* 2001, vol. 287, 366
- **N. E. ZACHARA ; N. O'DONNELL ; W. D. CHEUNG ; J. J. MERCER ; J. D. MARTH ; G. W. HART.** *J Biol Chem,* 2004, vol. 279, 30133
- **J. A. HANOVER ; Z. LAI ; G. LEE ; W. A. LUBAS ; S. M. SATO.** *Arch Biochem Biophys,* 1999, vol. 362, 38
- **K. LIU ; A. J. PATERSON ; R. J. KONRAD ; A. F. PARLOW ; S. JIMI ; M. ROH ; E. CHIN, JR. ; J. E. KUDLOW.** *Mol Cell Endocrinol,* 2002, vol. 194, 135
- **M. S. MACAULEY ; G. E. WHITWORTH ; A. W. DEBOWSKI ; D. CHIN ; D. J. VOCADLO.** *J Biol Chem,* 2005, vol. 280, 25313
- **B. L. MARK ; D. J. VOCADLO ; S. KNAPP ; B. L. TRIGGS-RAINE ; S. G. WITHERS ; M. N. JAMES.** *J Biol Chem,* 2001, vol. 276, 10330
- **R. S. HALTIWANGER ; K. GROVE ; G. A. PHILIPSBERG.** *J Biol Chem,* 1998, vol. 273, 3611

- **D. J. MILLER ; X. GONG ; B. D. SHUR.** *Development,* 1993, vol. 118, 1279
- **L. Y. ZOU ; S. L. YANG ; S. H. HU ; I. H. CHAUDRY ; R. B. MARCHASE ; J. C. CHATHAM.** *Shock,* 2007, vol. 27, 402
- **J. B. HUANG ; A. J. CLARK ; H. R. PETTY.** *Cellular Immunology,* 2007, vol. 245, 1
- *US/Japan Glyco 2004 Conference,* 2004
- **L. Y. ZOU ; S. L. YANG ; S. H. HU ; I. H. CHAUDRY ; R. B. MARCHASE ; J. C. CHATHAM.** *Faseb Journal,* 2006, vol. 20, A1471
- **V. CHAMPATTANACHAI ; R. B. MARCHASE ; J. C. CHATHAM.** *American Journal of Physiology-Cell Physiology,* 2007, vol. 292, C178
- **V. CHAMPATTANACHAI ; R. B. MARCHASE ; J. C. CHATHAM.** *American Journal of Physiology-Cell Physiology,* 2008, vol. 294, C1509
- **I. KHLISTUNOVA ; M. PICKHARDT ; J. BIERNAT ; Y. P. WANG ; E. M. MANDELKOW ; E. MANDELKOW.** *Current Alzheimer Research,* 2007, vol. 4, 544
- **P. FRIEDHOFF ; A. SCHNEIDER ; E. M. MANDELKOW ; E. MANDELKOW.** *Biochemistry,* 1998, vol. 37, 10223
- **M. PICKHARDT ; Z. GAZOVA ; M. VON BERGEN ; I. KHLISTUNOVA, Y. P ; WANG, A. HASCHER ; E. M. MANDELKOW ; J. BIERNAT ; E. MANDELKOW.** *Journal of Biological Chemistry,* 2005, vol. 280, 3628
- **P. H. LIANG ; W. C. CHENG ; Y. L. LEE ; H. P. YU ; Y. T. WU ; Y. L. LIN ; C. H. WONG.** *Chembiochem,* 2006, vol. 7, 165
- **J. J. LIU ; M. M. D. NUMA ; H. T. LIU ; S. J. HUANG ; P. SEARS ; A. R. SHIKHMAN ; C. H. WONG.** *Journal of Organic Chemistry,* 2004, vol. 69, 6273
- **Y. TAKAOKA ; T. KAJIMOTO ; C. H. WONG.** *Journal of Organic Chemistry,* 1993, vol. 58, 4809
- **T. M. WRODNIGG ; A. E. STUTZ ; S. G. WITHERS.** *Tetrahedron Letters,* 1997, vol. 38, 5463